# EUROPEAN PATENT APPLICATION

(11) **EP 4 410 830 A1**
(43) Date of publication of application: **07.08.2024**
(21) Application number: 22876568.1
(22) Date of filing: 30.09.2022
(51) Int. Cl.: C07K 16/00, A61K 31/165, A61K 39/395, A61K 45/00, A61K 47/65, A61K 47/68, A61K 49/00, A61P 35/00, A61P 43/00, C07K 5/06, C07K 5/08, C07K 7/06, C07K 19/00

(54) **CONJUGATE OF ANTIBODY AND FUNCTIONAL SUBSTANCE OR SALT THEREOF, AND ANTIBODY DERIVATIVE AND COMPOUND OR SALTS THEREOF TO BE USED IN PRODUCING CONJUGATE OR SALT THEREOF**

(30) Priority: 30.09.2021 JP 2021162299; 21.12.2021 JP 2021207541
(71) Applicant: AJINOMOTO CO., INC., Chuo-ku Tokyo 104-8315 (JP)
(72) Inventor: MATSUDA, Yutaka, Kawasaki-shi, Kanagawa 210-8681 (JP); WATANABE, Tomohiro, Kawasaki-shi, Kanagawa 210-8681 (JP); HATADA, Noriko, Kawasaki-shi, Kanagawa 210-8681 (JP); FUJII, Tomohiro, Kawasaki-shi, Kanagawa 210-8681 (JP)
(74) Representative: Mewburn Ellis LLP
(86) International application number: PCT/JP2022/036835
(87) International publication number: WO 2023/054706

(57) **Abstract**

The present invention provides a conjugate of an antibody and a functional substance excellent in desired properties, or a salt thereof. More specifically, the present invention provides
a conjugate of an antibody and a functional substance, comprising a structural unit represented by the following Formula (1): 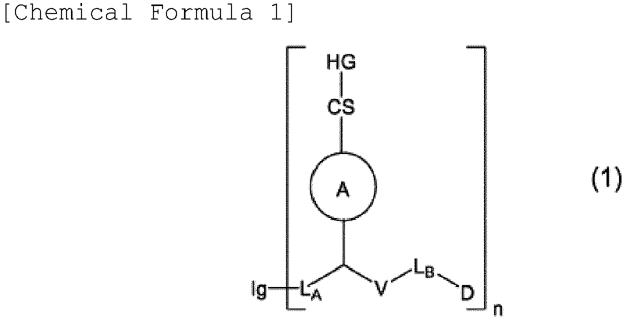 wherein
Ig represents an immunoglobulin unit comprising two heavy chains and two light chains, and is bonded to L_{A} adjacent to Ig via a thiol group in side chains of a plurality of cysteine residues in the two heavy chains and the two light chains,
HG represents a hydrophilic group or a monovalent group comprising a hydrophilic group,
CS represents a divalent group comprising a cleavable site,
ring A represents a divalent aromatic ring group optionally having a substituent wherein the divalent aromatic ring group constitutes a π electron conjugated system with the cleavable site,
V represents an oxygen atom, a sulfur atom, or an amino group (NH),
L_{A} and L_{B} each independently represent a divalent group,
D represents a functional substance, and
an average number n of the bonds per the immunoglobulin unit is 1.5 or more, or
a salt thereof, and
a substance associated therewith.

## Description

### Field

The present invention relates to a conjugate of an antibody and a functional substance or a salt thereof, a compound used in production of the same or a salt thereof, and the like.

### Background

In recent years, research and development of an antibody drug conjugate (ADC) have been actively performed. An ADC, as implied by the name, is a medicine in which a drug (e.g., an anti-cancer agent) is conjugated with an antibody and has a direct cytotoxic activity on cancer cells and the like. A typical ADC is T-DM1 (trade name: Kadcyla (registered trademark)) jointly developed by Immunogene Inc. and Roche Inc.

An ADC is produced by bonding a functional group in a side chain of a specific amino acid residue present in an antibody to a drug. Examples of such a functional group used in production of an ADC include an amino group in a side chain of a lysine residue present in an antibody. Several techniques have been reported as a technique for modifying a lysine group (e.g., a lysine residue at position 246/248, position 288/290, or position 317) in an antibody (e.g., Patent Literatures 1 to 4).

Another example of such a functional group used in production of an ADC is a thiol group. An antibody has disulfide groups because heavy chains are linked to each other and the heavy chains are linked to light chains by disulfide bonds. For example, an IgG antibody comprising two heavy chains and two light chains has four disulfide groups because the heavy chains are linked to each other and the heavy chains are linked to the light chains by four disulfide bonds. Such a disulfide bond can be cleaved by a reducing agent. For example, when all the four disulfide bonds in IgG are cleaved, an IgG antibody having eight thiol groups is generated. Even when all the four interchain disulfide bonds are cleaved, the heavy chains and the light chains of the antibody are not dissociated. This is because a higher order structure of the antibody can be maintained by a non-covalent bond between the chains. Antibody properties (ability to bond to a target) can also be maintained by this non-covalent bond. For example, trastuzumab delkstecan (Patent Literature 5) known as Enhartz (registered trademark) is an ADC having a drug/antibody ratio (DAR) = 8 in which all four interchain disulfide bonds are cleaved (reduced) and then a drug is bonded. Such an ADC is known to act as an antigen-specific agent by maintaining antibody properties thereof.

In an ADC, an antibody and a drug are linked to each other via a linker. There are various linkers in an ADC. For example, in an ADC used as an anti-cancer agent, there is a linker comprising a dipeptide consisting of valine-citrulline (Val-Cit: VC structure) as a linker that is stable in human plasma and has a structure cleavable by a specific enzyme for releasing a drug in cancer cells. A linker comprising such a dipeptide is stable in human plasma as illustrated in the following (A). However, as illustrated in the following (B), cathepsin B in lysosomes in human cancer cells recognizes a VC structure and cleaves an amide bond present on a carboxy terminal side of citrulline. Therefore, an ADC having a linker comprising such a dipeptide can release a drug and exhibit a drug efficacy in human cancer cells.

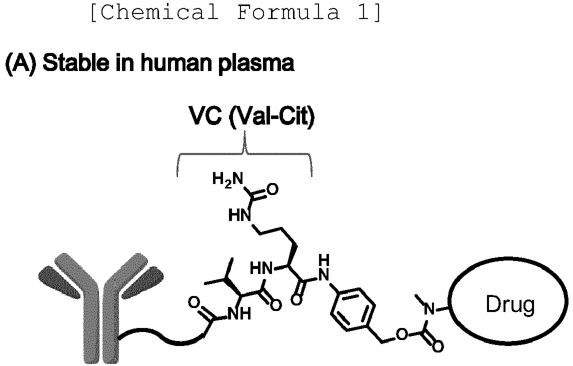

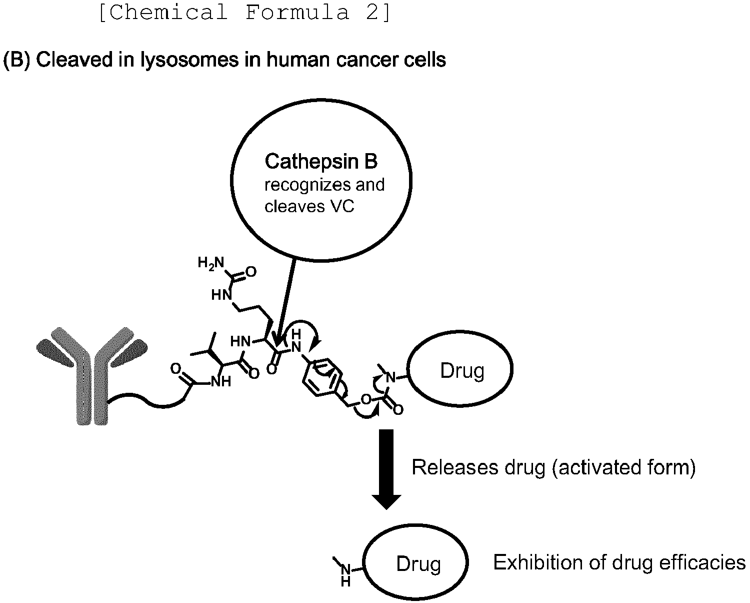

However, an ADC having a linker comprising such a dipeptide as described above is unstable in mouse plasma (Non Patent Literatures 1 and 2). This is because Cesic, which is a carboxylase that recognizes a VC structure and cleaves an amide bond present on a carboxy terminal side of citrulline, is present in mouse plasma, and therefore a linker comprising such a dipeptide as described above is cleaved in the plasma by Ceslc. Therefore, the ADC having a linker comprising such a dipeptide as described above largely differs in pharmacokinetics between mice and humans. Therefore, with mice, there is a problem that it is difficult to evaluate a drug efficacy in humans.

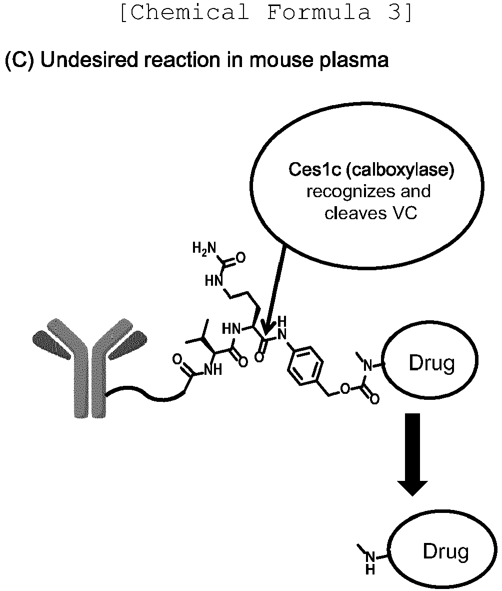

In order to improve instability in mouse plasma for an ADC having a structure "antibody-spacer-VC structure-spacer-drug" as described above, attempts have been made to stabilize the ADC by modifying a linker (that is, spacer-VC structure-spacer), and from such a viewpoint, an ADC in which an antibody and a drug or a mimic thereof are linked to each other via the linker has been reported. For example, the following has been reported as an ADC comprising the linker not in a main chain linking an antibody and a drug or a mimic thereof but in a side chain of the main chain (Patent Literature 6). 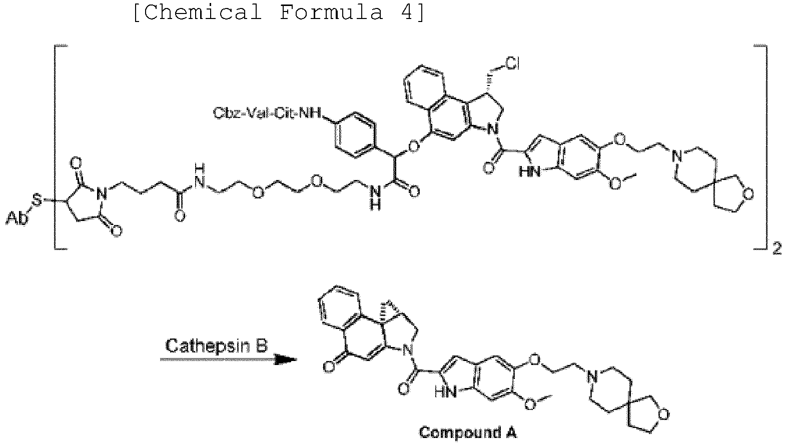
Ab: Antibody
Cbz: Benzyloxycarbonyl
Val: Valine residue
Cit: Citrulline residue

An example of the VC structure typically cleaved by cathepsin B has been described above as a peptide structure cleaved by protease in vivo. However, it is known that a peptide structure other than the VC structure is also present as the peptide structure cleaved by cathepsin B, and the other peptide structure is cleaved also by an enzyme in vivo other than cathepsin B.

By the way, Non Patent Literature 3 describes that the higher hydrophobicity of an ADC, the faster a plasma clearance, and that the hydrophobicity of the ADC can be evaluated by hydrophobic interaction chromatography (HIC)-HPLC.

### Citation List

### Patent Literature

Patent Literature 1: WO 2018/199337 A
Patent Literature 2: WO 2019/240288 A
Patent Literature 3: WO 2019/240287 A
Patent Literature 4: WO 2020/090979 A
Patent Literature 5: WO 2014/057687 A
Patent Literature 6: WO 2015/038426 A

### Non Patent Literature

Non Patent Literature 1: Dorywalska et al., Bioconjugate Chem., 2015, 26(4), 650-659
Non Patent Literature 2: Dorywalska et al., Mol Cancer Ther., 2016, 15(5), 958-70
Non Patent Literature 3: Lyon et al., Nat Biotechnol., 2015, 33(7), 733-5

### Summary

### Technical Problem

An object of the present invention is to provide a conjugate of an antibody and a functional substance excellent in desired properties, or a salt thereof.

### Solution to Problem

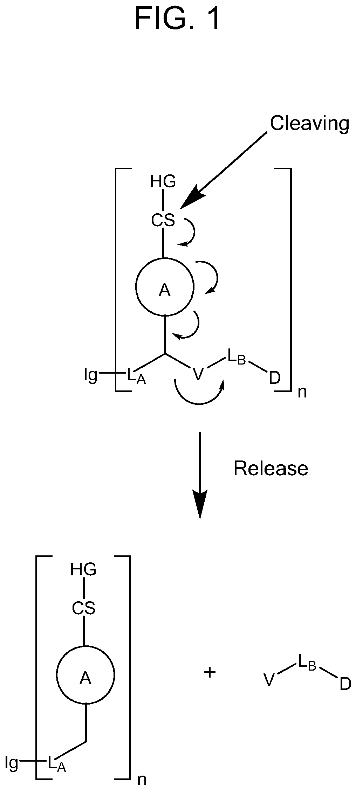

As a result of intensive studies, the present inventors have found that a conjugate comprising a linker having a specific structure (a linker that allows release of a drug (an active drug in which steric hindrance by an antibody is eliminated) by cleavage of a cleavable site and interlocking of a π electron resonance system), see FIG. 1) in a side chain of a main chain linking an antibody and a drug (or a drug mimic), in which the linker is linked to a thiol group generated by cleavage of disulfide bonds between heavy chains and between a heavy chain and a light chain in an antibody (immunoglobulin unit), has excellent properties. For example, such a conjugate or a salt thereof can have an excellent clearance (long residence time in body) and a low aggregation ratio (high monomer ratio). In addition, by using such a linker as described above, a hydrophilic group can be retained in the antibody after cleavage. That is, before cleavage, the drug is linked to a hydrophilic group in a form of a conjugate with the antibody, and therefore hydrophobicity of the drug can be suppressed (masking by the hydrophilic group), and after cleavage, the hydrophilic group is dissociated from the drug, and therefore the drug can exhibit original hydrophobicity of the drug. Therefore, for example, when the drug comprised in the conjugate is a hydrophobic compound that can be expected to have cell permeability (so-called bystander effect), the present invention using such a linker as described above has an advantage that it is easy to exhibit the cell permeability of the drug. Here, an ADC having such a linker may be referred to as an exo-type ADC.

The present inventors have also succeeded in developing an antibody derivative and a compound useful for production of such a conjugate. A conjugate, an antibody derivative, and a compound of the present invention represented by the structures of Formulas (1) to (7) have a technical feature of sharing partial structural units excluding X and Y among structural units represented in Formula (5). The present inventors have succeeded in developing a series of inventions having such a technical feature, and have completed the present invention. Related art neither describes nor suggests the chemical structure of the conjugate of the present invention and a relationship between such a chemical structure and excellent properties as described above. Related art neither describes nor suggests the antibody derivative and the compound of the present invention that can be used for production of such a conjugate.

That is, the present invention is as follows.

A first embodiment of the present invention provides
a conjugate of an antibody and a functional substance or functional substances, comprising a structural unit represented by the following Formula (1): 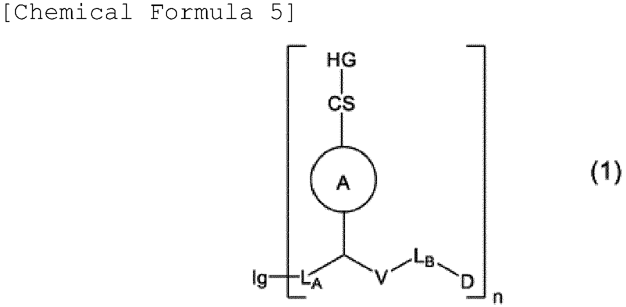
wherein
Ig represents an immunoglobulin unit comprising two heavy chains and two light chains, and is bonded to L_{A} adjacent to Ig via a thiol group in side chains of a plurality of cysteine residues in the two heavy chains and the two light chains,
HG represents a hydrophilic group or a monovalent group comprising a hydrophilic group,
CS represents a divalent group comprising a cleavable site,
ring A represents a divalent aromatic ring group optionally having a substituent wherein the divalent aromatic ring group constitutes a π electron conjugated system with the cleavable site,
V represents an oxygen atom, a sulfur atom, or an amino group (NH),
L_{A} and L_{B} each independently represent a divalent group,
D represents a functional substance, and
an average number n of the bonds per the immunoglobulin unit is 1.5 or more, or
a salt thereof.

In a specific embodiment, the structural unit represented by Formula (1) may be represented by the following Formula (1'): 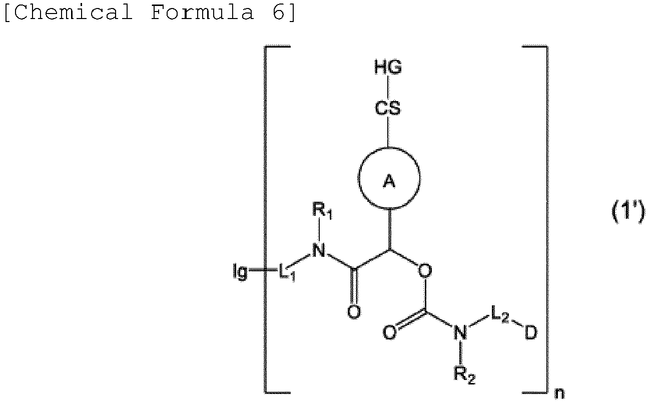 wherein
Ig represents an immunoglobulin unit comprising two heavy chains and two light chains, and is bonded to L₁ adjacent to Ig via a thiol group in side chains of a plurality of cysteine residues in the two heavy chains and the two light chains,
HG represents a hydrophilic group or a monovalent group comprising a hydrophilic group,
CS represents a divalent group comprising a cleavable site,
ring A represents a divalent aromatic ring group optionally having a substituent wherein the divalent aromatic ring group constitutes a π electron conjugated system with the cleavable site,
R₁ and R₂ each independently represent a hydrogen atom or a monovalent group,
L₁ and L₂ each independently represent a divalent group,
D represents a functional substance, and
an average number n of the bonds per the immunoglobulin unit is 1.5 or more.

In a specific embodiment, the structural unit represented by Formula (1) may be represented by the following Formula (1''): 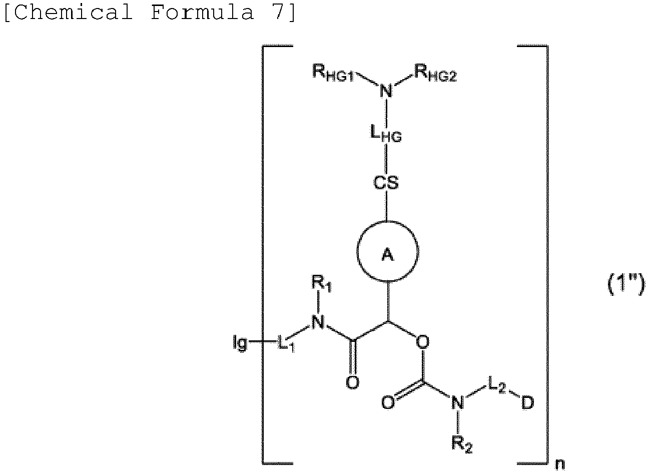 wherein
Ig represents an immunoglobulin unit comprising two heavy chains and two light chains, and is bonded to L₁ adjacent to Ig via a thiol group in side chains of a plurality of cysteine residues in the two heavy chains and the two light chains,
R_{HG1} and R_{HG2} each independently represent a hydrogen atom, a hydrophilic group, or a monovalent group optionally comprising a hydrophilic group,
L_{HG} represents a bond or a divalent group optionally comprising a hydrophilic group,
CS represents a divalent group comprising a cleavable site,
ring A represents a divalent aromatic ring group optionally having a substituent wherein the divalent aromatic ring group constitutes a π electron conjugated system with the cleavable site,
R₁ and R₂ each independently represent a hydrogen atom or a monovalent group,
L₁ and L₂ each independently represent a divalent group,
D represents a functional substance, and
an average number n of the bonds per the immunoglobulin unit is 1.5 or more.

In a specific embodiment, the structural unit represented by Formula (1) may be represented by the following Formula (1a): 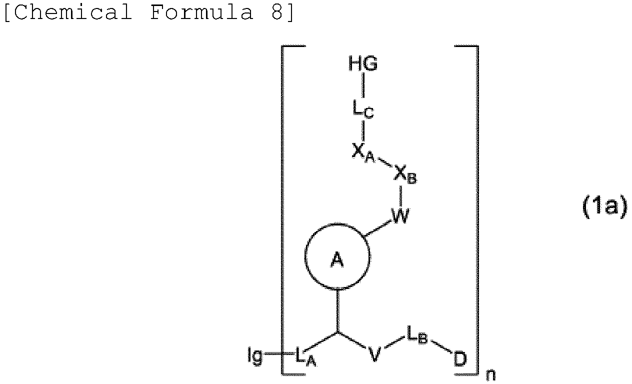 wherein
Ig, HG, ring A, V, L_{A}, L_{B}, D, and n are the same as those represented in Formula (1), respectively,
L_{C} represents a bond or a divalent group,
X_{A} and X_{B} each independently represent an amino acid residue, and
W represents an oxygen atom, a sulfur atom, or an amino group (NH).

In a specific embodiment, the structural unit represented by Formula (1a) may be represented by the following Formula (1b): 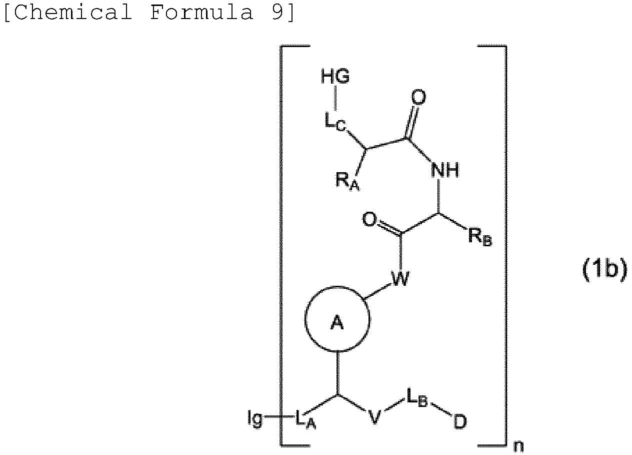 wherein
Ig, HG, ring A, V, L_{A}, L_{B}, D, and n are the same as those represented in Formula (1), respectively,
L_{C} and W are the same as those represented in Formula (1a), respectively,
R_{A} represents a side chain of a valine residue, a phenylalanine residue, a threonine residue, a leucine residue, or an alanine residue, and
R_{B} represents a side chain of a citrulline residue, an alanine residue, a glutamic acid residue, a glutamine residue, a lysine residue, an arginine residue, a threonine residue, or a methionine residue.

In a specific embodiment, the structural unit represented by Formula (1b) may be represented by the following Formula (1c): 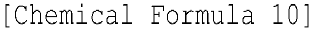 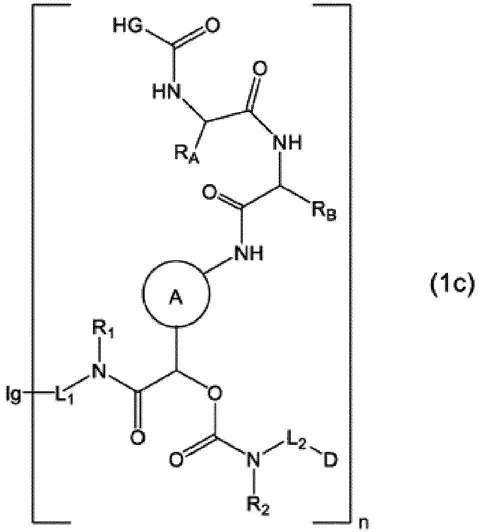 wherein
Ig, HG, ring A, D, and n are the same as those represented in Formula (1), respectively,
R_{A} and R_{B} are the same as those represented in Formula (1b), respectively,
R₁ and R₂ each independently represent a hydrogen atom or a monovalent group, and
L₁ and L₂ each independently represent a divalent group.

In a specific embodiment, the structural unit represented by Formula (1c) may be represented by the following Formula (1d): 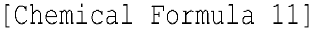 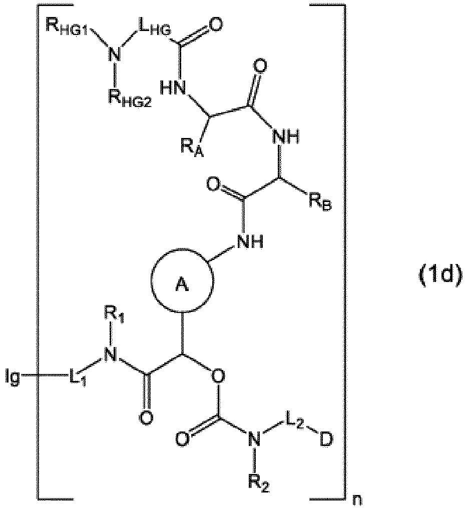 wherein
Ig, ring A, D, and n are the same as those represented in Formula (1), respectively,
R_{A} and R_{B} are the same as those represented in Formula (1b), respectively,
R₁, R₂, L₁, and L₂ are the same as those represented in Formula (1c), respectively,
L_{HG} represents a bond or a divalent group optionally comprising a hydrophilic group,
R_{HG1} and R_{HG2} each independently represent a hydrogen atom, a hydrophilic group, or a monovalent group optionally comprising a hydrophilic group, and
at least one hydrophilic group is comprised in one or more sites selected from the group consisting of L_{HG}, R_{HG1}, and R_{HG2}.

In a preferred embodiment, the conjugate may exhibit an aggregation ratio of 2.6% or less when being analyzed by size exclusion chromatography.

A second embodiment of the present invention provides
an antibody derivative having a bioorthogonal functional group or bioorthogonal functional groups, comprising a structural unit represented by the following Formula (2): 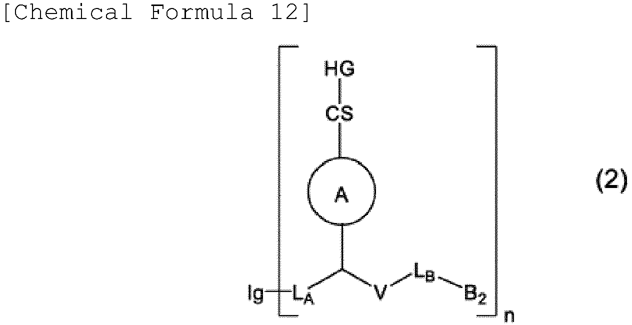 wherein
Ig represents an immunoglobulin unit comprising two heavy chains and two light chains, and is bonded to L_{A} adjacent to Ig via a thiol group in side chains of a plurality of cysteine residues in the two heavy chains and the two light chains,
HG represents a hydrophilic group or a monovalent group comprising a hydrophilic group,
CS represents a divalent group comprising a cleavable site,
ring A represents a divalent aromatic ring group optionally having a substituent wherein the divalent aromatic ring group constitutes a π electron conjugated system with the cleavable site,
V represents an oxygen atom, a sulfur atom, or an amino group (NH),
L_{A} and L_{B} each independently represent a divalent group,
B₂ represents a bioorthogonal functional group, and
an average number n of the bonds per the immunoglobulin unit is 1.5 or more, or
a salt thereof.

In a specific embodiment, the structural unit represented by Formula (2) may be represented by the following Formula (2'): 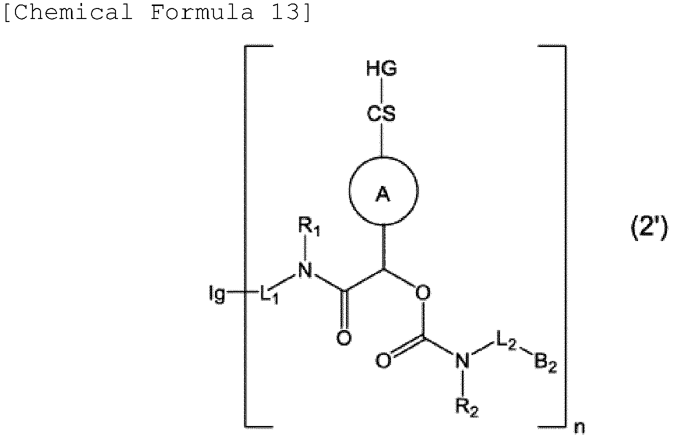 wherein
Ig represents an immunoglobulin unit comprising two heavy chains and two light chains, and is bonded to L₁ adjacent to Ig via a thiol group in side chains of a plurality of cysteine residues in the two heavy chains and the two light chains,
HG represents a hydrophilic group or a monovalent group comprising a hydrophilic group,
CS represents a divalent group comprising a cleavable site by cathepsin B,
ring A represents a divalent aromatic ring group optionally having a substituent wherein the divalent aromatic ring group constitutes a π electron conjugated system with the cleavable site,
R₁ and R₂ each independently represent a hydrogen atom or a monovalent group,
L₁ and L₂ each independently represent a divalent group,
B₂ represents a bioorthogonal functional group, and
an average number n of the bonds per the immunoglobulin unit is 1.5 or more.

In a specific embodiment, the structural unit represented by Formula (2) may be represented by the following Formula (2''): 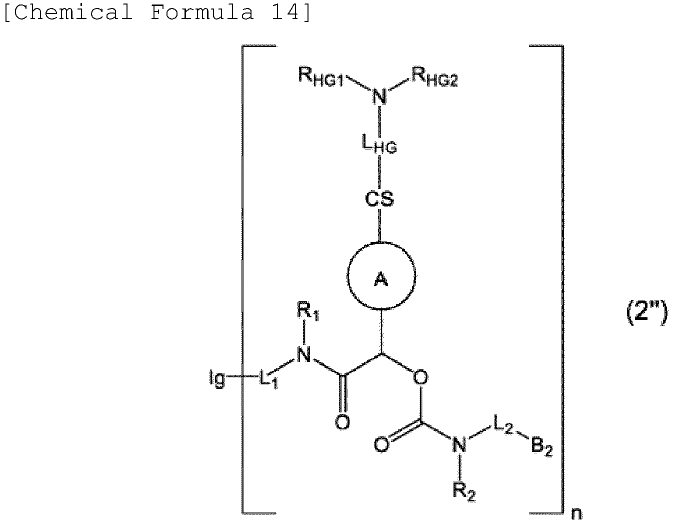 wherein
Ig represents an immunoglobulin unit comprising two heavy chains and two light chains, and is bonded to L₁ adjacent to Ig via a thiol group in side chains of a plurality of cysteine residues in the two heavy chains and the two light chains,
R_{HG1} and R_{HG2} each independently represent a hydrogen atom, a hydrophilic group, or a monovalent group optionally comprising a hydrophilic group,
L_{HG} represents a bond or a divalent group optionally comprising a hydrophilic group,
CS represents a divalent group comprising a cleavable site by cathepsin B,
ring A represents a divalent aromatic ring group optionally having a substituent wherein the divalent aromatic ring group constitutes a π electron conjugated system with the cleavable site,
R₁ and R₂ each independently represent a hydrogen atom or a monovalent group,
L₁ and L₂ each independently represent a divalent group,
B₂ represents a bioorthogonal functional group, and
an average number n of the bonds per the immunoglobulin unit is 1.5 or more.

In a specific embodiment, the structural unit represented by Formula (2) may be represented by the following Formula (2a): 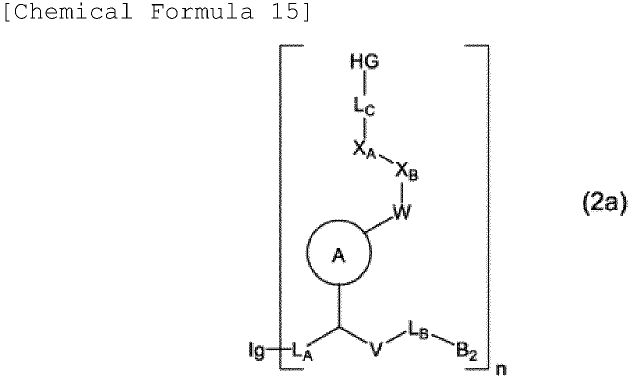 wherein
Ig, HG, ring A, V, L_{A}, L_{B}, B₂, and n are the same as those represented in Formula (2), respectively,
L_{C} represents a bond or a divalent group,
X_{A} and X_{B} each independently represent an amino acid residue, and
W represents an oxygen atom, a sulfur atom, or an amino group (NH).

In a specific embodiment, the structural unit represented by Formula (2a) may be represented by the following Formula (2b): 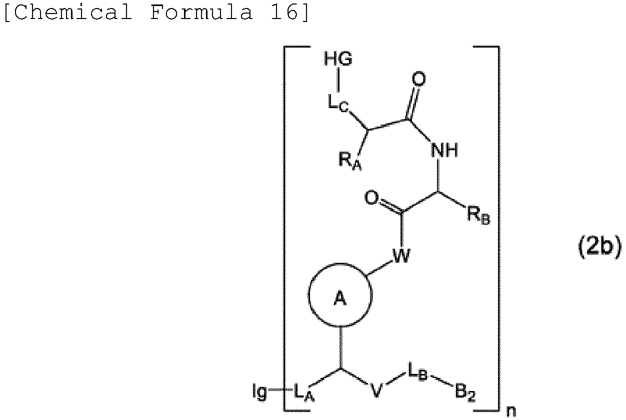 wherein
Ig, HG, ring A, V, L_{A}, L_{B}, B₂, and n are the same as those represented in Formula (2), respectively,
L_{C} and W are the same as those represented in Formula (2a), respectively,
R_{A} represents a side chain of a valine residue, a phenylalanine residue, a threonine residue, a leucine residue, or an alanine residue, and
R_{B} represents a side chain of a citrulline residue, an alanine residue, a glutamic acid residue, a glutamine residue, a lysine residue, an arginine residue, a threonine residue, or a methionine residue.

In a specific embodiment, the structural unit represented by Formula (2b) may be represented by the following Formula (2c): 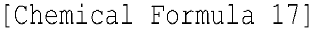 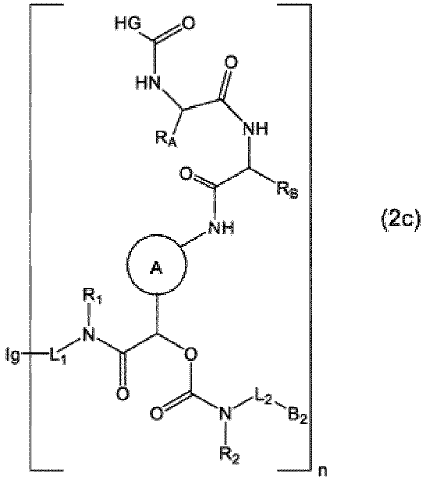 wherein
Ig, HG, ring A, B₂, and n are the same as those represented in Formula (2), respectively,
R_{A} and R_{B} are the same as those represented in Formula (2b), respectively,
R₁ and R₂ each independently represent a hydrogen atom or a monovalent group, and
L₁ and L₂ each independently represent a divalent group.

In a specific embodiment, the structural unit represented by Formula (2c) may be represented by the following Formula (2d): 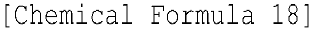 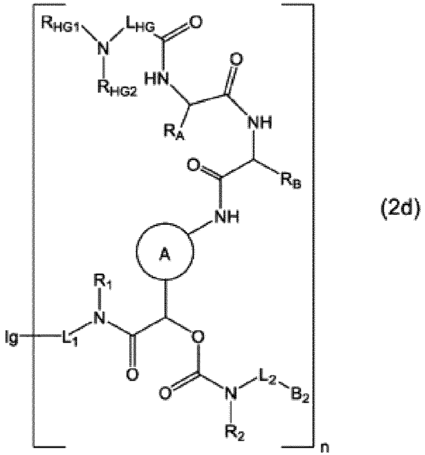 wherein
Ig, ring A, B₂, and n are the same as those represented in Formula (2), respectively,
R_{A} and R_{B} are the same as those represented in Formula (2b), respectively,
R₁, R₂, L₁, and L₂ are the same as those represented in Formula (2c), respectively,
L_{HG} represents a bond or a divalent group optionally comprising a hydrophilic group,
R_{HG1} and R_{HG2} each independently represent a hydrogen atom, a hydrophilic group, or a monovalent group optionally comprising a hydrophilic group, and
at least one hydrophilic group is comprised in one or more sites selected from the group consisting of L_{HG}, R_{HG1}, and R_{HG2}.

A third embodiment of the present invention provides a compound having a bioorthogonal functional group and a functional substance, represented by the following Formula (3): 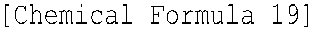 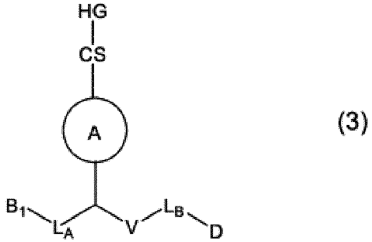 wherein
HG represents a hydrophilic group or a monovalent group comprising a hydrophilic group,
CS represents a divalent group comprising a cleavable site,
ring A represents a divalent aromatic ring group optionally having a substituent wherein the divalent aromatic ring group constitutes a π electron conjugated system with the cleavable site,
V represents an oxygen atom, a sulfur atom, or an amino group (NH),
L_{A} and L_{B} each independently represent a divalent group,
B₁ represents a bioorthogonal functional group, and
D represents a functional substance, or
a salt thereof.

In a specific embodiment, the compound represented by Formula (3) may be represented by the following Formula (3'): 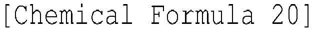 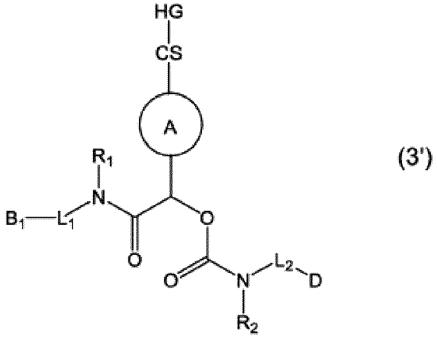 wherein
HG represents a hydrophilic group or a monovalent group comprising a hydrophilic group,
CS represents a divalent group comprising a cleavable site by cathepsin B,
ring A represents a divalent aromatic ring group optionally having a substituent wherein the divalent aromatic ring group constitutes a π electron conjugated system with the cleavable site,
R₁ and R₂ each independently represent a hydrogen atom or a monovalent group,
L₁ and L₂ each independently represent a divalent group,
B₁ represents a bioorthogonal functional group, and
D represents a functional substance.

In a specific embodiment, the compound represented by Formula (3) may be represented by the following Formula (3''): 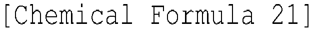 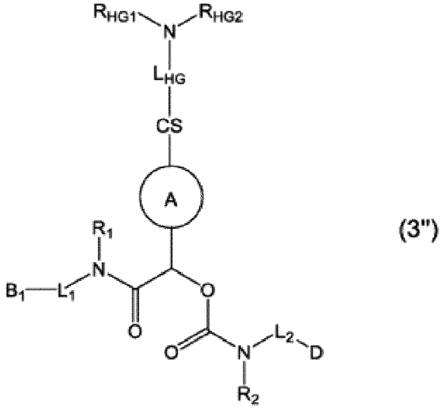 wherein
R_{HG1} and R_{HG2} each independently represent a hydrogen atom, a hydrophilic group, or a monovalent group optionally comprising a hydrophilic group,
L_{HG} represents a bond or a divalent group optionally comprising a hydrophilic group,
CS represents a divalent group comprising a cleavable site by cathepsin B,
ring A represents a divalent aromatic ring group optionally having a substituent wherein the divalent aromatic ring group constitutes a π electron conjugated system with the cleavable site,
R₁ and R₂ each independently represent a hydrogen atom or a monovalent group,
L₁ and L₂ each independently represent a divalent group,
B₁ represents a bioorthogonal functional group, and
D represents a functional substance.

In a specific embodiment, the compound represented by Formula (3) may be represented by the following Formula (3a): 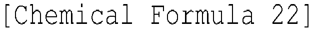 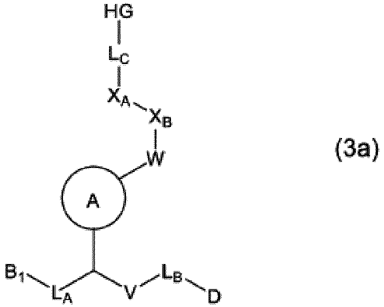 wherein
HG, ring A, V, L_{A}, L_{B}, B₁, and D are the same as those represented in Formula (3), respectively,
L_{C} represents a bond or a divalent group,
X_{A} and X_{B} each independently represent an amino acid residue, and
W represents an oxygen atom, a sulfur atom, or an amino group (NH).

In a specific embodiment, the compound represented by Formula (3a) may be represented by the following Formula (3b): 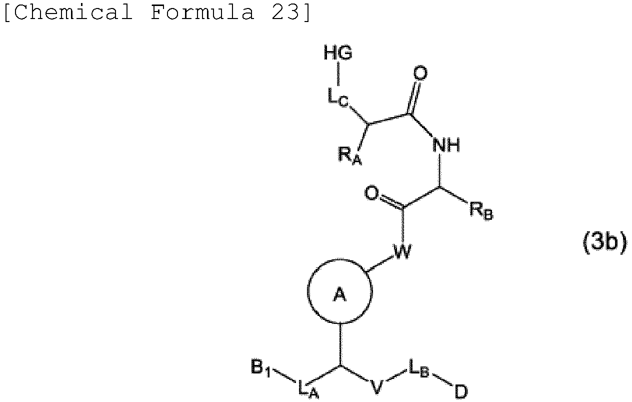 wherein
HG, ring A, V, L_{A}, L_{B}, B₁, and D are the same as those represented in Formula (3), respectively,
L_{C} and W are the same as those represented in Formula (3a), respectively,
R_{A} represents a side chain of a valine residue, a phenylalanine residue, a threonine residue, a leucine residue, or an alanine residue, and
R_{B} represents a side chain of a citrulline residue, an alanine residue, a glutamic acid residue, a glutamine residue, a lysine residue, an arginine residue, a threonine residue, or a methionine residue.

In a specific embodiment, the compound represented by Formula (3b) may be represented by the following Formula (3c): 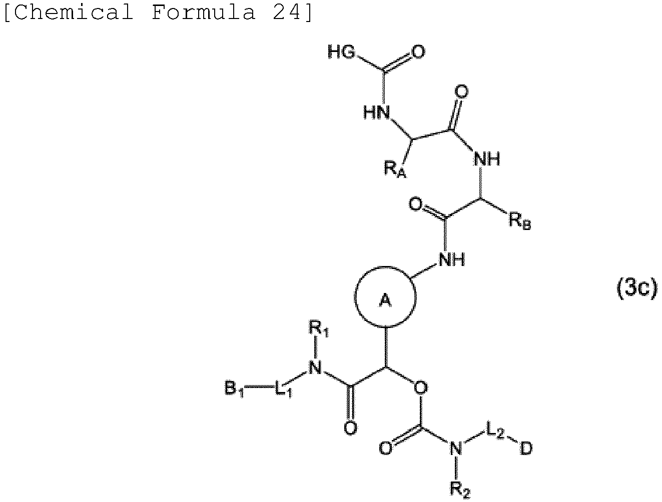 wherein
HG, ring A, B₁, and D are the same as those represented in Formula (3), respectively,
R_{A} and R_{B} are the same as those represented in Formula (3b), respectively,
R₁ and R₂ each independently represent a hydrogen atom or a monovalent group, and
L₁ and L₂ each independently represent a divalent group.

In a specific embodiment, the compound represented by Formula (3c) may be represented by the following Formula (3d): 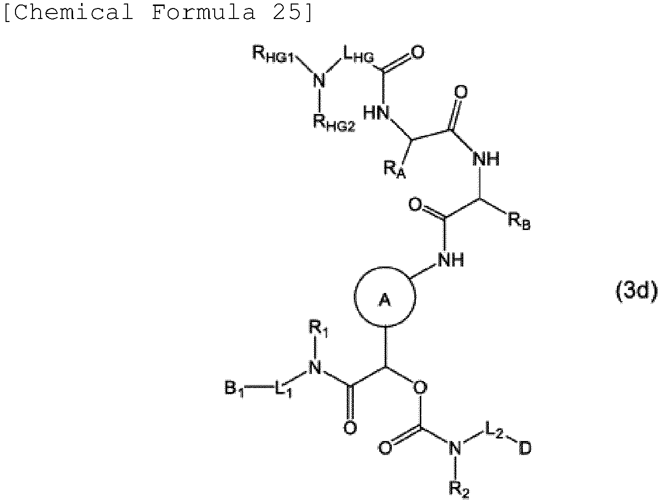 wherein
ring A, B₁, and D are the same as those represented in Formula (3), respectively,
R_{A} and R_{B} are the same as those represented in Formula (3b), respectively,
R₁, R₂, L₁, and L₂ are the same as those represented in Formula (3c), respectively,
L_{HG} represents a bond or a divalent group optionally comprising a hydrophilic group,
R_{HG1} and R_{HG2} each independently represent a hydrogen atom, a hydrophilic group, or a monovalent group optionally comprising a hydrophilic group, and
at least one hydrophilic group is comprised in one or more sites selected from the group consisting of L_{HG}, R_{HG1}, and R_{HG2}.

The present invention also provides a reagent for derivatizing an antibody, the reagent comprising a compound represented by the above Formula (3) or a formula of a subordinate concept thereof, or a salt thereof.

A fourth embodiment of the present invention provides a compound having a first bioorthogonal functional group and a second bioorthogonal functional group, represented by the following Formula (4): 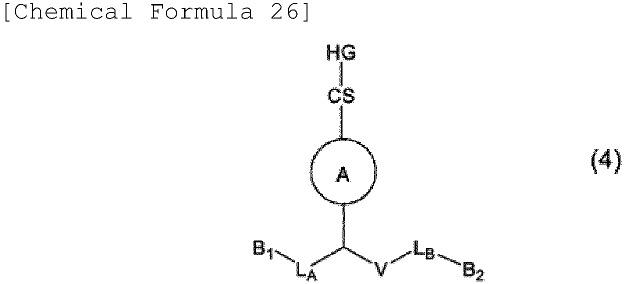 wherein
HG represents a hydrophilic group or a monovalent group comprising a hydrophilic group,
CS represents a divalent group comprising a cleavable site,
ring A represents a divalent aromatic ring group optionally having a substituent wherein the divalent aromatic ring group constitutes a π electron conjugated system with the cleavable site,
V represents an oxygen atom, a sulfur atom, or an amino group (NH),
L_{A} and L_{B} each independently represent a divalent group,
B₁ represents a first bioorthogonal functional group, and
B₂ represents a second bioorthogonal functional group, or
a salt thereof.

In a specific embodiment, the compound represented by Formula (4) may be represented by the following Formula (4'): 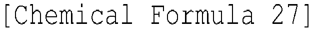 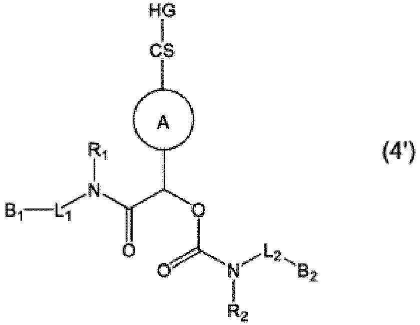 wherein
HG represents a hydrophilic group or a monovalent group comprising a hydrophilic group,
CS represents a divalent group comprising a cleavable site,
ring A represents a divalent aromatic ring group optionally having a substituent wherein the divalent aromatic ring group constitutes a π electron conjugated system with the cleavable site,
R₁ and R₂ each independently represent a hydrogen atom or a monovalent group,
L₁ and L₂ each independently represent a divalent group,
B₁ represents a first bioorthogonal functional group, and
B₂ represents a second bioorthogonal functional group.

In a specific embodiment, the compound represented by Formula (4) may be represented by the following Formula (4"): 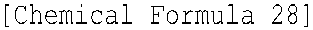 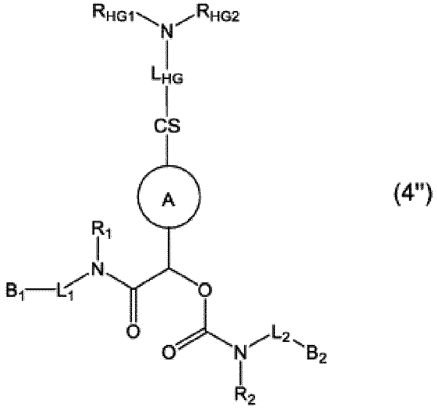 wherein
R_{HG1} and R_{HG2} each independently represent a hydrogen atom, a hydrophilic group, or a monovalent group optionally comprising a hydrophilic group,
L_{HG} represents a bond or a divalent group optionally comprising a hydrophilic group,
CS represents a divalent group comprising a cleavable site,
ring A represents a divalent aromatic ring group optionally having a substituent wherein the divalent aromatic ring group constitutes a π electron conjugated system with the cleavable site,
R₁ and R₂ each independently represent a hydrogen atom or a monovalent group,
L₁ and L₂ each independently represent a divalent group,
B₁ represents a first bioorthogonal functional group, and
B₂ represents a second bioorthogonal functional group.

In a specific embodiment, the compound represented by Formula (4) may be represented by the following Formula (4a): 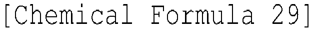 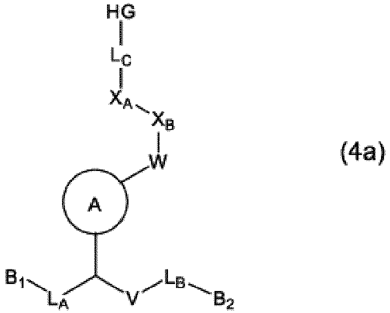 wherein
HG, ring A, V, L_{A}, L_{B}, B₁, and B₂ are the same as those represented in Formula (4), respectively,
L_{C} represents a bond or a divalent group,
X_{A} and X_{B} each independently represent an amino acid residue, and
W represents an oxygen atom, a sulfur atom, or an amino group (NH).

In a specific embodiment, the compound represented by Formula (4a) may be represented by the following Formula (4b): 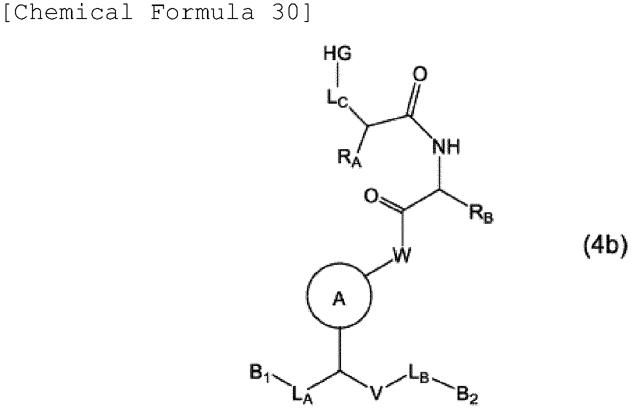 wherein
HG, ring A, V, L_{A}, L_{B}, B₁, and B₂ are the same as those represented in Formula (4), respectively,
L_{C} and W are the same as those represented in Formula (4a), respectively,
R_{A} represents a side chain of a valine residue, a phenylalanine residue, a threonine residue, a leucine residue, or an alanine residue, and
R_{B} represents a side chain of a citrulline residue, an alanine residue, a glutamic acid residue, a glutamine residue, a lysine residue, an arginine residue, a threonine residue, or a methionine residue.

In a specific embodiment, the compound represented by Formula (4b) may be represented by the following Formula (4c): 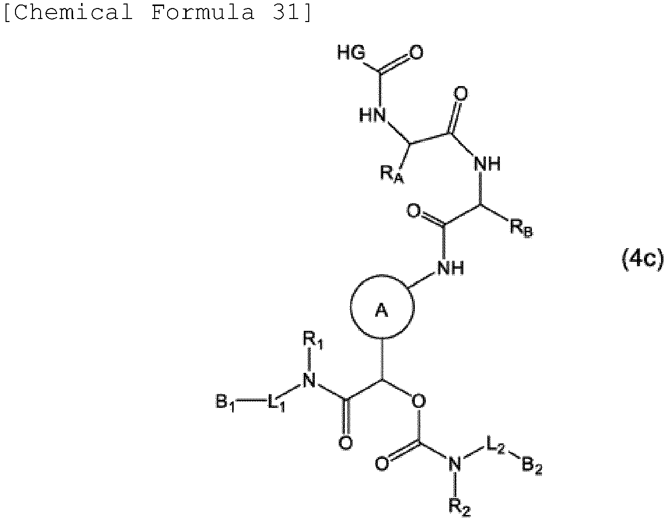 wherein
HG, ring A, B₁, and B₂ are the same as those represented in Formula (4), respectively,
R_{A} and R_{B} are the same as those represented in Formula (4b), respectively,
R₁ and R₂ each independently represent a hydrogen atom or a monovalent group, and
L₁ and L₂ each independently represent a divalent group.

In a specific embodiment, the compound represented by Formula (4c) may be represented by the following Formula (4d): 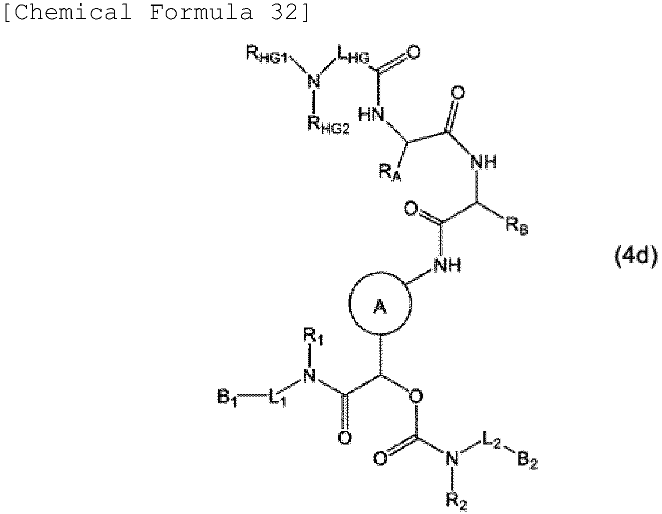 wherein
ring A, B₁, and B₂ are the same as those represented in Formula (4), respectively,
R_{A} and R_{B} are the same as those represented in Formula (4b), respectively,
R₁, R₂, L₁, and L₂ are the same as those represented in Formula (4c), respectively,
L_{HG} represents a bond or a divalent group optionally comprising a hydrophilic group,
R_{HG1} and R_{HG2} each independently represent a hydrogen atom, a hydrophilic group, or a monovalent group optionally comprising a hydrophilic group, and
at least one hydrophilic group is comprised in one or more sites selected from the group consisting of L_{HG}, R_{HG1}, and R_{HG2}.

The present invention also provides a reagent for derivatizing an antibody or a functional substance, the reagent comprising a compound represented by the above Formula (4) or a formula of a subordinate concept thereof, or a salt thereof.

A fifth embodiment of the present invention provides a compound or a salt thereof of the following (A), (B), or (C):
(A) a compound represented by the following Formula (5) : 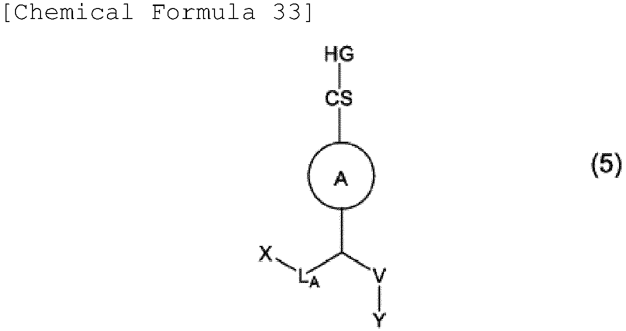 wherein
   HG represents a hydrophilic group or a monovalent group comprising a hydrophilic group,
   CS represents a divalent group comprising a cleavable site,
   ring A represents a divalent aromatic ring group optionally having a substituent wherein the divalent aromatic ring group constitutes a π electron conjugated system with the cleavable site,
   V represents an oxygen atom, a sulfur atom, or an amino group (NH),
   L_{A} represents a divalent group, and
   X and Y each independently represent a monovalent group, or
   a salt thereof;
(B) a compound having a bioorthogonal functional group, represented by the following Formula (6): 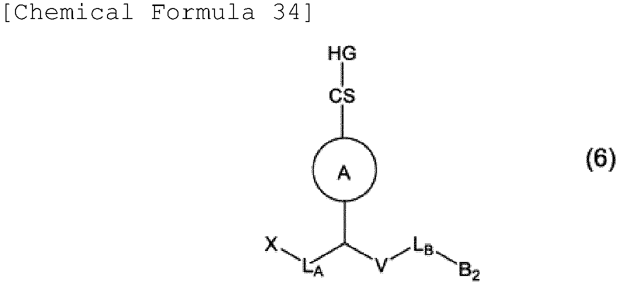 wherein
   HG, CS, ring A, V, L_{A}, and X are the same as those represented in Formula (5), respectively,
   L_{B} represents a divalent group, and
   B₂ represents the bioorthogonal functional group, or
   a salt thereof; or
(C) a compound having a bioorthogonal functional group, represented by the following Formula (7): 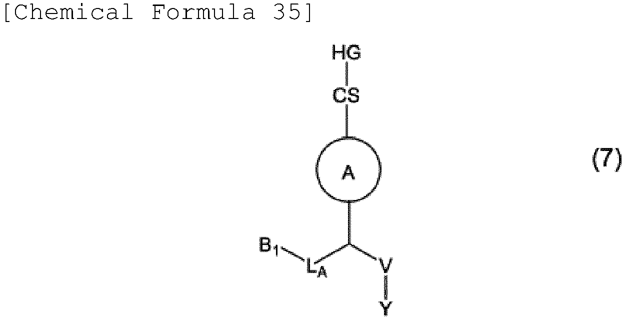 wherein
   HG, CS, Ring A, V, L_{A}, and Y are the same as those represented in Formula (5), respectively, and
   B₁ represents the bioorthogonal functional group, or
   a salt thereof.

In a specific embodiment, the above compound of (A) may be represented by the following Formula (5'): 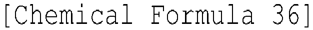 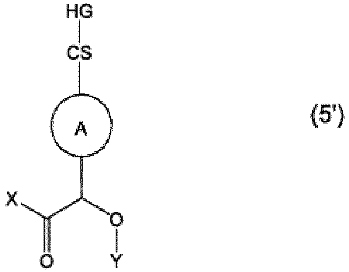 wherein
HG represents a hydrophilic group or a monovalent group comprising a hydrophilic group,
CS represents a divalent group comprising a cleavable site,
ring A represents a divalent aromatic ring group optionally having a substituent wherein the divalent aromatic ring group constitutes a π electron conjugated system with the cleavable site, and
X and Y each independently represent a monovalent group.

In a specific embodiment, the above compound of (A) may be represented by the following Formula (5''): 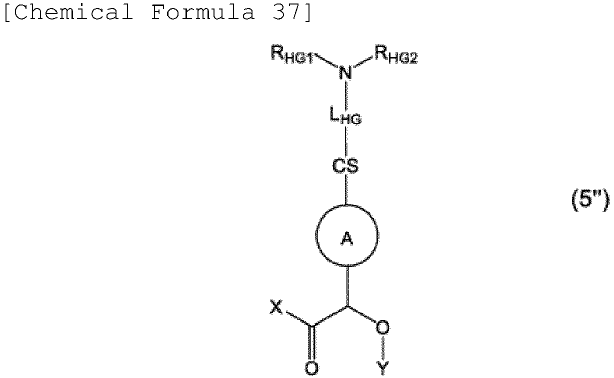 wherein
R_{HG1} and R_{HG2} each independently represent a hydrogen atom, a hydrophilic group, or a monovalent group optionally comprising a hydrophilic group,
L_{HG} represents a bond or a divalent group optionally comprising a hydrophilic group,
CS represents a divalent group comprising a cleavable site,
ring A represents a divalent aromatic ring group optionally having a substituent wherein the divalent aromatic ring group constitutes a π electron conjugated system with the cleavable site, and
X and Y each independently represent a monovalent group.

In a specific embodiment, the above compound of (B) may be represented by the following Formula (6'): 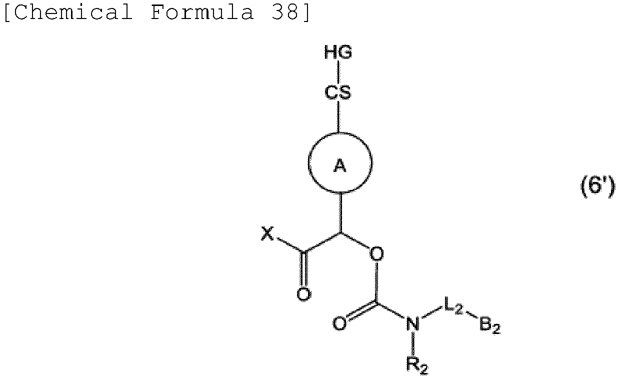 wherein
HG, CS, ring A, and X are the same as those represented in Formula (5), respectively,
R₂ represents a hydrogen atom or a monovalent group,
L₂ represents a divalent group, and
B₂ represents a bioorthogonal functional group.

In a specific embodiment, the above compound of (B) may be represented by the following Formula (6''): 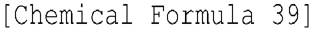 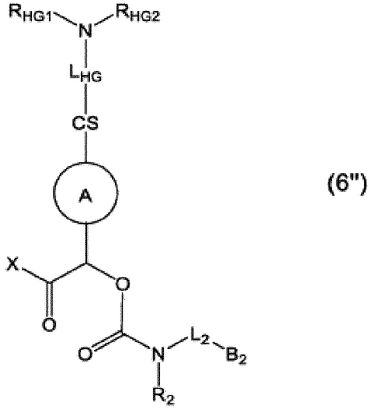 wherein
CS, ring A, and X are the same as those represented in Formula (5), respectively,
R_{HG1} and R_{HG2} each independently represent a hydrogen atom, a hydrophilic group, or a monovalent group optionally comprising a hydrophilic group,
L_{HG} represents a bond or a divalent group optionally comprising a hydrophilic group,
R₂ represents a hydrogen atom or a monovalent group,
L₂ represents a divalent group, and
B₂ represents a bioorthogonal functional group.

In a specific embodiment, the above compound of (C) may be represented by the following Formula (7'): 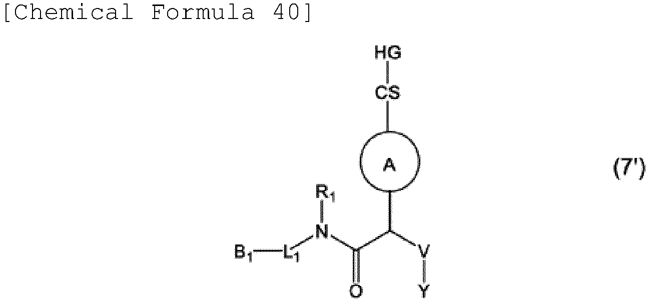 wherein
HG, CS, ring A, V, and Y are the same as those represented in Formula (5), respectively,
R₁ represents a hydrogen atom or a monovalent group,
L₁ represents a divalent group, and
B₁ represents a bioorthogonal functional group.

In a specific embodiment, the above compound of (C) may be represented by the following Formula (7''): 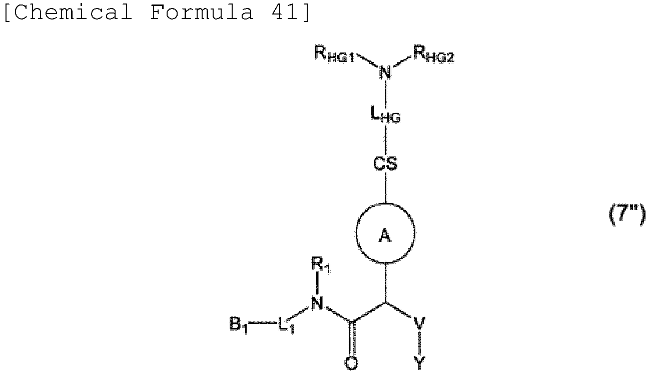 wherein
CS, ring A, V, and Y are the same as those represented in Formula (5), respectively,
R_{HG1} and R_{HG2} each independently represent a hydrogen atom, a hydrophilic group, or a monovalent group optionally comprising a hydrophilic group,
L_{HG} represents a bond or a divalent group optionally comprising a hydrophilic group,
R₁ represents a hydrogen atom or a monovalent group,
L₁ represents a divalent group, and
B₁ represents a bioorthogonal functional group.

In a specific embodiment, the above compounds of (A), (B), and (C) may be the following compounds of (A-1), (B-1), and (C-1), respectively:
(A-1) a compound represented by the following Formula (5a) : 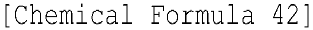 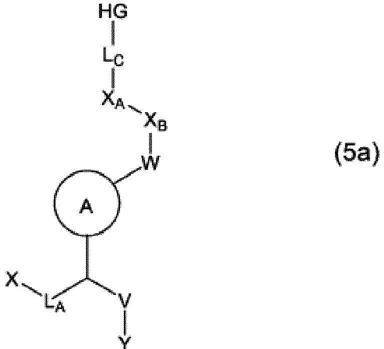 wherein
   HG, ring A, V, L_{A}, X, and Y are the same as those represented in Formula (5), respectively,
   L_{C} represents a bond or a divalent group,
   X_{A} and X_{B} each independently represent an amino acid residue, and
   W represents an oxygen atom, a sulfur atom, or an amino group (NH), or
   a salt thereof;
(B-1) a compound represented by the following Formula (6a) : 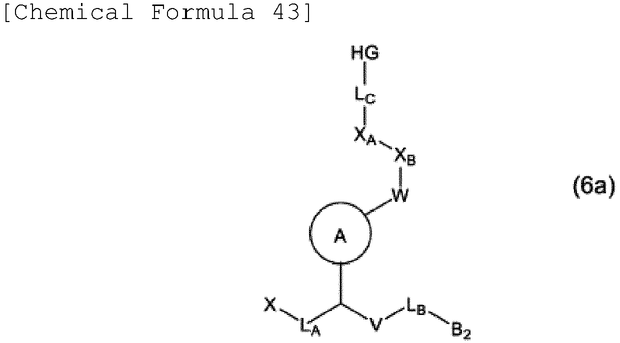 wherein
   HG, ring A, V, L_{A}, and X are the same as those represented in Formula (5), respectively,
   L_{C}, X_{A}, X_{B}, and W are the same as those represented in Formula (5a), respectively, and
   L_{B} and B₂ are the same as those represented in Formula (6), respectively, or
   a salt thereof; and
(C-1) a compound represented by the following Formula (7a) : 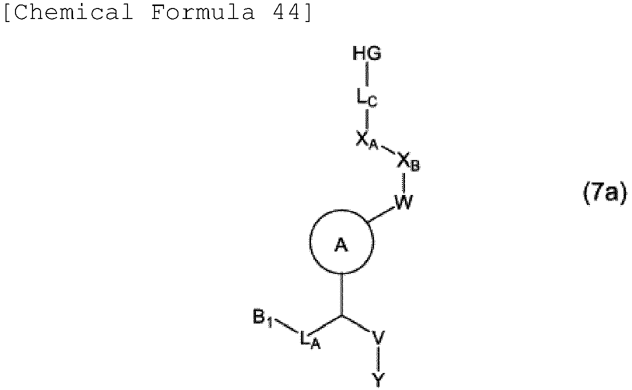 wherein
HG, ring A, V, L_{A}, and Y are the same as those represented in Formula (5), respectively,
L_{C}, X_{A}, X_{B}, and W are the same as those represented in Formula (5a), respectively, and
B₁ is the same as that represented in Formula (7), or
a salt thereof.

In a specific embodiment, the above compounds of (A-1), (B-1), and (C-1) may be the following compounds of (A-2), (B-2), and (C-2), respectively:
(A-2) a compound represented by the following Formula (5b) : 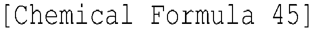 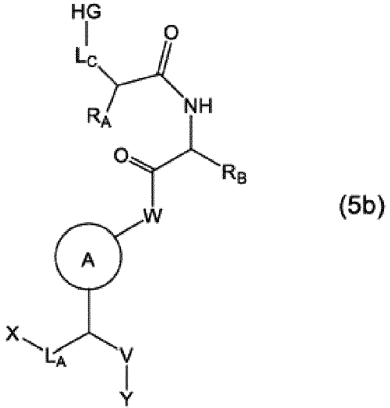 wherein
   HG, ring A, V, L_{A}, X, and Y are the same as those represented in Formula (5), respectively,
   L_{C} and W are the same as those represented in Formula (5a), respectively,
   R_{A} represents a side chain of a valine residue, a phenylalanine residue, a threonine residue, a leucine residue, or an alanine residue, and
   R_{B} represents a side chain of a citrulline residue, an alanine residue, a glutamic acid residue, a glutamine residue, a lysine residue, an arginine residue, a threonine residue, or a methionine residue, or
   a salt thereof;
(B-2) a compound represented by the following Formula (6b) : 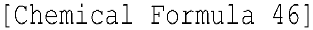 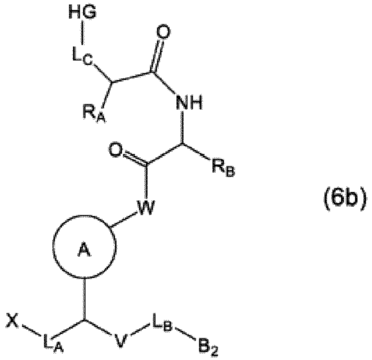 wherein
   HG, ring A, V, L_{A}, and X are the same as those represented in Formula (5), respectively,
   L_{C} and W are the same as those represented in Formula (5a), respectively,
   R_{A} and R_{B} are the same as those represented in Formula (5b), respectively, and
   L_{B} and B₂ are the same as those represented in Formula (6), respectively, or
   a salt thereof; and
(C-2) a compound represented by the following Formula (7b) : 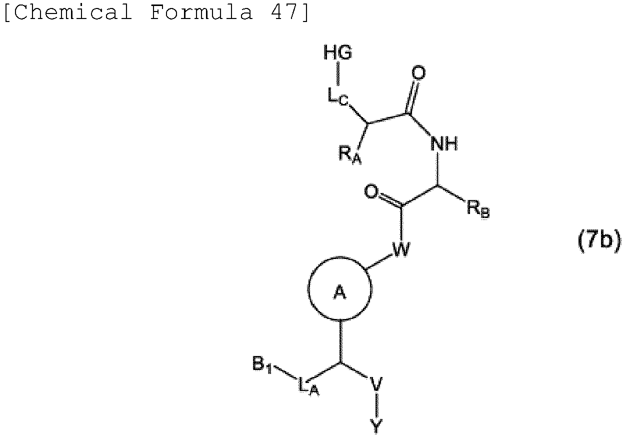 wherein
   HG, ring A, V, L_{A}, and Y are the same as those represented in Formula (5), respectively,
   L_{C}, X_{A}, X_{B}, and W are the same as those represented in Formula (5a), respectively,
   R_{A} and R_{B} are the same as those represented in Formula (5b), respectively, and
   B₁ is the same as that represented in Formula (7), or
   a salt thereof.

In a specific embodiment, the above compounds of (A-2), (B-2), and (C-2) may be the following compounds of (A-3), (B-3), and (C-3), respectively:
(A-3) a compound represented by the following Formula (5c) : 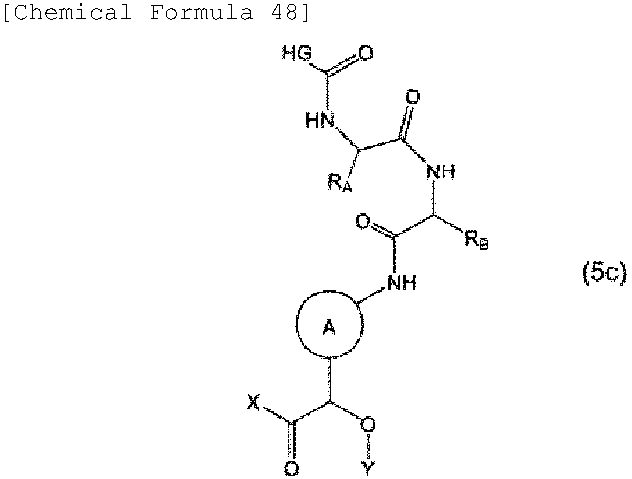 wherein
   HG, ring A, X, and Y are the same as those represented in Formula (5), respectively, and
   R_{A} and R_{B} are the same as those represented in Formula (5b), respectively, or
   a salt thereof;
(B-3) a compound represented by the following Formula (6c) : 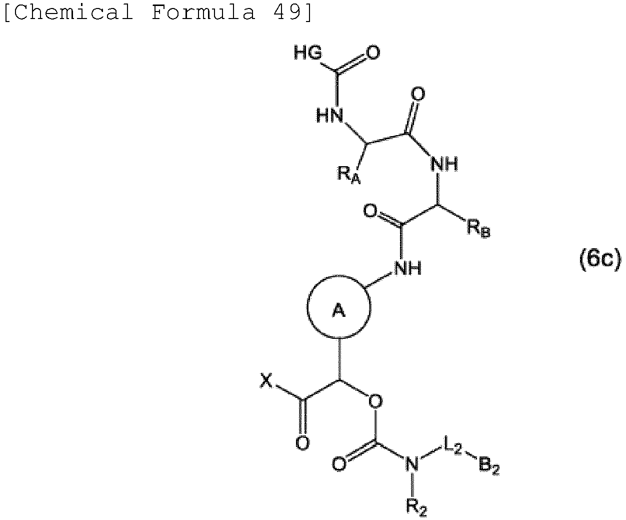 wherein
   HG, ring A, and X are the same as those represented in Formula (5), respectively,
   R_{A} and R_{B} are the same as those represented in Formula (5b), respectively,
   B₂ is the same as that represented in Formula (6),
   R₂ represents a hydrogen atom or a monovalent group, and
   L₂ represents a divalent group, or
   a salt thereof; and
(C-3) a compound represented by the following Formula (7c) : 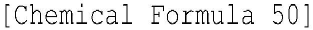 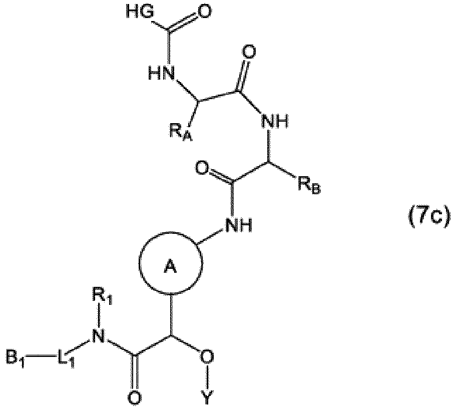
wherein
HG, ring A, and Y are the same as those represented in Formula (5), respectively,
R_{A} and R_{B} are the same as those represented in Formula (5b), respectively,
B₁ is the same as that represented in Formula (7),
R₁ represents a hydrogen atom or a monovalent group, and
L₁ represents a divalent group, or
a salt thereof.

In a specific embodiment, the above compounds of (A-3), (B-3), and (C-3) may be the following compounds of (A-4), (B-4), and (C-4), respectively:
(A-4) a compound represented by the following Formula (5d) : 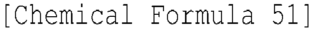 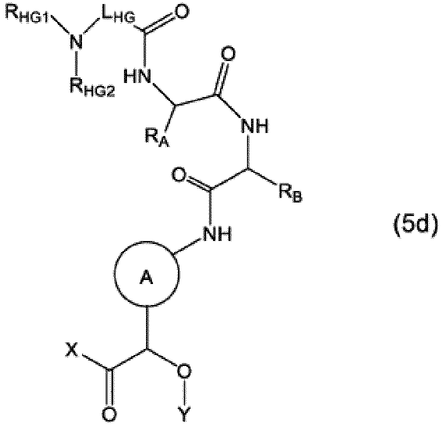 wherein
   ring A, X, and Y are the same as those represented in Formula (5), respectively,
   R_{A} and R_{B} are the same as those represented in Formula (5b), respectively,
   L_{HG} represents a bond or a divalent group optionally comprising a hydrophilic group,
   R_{HG1} and R_{HG2} each independently represent a hydrogen atom, a hydrophilic group, or a monovalent group optionally comprising a hydrophilic group, and
   at least one hydrophilic group is comprised in one or more sites selected from the group consisting of L_{HG}, R_{HG1}, and R_{HG2}, or
   a salt thereof;
(B-4) a compound represented by the following Formula (6d) : 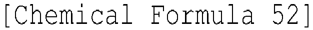 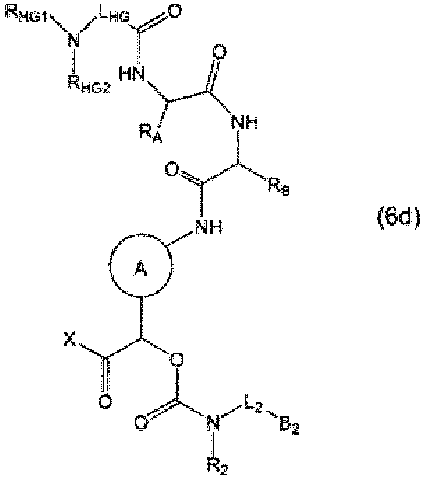 wherein
   ring A and X are the same as those represented in Formula (5), respectively,
   R_{A} and R_{B} are the same as those represented in Formula (5b), respectively,
   L_{HG}, R_{HG1}, and R_{HG2} are the same as those represented in Formula (5d), respectively,
   B₂ is the same as that represented in Formula (6), and
   R₂ and L₂ are the same as those represented in Formula (6c), respectively, or
   a salt thereof; and
(C-4) a compound represented by the following Formula (7d) : 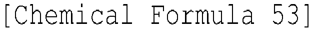 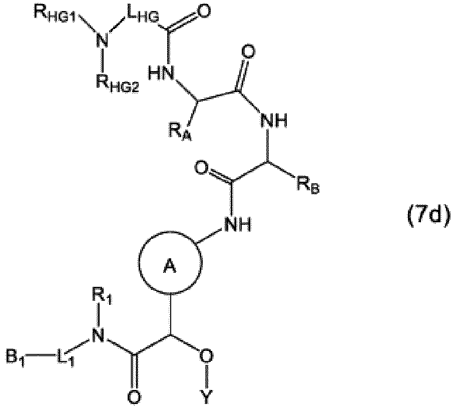
wherein
ring A and Y are the same as those represented in Formula (5), respectively,
R_{A} and R_{B} are the same as those represented in Formula (5b), respectively,
L_{HG}, R_{HG1}, and R_{HG2} are the same as those represented in Formula (5d), respectively,
B₁ is the same as that represented in Formula (7), and
R₁ and L₁ are the same as those represented in Formula (7c), respectively, or
a salt thereof.

In a preferred embodiment, the immunoglobulin unit may be a human immunoglobulin unit.

In a preferred embodiment, the human immunoglobulin unit may be a human IgG antibody.

In a preferred embodiment, n may be 2.0 to 8.0.

In a preferred embodiment, n may be 6.0 to 8.0.

In a preferred embodiment, n may be 7.0 to 8.0.

In a preferred embodiment, the hydrophilic group may be one or more groups selected from the group consisting of a carboxylic acid group, a sulfonate group, a hydroxy group, a polyethylene glycol group, a polysarcosine group, and a sugar portion.

In a preferred embodiment, the cleavable site may be a cleavable site by an enzyme.

In a preferred embodiment, the enzyme may be cathepsin B.

In a preferred embodiment, the divalent group comprising the cleavable site may satisfy the following conditions (i) to (iii):
(i) the cleavable site is -CO-W- wherein the hyphen (-) represents a bond, and W represents an oxygen atom, a sulfur atom, or an amino group (NH), and is bonded to the divalent aromatic ring group;
(ii) a bond between CO and W is a site to be cleaved; and
(iii) the divalent aromatic ring group constitutes a π electron conjugated system with W.

In a preferred embodiment, ring A may be a phenylene group optionally having a substituent.

In a preferred embodiment, the functional substance may be a medicament, a labelling substance, or a stabilizer.

In a preferred embodiment, X_{A} may represent a valine residue, a phenylalanine residue, a threonine residue, a leucine residue or an alanine residue, and
X_{B} may represent a citrulline residue, an alanine residue, a glutamic acid residue, a glutamine residue, a lysine residue, an arginine residue, a threonine residue, or a methionine residue.

In a preferred embodiment, the divalent group (-L_{HG}-) optionally comprising a hydrophilic group may be
a divalent group represented by the following Formula (a) :

   -(C(R_{HG})₂)ₙ₁-(C=O)ₙ₂-(NR_{HG})ₙ₃-(C(R_{HG})₂)ₙ₄- (a)

   wherein
a plurality of R_{HG} each independently represent a hydrogen atom, a hydrophilic group, or a monovalent group optionally comprising a hydrophilic group,
n1 is an integer of 0 to 3,
n2 is an integer of 0 or 1,
n3 is an integer of 0 or 1, and
n4 is an integer of 0 to 3.

In a preferred embodiment, the divalent group represented by Formula (a) may be the following Formula (a1), (a2), or (a3) :

(a1) -(C(R_{HC})₂)-;

(a2) -(C(R_{HC})₂)-(C=O)-(NR_{HG})-(C(R_{HC})₂)-;

or

(a3) -(C=O)-(C(R_{HC})₂)₂-,

wherein
a plurality of R_{HG} each independently represent a hydrogen atom, a hydrophilic group, or a C₁₋₆ alkyl group comprising a hydrophilic group.

In a preferred embodiment, the hydrophilic groups may each independently represent a carboxylic acid group, a sulfonate group, or a hydroxy group.

In a preferred embodiment, the hydrophilic group may be a carboxylic acid group.

In a preferred embodiment, the bioorthogonal functional group represented by B₂ may be a furan residue, an alkene residue, an alkyne residue, an azide residue, or a tetrazine residue.

### Effects of Invention

The conjugate of the present invention or a salt thereof can have excellent properties such as a long residence time in body and a high monomer ratio (low aggregation ratio).

The antibody derivative and the compound or salts thereof and the reagent of the present invention are useful as synthetic intermediates in production of the conjugate.

### Brief Description of Drawings

FIG. 1 is a diagram illustrating an example of release of a drug (an active drug in which steric hindrance by an antibody is eliminated) by cleavage of a cleavable site and interlocking of a π electron resonance system. The conjugate, the antibody derivative, and the compound of the present invention are designed so as to allow such release (see also description of an action of cathepsin B in Background as appropriate). The antibody can retain a hydrophilic group even after cleavage. That is, before cleavage, the drug is linked to a hydrophilic group in a form of a conjugate with the antibody, and therefore hydrophobicity of the drug can be suppressed (masking by the hydrophilic group), and after cleavage, the hydrophilic group is dissociated from the drug, and therefore the drug can exhibit original hydrophobicity of the drug.

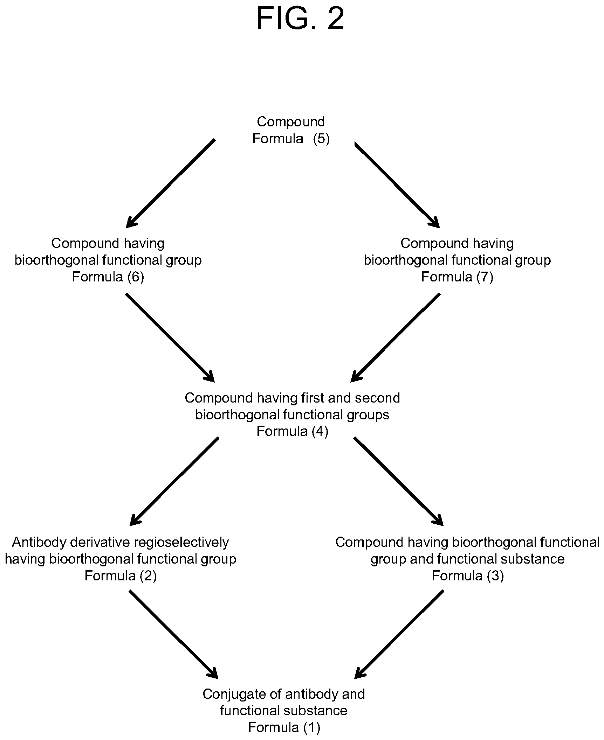

FIG. 2 is a diagram illustrating a correlation among a conjugate of the present invention represented by Formula (1), an antibody derivative of the present invention represented by Formula (2), and compounds of the present invention represented by Formulas (3) to (7). These substances share partial structural units excluding X and Y among structural units represented in Formula (5). In addition, these substances can be synthesized by a scheme illustrated in FIG. 2. Therefore, the present invention provides a series of inventions having a relation of a synthetic intermediate and a final synthetic product.
FIG. 3 is a diagram illustrating a synthesis outline of the conjugate of the present invention represented by Formula (1), the antibody derivative of the present invention represented by Formula (2), and the compounds of the present invention represented by Formulas (3) and (4).
FIG. 4 is a diagram illustrating an example of a synthesis outline of compounds of the present invention represented by Formulas (4) to (7).
FIG. 5 is a diagram illustrating an example of a synthesis outline of compounds represented by Formulas (4c) to (7c) which are preferred examples of the compound of the present invention represented by Formulas (4) to (7). DIPEA: N,N-diisopropylethylamine; DMF: N,N-dimethylformamide

### Description of Embodiments

### 1. Definitions of general terms

In the present invention, the term "antibody" is as follows. The term "immunoglobulin unit" corresponds to a divalent monomer unit that is a basic constituent element of such an antibody, and is a unit comprising two heavy chains and two light chains. Therefore, for the immunoglobulin unit, definitions, examples, and preferred examples of the origin, type (polyclonal or monoclonal, isotype, and full-length antibody or antibody fragment), antigen, and position of a cysteine residue are similar to those for the antibody described below.

The origin of the antibody is not particularly limited, and for example, the antibody may be derived from an animal such as a mammal or a bird (e.g., a domestic fowl). The immunoglobulin unit is preferably derived from a mammal. Examples of such a mammal include primates (e.g., humans, monkeys, and chimpanzees), rodents (e.g., mice, rats, guinea pigs, hamsters, and rabbits), pets (e.g., dogs and cats), domestic animals (e.g., cows, pigs, and goats), and work animals (e.g., horses and sheep). Primates and rodents are preferred, and humans are more preferred.

The type of the antibody may be a polyclonal antibody or a monoclonal antibody. The antibody may be a divalent antibody (e.g., IgG, IgD, or IgE) or a tetravalent or higher antibody (e.g., IgA antibody or IgM antibody). The antibody is preferably a monoclonal antibody. Examples of the monoclonal antibody include chimeric antibodies, humanized antibodies, human antibodies, antibodies with a certain sugar chain added (e.g., an antibody modified so as to have a sugar chain-bonding consensus sequence such as an N-type sugar chain-bonding consensus sequence), bi-specific antibodies, Fc region proteins, and Fc-fusion proteins. Examples of the isotype of the monoclonal antibody include IgG (e.g., IgG1, IgG2, IgG3, and IgG4), IgM, IgA, IgD, IgE, and IgY. In the present invention, as the monoclonal antibody, a full-length antibody or an antibody fragment comprising a variable region and a CH1 domain and a CH2 domain can be used, but a full-length antibody is preferred. The antibody is preferably a human IgG monoclonal antibody, and more preferably a human IgG full-length monoclonal antibody.

As an antigen of the antibody, any antigen can be used. Examples of such an antigen include a protein [which comprises an oligopeptide and a polypeptide, and may be a protein modified with a biomolecule such as a sugar (e.g., a glycoprotein)], a sugar chain, a nucleic acid, and a small compound. The antibody may be preferably an antibody with a protein as an antigen. Examples of the protein include cell membrane receptors, cell membrane proteins other than cell membrane receptors (e.g., extracellular matrix proteins), ligands, and soluble receptors.

More specifically, the protein as the antigen of the antibody may be a disease target protein. Examples of the disease target protein include the following.
(1) Cancerous Region
   PD-L1, GD2, PDGFRα (a platelet-derived growth factor receptor), CD22, HER2, phosphatidyl serine (PS), EpCAM, fibronectin, PD-1, VEGFR-2, CD33, HGF, gpNMB, CD27, DEC-205, folic acid receptors, CD37, CD19, Trop2, CEACAM5, S1P, HER3, IGF-1R, DLL4, TNT-1/B, CPAAs, PSMA, CD20, CD105 (Endoglin), ICAM-1, CD30, CD16A, CD38, MUC1, EGFR, KIR2DL1, KIR2DL2, NKG2A, tenascin-C, IGF (insulin-like growth factor), CTLA-4, mesothelin, CD138, c-Met, Ang2, VEGF-A, CD79b, ENPD3, folic acid receptor α, TEM-1, GM2, Glypican 3, macrophage inhibitory factor, CD74, Notch1, Notch2, Notch3, CD37, TLR-2, CD3, CSF-1R, FGFR2b, HLA-DR, GM-CSF, EphA3, B7-H3, CD123, gpA33, Frizzled7 receptor, DLL4, VEGF, RSPO, LIV-1, SLITRK6, Nectin-4, CD70, CD40, CD19, SEMA4D (CD100), CD25, MET, Tissue Factor, IL-8, EGFR, cMet, KIR3DL2, Bst1 (CD157), P-Cadherin, CEA, GITR, TAM (tumor associated macrophage), CEA, DLL4, Ang2, CD73, FGFR2, CXCR4, LAG-3, GITR, Fucosyl GM1, IGF-1, Angiopoietin 2, CSF-1R, FGFR3, OX40, BCMA, ErbB3, CD137 (4-1BB), PTK7, EFNA4, FAP, DR5, CEA, Ly6E, CA6, CEACAM5, LAMP1, tissue factor, EPHA2, DR5, B7-H3, FGFR4, FGFR2, α2-PI, A33, GDF15, CAIX, CD166, ROR1, GITR, BCMA, TBA, LAG-3, EphA2, TIM-3, CD-200, EGFRvIII, CD16A, CD32B, PIGF, Axl, MICA/B, Thomsen-Friedenreich, CD39, CD37, CD73, CLEC12A, Lgr3, transferrin receptors, TGFβ, IL-17, 5T4, RTK, Immune Suppressor Protein, NaPi2b, Lewis blood group B antigen, A34, Lysil-Oxidase, DLK-1, TROP-2, α9 Integrin, TAG-72 (CA72-4), and CD70.
(2) Autoimmune Diseases and Inflammatory Diseases
   IL-17, IL-6R, IL-17R, INF-α, IL-5R, IL-13, IL-23, IL-6, ActRIIB, β7-Integrin, IL-4αR, HAS, Eotaxin-1, CD3, CD19, TNF-α, IL-15, CD3ε, Fibronectin, IL-1β, IL-1α, IL-17, TSLP (Thymic Stromal Lymphopoietin), LAMP (Alpha4 Beta 7 Integrin), IL-23, GM-CSFR, TSLP, CD28, CD40, TLR-3, BAFF-R, MAdCAM, IL-31R, IL-33, CD74, CD32B, CD79B, IgE (immunoglobulin E), IL-17A, IL-17F, C5, FcRn, CD28, TLR4, MCAM, B7RP1, CXCR1,2 Ligands, IL-21, Cadherin-11, CX3CL1, CCL20, IL-36R, IL-10R, CD86, TNF-α, IL-7R, Kv1.3, α9 Integrin, and LIFHT.
(3) Brain or Nerve Diseases
   CGRP, CD20, β amyloid, β amyloid protofibril, Calcitonin Gene-Related Peptide Receptor, LINGO (Ig Domain Containing 1), α Synuclein, extracellular tau, CD52, insulin receptors, tau protein, TDP-43, SOD1, TauC3, and JC virus.
(4) Infectious Diseases
   Clostridium Difficile toxin B, cytomegalovirus, RS viruses, LPS, S.Aureus Alpha-toxin, M2e protein, Psl, PcrV, S. Aureus toxin, influenza A, Alginate, Staphylococcus aureus, PD-L1, influenza B, Acinetobacter, F-protein, Env, CD3, enteropathogenic Escherichia coli, Klebsiella, and Streptococcus pneumoniae.
(5) Hereditary Rare Diseases
   Amyloid AL, SEMA4D (CD100), insulin receptors, ANGPTL3, IL4, IL13, FGF23, adrenocorticotropic hormone, transthyretin, and huntingtin.
(6) Eye Diseases
   Factor D, IGF-1R, PGDFR, Ang2, VEGF-A, CD-105 (Endoglin), IGF-1R, and β amyloid.
(7) Bone and Orthopedic Region
   Sclerostin, Myostatin, Dickkopf-1, GDF8, RNAKL, HAS, Siglec-15
(8) Blood Diseases
   vWF, Factor IXa, Factor X, IFNγ, C5, BMP-6, Ferroportin, TFPI
(9) Other Diseases
   BAFF (B cell activating factor), IL-1β, PCSK9, NGF, CD45, TLR-2, GLP-1, TNFR1, C5, CD40, LPA, prolactin receptors, VEGFR-1, CB1, Endoglin, PTH1R, CXCL1, CXCL8, IL-1β, AT2-R, and IAPP.

Specific examples of the monoclonal antibody include specific chimeric antibodies (e.g., rituximab, basiliximab, infliximab, cetuximab, siltuximab, dinutuximab, and altertoxaximab), specific humanized antibodies (e.g., daclizumab, palivizumab, trastuzumab, alemtuzumab, omalizumab, efalizumab, bevacizumab, natalizumab (IgG4), tocilizumab, eculizumab (IgG2), mogamulizumab, pertuzumab, obinutuzumab, vedolizumab, pembrolizumab (IgG4), mepolizumab, elotuzumab, daratumumab, ixekizumab (IgG4), reslizumab (IgG4), and atezolizumab), and specific human antibodies (e.g., adalimumab (IgG1), panitumumab, golimumab, ustekinumab, canakinumab, ofatumumab, denosumab (IgG2), ipilimumab, belimumab, raxibacumab, ramucirumab, nivolumab, dupilumab (IgG4), secukinumab, evolocumab (IgG2), alirocumab, necitumumab, brodalumab (IgG2), and olaratumab) (cases not referring to the IgG subtype indicate that they are IgG1).

For example, an immunoglobulin unit (e.g., a divalent antibody such as IgG) comprising two heavy chains and two light chains has four disulfide groups because the heavy chains are linked to each other and the heavy chains are linked to the light chains by four disulfide bonds. When a reducing agent sufficiently acts on such an immunoglobulin unit, eight thiol groups are generated from the four disulfide bonds. As the reducing agent, any reducing agent capable of cleaving a disulfide bond to form a thiol group can be used, and examples thereof include tricarboxylethylphosphine (TCEP), cysteine, dithiothreitol, reduced glutathione, and β-mercaptoethanol. Note that even when all the four disulfide bonds are reduced, the heavy chains and the light chains of the antibody are not dissociated. This is because a higher order structure of the antibody can be maintained by a non-covalent bond between the chains. Antibody properties (target and bonding force) can also be maintained by this non-covalent bond. For example, trastuzumab delkstecan known as Enhartz (registered trademark) is an ADC of DAR = 8 in which a drug is bonded to a thiol group generated by reducing (cleaving) all four interchain disulfide bonds. Such an ADC is known to act as an antigen-specific agent by maintaining antibody properties thereof.

In the present invention, an immunoglobulin unit comprising two heavy chains and two light chains can be bonded to a modifying group (e.g., LA or L1 described later) adjacent to Ig via a thiol group in side chains of a plurality of cysteine residues (a plurality of cysteine residues in two heavy chains and two light chains comprised in the immunoglobulin unit) generated by causing a reducing agent to act on the immunoglobulin unit. The number corresponding to the plurality is, for example, 2 or more (e.g., 2 to 8), preferably 3 or more (e.g., 3 to 8), more preferably 4 or more (e.g., 4 to 8), still more preferably 5 or more (e.g., 5 to 8), and particularly preferably 6 or more (e.g., 6 to 8), 7 or more (e.g., 7 to 8), or 8.

In the present invention, as long as such a cysteine residue as described above in an immunoglobulin unit or an antibody is modified, a specific amino acid residue at another position may be further modified regioselectively. For example, a method for regioselectively modifying a specific amino acid residue at a predetermined position in an immunoglobulin unit or an antibody is described in WO 2018/199337 A, WO 2019/240288 A, WO 2019/240287 A, and WO 2020/090979 A. As such a specific amino acid residue, an amino acid residue (e.g., a lysine residue, an aspartic acid residue, a glutamic acid residue, an asparagine residue, a glutamine residue, a threonine residue, a serine residue, or a tyrosine residue) having a side chain that is easily modified (e.g., an amino group, a carboxy group, an amide group, or a hydroxy group) can be used. However, a lysine residue having a side chain comprising an amino group, a tyrosine residue having a side chain comprising a hydroxy group, a serine residue, or a threonine residue may be preferred, and a lysine residue may be more preferred (e.g., lysine residues at positions 246/248, 288/290, and 317).

### (Halogen atom)

Examples of the halogen atom include a fluorine atom, a chlorine atom, a bromine atom, and an iodine atom.

### (Monovalent group)

Examples of the monovalent group include a monovalent hydrocarbon group and a monovalent heterocyclic group.

The monovalent group may have one or more (e.g., 1 to 10, preferably 1 to 8, more preferably 1 to 6, still more preferably 1 to 5, particularly preferably 1 to 3) substituents described later.

### (Monovalent hydrocarbon group and terms related thereto)

Examples of the monovalent hydrocarbon group include a monovalent chain hydrocarbon group, a monovalent alicyclic hydrocarbon group, and a monovalent aromatic hydrocarbon group.

The monovalent chain hydrocarbon group means a hydrocarbon group comprising only a chain structure and does not comprise a cyclic structure in a main chain thereof. Note that the chain structure may be linear or branched. Examples of the monovalent chain hydrocarbon group include an alkyl, an alkenyl, and an alkynyl. The alkyl, alkenyl, and alkynyl may be linear or branched.

The alkyl is preferably a C₁₋₁₂ alkyl, more preferably a C₁₋₆ alkyl, and still more preferably a C₁₋₄ alkyl. When the alkyl has a substituent, the number of carbon atoms does not comprise the number of carbon atoms of the substituent. Examples of the C₁₋₁₂ alkyl include methyl, ethyl, n-propyl, i-propyl, n-butyl, s-butyl, isobutyl, t-butyl, pentyl, hexyl, heptyl, octyl, nonyl, decyl, and dodecyl.

The alkenyl is preferably a C₂₋₁₂ alkenyl, more preferably a C₂₋₆ alkenyl, and still more preferably a C₂₋₄ alkenyl. When the alkenyl has a substituent, the number of carbon atoms does not comprise the number of carbon atoms of the substituent. Examples of the C₂₋₁₂ alkenyl include vinyl, propenyl, and n-butenyl.

The alkynyl is preferably a C₂₋₁₂ alkynyl, more preferably a C₂₋₆ alkynyl, and still more preferably a C₂₋₄ alkynyl. When the alkynyl has a substituent, the number of carbon atoms does not comprise the number of carbon atoms of the substituent. Examples of the C₂₋₁₂ alkynyl include ethynyl, propynyl, and n-butynyl.

The monovalent chain hydrocarbon group is preferably an alkyl.

The monovalent alicyclic hydrocarbon group means a hydrocarbon group comprising only an alicyclic hydrocarbon as a cyclic structure and not comprising any aromatic ring, in which the alicyclic hydrocarbon may be monocyclic or polycyclic. Note that the monovalent alicyclic hydrocarbon group is not necessarily required to comprise only an alicyclic hydrocarbon but may comprise a chain structure in a part thereof. Examples of the monovalent alicyclic hydrocarbon group include cycloalkyl, cycloalkenyl, and cycloalkynyl, which may be monocyclic or polycyclic.

The cycloalkyl is preferably a C₃₋₁₂ cycloalkyl, more preferably a C₃₋₆ cycloalkyl, and still more preferably a C₅₋₆ cycloalkyl. When the cycloalkyl has a substituent, the number of carbon atoms does not comprise the number of carbon atoms of the substituent. Examples of the C₃₋₁₂ cycloalkyl include cyclopropyl, cyclobutyl, cyclopentyl, and cyclohexyl.

The cycloalkenyl is preferably a C₃₋₁₂ cycloalkenyl, more preferably a C₃₋₆ cycloalkenyl, and still more preferably a C₅₋₆ cycloalkenyl. When the cycloalkenyl has a substituent, the number of carbon atoms does not comprise the number of carbon atoms of the substituent. Examples of the C₃₋₁₂ cycloalkenyl include cyclopropenyl, cyclobutenyl, cyclopentenyl, and cyclohexenyl.

The cycloalkynyl is preferably a C₃₋₁₂ cycloalkynyl, more preferably a C₃₋₆ cycloalkynyl, and still more preferably a C₅₋₆ cycloalkynyl. When the cycloalkynyl has a substituent, the number of carbon atoms does not comprise the number of carbon atoms of the substituent. Examples of the C₃₋₁₂ cycloalkynyl include cyclopropynyl, cyclobutynyl, cyclopentynyl, and cyclohexynyl.

The monovalent alicyclic hydrocarbon group is preferably a cycloalkyl.

The monovalent aromatic hydrocarbon group means a hydrocarbon group comprising an aromatic cyclic structure. Note that the monovalent aromatic hydrocarbon group is not necessarily required to comprise only an aromatic ring and may comprise a chain structure or alicyclic hydrocarbon in a part thereof, in which the aromatic ring may be monocyclic or polycyclic. The monovalent aromatic hydrocarbon group is preferably C₆₋₁₂ aryl, more preferably C₆₋₁₀ aryl, and still more preferably C₆ aryl. When the monovalent aromatic hydrocarbon group has a substituent, the number of carbon atoms does not comprise the number of carbon atoms of the substituent. Examples of the C₆₋₁₂ aryl include phenyl and naphthyl.

The monovalent aromatic hydrocarbon group is preferably phenyl.

Among these groups, the monovalent hydrocarbon group is preferably alkyl, cycloalkyl, or aryl.

### (Monovalent heterocyclic group and terms related thereto)

The monovalent heterocyclic group refers to a group obtained by removing one hydrogen atom from a heterocycle of a heterocyclic compound. The monovalent heterocyclic group is a monovalent aromatic heterocyclic group or a monovalent nonaromatic heterocyclic group. The monovalent heterocyclic group preferably comprises one or more selected from the group consisting of an oxygen atom, a sulfur atom, a nitrogen atom, a phosphorus atom, a boron atom, and a silicon atom and more preferably comprises one or more selected from the group consisting of an oxygen atom, a sulfur atom, and a nitrogen atom as a hetero atom constituting the heterocyclic group.

The monovalent aromatic heterocyclic group is preferably a C₁₋₁₅ aromatic heterocyclic group, more preferably a C₁₋₉ aromatic heterocyclic group, and still more preferably a C₁₋₆ aromatic heterocyclic group. When the monovalent aromatic heterocyclic group has a substituent, the number of carbon atoms does not comprise the number of carbon atoms of the substituent. Examples of the monovalent aromatic heterocyclic group include pyrrolyl, furanyl, thiophenyl, pyridinyl, pyridazinyl, pyrimidinyl, pyrazinyl, triazinyl, pyrazolyl, imidazolyl, thiazolyl, isothiazolyl, oxazolyl, isoxazolyl, triazolyl, tetrazolyl, indolyl, purinyl, anthraquinolyl, carbazonyl, fluorenyl, quinolinyl, isoquinolinyl, quinazolinyl, and phthalazinyl.

The monovalent nonaromatic heterocyclic group is preferably a C₂₋₁₅ nonaromatic heterocyclic group, more preferably a C₂₋₉ nonaromatic heterocyclic group, and still more preferably a C₂₋₆ nonaromatic heterocyclic group. When the monovalent nonaromatic heterocyclic group has a substituent, the number of carbon atoms does not comprise the number of carbon atoms of the substituent. Examples of the monovalent nonaromatic heterocyclic group include oxiranyl, aziridinyl, azetidinyl, oxetanyl, thietanyl, pyrrolidinyl, dihydrofuranyl, tetrahydrofuranyl, dioxolanyl, tetrahydrothiophenyl, pyrolinyl, imidazolidinyl, oxazolidinyl, piperidinyl, dihydropyranyl, tetrahydropyranyl, tetrahydrothiopyranyl, morpholinyl, thiomorpholinyl, piperazinyl, dihydrooxazinyl, tetrahydrooxazinyl, dihydropyrimidinyl, and tetrahydropyrimidinyl.

Among these groups, the monovalent heterocyclic group is preferably a five-membered or six-membered heterocyclic group.

### (Divalent group)

The divalent group is a divalent linear hydrocarbon group, a divalent cyclic hydrocarbon group, a divalent heterocyclic group, one group selected from the group consisting of -C(=O)-, -C(=S)-, -NR₇-, -C(=O)-NR₇-, -NR₇-C(=O)-, -C(=S)-NR₇-, -NR₇-C(=S)-, -O-, -S-, -(O-R₈)ₘ-, and - (S-R₈)ₘ₁-, or a group having a main chain structure comprising two or more (e.g., 2 to 10, preferably 2 to 8, more preferably 2 to 6, still more preferably 2 to 5, particularly preferably 2 or 3) of these groups. R₇ represents a hydrogen atom or a substituent described later. R₈ represents a divalent linear hydrocarbon group, a divalent cyclic hydrocarbon group, or a divalent heterocyclic group. m1 is an integer of 1 to 10, preferably an integer of 1 to 8, more preferably an integer of 1 to 6, still more preferably an integer of 1 to 5, and particularly preferably an integer of 1 to 3.

The divalent linear hydrocarbon group is a linear alkylene, a linear alkenylene, or a linear alkynylene.

The linear alkylene is a C₁₋₆ linear alkylene, and is preferably a C₁₋₄ linear alkylene. Examples of the linear alkylene include methylene, ethylene, n-propylene, n-butylene, n-pentylene, and n-hexylene.

The linear alkenylene is a C₂₋₆ linear alkenylene, and is preferably a C₂₋₄ linear alkenylene. Examples of the linear alkenylene include ethylenylene, n-propynylene, n-butenylene, n-pentenylene, and n-hexenylene.

The linear alkynylene is a C₂₋₆ linear alkynylene, and is preferably a C₂₋₄ linear alkynylene. Examples of the linear alkynylene include ethynylene, n-propynylene, n-butynylene, n-pentynylene, and n-hexynylene.

The divalent linear hydrocarbon group is preferably a linear alkylene.

The divalent cyclic hydrocarbon group is an arylene or a divalent nonaromatic cyclic hydrocarbon group.

The arylene is preferably a C₆₋₁₄ arylene, more preferably a C₆₋₁₀ arylene, and particularly preferably a C₆ arylene. Examples of the arylene include phenylene, naphthylene, and anthracenylene.

The divalent nonaromatic cyclic hydrocarbon group is preferably a C₃₋₁₂ monocyclic or polycyclic divalent nonaromatic cyclic hydrocarbon group, more preferably a C₄₋₁₀ monocyclic or polycyclic divalent nonaromatic cyclic hydrocarbon group, and particularly preferably a C₅₋₈ monocyclic divalent nonaromatic cyclic hydrocarbon group. Examples of the divalent nonaromatic cyclic hydrocarbon group include cyclopropylene, cyclobutylene, cyclopentylene, cyclohexylene, cycloheptylene, and cyclooctylene.

The divalent cyclic hydrocarbon group is preferably an arylene.

The divalent heterocyclic group is a divalent aromatic heterocyclic group or a divalent nonaromatic heterocyclic group. The divalent heterocyclic group preferably comprises, as a hetero atom forming a heterocycle, one or more selected from the group consisting of an oxygen atom, a sulfur atom, a nitrogen atom, a phosphorous atom, a boron atom, and a silicon atom and more preferably comprises one or more selected from the group consisting of an oxygen atom, a sulfur atom, and a nitrogen atom.

The divalent aromatic heterocyclic group is preferably a C₃₋₁₅ divalent aromatic heterocyclic group, more preferably a C₃₋₉ divalent aromatic heterocyclic group, and particularly preferably a C₃₋₆ divalent aromatic heterocyclic group. Examples of the divalent aromatic heterocyclic group include pyrrolediyl, furandiyl, thiophenediyl, pyridinediyl, pyridazinediyl, pyrimidinediyl, pyrazinediyl, triazinediyl, pyrazolediyl, imidazolediyl, thiazolediyl, isothiazolediyl, oxazolediyl, isoxazolediyl, triazolediyl, tetrazolediyl, indolediyl, purinediyl, anthraquinonediyl, carbazolediyl, fluorenediyl, quinolinediyl, isoquinolinediyl, quinazolinediyl, and phthalazinediyl.

The divalent nonaromatic heterocyclic group is preferably a C₃₋₁₅ nonaromatic heterocyclic group, more preferably a C₃₋₉ nonaromatic heterocyclic group, and particularly preferably a C₃₋₆ nonaromatic heterocyclic group. Examples of the divalent nonaromatic heterocyclic group include pyrroldionediyl, pyrrolinedionediyl, oxiranediyl, aziridinediyl, azetidinediyl, oxetanediyl, thietanediyl, pyrrolidinediyl, dihydrofurandiyl, tetrahydrofurandiyl, dioxolanediyl, tetrahydrothiophenediyl, pyrrolinediyl, imidazolidinediyl, oxazolidinediyl, piperidinediyl, dihydropyrandiyl, tetrahydropyrandiyl, tetrahydrothiopyrandiyl, morpholinediyl, thiomorpholinediyl, piperazinediyl, dihydrooxazinediyl, tetrahydrooxazinediyl, dihydropyrimidinediyl, and tetrahydropyrimidinediyl.

The divalent heterocyclic group is preferably a divalent aromatic heterocyclic group.

The divalent group is preferably a divalent group having a main chain structure comprising one group selected from the group consisting of alkylene, arylene, -C(=O)-, - NR₇-, -C(=O)-NR₇-, -NR₇-C(=O)-, -O-, and -(O-R₈)ₘ-, or
a divalent group having a main chain structure comprising two or more groups selected from the group consisting of alkylene, arylene, -C(=O)-, -NR₇-, -C(=O)-NR₇-, -NR₇-C(=O)-, -O-, and -(O-R₈)ₘ₁-,
R₇ is a hydrogen atom or an alkyl,
R₈ is an alkylene or an arylene, and
m1 may be an integer of 1 to 5 (that is, 1, 2, 3, 4, or 5).

The alkylene, the arylene, and the alkyl are similar to those described above.

The main chain structure in the divalent group may have one or more (e.g., 1 to 10, preferably 1 to 8, more preferably 1 to 6, still more preferably 1 to 5, particularly preferably 1 to 3) substituents described later.

### (Substituent)

Examples of the substituent include:
(i) a halogen atom;
(ii) a monovalent hydrocarbon group;
(iii) a monovalent heterocyclic group;
(iv) an aralkyl;
(v) Rₐ-O-, Rₐ-C(=O)-, Rₐ-O-C(=O)-, or Rₐ-C(=O)-O-, (where Rₐ represents a hydrogen atom or a monovalent hydrocarbon group);
(vi) NR_{b}R_{c}-, NR_{b}R_{c}-C(=O) -, NR_{b}R_{c}-C(=O) -O-, or R_{b}-C(=O)-NR_{c}-, (where R_{b} and R_{c} are the same as or different from each other, and each represent a hydrogen atom or a monovalent hydrocarbon group); and
(vii) a nitro group, a sulfate group, a sulfonate group, a cyano group, and a carboxyl group.

Definitions, examples, and preferred examples of the halogen atom, the monovalent hydrocarbon group, and the monovalent heterocyclic group in the substituent are similar to those described above.

The aralkyl refers to arylalkyl. Definitions, examples, and preferred examples of the aryl and the alkyl in the arylalkyl are as described above. The aralkyl is preferably C₃₋₁₅ aralkyl. Examples of such an aralkyl include benzoyl, phenethyl, naphthylmethyl, and naphthylethyl.

The substituent may be preferably:
(i) a halogen atom;
(ii) a C₁₋₁₂ alkyl, a C₁₋₁₂ phenyl, or a C₁₋₁₂ naphthyl;
(iii) a C₃₋₁₅ aralkyl;
(iv) a 5- or 6-membered heterocycle;
(v) Rₐ-O-, Rₐ-C(=O)-, Rₐ-O-C(=O)-, or Rₐ-C(=O)-O-, (where Rₐ represents a hydrogen atom or a C₁₋₁₂ alkyl);
(vi) NR_{b}R_{c}-, NR_{b}R_{c}-C(=O) -, NR_{b}R_{c}-C(=O) -O-, or R_{b}-C(=O)-NR_{c}-, (where R_{b} and R_{c} are the same as or different from each other, and each represent a hydrogen atom or a C₁₋₁₂ alkyl); or
(vii) the same groups as listed in the above (vii).

The substituent may be more preferably:
(i) a halogen atom;
(ii) a C₁₋₁₂ alkyl;
(iii) Rₐ-O-, Rₐ-C(=O)-, Rₐ-O-C(=O)-, or Rₐ-C(=O)-O-, (where Rₐ represents a hydrogen atom or a C₁₋₁₂ alkyl);
(iv) NR_{b}R_{c}-, NR_{b}R_{c}-C(=O) -, NR_{b}R_{c}-C(=O) -O-, or R_{b}-C(=O)-NR_{c}-, (where R_{b} and R_{c} are the same as or different from each other, and each represent a hydrogen atom or a C₁₋₁₂ alkyl); or
(v) the same groups as listed in the above (vii).

The substituent may be still more preferably:
(i) a halogen atom;
(ii) a C₁₋₆ alkyl;
(iii) Rₐ-O-, Rₐ-C(=O)-, Rₐ-O-C(=O)-, or Rₐ-C(=O)-O-, (where Rₐ represents a hydrogen atom or a C₁₋₆ alkyl);
(iv) NR_{b}R_{c}-, NR_{b}R_{c}-C(=O) -, NR_{b}R_{c}-C(=O) -O-, or R_{b}-C(=O)-NR_{c}-, (where R_{b} and R_{c} are the same as or different from each other, and each represent a hydrogen atom or a C₁₋₆ alkyl); or
(v) the same groups as listed in the above (vii).

The substituent may be particularly preferably:
(i) a halogen atom;
(ii) a C₁₋₄ alkyl;
(iii) Rₐ-O-, Rₐ-C(=O)-, Rₐ-O-C(=O)-, or Rₐ-C(=O)-O-, (where Rₐ represents a hydrogen atom or a C₁₋₄ alkyl);
(iv) NR_{b}R_{c}-, NR_{b}R_{c}-C(=O) -, NR_{b}R_{c}-C(=O) -O-, or R_{b}-C(=O)-NR_{c}-, (where R_{b} and R_{c} are the same as or different from each other, and each represent a hydrogen atom or a C₁₋₄ alkyl); or
(v) the same groups as listed in the above (vii).

### (Hydrophilic group)

The hydrophilic group is a group that can make structural units represented by Formulas (1) to (7) or a formula of a subordinate concept thereof more hydrophilic. By having a hydrophilic group at a predetermined site in the structural unit, the properties of the conjugate can be further improved. Examples of such a hydrophilic group include a carboxylic acid group, a sulfonate group, a hydroxy group, a polyethylene glycol group, a polysarcosine group, and a sugar portion. The conjugate may comprise one or more (e.g., 1, 2, 3, 4, or 5) hydrophilic groups.

The polyethylene glycol (PEG) group is a divalent group represented by -(CH₂-CH₂-O-)ₖ₁-. When the conjugate has a polyethylene glycol group, the conjugate may have a monovalent group in which one bond of the polyethylene glycol group is bonded to a hydrogen atom or a monovalent group (e.g., a monovalent hydrocarbon group). k1 may be, for example, an integer of 3 or more, preferably an integer of 4 or more, more preferably an integer of 5 or more, and still more preferably an integer of 6 or more. k1 may also be an integer of 15 or less, preferably an integer of 12 or less, more preferably an integer of 10 or less, and still more preferably an integer of 9 or less. More specifically, k1 may be an integer of 3 to 15, preferably an integer of 4 to 12, more preferably an integer of 5 to 10, and still more preferably an integer of 4 to 9.

The polysarcosine group is a divalent group represented by -(NCH₃-CH₂-CO-)ₖ₂-. The polysarcosine group can be used as an alternative to PEG. k2 may be, for example, an integer of 3 or more, preferably an integer of 4 or more, more preferably an integer of 5 or more, and still more preferably an integer of 6 or more. k2 may also be an integer of 15 or less, preferably an integer of 12 or less, more preferably an integer of 10 or less, and still more preferably an integer of 9 or less. More specifically, k2 may be an integer of 3 to 15, preferably an integer of 4 to 12, more preferably an integer of 5 to 10, and still more preferably an integer of 4 to 9.

The sugar portion is a monosaccharide, an oligosaccharide (e.g., a disaccharide, a trisaccharide, a tetrasaccharide, or a pentasaccharide), or a polysaccharide. The sugar portion can comprise an aldose or a ketose, or a combination thereof. The sugar portion may be a monosaccharide such as ribose, deoxyribose, xylose, arabinose, glucose, mannose, galactose, fructose, or an amino sugar (e.g., glucosamine), or an oligosaccharide or a polysaccharide comprising such a monosaccharide.

In a specific embodiment, the sugar portion may be a low molecular weight hydrophilic group. The low molecular weight hydrophilic group refers to a hydrophilic group having a molecular weight of 1500 or less. The molecular weight of the low molecular weight hydrophilic group may be 1,200 or lower, 1,000 or lower, 800 or lower, 700 or lower, 600 or lower, 500 or lower, 400 or lower, 300 or lower, 200 or lower, or 100 or lower. Examples of the low molecular weight hydrophilic group include a carboxylic acid group, a sulfonate group, a hydroxy group, and a polyethylene glycol group, a polysarcosine group, and a sugar portion (e.g., a monosaccharide or an oligosaccharide) satisfying the above molecular weight.

### (Bioorthogonal functional group)

The bioorthogonal functional group refers to a group that does not react with biological components (e.g., amino acids, proteins, nucleic acids, lipids, sugars, and phosphoric acids) or has a low reaction rate to the biological components but selectively reacts with components other than the biological components. The bioorthogonal functional group is well known in the technical field concerned (see, for example, Sharpless K. B. et al., Angew. Chem. Int. Ed. 40, 2004 (2015); Bertozzi C. R. et al., Science 291, 2357 (2001); and Bertozzi C. R. et al., Nature Chemical Biology 1, 13 (2005)).

In the present invention, as the bioorthogonal functional group, a bioorthogonal functional group to a protein is used. This is because the antibody to be derivatized with a reagent of the present invention is a protein. The bioorthogonal functional group to a protein is a group that does not react with side chains of 20 types of natural amino acid residues forming proteins, or reacts with a target functional group although having a low reaction rate to the side chain. The 20 types of natural amino acids constituting the protein are alanine (A), asparagine (N), cysteine (C), glutamine (Q), glycine (G), isoleucine (I), leucine (L), methionine (M), phenylalanine (F), proline (P), serine (S), threonine (T), tryptophan (W), tyrosine (Y), valine (V), aspartic acid (D), glutamic acid (E), arginine (R), histidine (H), and lysine (K). Among these 20 types of natural amino acids, glycine, which has no side chain (that is, which has a hydrogen atom as a side chain), and alanine, isoleucine, leucine, phenylalanine, and valine, which each have a hydrocarbon group as a side chain (that is, which each comprise no hetero atom selected from the group consisting of a sulfur atom, a nitrogen atom, and an oxygen atom in a side chain thereof) are inactive to a normal reaction. Therefore, the bioorthogonal functional group to a protein is a group that does not react with, in addition to the side chains of these amino acids having side chains inactive to normal reactions, side chains of asparagine, glutamine, methionine, proline, serine, threonine, tryptophan, tyrosine, aspartic acid, glutamic acid, arginine, histidine, and lysine, or reacts with a target functional group although having a low reaction rate.

Examples of such a bioorthogonal functional group include an azide residue, an aldehyde residue, a thiol residue, an alkene residue (in other words, it is only required to have a vinylene (ethenylene) portion, which is a minimum unit having a carbon-carbon double bond. Hereinafter the same), an alkyne residue (in other words, it is only required to have an ethynylene portion, which is a minimum unit having a carbon-carbon triple bond. Hereinafter the same), a halogen residue, a tetrazine residue, a nitrone residue, a hydroxylamine residue, a nitrile residue, a hydrazine residue, a ketone residue, a boronic acid residue, a cyanobenzothiazole residue, an allyl residue, a phosphine residue, a maleimide residue, a disulfide residue, a thioester residue, an α-halocarbonyl residue (e.g., a carbonyl residue having a fluorine atom, a chlorine atom, a bromine atom, or an iodine atom at an α-position. Hereinafter the same), an isonitrile residue, a sydnone residue, and a selenium residue.

More specifically, the bioorthogonal functional group may correspond to any one chemical structure selected from the group consisting of the following: 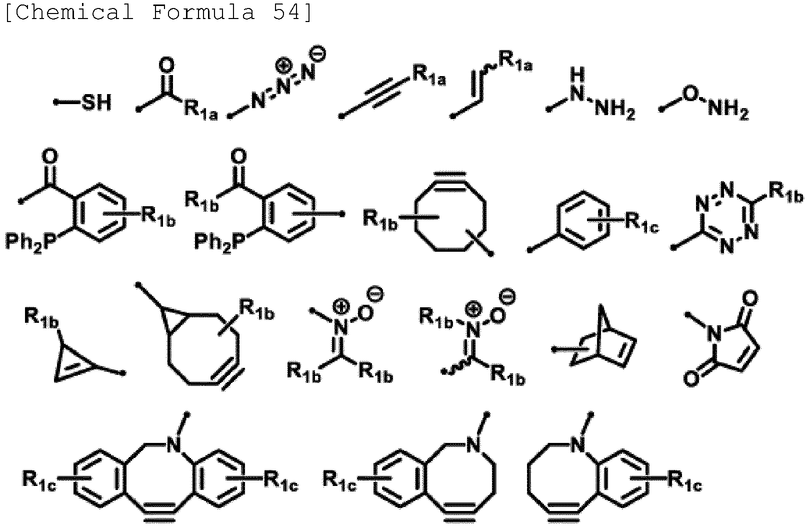 wherein
R₁ₐ, one or a plurality of R_{1b}, and one or a plurality of R_{1c} are the same as or different from each other, and each represent any one of the substituents described above or an electron-withdrawing group, and
- is a bond.

Examples of the electron-withdrawing group include a halogen atom, an alkyl substituted with a halogen atom (e.g., trifluoromethyl), a boronic acid residue, mesyl, tosyl, triflate, nitro, cyano, a phenyl group, and a keto group (e.g., acyl), and a halogen atom, a boronic acid residue, mesyl, tosyl, and triflate are preferred.

In a specific embodiment, the bioorthogonal functional group may be protected. The optionally protected bioorthogonal functional group refers to an unprotected bioorthogonal functional group or a protected bioorthogonal functional group. The unprotected bioorthogonal functional group corresponds to the bioorthogonal functional group described above. The protected bioorthogonal functional group is a group that generates a bioorthogonal functional group by cleavage of a protective group. The protective group can be cleaved by a specific treatment under a condition (a mild condition) incapable of causing denaturation or decomposition of proteins (e.g., cleavage of an amide bond). Examples of such a specific treatment include (a) a treatment with one or more substances selected from the group consisting of an acidic substance, a basic substance, a reducing agent, an oxidizing agent, and an enzyme, (b) a treatment with a physical and chemical stimulus selected from the group consisting of light, and (c) leaving a cleavable linker as it is when the cleavable linker comprises a self-degradable cleavable portion. Such a protective group and a cleavage condition therefor are common technical knowledge in the field concerned (e.g., G. Leriche, L. Chisholm, A. Wagner, Bioorganic & Medicinal Chemistry. 20,571 (2012); Feng P. et al., Jounal of American Chemical Society. 132,1500 (2010).; Bessodes M. et al., Journal of Controlled Release, 99, 423 (2004).; DeSimone, J. M., Journal of American Chemical Society. 132,17928 (2010); Thompson, D. H., Journal of Controlled Release, 91, 187 (2003); and Schoenmarks, R. G., Journal of Controlled Release, 95, 291 (2004)).

Examples of the protected bioorthogonal functional group include a disulfide residue, an ester residue, an acetal residue, a ketal residue, an imine residue, and a vicinaldiol residue.

More specifically, the protected bioorthogonal functional group may correspond to any one chemical structure selected from the group consisting of the following: 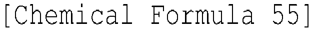 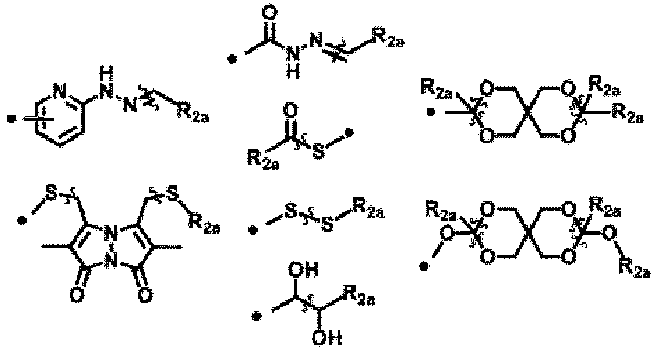
wherein the wavy line orthogonal to the bond indicates a cleavage site,
one or a plurality of R₂ₐ are the same as or different from each other, and each represent a hydrogen atom or a group selected from the group consisting of the substituents described above, and
   - is a bond.

The optionally protected bioorthogonal functional group is preferably an unprotected bioorthogonal functional group.

### (Functional substance)

The functional substance is not limited to a particular substance as long as it is a substance imparting any function to the antibody, and examples thereof include drugs, labelling substances, affinity substances, transporting substances, and stabilizers. Preferably, the functional substance may be a drug, a labelling substance, an affinity substance, or a transporting substance, or may be a drug or a labelling substance. The functional substance may be a single functional substance or a substance in which two or more functional substances are linked to each other.

The drug may be a drug to any disease. Examples of such a disease include cancer (for example, a lung cancer, a stomach cancer, a colon cancer, a pancreatic cancer, a renal cancer, a liver cancer, a thyroid cancer, a prostatic cancer, a bladder cancer, an ovarian cancer, a uterine cancer, a bone cancer, a skin cancer, a brain tumor, or melanoma), an autoimmune disease and an inflammatory disease (for example, an allergic disease, articular rheumatism, or systemic lupus erythematosus), a brain or nerve disease (for example, cerebral infarction, Alzheimer's disease, Parkinson disease, or amyotrophic lateral sclerosis), an infectious disease (for example, a microbial infectious disease or a viral infectious disease), a hereditary rare disease (for example, hereditary spherocytosis or nondystrophic myotonia), an eye disease (for example, age-related macular degeneration, diabetic retinopathy, or retinitis pigmentosa), a diseases in the bone and orthopedic field (for example, osteoarthritis), a blood disease (for example, leukosis or purpura), and other diseases (for example, diabetes, a metabolic disease such as hyperlipidemia, a liver disease, a renal disease, a lung disease, a circulatory system disease, or a digestive system disease). The drug may be a prophylactic or therapeutic agent for a disease, or a relief agent for a side effect.

More specifically, the drug may be an anti-cancer agent. Examples of the anti-cancer agent include chemotherapeutic agents, toxins, and radioisotopes or substances comprising them. Examples of the chemotherapeutic agent include a DNA injuring agent, an antimetabolite, an enzyme inhibitor, a DNA intercalating agent, a DNA cleaving agent, a topoisomerase inhibitor, a DNA bonding inhibitor, a tubulin bonding inhibitor, a cytotoxic nucleoside, and a platinum compound. Examples of the toxin include a bacteriotoxin (for example, a diphtheria toxin) and a phytotoxin (for example, ricin). Examples of the radioisotope include a radioisotope of a hydrogen atom (for example, ³H), a radioisotope of a carbon atom (for example, ¹⁴C), a radioisotope of a phosphorous atom (for example, ³²P), a radioisotope of a sulfur atom (for example, 35^{S}), a radioisotope of yttrium (for example, ⁹⁰Y), a radioisotope of technetium (for example, ^{99m}Tc), a radioisotope of indium (for example, ¹¹¹In), a radioisotope of an iodide atom (for example, ¹²³I, ¹²⁵I, ¹²⁹I, and ¹³¹I), a radioisotope of samarium (for example, ¹⁵³Sm), a radioisotope of rhenium (for example, ¹⁸⁶Re), a radioisotope of astatine (for example, ²¹¹At), and a radioisotope of bismuth (for example, ²¹²Bi). More specific examples of the drug include auristatin (MMAE or MMAF), maytansine (DM1 or DM4), PBD (pyrrolobenzodiazepine), IGN, a camptothecin analog, calicheamicin, duocarmycin, eribulin, anthracycline, dmDNA31, and tubricin.

The labelling substance is a substance that makes detection of a target (for example, a tissue, a cell, or a substance) possible. Examples of the labelling substance include an enzyme (for example, peroxidase, alkaline phosphatase, luciferase, or β-galactosidase), an affinity substance (for example, streptavidin, biotin, digoxigenin, or aptamer), a fluorescent substance (for example, fluorescein, fluorescein isothiocyanate, rhodamine, green-fluorescent protein, or red-fluorescent protein), a luminescent substance (for example, luciferin, aequorin, acridinium ester, tris(2,2'-bipyridyl) ruthenium, or luminol), a radioisotope (for example, those described above), and substances comprising these.

The affinity substance is a substance having affinity for a target. Examples of the affinity substance include an affinity protein or a peptide such as an antibody, an aptamer, a lectin, and a complementary strand to a target nucleic acid. The affinity substance may be preferably an affinity protein or an affinity peptide, and more preferably an antibody. The type of animal from which an antibody used as the functional substance is derived is similar to that described above.

The type of the antibody used as the functional substance may be a polyclonal antibody or a monoclonal antibody. The antibody may be a divalent antibody (e.g., IgG, IgD, or IgE) or a tetravalent or higher antibody (e.g., IgA antibody or IgM antibody). The antibody is preferably a monoclonal antibody. Examples of the monoclonal antibody include chimeric antibodies, humanized antibodies, human antibodies, antibodies with a certain sugar chain added (e.g., an antibody modified so as to have a sugar chain-bonding consensus sequence such as an N-type sugar chain-bonding consensus sequence), bi-specific antibodies, Fc region proteins, and Fc-fusion proteins. Examples of the isotype of the monoclonal antibody include IgG (e.g., IgG1, IgG2, IgG3, and IgG4), IgM, IgA, IgD, IgE, and IgY. Examples of the antibody used as the functional substance include a full-length antibody and a fragment thereof (fragment antibody). The fragment antibody only needs to maintain a bonding property to a desired antigen, and examples thereof include Fab, Fab', F(ab')₂, and scFv.

Antigenicity of the antibody used as the functional substance may be the same as or different from antigenicity of the immunoglobulin unit in the antibody, the antibody derivative, and the conjugate of the present invention, and is preferably different. In addition, an origin of the antibody used as the functional substance may be the same as or different from an origin of the immunoglobulin unit, and is preferably different. Therefore, the antibody used as the functional substance may be a specific chimeric antibody, a specific humanized antibody, or a specific human antibody mentioned in the specific examples of the monoclonal antibody described above, or an antibody derived therefrom. The antibody used as the functional substance may also be IgG1, IgG2, IgG3, or IgG4 mentioned in the specific examples of the monoclonal antibody described above, or an antibody derived therefrom.

The transporting substance is a substance having ability to transport a compound. The transporting substance is preferably a substance (e.g., a ferritin such as human ferritin, viral particles, and virus-like particles) capable of encapsulating a compound in a protein outer coat (e.g., multimers).

The stabilizer is a substance that makes stabilization of an antibody possible. Examples of the stabilizer include a diol, glycerin, a nonionic surfactant, an anionic surfactant, a natural surfactant, a saccharide, and a polyol.

The functional substance may also be a peptide, a protein, a nucleic acid, a low molecular weight organic compound, a sugar chain, a lipid, a high molecular polymer, a metal (e.g., gold), or a chelator. Examples of the peptide include a cell membrane permeable peptide, a blood-brain barrier permeable peptide, and a peptide medicament. Examples of the protein include enzymes, cytokines, fragment antibodies, lectins, interferons, serum albumin, and antibodies. Examples of the nucleic acid include DNA, RNA, and artificial nucleic acid. Examples of the nucleic acid also include RNA interference inducible nucleic acids (e.g., siRNA), aptamers, and antisense. Examples of the low molecular weight organic compound include proteolysis targeting chimeras, dyes, and photodegradable compounds.

In a specific embodiment, the functional substance may be a substance having an aromatic ring. Examples of the substance having an aromatic ring include monomethylauristatin [e.g., monomethyl auristatin E (MMAE) and monomethyl auristatin F (MMAF)] and Exatecan.

### (Salt)

In the present invention, examples of the term "salt" include salts with inorganic acids, salts with organic acids, salts with inorganic bases, salts with organic bases, and salts with amino acids. Examples of salts with inorganic acids include salts with hydrogen chloride, hydrogen bromide, phosphoric acid, sulfuric acid, and nitric acid. Examples of salts with organic acids include salts with formic acid, acetic acid, trifluoroacetic acid, lactic acid, tartaric acid, fumaric acid, oxalic acid, maleic acid, citric acid, succinic acid, malic acid, benzenesulfonic acid, and p-toluenesulfonic acid. Examples of salts with inorganic bases include salts with alkali metals (e.g., sodium and potassium), alkaline-earth metals (e.g., calcium and magnesium), other metals such as zinc and aluminum, and ammonium. Examples of salts with organic bases include salts with trimethylamine, triethylamine, propylenediamine, ethylenediamine, pyridine, ethanolamine, monoalkyl ethanolamine, dialkyl ethanolamine, diethanolamine, and triethanolamine. Examples of salts with amino acids include salts with basic amino acids (e.g., arginine, histidine, lysine, and ornithine) and acidic amino acids (e.g., aspartic acid and glutamic acid). The salt is preferably a salt with an inorganic acid (e.g., hydrogen chloride) or a salt with an organic acid (e.g., trifluoroacetic acid).

### 2. Conjugate or salt thereof

The present invention provides
a conjugate of an antibody and a functional substance or functional substances, comprising a structural unit represented by a conjugate of an antibody and a functional substance, or a salt thereof, comprising a structural unit represented by the following Formula (1): 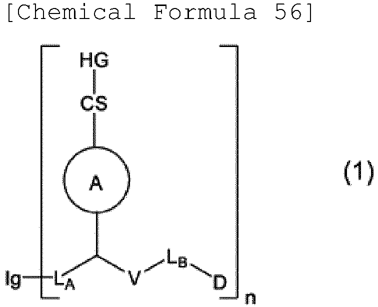 wherein
Ig represents an immunoglobulin unit comprising two heavy chains and two light chains, and is bonded to L_{A} adjacent to Ig via a thiol group in side chains of a plurality of cysteine residues in the two heavy chains and the two light chains,
HG represents a hydrophilic group or a monovalent group comprising a hydrophilic group,
CS represents a divalent group comprising a cleavable site,
ring A represents a divalent aromatic ring group optionally having a substituent wherein the divalent aromatic ring group constitutes a π electron conjugated system with the cleavable site,
V represents an oxygen atom, a sulfur atom, or an amino group (NH),
L_{A} and L_{B} each independently represent a divalent group,
D represents a functional substance, and
an average number n of the bonds per the immunoglobulin unit is 1.5 or more, or
a salt thereof.

In Formula (1) and other Formulas presented in relation to the present invention, -(hyphen) indicates that two units (e.g., atoms or groups) present on both sides thereof are covalently bonded to each other. When there is no unit on one side of - (hyphen), the - (hyphen) indicates a bond.

The antibody comprises such an immunoglobulin unit as described above. Examples of such an antibody include: an IgG antibody comprising two heavy chains and two light chains and comprising an immunoglobulin unit having a disulfide bond between the heavy chains and between the heavy chains and the light chains; an IgD antibody and an IgE antibody; an IgA antibody comprising four heavy chains and four light chains and comprising an immunoglobulin unit having a disulfide bond between the heavy chains and between the heavy chains and the light chains; and an IgM antibody comprising eight heavy chains and eight light chains and comprising an immunoglobulin unit having a disulfide bond between the heavy chains and between the heavy chains and the light chains, and an IgG antibody (e.g., IgG1, IgG2, IgG3, or IgG4) is preferred. The antibody is preferably a human IgG monoclonal antibody, and more preferably a human IgG full-length monoclonal antibody.

A bond between the antibody to the linker site (L_{A}) can be achieved by a bond between a thiol group in a side chain of a cysteine residue in the antibody and an atom or group (e.g., a maleimide residue, a carbonyl group, or a thiol group) capable of being bonded thereto.

In Formula (1), HG represents a hydrophilic group or a monovalent group comprising a hydrophilic group. The hydrophilic group and the monovalent group are as described above. HG may preferably represent a monovalent group comprising a hydrophilic group.

The cleavable site is a site that can be cleaved in an appropriate environment (e.g., intracellular or extracellular). Examples of the cleavable site include a cleavable site by an enzyme (e.g., US 6,214,345 B2; Dubowchik et al., Pharm.Therapeutics 83: 67-123 (1999); The FEBS Journal 287: 1936-1969 (2020)), a cleavable site by an acid (a locally acidic cleavable site present in a living body) (e.g., US 5,622,929 B2, US 5,122,368 B2; US 5,824,805 B2; The FEBS Journal 287: 1936-1969 (2020)), and a cleavable site by glutathione in blood (e.g., The FEBS Journal 287: 1936-1969 (2020)). The cleavable site may be self-immolative (e.g., WO 02/083180 A, WO 04/043493 A, and WO 05/112919 A).

The cleavable site is preferably a cleavable site by an enzyme. Examples of the cleavable site by an enzyme include a cleavable site by an intracellular protease (e.g., a protease present in a lysosome or an endosome) and a cleavable site by an extracellular protease (e.g., a secretory protease) (typically, a cleavable peptide site. More preferably, the cleavable site is a cleavable site by an intracellular protease (e.g., a protease present in a lysosome or an endosome). Still more preferably, the cleavable site is a cleavable site by a protease present in a lysosome. Particularly preferably, the cleavable site is a cleavable site by cathepsin B.

In a specific embodiment, the divalent group comprising a cleavable site may satisfy the following conditions (i) to (iii):
(i) the cleavable site is -CO-W- wherein the hyphen (-) represents a bond, and W represents an oxygen atom, a sulfur atom, or an amino group (NH), and is bonded to the divalent aromatic ring group;
(ii) a bond between CO and W is a site to be cleaved; and
(iii) the divalent aromatic ring group constitutes a π electron conjugated system with W.

When the divalent group comprising a cleavable site by cathepsin B satisfies the above conditions (i) to (iii), cleavage of the bond between CO and W interlocks with the π electron conjugated system and V in the divalent aromatic ring group (wherein the divalent aromatic ring group constitutes a π electron conjugated system with the cleavable site) in ring A, whereby a bond between a tertiary carbon atom located under ring A and V can be efficiently cleaved. W is preferably an oxygen atom or an amino group (NH), and more preferably an amino group (NH). Therefore, the cleavable site is preferably an amide bonding site.

The cleavable site may be a cleavable site comprising a peptide. The cleavable site comprising a peptide may be a cleavable site consisting of a cleavable peptide cleaved by an enzyme. The cleavable site comprising a peptide may further comprise a peptide not involved in cleavage in addition to the cleavable peptide cleaved by an enzyme. Even when the cleavable site comprising a peptide further comprises a peptide not involved in cleavage, the cleavable site can be cleaved as long as the cleavable site comprises a cleavable peptide cleaved by an enzyme.

The amino acid residue constituting the peptide in the cleavable site may be any amino acid residue. Examples of such any amino acid residue include an α-amino acid residue, a β-amino acid residue, and a γ-amino acid residue, and an α-amino acid residue is preferable. Examples of the α-amino acid residue include an alanine residue, an asparagine residue, a cysteine residue, a glutamine residue, a glycine residue, an isoleucine residue, a leucine residue, a methionine residue, a phenylalanine residue, a proline residue, a serine residue, a threonine residue, a tryptophan residue, a tyrosine residue, a valine residue, an aspartic acid residue, a glutamic acid residue, an arginine residue, a histidine residue, a lysine residue, and a citrulline residue. Such any amino acid residue may also be an L-amino acid residue or a D-amino acid residue, and an L-amino acid residue is preferable. Preferably, any amino acid residue may be an L-α-amino acid residue (e.g., an L-form of the specific α-amino acid residue described above) or a glycine residue.

In a specific embodiment, the cleavable site comprising a peptide may comprise a cleavable peptide comprising an amino acid residue selected from the group consisting of a valine residue, a phenylalanine residue, a threonine residue, a leucine residue, a citrulline residue, an alanine residue, a glutamic acid residue, a glutamine residue, a lysine residue, an arginine residue, a methionine residue, and a combination thereof.

The number of amino acid residues constituting the peptide in the cleavable site may be 2 or more, 3 or more, 4 or more, 5 or more, or 6 or more. The number of amino acid residues constituting the peptide in the cleavable site may also be 20 or less, 15 or less, 12 or less, 10 or less, 8 or less, 6 or less, or 4 or less. More specifically, the number of amino acid residues constituting the peptide may be 2 to 20, 2 to 15, 2 to 12, 2 to 10, 2 to 8, 2 to 6, or 2 to 4, may be 3 to 20, 3 to 15, 3 to 12, 3 to 10, 3 to 8, 3 to 6, or 3 to 4, or may be 4 to 20, 4 to 15, 4 to 12, 4 to 10, 4 to 8, or 4 to 6.

In a specific embodiment, the number of amino acid residues constituting the peptide in the cleavable site may be 2. The number of amino acid residues constituting the peptide in the cleavable site may also be 3 or 4. Alternatively, the number of amino acid residues constituting the cleavable peptide may be 2. The number of amino acid residues constituting the cleavable peptide may also be 3 or 4.

In a specific embodiment, the divalent group comprising a cleavable site, represented by CS, may be -Lc-CS'-W- [here, the hyphen (-) represents a bond, Lc represents a bond or a divalent group, CS' represents a cleavable site, and W represents an oxygen atom, a sulfur atom, or an amino group (NH)]. A definition, examples, and preferred examples of the cleavable site represented by CS' are similar to those of the cleavable site described above. W represents an oxygen atom, a sulfur atom, or an amino group (NH), preferably represents an oxygen atom or an amino group (NH), and more preferably represents an amino group (NH). The same applies to CS (a divalent group comprising a cleavable site) in other Formulas.

Ring A represents a divalent aromatic ring group wherein the divalent aromatic ring group constitutes a π electron conjugated system with the cleavable site) optionally having a substituent. The divalent aromatic ring group is the arylene or the divalent aromatic heterocyclic ring described above. The position of the divalent aromatic ring group to which two adjacent atoms (a carbon atom and an adjacent atom in CS) are bonded is not particularly limited as long as cleavage occurs between V and a carbon atom adjacent thereto by conjugation of a π electron when the cleavable site is cleaved (see FIG. 1). Such a position is common technical knowledge in the art, and can be easily determined by a person skilled in the art according to factors such as the cleavable site and the type of the divalent aromatic ring group.

Ring A may be preferably a divalent monocyclic aromatic ring group optionally having a substituent. The divalent aromatic ring group is a phenylene group or a divalent monocyclic aromatic heterocyclic group.

Ring A may be more preferably a divalent 6-membered ring type aromatic ring group. Examples of the 6-membered ring type aromatic ring group include the various groups described above. In this case, the position of the divalent 6-membered ring type aromatic ring group to which two adjacent atoms are bonded is an ortho position or a para position, and preferably a para position.

Ring A may be still more preferably a phenylene group optionally having a substituent. In this case, the position of the phenylene group to which two adjacent atoms are bonded is an ortho position or a para position, and preferably a para position.

The substituent in the divalent aromatic ring group optionally having a substituent is as described above. Such a substituent may be an electron-withdrawing group as described above.

V represents an oxygen atom, a sulfur atom, or an amino group (NH). V is preferably an oxygen atom or a sulfur atom, and more preferably an oxygen atom.

The divalent group represented by L_{A} is a divalent group capable of linking Ig and a carbon atom adjacent to ring A. The divalent group represented by L_{B} is a divalent group capable of linking D and V.

In a specific embodiment, the divalent group represented by L_{A} or L_{B} may comprise a portion generated by a reaction of two bioorthogonal functional groups capable of reacting with each other. Since a combination of two bioorthogonal functional groups capable of reacting with each other is well known, a person skilled in the art can appropriately select such a combination and appropriately set a divalent group comprising a portion generated by a reaction of the two bioorthogonal functional groups capable of reacting with each other. Examples of the combination of bioorthogonal functional groups capable of reacting with each other include a combination of a thiol residue and a maleimide residue, a combination of a furan residue and a maleimide residue, a combination of a thiol residue and a halocarbonyl residue (a halogen is replaced with a thiol by a substitution reaction), a combination of an alkyne residue (preferably, a ring group having a triple bond between carbon atoms, which may have such a substituent as described above) and an azide residue, a combination of a tetrazine residue and an alkene residue, a combination of a tetrazine residue and an alkyne residue, and a combination of a thiol residue and another thiol residue (disulfide bond). Therefore, the above portion may be a group generated by a reaction of a thiol residue and a maleimide residue, a group generated by a reaction of a furan residue and a maleimide residue, a group generated by a reaction of a thiol residue and a halocarbonyl residue, a group generated by a reaction of an alkyne residue and an azide residue, a group generated by a reaction of a tetrazine residue and an alkene residue, or a disulfide group generated by a combination of a thiol residue and another thiol residue.

In a specific embodiment, the above portion may be a divalent group represented by any one of the following structural Formulas. 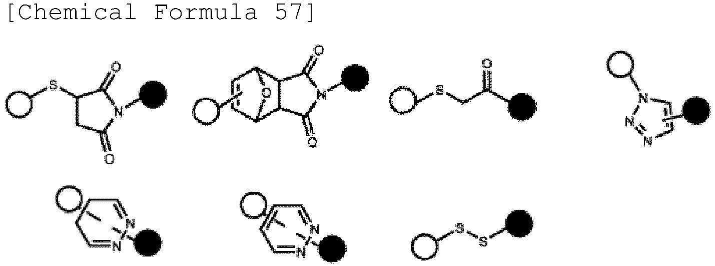 where a white circle and a black circle each represent a bond.

In L_{A}, when a bond of a white circle is bonded to an atom present on an Ig bonding portion side, a bond of a black circle may be bonded to an atom present on a carbon atom side adjacent to ring A, and
when the bond of the white circle is bonded to an atom present on the carbon atom side adjacent to ring A, the bond of the black circle may be bonded to an atom present on the Ig bonding portion side.

In L_{B}, when a bond of a white circle is bonded to an atom present on a V side, a bond of a black circle may be bonded to an atom present on a functional substance (D) bonding portion side, and
when the bond of the white circle is bonded to an atom present on the functional substance (D) bonding portion side, the bond of the black circle may be bonded to an atom present on the V side.

In a preferred embodiment, the divalent group represented by L_{A} may be -L₁-N(-R₁)-CO-. Here, L₁ represents a divalent group, R₁ represents a hydrogen atom or a monovalent group, and a hyphen present on the left side of L₁ and a hyphen present on the right side of CO represent bonds. Preferably, the bond of L₁ is bonded to Ig, and the bond of CO is bonded to a carbon atom adjacent to ring A.

In another preferred embodiment, the divalent group represented by L_{B} may be -L₂-N(-R₂)-CO-. Here, L₂ represents a divalent group, R₂ represents a hydrogen atom or a monovalent group, and a hyphen present on the left side of L₂ and a hyphen present on the right side of CO represent bonds. Preferably, the bond of L₂ is bonded to the functional substance (D), and the bond of CO is bonded to V.

In a specific embodiment, the divalent group represented by L₁ or L₂ may comprise a portion generated by a reaction of two bioorthogonal functional groups capable of reacting with each other. The portion generated by a reaction of two bioorthogonal functional groups capable of reacting with each other, which may be comprised in the divalent group represented by L₁ or L₂, is similar to the portion generated by a reaction of two bioorthogonal functional groups capable of reacting with each other, which may be comprised in the divalent group represented by L_{A} or L_{B}, as described above.

R₁ and R₂ each independently represent a hydrogen atom or a monovalent group. The monovalent group is as described above. The monovalent group in R₁ and R₂ is preferably a monovalent hydrocarbon group optionally having a substituent, more preferably an alkyl optionally having a substituent, and still more preferably an alkyl. As the alkyl, those described above are preferable.

In a specific embodiment, the monovalent group represented by R₁ or R₂ may be a protective group of an amino group. Examples of such a protective group include an alkylcarbonyl group (an acyl group) (e.g., an acetyl group, a propoxy group, and a butoxycarbonyl group such as a tert-butoxycarbonyl group), an alkyloxycarbonyl group (e.g., a fluorenylmethoxycarbonyl group), an aryloxycarbonyl group, and an arylalkyl(aralkyl)oxycarbonyl group (e.g., a benzyloxycarbonyl group).

In a preferred embodiment, R₁ and R₂ each independently represent a hydrogen atom or a protective group of an amino group. R₁ and R₂ may each be preferably a hydrogen atom.

The functional substance represented by D is as described above.

n represents an average number of the bonds per immunoglobulin unit comprising two heavy chains and two light chains, and is 1.5 or more. Such an average number may be, for example, 2.0 or more, preferably 4.0 or more, more preferably 6.0 or more, still more preferably 7.0 or more, and particularly preferably 7.5 or more or 7.8 or more. Such an average number may also be 8.0 or less. More specifically, such an average number may be preferably 2.0 to 8.0, more preferably 4.0 to 8.0, still more preferably 6.0 to 8.0, and particularly preferably 7.0 to 8.0, 7.5 to 8.0, 7.8 to 8.0, or 8.0.

In a specific embodiment, the conjugate of the present invention or a salt thereof has a desired property of being less likely to aggregate and thus can be identified by an aggregation ratio. More specifically, the aggregation ratio of the conjugate of the present invention or a salt thereof may be 5% or less. This is because the present invention makes it easy to avoid aggregation of an antibody. The aggregation ratio is preferably 4.8% or less, more preferably 4.6% or less, still more preferably 4.4% or less, particularly preferably 4.2% or less, 4.0% or less, 3.8% or less, 3.6% or less, 3.40 or less, 3.20 or less, 3.0% or less, 2.8% or less, or 2.6% or less. The aggregation ratio of an antibody can be measured by size exclusion chromatography (SEC)-HPLC (refer to Examples and Chemistry Select, 2020, 5, 8435-8439).

In a preferred embodiment, the aggregation ratio of the conjugate of the present invention or a salt thereof may be 2.6% or less. The aggregation ratio may also be 2.4% or less, 2.2% or less, 2.0% or less, 1.8% or less, 1.6% or less, 1.40 or less, 1.20 or less, 1.0% or less, or 0.8% or less.

Preferably, the structural unit represented by Formula (1) may be represented by the following Formula (1'): 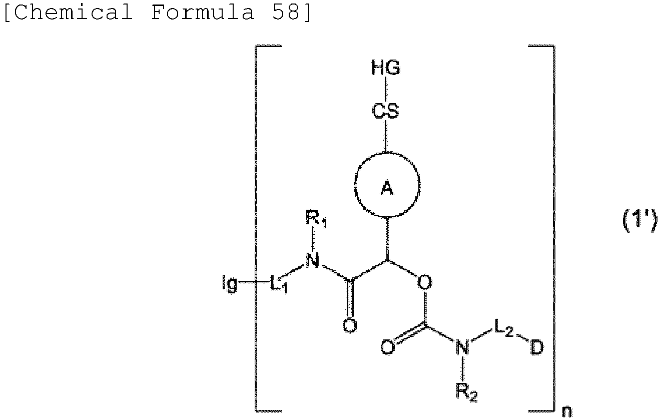 wherein
Ig, HG, CS, ring A, D, and n are the same as those represented in Formula (1), respectively,
R₁ and R₂ each independently represent a hydrogen atom or a monovalent group, and
L₁ and L₂ each independently represent a divalent group, or
may be represented by the following Formula (1''): 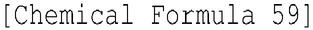 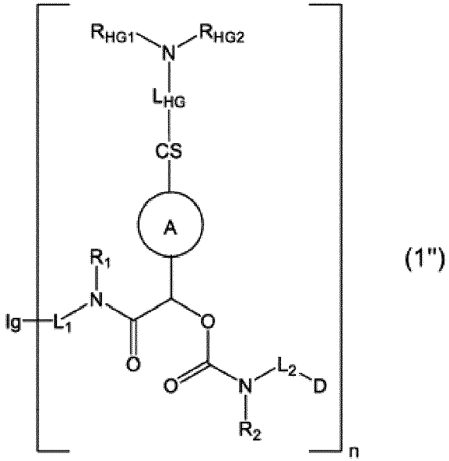 wherein
   Ig, CS, ring A, D, and n are the same as those represented in Formula (1), respectively,
   R_{HG1} and R_{HG2} each independently represent a hydrogen atom, a hydrophilic group, or a monovalent group optionally comprising a hydrophilic group,
   L_{HG} represents a bond or a divalent group optionally comprising a hydrophilic group,
   R₁ and R₂ each independently represent a hydrogen atom or a monovalent group, and
   L₁ and L₂ each independently represent a divalent group.

A definition, examples, and preferred examples of the hydrophilic group or the monovalent group optionally comprising a hydrophilic group, represented by R_{HG1} or R_{HG2}, are similar to those described above.

A definition, examples, and preferred examples of the divalent group optionally comprising a hydrophilic group, represented by L_{HG}, are similar to those described above.

A definition, examples, and preferred examples of the monovalent group, represented by R₁ or R₂, are similar to those of the monovalent group represented by R₁ or R₂ described above.

A definition, examples, and preferred examples of the divalent group, represented by L₁ or L₂, are similar to those of the divalent group represented by L₁ or L₂ described above.

Preferably, the structural unit represented by Formula (1) may be represented by the following Formula (1a) : 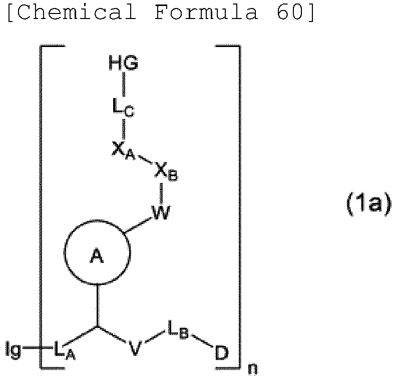 wherein
Ig, HG, ring A, V, L_{A}, L_{B}, D, and n are the same as those represented in Formula (1), respectively,
L_{C} represents a bond or a divalent group,
X_{A} and X_{B} each independently represent an amino acid residue, and
W represents an oxygen atom, a sulfur atom, or an amino group (NH).

L_{C} represents a bond or a divalent group. The divalent group is as described above.

Since X_{A} and X_{B} each independently represent an amino acid residue, the structural unit of X_{A}-X_{B} represented by Formula (1a) corresponds to a dipeptide.

W is preferably an oxygen atom or an amino group (NH), and more preferably an amino group (NH).

The structural unit of X_{A}-X_{B}-W represented by Formula (1a) can be cleaved between X_{A}-X_{B} and W. A person skilled in the art can appropriately set the amino acid residues represented by X_{A} and X_{B} so as to correspond to a cleavable site by an enzyme (e.g., an intracellular protease such as cathepsin B), an acid, or glutathione in blood, or a self-immolative cleavable site as described above. For example, X_{A} may be a valine residue, a phenylalanine residue, a threonine residue, a leucine residue, or an alanine residue. X_{B} may be a citrulline residue, an alanine residue, a glutamic acid residue, a glutamine residue, a lysine residue, an arginine residue, a threonine residue, or a methionine residue. Each of steric configurations of the amino acid residues in X_{A} and X_{B} may be an L-form or a D-form, and is preferably an L-form. Preferably, X_{A} is a valine residue and X_{B} is a citrulline residue or an alanine residue.

Preferably, the structural unit represented by Formula (1a) may be represented by the following Formula (1b) : 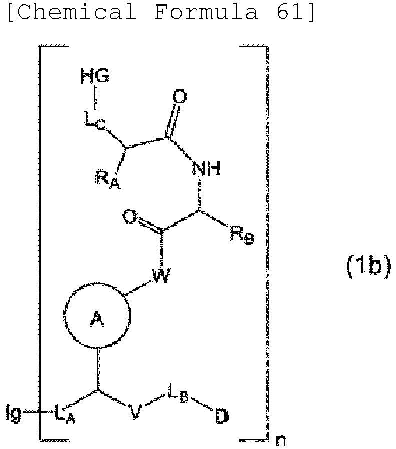 wherein
Ig, HG, ring A, V, L_{A}, L_{B}, D, and n are the same as those represented in Formula (1), respectively,
L_{C} and W are the same as those represented in Formula (1a), respectively,
R_{A} represents a side chain of a valine residue, a phenylalanine residue, a threonine residue, a leucine residue, or an alanine residue, and
R_{B} represents a side chain of a citrulline residue, an alanine residue, a glutamic acid residue, a glutamine residue, a lysine residue, an arginine residue, a threonine residue, or a methionine residue.

R_{A} represents a side chain of a valine residue (that is, -CH(CH₃)₂). Alternatively, R_{A} may be a side chain of a phenylalanine residue, a threonine residue, a leucine residue, or an alanine residue. A steric configuration of the amino acid residue in R_{A} may be L-form or D-form, and is preferably L-form.

R_{B} represents a side chain of a citrulline residue (that is, -CH₂CH₂CH₂NHCONH₂) or a side chain of an alanine residue (that is, -CH₃). Alternatively, R_{B} may be a side chain of a glutamic acid residue, a glutamine residue, a lysine residue, an arginine residue, a threonine residue, or a methionine residue. Each of steric configurations of the amino acid residues in R_{B} may be L-form or D-form, and is preferably L-form.

A combination of R_{A} and R_{B} is preferably a combination in which R_{A} is a side chain of a valine residue and R_{B} is a side chain of a citrulline residue or an alanine residue. Alternatively, other preferred examples of the combination of R_{A} and R_{B} include:
(a) a combination in which R_{A} is a side chain of a valine residue and R_{B} is a side chain of a glutamic acid residue, a lysine residue, an arginine residue, or a threonine residue;
(b) a combination in which R_{A} is a side chain of a phenylalanine residue and R_{B} is a side chain of a lysine residue, an arginine residue, or a glutamine residue;
(c) a combination in which R_{A} is a side chain of a threonine residue and R_{B} is a side chain of a threonine residue or a methionine residue;
(d) a combination in which R_{A} is a side chain of a leucine residue and R_{B} is a side chain of a glutamic acid residue; and
(e) a combination in which R_{A} is a side chain of an alanine residue and R_{B} is a side chain of an alanine residue.

Preferably, the structural unit represented by Formula (1b) may be represented by the following Formula (1c) : 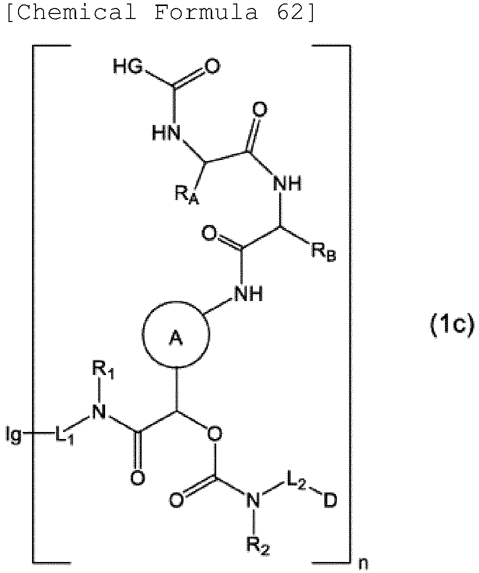 wherein
Ig, HG, ring A, D, and n are the same as those represented in Formula (1), respectively,
R_{A} and R_{B} are the same as those represented in Formula (1b), respectively,
R₁ and R₂ each independently represent a hydrogen atom or a monovalent group, and
L₁ and L₂ each independently represent a divalent group.

A definition, examples, and preferred examples of the monovalent group, represented by R₁ or R₂, are similar to those of the monovalent group represented by R₁ or R₂ described above.

A definition, examples, and preferred examples of the divalent group, represented by L₁ or L₂, are similar to those of the divalent group represented by L₁ or L₂ described above.

Preferably, the structural unit represented by Formula (1c) may be represented by the following Formula (1d) : 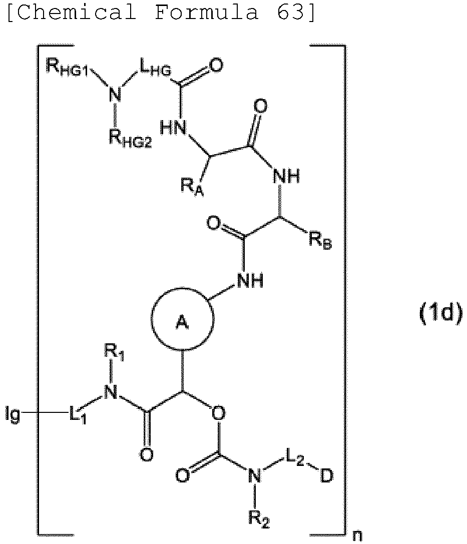 wherein
Ig, ring A, D, and n are the same as those represented in Formula (1), respectively,
R_{A} and R_{B} are the same as those represented in Formula (1b), respectively,
R₁, R₂, L₁, and L₂ are the same as those represented in Formula (1c), respectively,
L_{HG} represents a bond or a divalent group optionally comprising a hydrophilic group,
R_{HG1} and R_{HG2} each independently represent a hydrogen atom, a hydrophilic group, or a monovalent group optionally comprising a hydrophilic group, and
at least one hydrophilic group is comprised in one or more sites selected from the group consisting of L_{HG}, R_{HG1}, and R_{HG2}.

In Formula (1d), L_{HG} represents a bond or a divalent group optionally comprising a hydrophilic group. The hydrophilic group and the divalent group are as described above. The divalent group optionally comprising a hydrophilic group may be comprised in a main chain linking a nitrogen atom and a carbon atom adjacent to L_{HG} or in a side chain of the main chain, and is preferably comprised in the side chain of the main chain.

Preferably, the divalent group (-L_{HG}-) optionally comprising a hydrophilic group may be a divalent group represented by the following Formula (a):

- (C(R_{HC})₂)ₙ₁-(C=O)ₙ₂-(NR_{HG})ₙ₃-(C(R_{HG})₂)ₙ₄- (a)

(hyphens (-) located at both terminals indicate bonds).

In Formula (a), a plurality of R_{HG} each independently represent a hydrogen atom, a hydrophilic group, or a monovalent group optionally comprising a hydrophilic group. The hydrophilic group and the monovalent group are as described above.

n1 is an integer of 0 to 3, preferably an integer of 0 to 2, and more preferably an integer of 0 or 1.

n2 is an integer of 0 or 1.

n3 is an integer of 0 or 1.

n4 is an integer of 0 to 3, preferably an integer of 0 to 2, and more preferably an integer of 0 or 1.

The divalent group (-L_{HG}-) optionally comprising a hydrophilic group may be still more preferably a divalent group represented by the following Formula (a1), (a2), or (a3) :

(a1) -(C(R_{HG})₂)-;

(a2) -(C(R_{HC})₂)-(C=O)-(NR_{HG})-(C(R_{HC})₂)-;

or

(a3) -(C=O)-(C(R_{HG})₂)₂-.

In Formula (a1), (a2), or (a3), a plurality of R_{HG} each independently represent a hydrogen atom, a hydrophilic group, or a C₁₋₆ alkyl group comprising a hydrophilic group. The hydrophilic group and the C₁₋₆ alkyl group are as described above.

In Formula (1d), R_{HG1} and R_{HG2} each independently represent a hydrogen atom, a hydrophilic group, or a monovalent group optionally comprising a hydrophilic group. The hydrophilic group and the monovalent group are as described above.

In a specific embodiment, the monovalent group optionally comprising a hydrophilic group, represented by R_{HG1} or R_{HG2} may be a protective group of an amino group. Examples of the protective group of an amino group include those described above for R₁ and R₂. For example, one of R_{HG1} and R_{HG2} may be a hydrogen atom, and the other may be a protective group of an amino group.

Alternatively, one of R_{HG1} and R_{HG2} may be a hydrogen atom, and the other may be a monovalent group comprising a hydrophilic group. Examples of the monovalent group comprising a hydrophilic group include an alkyl group comprising a hydrophilic group, a carboxyl group comprising a hydrophilic group, an alkylcarbonyl group comprising a hydrophilic group (e.g., groups described above), an alkyloxycarbonyl group comprising a hydrophilic group, and an oxycarbonyl group comprising a hydrophilic group.

In Formula (1d), at least one hydrophilic group is comprised in one or more sites selected from the group consisting of L_{HG}, R_{HG1}, and R_{HG2}. Examples of the site comprising at least one hydrophilic group and a combination thereof include:
(i) L_{HG} alone;
(ii) R_{HG1} alone;
(iii) R_{HG2} alone;
(iv) combination of L_{HG} and L_{HG1};
(v) combination of L_{HG} and L_{HG2};
(vi) combination of L_{HG1} and L_{HG2}; and
(vii) combination of L_{HG}, L_{HG1} and L_{HG2}.

One hydrophilic group may be comprised in each of the L_{HG} site, the R_{HG1} site, and the R_{HG2} site, or two or more hydrophilic groups may be comprised therein.

The definitions, examples, and preferred examples of the symbols described in the above series of Formulas and the elements in the symbols (e.g., specific elements such as cleavable sites or specific Formulas) are similarly applied to other Formulas.

The conjugate of the present invention or a salt thereof is useful as, for example, a medicament or a reagent (e.g., a diagnostic medicament or a research reagent).

The conjugate of the present invention or a salt thereof may be provided in a form of a pharmaceutical composition. Such a pharmaceutical composition may comprise a pharmaceutically allowable carrier in addition to the conjugate of the present invention or a salt thereof. Examples of the pharmaceutically allowable carrier include, but are not limited to, excipients such as sucrose, starch, mannitol, sorbitol, lactose, glucose, cellulose, talc, calcium phosphate, and calcium carbonate; binders such as cellulose, methylcellulose, hydroxypropylcellulose, polypropylpyrrolidone, gelatin, gum arabic, polyethylene glycol, sucrose, and starch; disintegrators such as starch, carboxymethylcellulose, hydroxypropyl starch, sodium hydrogencarbonate, calcium phosphate, and calcium citrate; lubricants such as magnesium stearate, Aerosil, talc, sodium lauryl sulfate; aromatics such as citric acid, menthol, glycyl lysine ammonium salts, glycine, and orange powder; preservatives such as sodium benzoate, sodium hydrogen sulfite, methylparaben, and propylparaben; stabilizers such as citric acid, sodium citrate, and acetic acid, suspensions such as methylcellulose, polyvinylpyrrolidone, and aluminum stearate; dispersants such as surfactants; diluents such as water, a physiological saline solution, and orange juice; and base waxes such as cacao butter, polyethylene glycol, and refined kerosene. The conjugate of the present invention or a salt thereof may also have any modification (e.g., PEGylation) achieving stability.

Examples of preparations suitable for oral administration include liquid medicines dissolving an effective amount of a ligand in a diluted solution such as water, a physiological saline solution, or orange juice; capsules, sachets, and tablets comprising an effective amount of a ligand as a solid or granules; suspension medicines suspending an effective amount of an active ingredient in an appropriate dispersion medium; and emulsions dispersing a solution dissolving an effective amount of an active ingredient in an appropriate dispersion medium to be emulsified.

The pharmaceutical composition is suitable for nonoral administration (e.g., intravenous injection, hypodermic injection, intramuscular injection, local injection, and intraperitoneal administration). Examples of the pharmaceutical composition suitable for such nonoral administration include aqueous or nonaqueous, isotonic, aseptic injection medicines, which may comprise an antioxidant, a buffer, a bacteriostat, a tonicity agent, or the like. Examples thereof also include aqueous or nonaqueous, aseptic suspension medicines, which may comprise a suspension, a solubilizing agent, a thickener, a stabilizer, an antiseptic, or the like.

The dose of the pharmaceutical composition, which varies by the type and activity of an active ingredient, the severity of diseases, an animal type as a subject to be dosed, the drug receptivity, body weight, and age of a subject to be dosed, or the like, can be set appropriately.

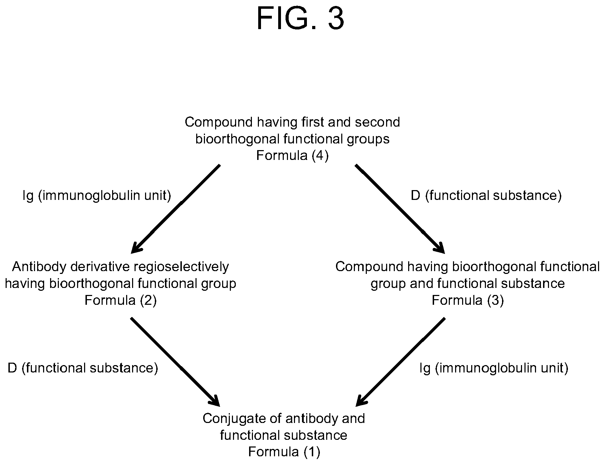

In one embodiment, the conjugate of the present invention or a salt thereof can be produced by causing an antibody derivative having a bioorthogonal functional group or a salt thereof to react with a functional substance (FIG. 3). Such a reaction can be advanced by a reaction between the bioorthogonal functional group in the antibody derivative and the functional substance.

When the functional substance has a functional group that easily reacts with the bioorthogonal functional group, the functional group of the functional substance and the bioorthogonal functional group in the antibody derivative can be caused to react with each other appropriately. The functional group that easily reacts with the bioorthogonal functional group can vary depending on a specific type of bioorthogonal functional group. A person skilled in the art can select an appropriate functional group as the functional group that easily reacts with the bioorthogonal functional group appropriately (for example, Boutureira et al., Chem. Rev., 2015, 115, 2174-2195). Examples of the functional group that easily reacts with the bioorthogonal functional group include, but are not limited to, an alkyne residue when the bioorthogonal functional group is an azide residue, a maleimide residue and a disulfide residue when the bioorthogonal functional group is a thiol residue, a hydrazine residue when the bioorthogonal functional group is an aldehyde residue or a ketone residue, an azide residue when the bioorthogonal functional group is a norbornene residue, and an alkyne residue when the bioorthogonal functional group is a tetrazine residue. Of course, in each of the above combinations of the bioorthogonal functional group and the functional group that easily reacts therewith, it is also possible to exchange the bioorthogonal functional group and the functional group with each other. Therefore, when the bioorthogonal functional group and the functional group that easily reacts therewith are exchanged with each other in the first example of the above combinations, a combination of an alkyne residue as the bioorthogonal functional group and an azide residue as the functional group that easily reacts with the bioorthogonal functional group can be used.

When the functional substance does not have a functional group that easily reacts with the bioorthogonal functional group in the antibody derivative, the drug may be derivatized so as to have such a functional group. Derivatization is common technical knowledge in the field concerned (e.g., WO 2004/010957 A, United States Patent Application Publication No. 2006/0074008, and United States Patent Application Publication No. 2005/0238649). Derivatization may be performed using any cross-linking agent, for example. Alternatively, derivatization may be performed using a specific linker having a desired functional group. In the present invention, the derivatized functional substance can also be referred to simply as "functional substance" because the derivatized functional substance is only one type of functional substance.

The reaction can be appropriately performed under a condition incapable of causing denaturation or decomposition (e.g., cleavage of an amide bond) of proteins (a mild condition). Such a reaction can be performed in an appropriate reaction system, for example, in a buffer at room temperature (e.g., about 15°C to 30°C), for example. The pH of the buffer is e.g., 5 to 9, preferably 5.5 to 8.5, and more preferably 6.0 to 8.0. The buffer may comprise an appropriate catalyst. The reaction time is e.g., 1 minute to 20 hours, preferably 10 minutes to 15 hours, more preferably 20 minutes to 10 hours, and still more preferably 30 minutes to 8 hours. For the details of such a reaction, refer to G. J. L. Bernardes et al., Chem. Rev., 115,2174 (2015); G. J. L. Bernardes et al., Chem. Asian. J., 4,630 (2009); B. G. Davies et al., Nat. Commun., 5,4740 (2014); A. Wagner et al., Bioconjugate. Chem., 25,825 (2014), for example.

In another embodiment, the conjugate of the present invention or a salt thereof can be produced by causing a compound having a bioorthogonal functional group and a functional substance or a salt thereof to react with a raw material antibody having Ig (immunoglobulin unit) (FIG. 3). Such a raw material antibody can be prepared by bringing an antibody comprising an immunoglobulin unit (comprising four disulfide bonds) comprising two heavy chains and two light chains into contact with a reducing agent to generate an antibody comprising a thiol group. As the reducing agent, any reducing agent capable of cleaving a disulfide bond to form a thiol group can be used, and examples thereof include tricarboxylethylphosphine (TCEP), cysteine, dithiothreitol, reduced glutathione, and β-mercaptoethanol. In the present embodiment, the bioorthogonal functional group of the compound is preferably a group capable of efficiently reacting with a thiol group (e.g., a maleimide residue, a halocarbonyl group, or a thiol group). The reaction between the compound having a bioorthogonal functional group and a functional substance or a salt thereof and the raw material antibody can be appropriately performed under the above-described condition (a mild condition) that cannot cause denaturation or decomposition of a protein (e.g., cleavage of an amide bond).

Production of the conjugate or a salt thereof can be confirmed by, for example, reverse phase HPLC under reducing conditions or mass spectrometry, depending on a specific raw material thereof and the molecular weight of a product. The conjugate or a salt thereof can be appropriately purified by any purification method such as chromatography (e.g., gel filtration chromatography, ion exchange chromatography, reverse phase column chromatography, high performance liquid chromatography, or affinity chromatography).

### 3. Antibody derivative or salt thereof

The present invention also provides
an antibody derivative having a bioorthogonal functional group or bioorthogonal functional groups, comprising a structural unit represented by the following Formula (2): 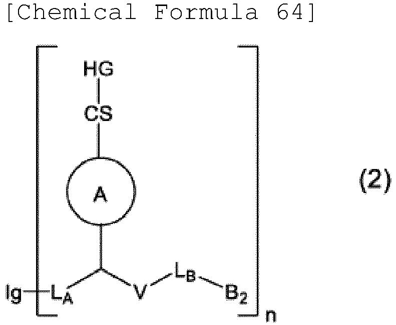 wherein
Ig represents an immunoglobulin unit comprising two heavy chains and two light chains, and is bonded to L_{A} adjacent to Ig via a thiol group in side chains of a plurality of cysteine residues in the two heavy chains and the two light chains,
HG represents a hydrophilic group or a monovalent group comprising a hydrophilic group,
CS represents a divalent group comprising a cleavable site by cathepsin B,
ring A represents a divalent aromatic ring group optionally having a substituent wherein the divalent aromatic ring group constitutes a π electron conjugated system with the cleavable site,
V represents an oxygen atom, a sulfur atom, or an amino group (NH),
L_{A} and L_{B} each independently represent a divalent group,
B₂ represents a bioorthogonal functional group, and
an average number n of the bonds per the immunoglobulin unit is 1.5 or more, or
a salt thereof.

In Formula (2), Ig, HG, CS, ring A, V, L_{A}, L_{B}, and n are as described above in Formula (1). Therefore, definitions, examples, and preferred examples of these elements and other elements associated therewith are the same as those described above in Formula (1).

The bioorthogonal functional group represented by B₂ is as described above.

In a specific embodiment, the bioorthogonal functional group represented by B₂ may be a maleimide residue, a thiol residue, a furan residue, a halocarbonyl residue, an alkene residue, an alkyne residue, an azide residue, or a tetrazine residue. Alternatively, the bioorthogonal functional group represented by B₂ may be a maleimide residue, a thiol residue, a furan residue, a halocarbonyl residue, an alkene residue, an alkyne residue, an azide residue, or a tetrazine residue. These bioorthogonal functional groups are preferable because the bioorthogonal functional groups have excellent reaction efficiency and high versatility.

Preferably, the structural unit represented by Formula (2) may be represented by the following Formula (2'): 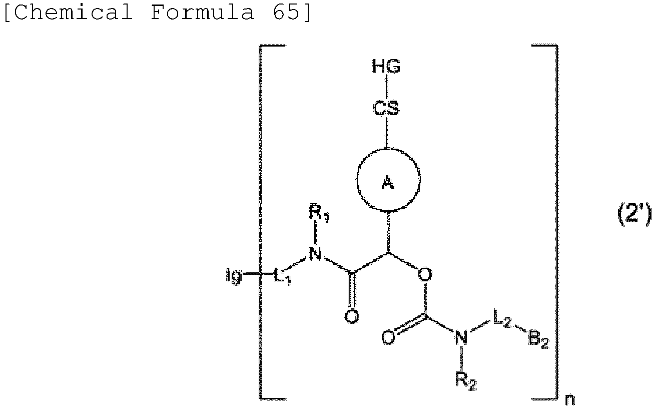 wherein
Ig, HG, CS, ring A, B₂, and n are the same as those represented in Formula (2), respectively,
R₁ and R₂ each independently represent a hydrogen atom or a monovalent group, and
L₁ and L₂ each independently represent a divalent group, or
may be represented by the following Formula (2''): 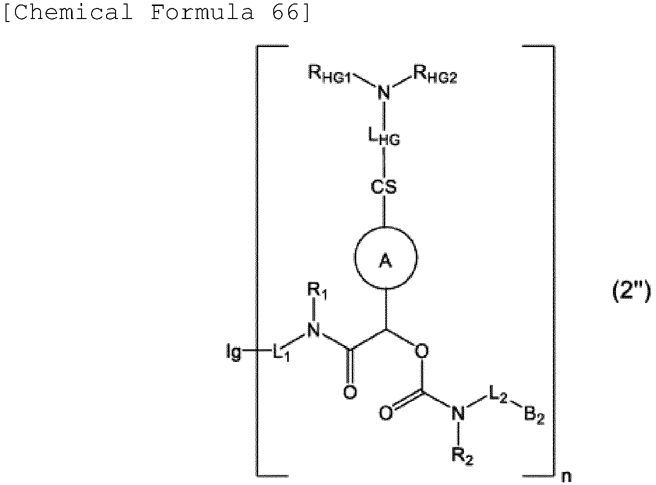 wherein
Ig, CS, ring A, B₂, and n are the same as those represented in Formula (2), respectively,
R_{HG1} and R_{HG2} each independently represent a hydrogen atom, a hydrophilic group, or a monovalent group optionally comprising a hydrophilic group,
L_{HG} represents a bond or a divalent group optionally comprising a hydrophilic group,
R₁ and R₂ each independently represent a hydrogen atom or a monovalent group, and
L₁ and L₂ each independently represent a divalent group.

In these Formulas, R_{HG1}, R_{HG2}, L_{HG}, R₁, R₂, L₁, and L₂ are as described above. Therefore, definitions, examples, and preferred examples of these elements and other elements associated therewith are the same as those described above.

Preferably, the structural unit represented by Formula (2) may be represented by the following Formula (2a) : 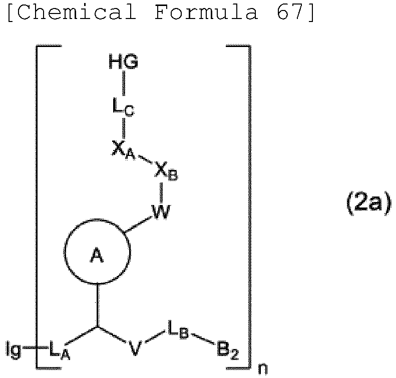 wherein
Ig, HG, ring A, V, L_{A}, L_{B}, B₂, and n are the same as those represented in Formula (2), respectively,
L_{C} represents a bond or a divalent group,
X_{A} and X_{B} each independently represent an amino acid residue, and
W represents an oxygen atom, a sulfur atom, or an amino group (NH).

In Formula (2a), L_{C}, X_{A}, X_{B}, and W are as described above in Formula (1a). Therefore, definitions, examples, and preferred examples of these elements and other elements associated therewith are the same as those described above in Formula (1a).

Preferably, the structural unit represented by Formula (2a) may be represented by the following Formula (2b) : 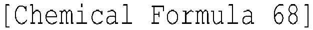 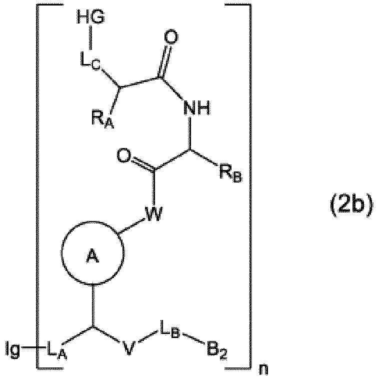 wherein
Ig, HG, ring A, V, L_{A}, L_{B}, B₂, and n are the same as those represented in Formula (2), respectively,
L_{C} and W are the same as those represented in Formula (2a), respectively,
R_{A} represents a side chain of a valine residue, a phenylalanine residue, a threonine residue, a leucine residue, or an alanine residue, and
R_{B} represents a side chain of a citrulline residue, an alanine residue, a glutamic acid residue, a glutamine residue, a lysine residue, an arginine residue, a threonine residue, or a methionine residue.

In Formula (2b), R_{A} and R_{B} are as described above in Formula (1b). Therefore, definitions, examples, and preferred examples of these elements and other elements associated therewith are the same as those described above in Formula (1b).

Preferably, the structural unit represented by Formula (2b) may be represented by the following Formula (2c) : 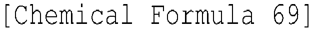 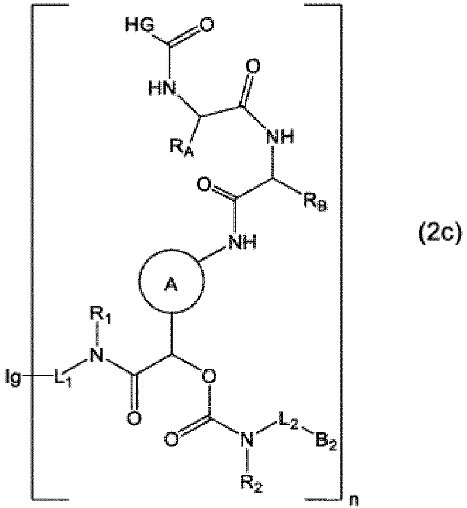 wherein
Ig, HG, ring A, B₂, and n are the same as those represented in Formula (2), respectively,
R_{A} and R_{B} are the same as those represented in Formula (2b), respectively,
R₁ and R₂ each independently represent a hydrogen atom or a monovalent group, and
L₁ and L₂ each independently represent a divalent group.

In Formula (2c), R₁, R₂, L₁, and L₂ are as described above in Formula (1c). Therefore, definitions, examples, and preferred examples of these elements and other elements associated therewith are the same as those described above in Formula (1c).

Preferably, the structural unit represented by Formula (2c) may be represented by the following Formula (2d) : 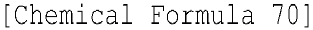 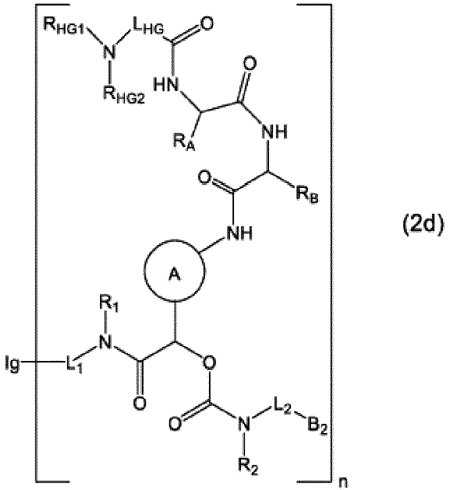 wherein
Ig, ring A, B₂, and n are the same as those represented in Formula (2), respectively,
R_{A} and R_{B} are the same as those represented in Formula (2b), respectively,
R₁, R₂, L₁, and L₂ are the same as those represented in Formula (2c), respectively,
L_{HG} represents a bond or a divalent group optionally comprising a hydrophilic group,
R_{HG1} and R_{HG2} each independently represent a hydrogen atom, a hydrophilic group, or a monovalent group optionally comprising a hydrophilic group, and
at least one hydrophilic group is comprised in one or more sites selected from the group consisting of L_{HG}, R_{HG1}, and R_{HG2}.

In Formula (2d), L_{HG}, R_{HG1}, and R_{HG2} are as described above in Formula (1d). Therefore, definitions, examples, and preferred examples of these elements and other elements associated therewith are the same as those described above in Formula (1d).

The antibody derivative of the present invention or a salt thereof is useful as, for example, an intermediate for production of the conjugate of the present invention or a salt thereof.

The antibody derivative of the present invention or a salt thereof can be produced, for example, by causing a compound having a first bioorthogonal functional group and a second bioorthogonal functional group or a salt thereof to react with a raw material antibody having Ig (immunoglobulin unit) (FIG. 3). The raw material antibody is the same as that described above.

The reaction between the compound having a first bioorthogonal functional group and a second bioorthogonal functional group or a salt thereof and the raw material antibody can be appropriately performed under the above-described condition (a mild condition) that cannot cause denaturation or decomposition of a protein (e.g., cleavage of an amide bond).

Production of the antibody derivative or a salt thereof can be confirmed in a similar manner to the method described for the conjugate of the present invention. The antibody derivative or a salt thereof can be appropriately purified by any purification method as described for the conjugate of the present invention.

### 4. Compound having bioorthogonal functional group or bioorthogonal functional groups and functional substance or functional substances or salt thereof

The present invention also provides a compound having a bioorthogonal functional group or bioorthogonal functional groups and a functional substance or functional substances, represented by the following Formula (3): 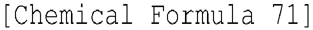 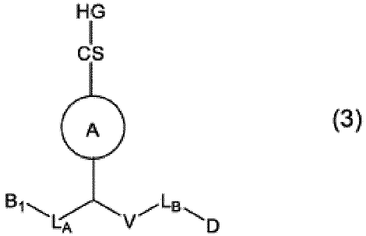 wherein
HG represents a hydrophilic group or a monovalent group comprising a hydrophilic group,
CS represents a divalent group comprising a cleavable site by cathepsin B,
ring A represents a divalent aromatic ring group optionally having a substituent wherein the divalent aromatic ring group constitutes a π electron conjugated system with the cleavable site,
V represents an oxygen atom, a sulfur atom, or an amino group (NH),
L_{A} and L_{B} each independently represent a divalent group,
B₁ represents a bioorthogonal functional group, and
D represents a functional substance, or
a salt thereof.

In Formula (3), HG, CS, ring A, V, L_{A}, L_{B}, and D are as described above in Formula (1). Therefore, definitions, examples, and preferred examples of these elements and other elements associated therewith are the same as those described above in Formula (1).

B₁ represents a bioorthogonal functional group. The bioorthogonal functional group is as described above. Preferably, the bioorthogonal functional group is preferably a group capable of efficiently reacting with a thiol group (e.g., a maleimide residue, a halocarbonyl group, or a thiol group).

Preferably, the structural unit represented by Formula (3) may be represented by the following Formula (3'): 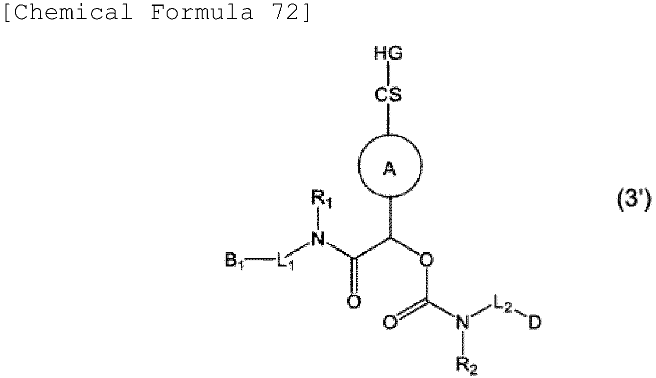 wherein
HG, CS, ring A, B₁, and D are the same as those represented in Formula (3), respectively,
R₁ and R₂ each independently represent a hydrogen atom or a monovalent group, and
L₁ and L₂ each independently represent a divalent group, or
may be represented by the following Formula (3''): 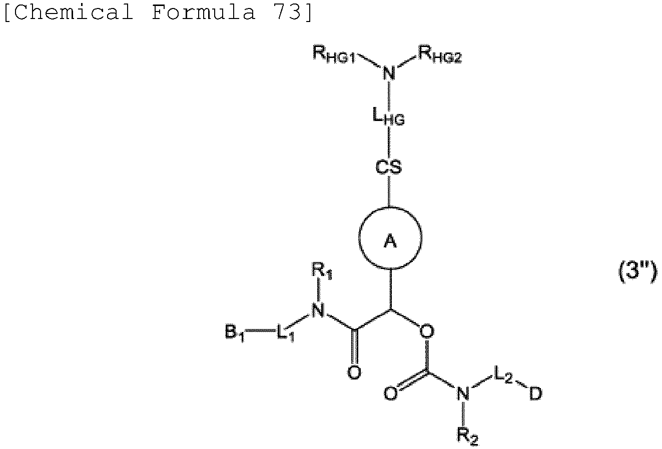 wherein
CS, ring A, B₁, and D are the same as those represented in Formula (3), respectively,
R_{HG1} and R_{HG2} each independently represent a hydrogen atom, a hydrophilic group, or a monovalent group optionally comprising a hydrophilic group,
L_{HG} represents a bond or a divalent group optionally comprising a hydrophilic group,
R₁ and R₂ each independently represent a hydrogen atom or a monovalent group, and
L₁ and L₂ each independently represent a divalent group.

In these Formulas, R_{HG1}, R_{HG2}, L_{HG}, R₁, R₂, L₁, and L₂ are as described above. Therefore, definitions, examples, and preferred examples of these elements and other elements associated therewith are the same as those described above.

Preferably, the compound represented by Formula (3) may be represented by the following Formula (3a): 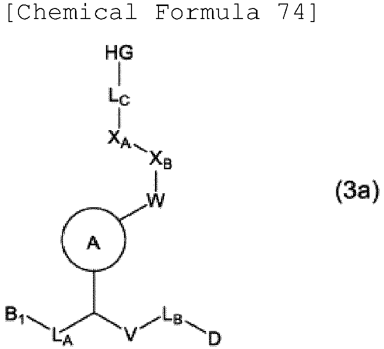 wherein
HG, ring A, V, L_{A}, L_{B}, B₁, and D are the same as those represented in Formula (3), respectively,
L_{C} represents a bond or a divalent group,
X_{A} and X_{B} each independently represent an amino acid residue, and
W represents an oxygen atom, a sulfur atom, or an amino group (NH).

In Formula (3a), L_{C}, X_{A}, X_{B}, and W are as described above in Formula (1a). Therefore, definitions, examples, and preferred examples of these elements and other elements associated therewith are the same as those described above in Formula (1a).

Preferably, the compound represented by Formula (3a) may be represented by the following Formula (3b): 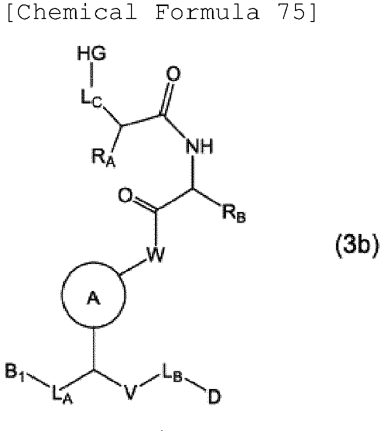 wherein
HG, ring A, V, L_{A}, L_{B}, B₁, and D are the same as those represented in Formula (3), respectively,
L_{C} and W are the same as those represented in Formula (3a), respectively,
R_{A} represents a side chain of a valine residue, a phenylalanine residue, a threonine residue, a leucine residue, or an alanine residue, and
R_{B} represents a side chain of a citrulline residue, an alanine residue, a glutamic acid residue, a glutamine residue, a lysine residue, an arginine residue, a threonine residue, or a methionine residue.

In Formula (3b), R_{A} and R_{B} are as described above in Formula (1b). Therefore, definitions, examples, and preferred examples of these elements and other elements associated therewith are the same as those described above in Formula (1b).

Preferably, the compound represented by Formula (3b) may be represented by the following Formula (3c): 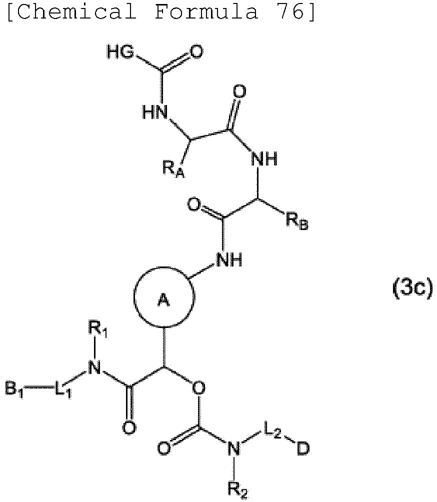 wherein
HG, ring A, B₁, and D are the same as those represented in Formula (3), respectively,
R_{A} and R_{B} are the same as those represented in Formula (3b), respectively,
R₁ and R₂ each independently represent a hydrogen atom or a monovalent group, and
L₁ and L₂ each independently represent a divalent group.

In Formula (3c), R₁, R₂, L₁, and L₂ are as described above in Formula (1c). Therefore, definitions, examples, and preferred examples of these elements and other elements associated therewith are the same as those described above in Formula (1c).

Preferably, the compound represented by Formula (3c) may be represented by the following Formula (3d): 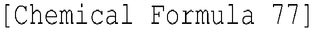 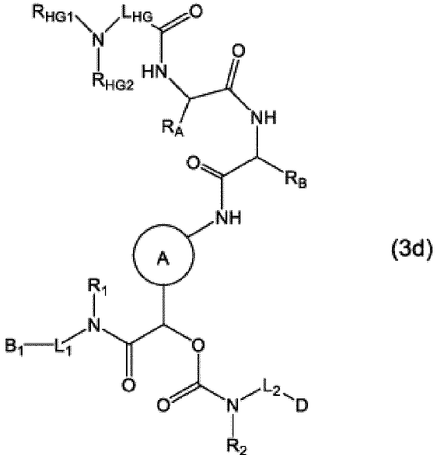 wherein
ring A, B₁, and D are the same as those represented in Formula (3), respectively,
R_{A} and R_{B} are the same as those represented in Formula (3b), respectively,
R₁, R₂, L₁, and L₂ are the same as those represented in Formula (3c), respectively,
L_{HG} represents a bond or a divalent group optionally comprising a hydrophilic group,
R_{HG1} and R_{HG2} each independently represent a hydrogen atom, a hydrophilic group, or a monovalent group optionally comprising a hydrophilic group, and
at least one hydrophilic group is comprised in one or more sites selected from the group consisting of L_{HG}, R_{HG1}, and R_{HC2}.

In Formula (3d), L_{HG}, R_{HG1}, and R_{HG2} are as described above in Formula (1d). Therefore, definitions, examples, and preferred examples of these elements and other elements associated therewith are the same as those described above in Formula (1d).

The compound of the present invention represented by Formula (3) or a salt thereof is useful as, for example, an intermediate for production of the conjugate of the present invention. The compound of the present invention represented by Formula (3) or a salt thereof is also useful, for example, for derivatization of any substance such as a biomolecule (e.g., a protein such as an antibody, a saccharide, a nucleic acid, or a lipid).

The compound having a bioorthogonal functional group or bioorthogonal functional groups and a functional substance or functional substances or a salt thereof can be produced, for example, by causing the compound having a first bioorthogonal functional group and a second bioorthogonal functional group or a salt thereof to react with a functional substance (FIG. 3). Details of the functional substance are as described above.

The reaction between the compound having a first bioorthogonal functional group and a second bioorthogonal functional group or a salt thereof and the functional substance or functional substances can be performed at an appropriate temperature (e.g., about 15 to 200°C) in an appropriate reaction system, for example, in an organic solvent system or an aqueous solution (e.g., buffer) system. The reaction system may comprise an appropriate catalyst. The reaction time is e.g., 1 minute to 20 hours, preferably 10 minutes to 15 hours, more preferably 20 minutes to 10 hours, and still more preferably 30 minutes to 8 hours. Of course, such a reaction can also be performed under the mild condition described above.

Production of the compound having a bioorthogonal functional group and a functional substance or a salt thereof can be confirmed by, for example, NMR, HPLC, or mass spectrometry, depending on a specific raw material thereof and the molecular weight of a product. Such a compound or a salt thereof can be appropriately purified by any purification method such as chromatography (e.g., gel filtration chromatography, ion exchange chromatography, reverse phase column chromatography, high performance liquid chromatography, or affinity chromatography).

### 5. Compound having first bioorthogonal functional group and second bioorthogonal functional group or salt thereof

The present invention also provides a compound having a first bioorthogonal functional group and a second bioorthogonal functional group, represented by the following Formula (4): 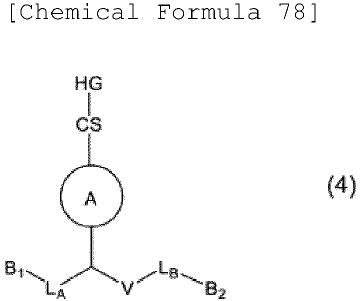 wherein
HG represents a hydrophilic group or a monovalent group comprising a hydrophilic group,
CS represents a divalent group comprising a cleavable site by cathepsin B,
ring A represents a divalent aromatic ring group optionally having a substituent wherein the divalent aromatic ring group constitutes a π electron conjugated system with the cleavable site,
V represents an oxygen atom, a sulfur atom, or an amino group (NH),
L_{A} and L_{B} each independently represent a divalent group,
B₁ represents a first bioorthogonal functional group, and
B₂ represents a second bioorthogonal functional group, or
a salt thereof.

In Formula (4), HG, CS, ring A, V, L_{A}, L_{B}, and D are as described above in Formula (1). Therefore, definitions, examples, and preferred examples of these elements and other elements associated therewith are the same as those described above in Formula (1).

B₁ represents a first bioorthogonal functional group. The first bioorthogonal functional group is the same as the bioorthogonal functional group described above. Preferably, the bioorthogonal functional group is preferably a group capable of efficiently reacting with a thiol group (e.g., a maleimide residue, a halocarbonyl group, or a thiol group).

B₂ represents a second bioorthogonal functional group. The second bioorthogonal functional group is the same as the bioorthogonal functional group described above.

In a specific embodiment, the bioorthogonal functional group represented by B₂ may be a maleimide residue, a thiol residue, a furan residue, a halocarbonyl residue, an alkene residue, an alkyne residue, an azide residue, or a tetrazine residue. Alternatively, the bioorthogonal functional group represented by B₂ may be a maleimide residue, a thiol residue, a furan residue, a halocarbonyl residue, an alkene residue, an alkyne residue, an azide residue, or a tetrazine residue. These bioorthogonal functional groups are preferable because the bioorthogonal functional groups have excellent reaction efficiency and high versatility.

The second bioorthogonal functional group may be preferably a bioorthogonal functional group that does not react with the first bioorthogonal functional group or has low reactivity to the first bioorthogonal functional group. In this case, an intermolecular reaction of the compound represented by Formula (4) or a salt thereof can be suppressed. Therefore, the first and second bioorthogonal functional groups can be used in a combination in which the first and second bioorthogonal functional groups do not react with each other or have low reactivity to each other. Such a combination of bioorthogonal functional groups is well known in the art. For example, for a maleimide residue, a thiol residue, a furan residue, a halocarbonyl residue, an alkene residue, an alkyne residue, an azide residue, and a tetrazine residue, which are preferred bioorthogonal functional groups, examples of such a combination are as follows.

**[Table A]**

| Table A. Examples of combination of first and second bioorthogonal functional groups which do not react with each other or have low reactivity to each other | |
|---|---|
| First bioorthogonal functional group | Second bioorthogonal functional group |
| Maleimide residue | Halocarbonyl residue, alkene residue, or alkyne residue |
| Thiol residue | Furan residue, alkene residue, alkyne residue, azide residue, or tetrazine residue |
| Furan residue | Thiol residue, or halocarbonyl residue |
| Halocarbonyl residue | Maleimide residue, furan residue, alkene residue, alkyne residue, azide residue, or tetrazine residue |
| Alkene residue | Maleimide residue, thiol residue, halocarbonyl residue, or alkyne residue |
| Alkyne residue | Maleimide residue, thiol residue, halocarbonyl residue, or alkene residue |
| Azide residue | Thiol residue, or halocarbonyl residue, |
| Tetrazine residue | Thiol residue, or halocarbonyl residue, |

Preferably, the structural unit represented by Formula (4) may be represented by the following Formula (4'): 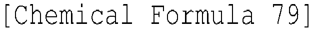 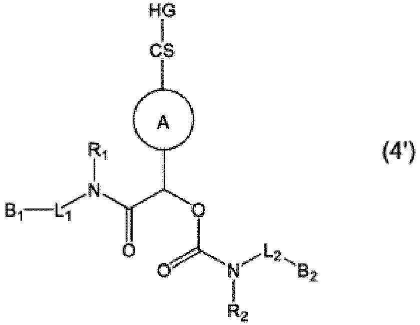 wherein
HG, CS, ring A, B₁, and B₂ are the same as those represented in Formula (4), respectively,
R₁ and R₂ each independently represent a hydrogen atom or a monovalent group, and
L₁ and L₂ each independently represent a divalent group, or
may be represented by the following Formula (4''): 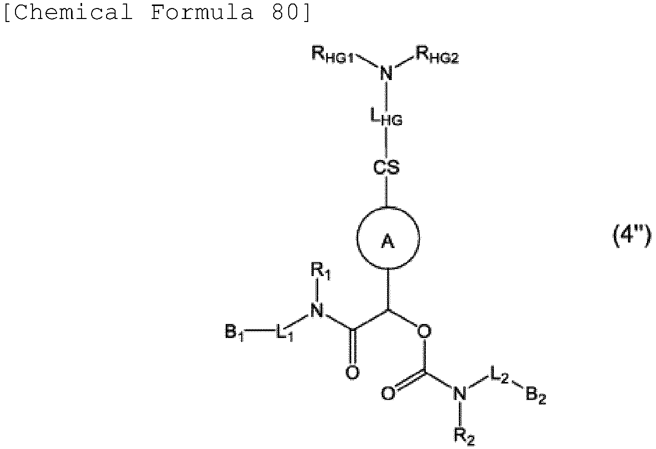 wherein
CS, ring A, B₁, and B₂ are the same as those represented in Formula (4), respectively,
R_{HG1} and R_{HG2} each independently represent a hydrogen atom, a hydrophilic group, or a monovalent group optionally comprising a hydrophilic group,
L_{HG} represents a bond or a divalent group optionally comprising a hydrophilic group,
R₁ and R₂ each independently represent a hydrogen atom or a monovalent group, and
L₁ and L₂ each independently represent a divalent group.

In these Formulas, R_{HG1}, R_{HG2}, L_{HG}, R₁, R₂, L₁, and L₂ are as described above. Therefore, definitions, examples, and preferred examples of these elements and other elements associated therewith are the same as those described above.

Preferably, the compound represented by Formula (4) may be represented by the following Formula (4a): 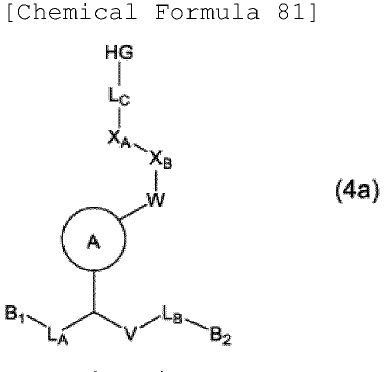 wherein
HG, ring A, V, L_{A}, L_{B}, B₁, and B₂ are the same as those represented in Formula (4), respectively,
L_{C} represents a bond or a divalent group,
X_{A} and X_{B} each independently represent an amino acid residue, and
W represents an oxygen atom, a sulfur atom, or an amino group (NH).

In Formula (4a), L_{C}, X_{A}, X_{B}, and W are as described above in Formula (1a). Therefore, definitions, examples, and preferred examples of these elements and other elements associated therewith are the same as those described above in Formula (1a).

Preferably, the compound represented by Formula (4a) may be represented by the following Formula (4b): 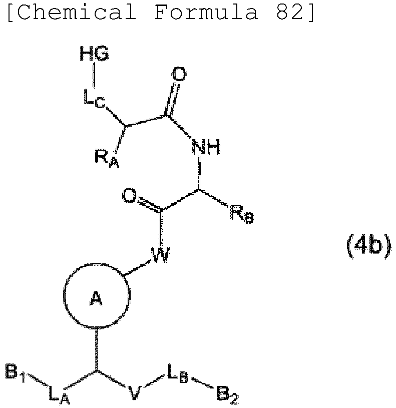 wherein
HG, ring A, V, L_{A}, L_{B},B₁, and B₂ are the same as those represented in Formula (4), respectively,
L_{C} and W are the same as those represented in Formula (4a), respectively,
R_{A} represents a side chain of a valine residue, a phenylalanine residue, a threonine residue, a leucine residue, or an alanine residue, and
R_{B} represents a side chain of a citrulline residue, an alanine residue, a glutamic acid residue, a glutamine residue, a lysine residue, an arginine residue, a threonine residue, or a methionine residue.

In Formula (4b), R_{A} and R_{B} are as described above in Formula (1b). Therefore, definitions, examples, and preferred examples of these elements and other elements associated therewith are the same as those described above in Formula (1b).

Preferably, the compound represented by Formula (4b) may be represented by the following Formula (4c): 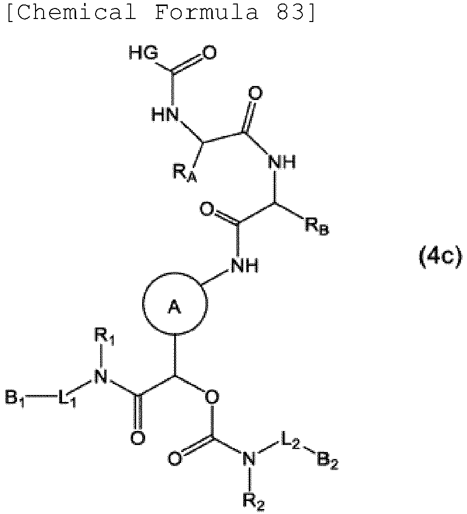 wherein
HG, ring A, B₁, and B₂ are the same as those represented in Formula (4), respectively,
R_{A} and R_{B} are the same as those represented in Formula (4b), respectively,
R₁ and R₂ each independently represent a hydrogen atom or a monovalent group, and
L₁ and L₂ each independently represent a divalent group.

In Formula (4c), R₁, R₂, L₁, and L₂ are as described above in Formula (1c). Therefore, definitions, examples, and preferred examples of these elements and other elements associated therewith are the same as those described above in Formula (1c).

Preferably, the compound represented by Formula (4c) may be represented by the following Formula (4d): 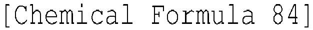 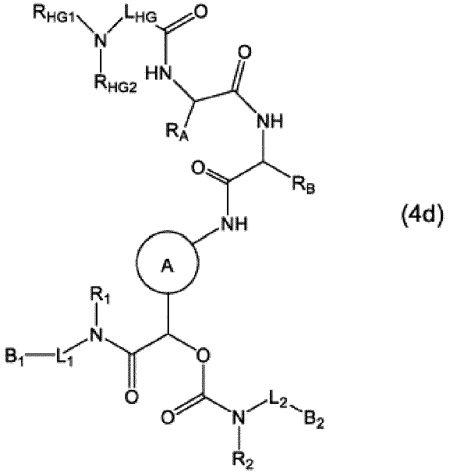 wherein
ring A, B₁, and B₂ are the same as those represented in Formula (4), respectively,
R_{A} and R_{B} are the same as those represented in Formula (4b), respectively,
R₁, R₂, L₁, and L₂ are the same as those represented in Formula (4c), respectively,
L_{HG} represents a bond or a divalent group optionally comprising a hydrophilic group,
R_{HG1} and R_{HG2} each independently represent a hydrogen atom, a hydrophilic group, or a monovalent group optionally comprising a hydrophilic group, and
at least one hydrophilic group is comprised in one or more sites selected from the group consisting of L_{HG}, R_{HG1}, and R_{HC2}.

In Formula (4d), L_{HG}, R_{HG1}, and R_{HG2} are as described above in Formula (1d). Therefore, definitions, examples, and preferred examples of these elements and other elements associated therewith are the same as those described above in Formula (1d).

The compound of the present invention represented by Formula (4) or a salt thereof is useful as, for example, an intermediate for production of the antibody derivative of the present invention and the compound of the present invention represented by Formula (3). The compound of the present invention represented by Formula (4) or a salt thereof is also useful, for example, for derivatization of any substance such as a biomolecule (e.g., a protein such as an antibody, a saccharide, a nucleic acid, or a lipid) or a functional substance.

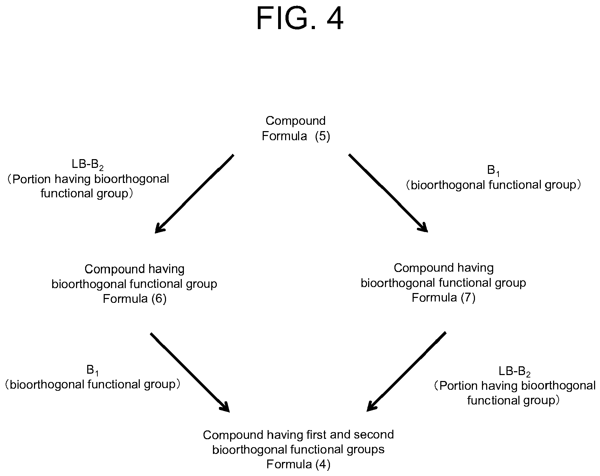

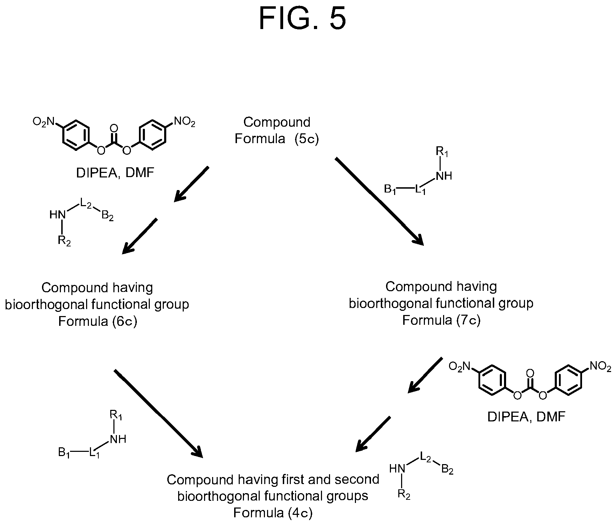

In one embodiment, the compound having a first bioorthogonal functional group and a second bioorthogonal functional group or a salt thereof can be produced, for example, by causing the compound having a bioorthogonal functional group or bioorthogonal functional groups, represented by Formula (6) or a salt thereof to react with an appropriate compound having B₁ (e.g., a compound represented by B₁-L₁-NH-R₁) (FIGS. 4 and 5). Definitions, examples, and preferred examples of B₁, L₁, and R₁ are as described above.

In another embodiment, the compound having a first bioorthogonal functional group and a second bioorthogonal functional group or a salt thereof can be produced, for example, by causing the compound having a bioorthogonal functional group or bioorthogonal functional groups, represented by Formula (7) or a salt thereof to react with an appropriate compound having L_{B}-B₂ (for example, by causing the compound having a bioorthogonal functional group or bioorthogonal functional groups, represented by Formula (7) or a salt thereof to react with bis(4-nitriphenyl) carbonate and N,N-diisopropylethylamine (DIPEA), and then causing the resulting product to react with a compound represented by B₂-L₂-NH-R₂ (FIGS. 4 and 5). Definitions, examples, and preferred examples of B₂, L_{B}, and R₂ are as described above.

The reaction can be performed in an appropriate reaction system, for example, in an organic solvent system or an aqueous solution (e.g., buffer) system at an appropriate temperature (e.g., about 15°C to 200°C). The reaction system may comprise an appropriate catalyst. The reaction time is e.g., 1 minute to 20 hours, preferably 10 minutes to 15 hours, more preferably 20 minutes to 10 hours, and still more preferably 30 minutes to 8 hours. Of course, such a reaction can also be performed under the mild condition described above.

Production of the compound having a first bioorthogonal functional group and a second bioorthogonal functional group or a salt thereof can be confirmed by, for example, NMR, HPLC, or mass spectrometry, depending on a specific raw material thereof and the molecular weight of a product. Such a compound or a salt thereof can be appropriately purified by any purification method such as chromatography (e.g., gel filtration chromatography, ion exchange chromatography, reverse phase column chromatography, high performance liquid chromatography, or affinity chromatography).

### 6. Series of compounds or salts thereof

### (1) Compound or salt thereof

The present invention also provides a compound represented by the following Formula (5): 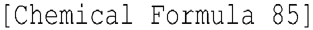 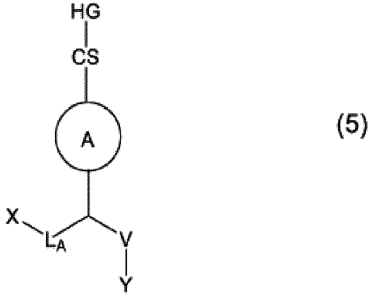 wherein
HG represents a hydrophilic group or a monovalent group comprising a hydrophilic group,
CS represents a divalent group comprising a cleavable site by cathepsin B,
ring A represents a divalent aromatic ring group optionally having a substituent wherein the divalent aromatic ring group constitutes a π electron conjugated system with the cleavable site,
V represents an oxygen atom, a sulfur atom, or an amino group (NH),
L_{A} represents a divalent group, and
X and Y each independently represent a monovalent group, or
a salt thereof.

In Formula (5), HG, CS, ring A, V, and L_{A} are as described above in Formula (1). Therefore, definitions, examples, and preferred examples of these elements and other elements associated therewith are the same as those described above in Formula (1).

X and Y each independently represent a monovalent group. The monovalent group is as described above.

Preferably, the structural unit represented by Formula (5) may be represented by the following Formula (5'): 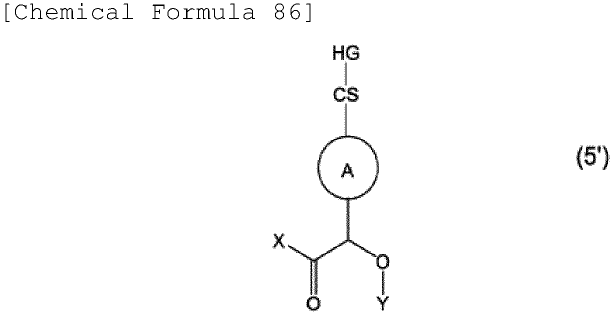 wherein
HG, CS, ring A, X, and Y are the same as those represented in Formula (5), respectively, or
may be represented by the following Formula (5''): 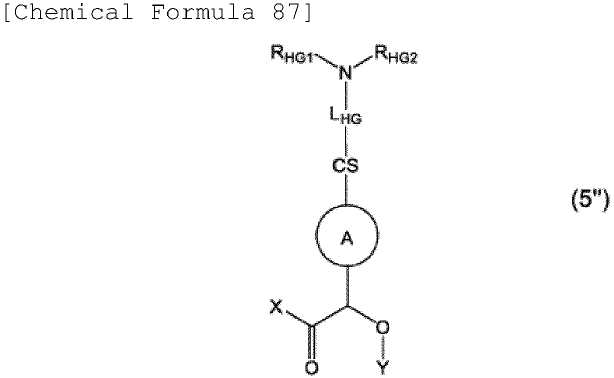 wherein
CS, ring A, X, and Y are the same as those represented in Formula (5), respectively,
R_{HG1} and R_{HG2} each independently represent a hydrogen atom, a hydrophilic group, or a monovalent group optionally comprising a hydrophilic group, and
L_{HG} represents a bond or a divalent group optionally comprising a hydrophilic group.

In these Formulas, R_{HG1}, R_{HG2}, and L_{HG} are as described above. Therefore, definitions, examples, and preferred examples of these elements and other elements associated therewith are the same as those described above.

Preferably, the compound represented by Formula (5) may be represented by the following Formula (5a): 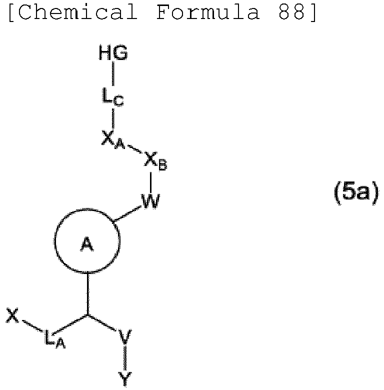 wherein
HG, ring A, V, L_{A}, X, and Y are the same as those represented in Formula (5), respectively,
L_{C} represents a bond or a divalent group,
X_{A} and X_{B} each independently represent an amino acid residue, and
W represents an oxygen atom, a sulfur atom, or an amino group (NH).

In Formula (5a), L_{C}, X_{A}, X_{B}, and W are as described above in Formula (1a). Therefore, definitions, examples, and preferred examples of these elements and other elements associated therewith are the same as those described above in Formula (1a).

Preferably, the compound represented by Formula (5a) may be represented by the following Formula (5b): 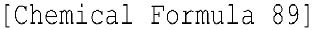 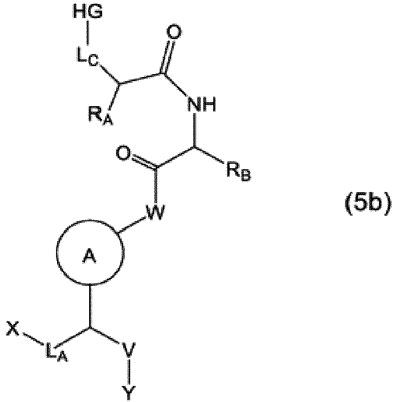 wherein
HG, ring A, V, L_{A}, X, and Y are the same as those represented in Formula (5), respectively,
L_{C} and W are the same as those represented in Formula (5a), respectively,
R_{A} represents a side chain of a valine residue, a phenylalanine residue, a threonine residue, a leucine residue, or an alanine residue, and
R_{B} represents a side chain of a citrulline residue, an alanine residue, a glutamic acid residue, a glutamine residue, a lysine residue, an arginine residue, a threonine residue, or a methionine residue.

In Formula (5b), R_{A} and R_{B} are as described above in Formula (1b). Therefore, definitions, examples, and preferred examples of these elements and other elements associated therewith are the same as those described above in Formula (1b).

Preferably, the compound represented by Formula (5b) may be represented by the following Formula (5c): 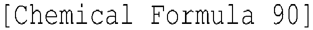 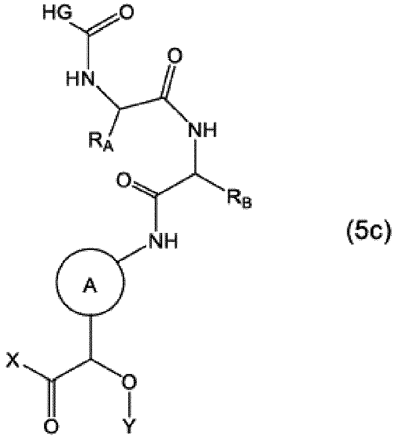 wherein
HG, ring A, X, and Y are the same as those represented in Formula (5), respectively, and
R_{A} and R_{B} are the same as those represented in Formula (5b), respectively.

Preferably, the compound represented by Formula (5c) may be represented by the following Formula (5d): 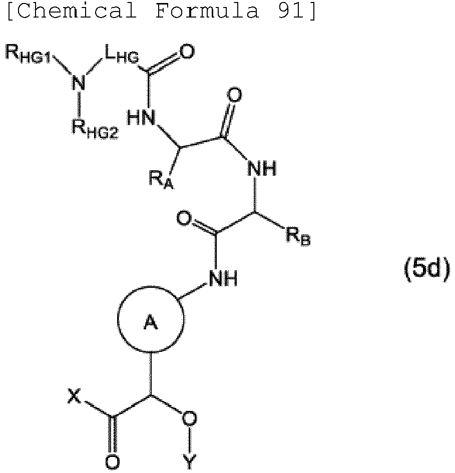 wherein
ring A, X, and Y are the same as those represented in Formula (5), respectively,
R_{A} and R_{B} are the same as those represented in Formula (5b), respectively,
L_{HG} represents a bond or a divalent group optionally comprising a hydrophilic group,
R_{HG1} and R_{HG2} each independently represent a hydrogen atom, a hydrophilic group, or a monovalent group optionally comprising a hydrophilic group, and
at least one hydrophilic group is comprised in one or more sites selected from the group consisting of L_{HG}, R_{HG1}, and R_{HC2}.

In Formula (5d), L_{HG}, R_{HG1}, and R_{HG2} are as described above in Formula (1d). Therefore, definitions, examples, and preferred examples of these elements and other elements associated therewith are the same as those described above in Formula (1d).

The compound represented by Formula (5) or a salt thereof is useful as, for example, synthetic intermediates of the conjugate and the antibody derivative of the present invention and another compound of the present invention.

The compound represented by Formula (5) or a salt thereof can be produced, for example, by causing a compound represented by the following Formula (5-1): 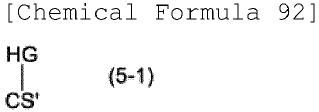 wherein
HG represents a hydrophilic group or a monovalent group comprising a hydrophilic group, and
CS' represents a reactive divalent group comprising a cleavable site, or
a salt thereof
to react with a compound represented by the following Formula (5-2) 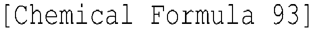 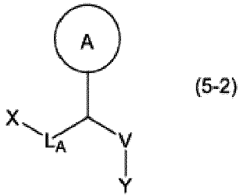 wherein
ring A represents a divalent aromatic ring group capable of reacting with CS' and optionally having a substituent, and
X and Y each independently represent a monovalent group, or
a salt thereof. Definitions, examples, and preferred examples of HG and the cleavable site and the divalent group in CS' in Formula (5-1) and ring A, L_{A}, V, X, and Y in Formula (5-2) are as described above. Such a reaction can be performed under a similar condition to the reaction condition described above for production of the compound having a first bioorthogonal functional group and a second bioorthogonal functional group or a salt thereof.

Preferably, the compound represented by the formula (5-1) may be represented by the following formula (5-1'): 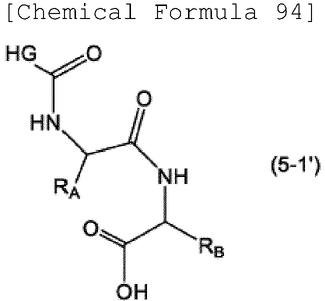 wherein
HG represents a hydrophilic group or a monovalent group comprising a hydrophilic group,
R_{A} represents a side chain of a valine residue, and
R_{B} represents a side chain of a citrulline residue or an alanine residue.

Preferably, the compound represented by the formula (5-2) may be represented by the following formula (5-2'): 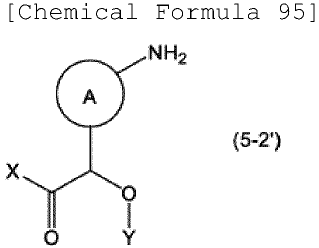 wherein
ring A represents a divalent aromatic ring group optionally having a substituent, and
X and Y each independently represent a monovalent group.

### (2) Compound having bioorthogonal functional group or bioorthogonal functional groups, represented by Formula (6), or salt thereof

The present invention also provides a compound having a bioorthogonal functional group or bioorthogonal functional groups, represented by the following Formula (6) : 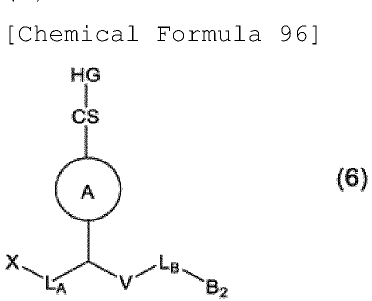 wherein
HG represents a hydrophilic group or a monovalent group comprising a hydrophilic group,
CS represents a divalent group comprising a cleavable site by cathepsin B,
ring A represents a divalent aromatic ring group optionally having a substituent wherein the divalent aromatic ring group constitutes a π electron conjugated system with the cleavable site,
V represents an oxygen atom, a sulfur atom, or an amino group (NH),
L_{A} and L_{B} each independently represent a divalent group,
X represents a monovalent group, and
B₂ represents a bioorthogonal functional group, or
a salt thereof.

In Formula (6), HG, CS, ring A, V, L_{A}, and L_{B} are as described above in Formula (1). Therefore, definitions, examples, and preferred examples of these elements and other elements associated therewith are the same as those described above in Formula (1).

The monovalent group represented by X is as described above.

The bioorthogonal functional group represented by B₂ is as described above.

Preferably, the compound represented by Formula (6) may be represented by the following Formula (6'): 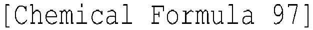 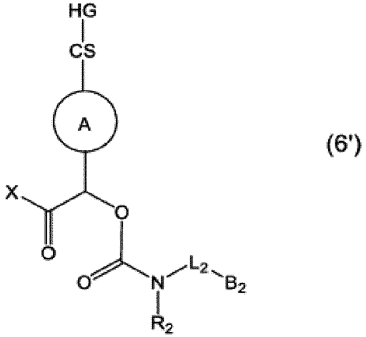 wherein
HG, CS, ring A, and X are the same as those represented in Formula (5), respectively,
R₂ represents a hydrogen atom or a monovalent group,
L₂ represents a divalent group, and
B₂ represents a bioorthogonal functional group, or
may be represented by the following Formula (6''): 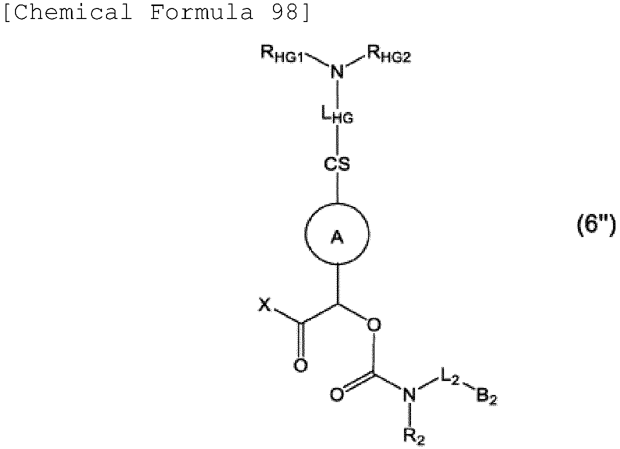 wherein
CS, ring A, X are the same as those represented in Formula (5), respectively,
R_{HG1} and R_{HG2} each independently represent a hydrogen atom, a hydrophilic group, or a monovalent group optionally comprising a hydrophilic group,
L_{HG} represents a bond or a divalent group optionally comprising a hydrophilic group,
R₂ represents a hydrogen atom or a monovalent group,
L₂ represents a divalent group, and
B₂ represents a bioorthogonal functional group.

In these Formulas, R_{HG1}, R_{HG2}, L_{HG}, R₂, L₂, and B₂ are as described above. Therefore, definitions, examples, and preferred examples of these elements and other elements associated therewith are the same as those described above.

Preferably, the compound represented by Formula (6) may be represented by the following Formula (6a): 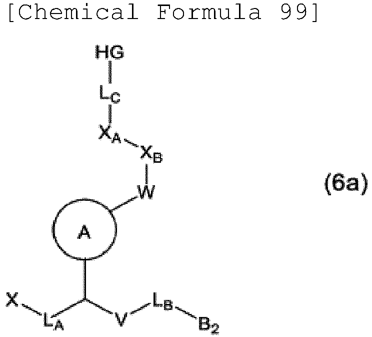 wherein
HG, ring A, V, L_{A}, X, L_{B}, and B₂ are the same as those represented in Formula (6), respectively,
L_{C} represents a bond or a divalent group,
X_{A} and X_{B} each independently represent an amino acid residue, and
W represents an oxygen atom, a sulfur atom, or an amino group (NH).

In Formula (6a), L_{C}, X_{A}, X_{B}, and W are as described above in Formula (1a). Therefore, definitions, examples, and preferred examples of these elements and other elements associated therewith are the same as those described above in Formula (1a).

Preferably, the compound represented by Formula (6a) may be represented by the following Formula (6b): 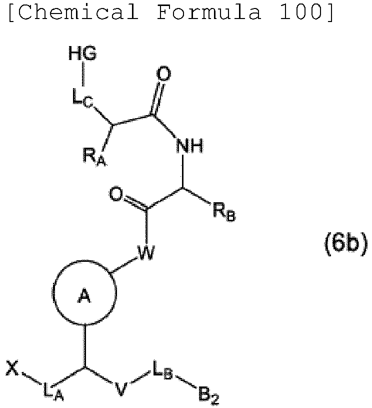 wherein
HG, ring A, V, L_{A}, X, L_{B}, and B₂ are the same as those represented in Formula (6), respectively,
L_{C} and W are the same as those represented in Formula (6a), respectively,
R_{A} represents a side chain of a valine residue, a phenylalanine residue, a threonine residue, a leucine residue, or an alanine residue, and
R_{B} represents a side chain of a citrulline residue, an alanine residue, a glutamic acid residue, a glutamine residue, a lysine residue, an arginine residue, a threonine residue, or a methionine residue.

In Formula (6b), R_{A} and R_{B} are as described above in Formula (1b). Therefore, definitions, examples, and preferred examples of these elements and other elements associated therewith are the same as those described above in Formula (1b).

Preferably, the compound represented by Formula (6b) may be represented by the following Formula (6c): 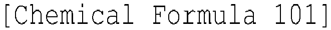 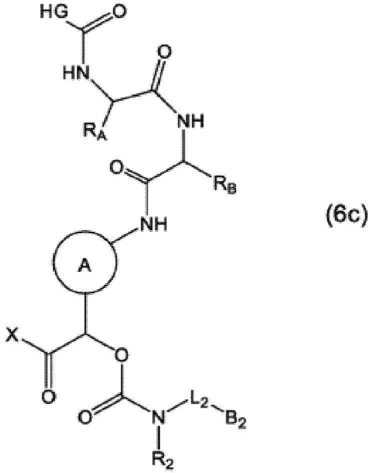 wherein
HG, ring A, X, and B₂ are the same as those represented in Formula (6), respectively,
R_{A} and R_{B} are the same as those represented in Formula (6b), respectively,
R₂ represents a hydrogen atom or a monovalent group, and
L₂ represents a divalent group.

In Formula (6c), R₂ and L₂ are as described above in Formula (1c). Therefore, definitions, examples, and preferred examples of these elements and other elements associated therewith are the same as those described above in Formula (1c).

Preferably, the compound represented by Formula (6c) may be represented by the following Formula (6d): 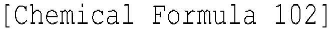 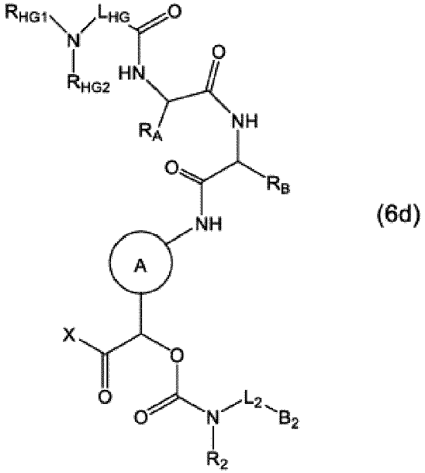 wherein
ring A, X, and B₂ are the same as those represented in Formula (6), respectively,
R_{A} and R_{B} are the same as those represented in Formula (6b), respectively,
R₂ and L₂ are the same as those represented in Formula (6c), respectively,
L_{HG} represents a bond or a divalent group optionally comprising a hydrophilic group,
R_{HG1} and R_{HG2} each independently represent a hydrogen atom, a hydrophilic group, or a monovalent group optionally comprising a hydrophilic group, and
at least one hydrophilic group is comprised in one or more sites selected from the group consisting of L_{HG}, R_{HG1}, and R_{HC2}.

In Formula (6d), L_{HG}, R_{HG1}, and R_{HG2} are as described above in Formula (1d). Therefore, definitions, examples, and preferred examples of these elements and other elements associated therewith are the same as those described above in Formula (1d).

The compound represented by Formula (6) or a salt thereof is useful as, for example, synthetic intermediates of the conjugate and the antibody derivative of the present invention and a predetermined compound of the present invention. Such a compound or a salt thereof are also useful, for example, for derivatization of a functional substance.

The compound represented by Formula (6) or a salt thereof can be produced, for example, by causing the compound represented by Formula (5) or a salt thereof to react with an appropriate compound having L_{B}-B₂ (for example, by causing the compound represented by Formula (5) or a salt thereof to react with bis(4-nitriphenyl) carbonate and N,N-diisopropylethylamine (DIPEA), and then causing the resulting product to react with a compound represented by B₂-L₂-NH-R₂ (FIGS. 4 and 5). Definitions, examples, and preferred examples of B₂, L₂, and R₂ are as described above. Such a reaction can be performed under a similar condition to the reaction condition described above for production of the compound having a first bioorthogonal functional group and a second bioorthogonal functional group or a salt thereof.

### (3) Compound having bioorthogonal functional group or bioorthogonal functional groups, represented by Formula (7) or salt thereof

The present invention also provides a compound having a bioorthogonal functional group or bioorthogonal functional groups, represented by the following Formula (7) : 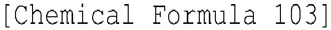 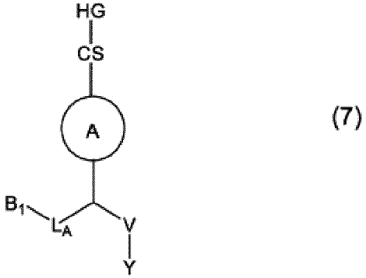 wherein
HG represents a hydrophilic group or a monovalent group comprising a hydrophilic group,
CS represents a divalent group comprising a cleavable site by cathepsin B,
ring A represents a divalent aromatic ring group optionally having a substituent wherein the divalent aromatic ring group constitutes a π electron conjugated system with the cleavable site,
V represents an oxygen atom, a sulfur atom, or an amino group (NH),
L_{A} represents a divalent group,
Y represents a monovalent group, and
B₁ represents a bioorthogonal functional group, or
a salt thereof.

In Formula (7), HG, CS, ring A, V, and L_{A} are as described above in Formula (1). Therefore, definitions, examples, and preferred examples of these elements and other elements associated therewith are the same as those described above in Formula (1).

The monovalent group represented by Y is as described above.

The bioorthogonal functional group represented by B₁ is as described above.

Preferably, the compound represented by Formula (7) may be represented by the following Formula (7'): 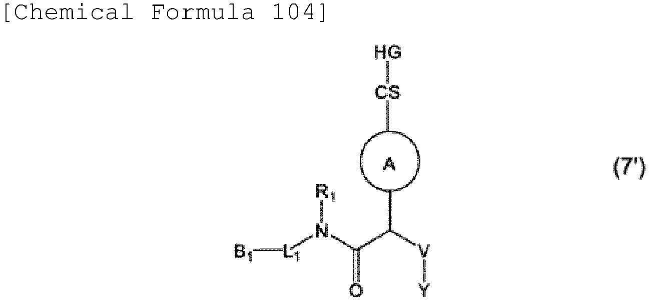 wherein
HG, CS, ring A, V, and Y are the same as those represented in Formula (5), respectively,
R₁ represents a hydrogen atom or a monovalent group,
L₁ represents a divalent group, and
B₁ represents a bioorthogonal functional group, or
may be represented by the following Formula (7''): 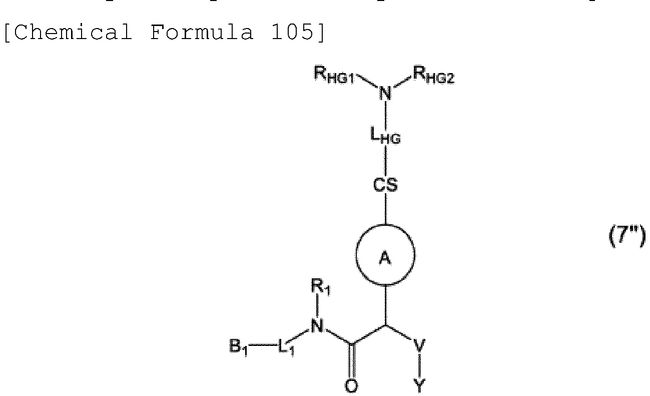 wherein
CS, ring A, V, and Y are the same as those represented in Formula (5), respectively,
R_{HG1} and R_{HG2} each independently represent a hydrogen atom, a hydrophilic group, or a monovalent group optionally comprising a hydrophilic group,
L_{HG} represents a bond or a divalent group optionally comprising a hydrophilic group,
R₁ represents a hydrogen atom or a monovalent group,
L₁ represents a divalent group, and
B₁ represents a bioorthogonal functional group.

In these Formulas, R_{HG1}, R_{HG2}, L_{HG}, R₁, L₁, and B₁ are as described above. Therefore, definitions, examples, and preferred examples of these elements and other elements associated therewith are the same as those described above.

Preferably, the compound represented by Formula (7) may be represented by the following Formula (7a): 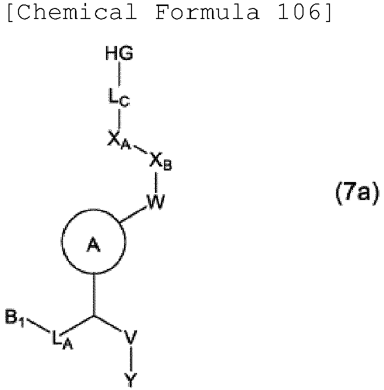 wherein
HG, ring A, V, L_{A}, Y, and B₁ are the same as those represented in Formula (7), respectively,
L_{C} represents a bond or a divalent group,
X_{A} and X_{B} each independently represent an amino acid residue, and
W represents an oxygen atom, a sulfur atom, or an amino group (NH).

In Formula (7a), L_{C}, X_{A}, X_{B}, and W are as described above in Formula (1a). Therefore, definitions, examples, and preferred examples of these elements and other elements associated therewith are the same as those described above in Formula (1a).

Preferably, the compound represented by Formula (7a) may be represented by the following Formula (7b): 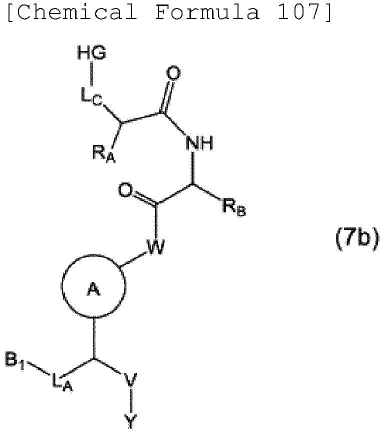 wherein
HG, ring A, V, L_{A}, Y, and B₁ are the same as those represented in Formula (7), respectively,
L_{C}, X_{A}, X_{B}, and W are the same as those represented in Formula (7a), respectively,
R_{A} represents a side chain of a valine residue, a phenylalanine residue, a threonine residue, a leucine residue, or an alanine residue, and
R_{B} represents a side chain of a citrulline residue, an alanine residue, a glutamic acid residue, a glutamine residue, a lysine residue, an arginine residue, a threonine residue, or a methionine residue.

In Formula (7b), R_{A} and R_{B} are as described above in Formula (1b). Therefore, definitions, examples, and preferred examples of these elements and other elements associated therewith are the same as those described above in Formula (1b).

Preferably, the compound represented by Formula (7b) may be represented by the following Formula (7c): 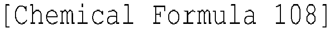 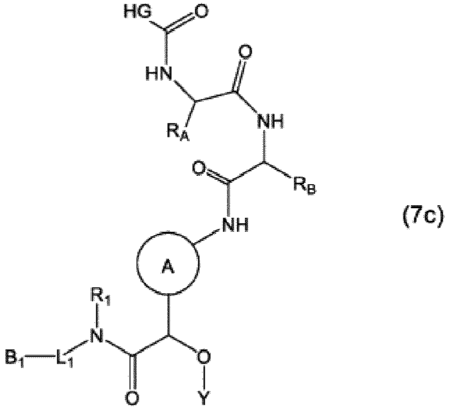 wherein
HG, ring A, Y, and B₁ are the same as those represented in Formula (7), respectively,
R_{A} and R_{B} are the same as those represented in Formula (7b), respectively,
R₁ represents a hydrogen atom or a monovalent group, and
L₁ represents a divalent group.

In Formula (7c), R₁ and L₁ are as described above in Formula (1c). Therefore, definitions, examples, and preferred examples of these elements and other elements associated therewith are the same as those described above in Formula (1c).

Preferably, the compound represented by Formula (7c) may be represented by the following Formula (7d): 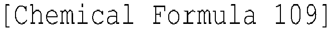 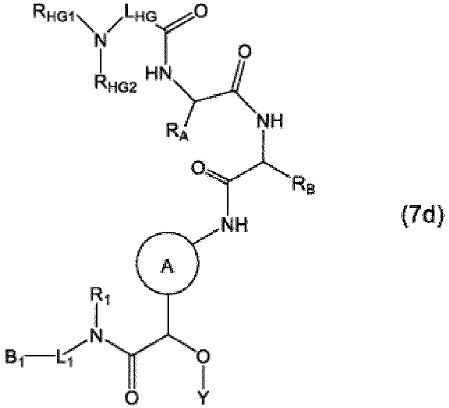 wherein
ring A, Y, and B₁ are the same as those represented in Formula (7), respectively,
R_{A} and R_{B} are the same as those represented in Formula (7b), respectively,
R₁ and L₁ are the same as those represented in Formula (7c), respectively,
L_{HG} represents a bond or a divalent group optionally comprising a hydrophilic group,
R_{HG1} and R_{HG2} each independently represent a hydrogen atom, a hydrophilic group, or a monovalent group optionally comprising a hydrophilic group, and
at least one hydrophilic group is comprised in one or more sites selected from the group consisting of L_{HG}, R_{HG1}, and R_{HG2}.

In Formula (7d), L_{HG}, R_{HG1}, and R_{HG2} are as described above in Formula (1d). Therefore, definitions, examples, and preferred examples of these elements and other elements associated therewith are the same as those described above in Formula (1d).

The compound represented by Formula (7) or a salt thereof is useful as, for example, synthetic intermediates of the conjugate and the antibody derivative of the present invention and a predetermined compound of the present invention. Such a compound or a salt thereof is also useful, for example, for derivatization of any substance such as a biomolecule (e.g., a protein such as an antibody, a saccharide, a nucleic acid, or a lipid).

The compound represented by Formula (7) or a salt thereof can be produced, for example, by causing the compound represented by Formula (7) or a salt thereof to react with an appropriate compound having B₁ (e.g., a compound represented by B₁-L₁-NH-R₁) (FIGS. 4 and 5) . Definitions, examples, and preferred examples of B₁, L₁, and R₁ are as described above. Such a reaction can be performed under a similar condition to the reaction condition described above for production of the compound having a first bioorthogonal functional group and a second bioorthogonal functional group or a salt thereof.

Production of the compound represented by Formula (5), (6), or (7) or a salt thereof can be confirmed by, for example, NMR, HPLC, or mass spectrometry, depending on a specific raw material thereof and the molecular weight of a product. Such a compound or a salt thereof can be appropriately purified by any purification method such as chromatography (e.g., gel filtration chromatography, ion exchange chromatography, reverse phase column chromatography, high performance liquid chromatography, or affinity chromatography).

### Examples

Next, the present invention will be described in more detail by describing Examples, but the present invention is not limited to the following Examples.

The following peptides, Ac-Glu(OtBu)-Val-Cit-OH, Z-Glu(OtBu)-Val-Cit-OH, Ac-Glu(OtBu)-Glu(OtBu)-Val-Cit-OH, and SCA(OtBu)-Glu(OtBu)-Val-Cit-OH in Example 1 were all prepared by methods similar to each other. Note that SCA(OtBu) stands for mono-tert-butyl succinate, and SCA stands for succinic acid.

By peptide solid phase synthesis using Cl-TCP(Cl) ProTide Resin (CEM) by a Fmoc method, a compound in which an N-terminal was capped with an acetyl group and a compound in which an N-terminal was capped with succinic acid were prepared, and stirred overnight with a solution of 20% HFIP/dichloromethane to perform cutting out from the resin while an amino acid side chain was protected. The resin was removed by filtration, and the solution was concentrated. Thereafter, the concentrate was purified by preparative HPLC to obtain a peptide as a product.

### Ac-Glu(OtBu)-Val-Cit-OH

¹H NMR(400 MHz,DMSO-d₆)δ12.50(brs,1H),8.22(d,J=7.2Hz,1H),8.05(d,J=8.0Hz,1H),7.6 9(d,J=8.8Hz,1H),5.95-5.93(m,1H),5.38(brs,2H),4.33-4.27(m,1H),4.22-4.18(m,1H),4.15-4.10(m,1H),2.96-2.95(m,2H),2.25-2.19(m,2H),2.00-1.93(m,1H),1.89-1.80(m,4H),1.73-1.66(m,2H),1.61-1.51(m,1H),1.46-1.34(m,11H),0.86(d,J=6.8Hz,3H),0.83(d,J=6.8Hz,3H).

MS(ESI)m/z:502.30[M+H]⁺

### Z-Glu(OtBu)-Val-Cit-OH

¹H NMR(400 MHz,DMSO-d₆)δ12.50(brs,1H),7.93-7.37(m,8H),6.05-6.00(m,1H),5.44(brs,2H),5.08(s,2H),4.17-3.81(m,3H),3.00-2.90(m,2H),2.31-2.27(m,2H),2.10-1.34(m,16H),0.89-0.83(m,6H).

MS(ESI)m/z:594.30[M+H]⁺

### Ac-Glu(OtBu)-Glu(OtBu)-Val-Cit-OH

¹H NMR(400 MHz,DMSO-d₆)δ12.50(brs,1H),8.21(d,J=7.2Hz,1H),8.08(d,J=8.0Hz,1H),8.0 4(d,J=8.0Hz,1H),7.68(d,J=8.0Hz,1H),5.95(brs,1H),5.37(brs,2H ),4.32-4.19(m,3H),4.16-4.11(m,1H),2.98-2.94(m,2H),2.27-2.13(m,4H),2.00-1.79(m,5H),1.76-1.52(m,5H),1.42-1.36(m,20H),0.86(d,J=6.8Hz,3H),0.82(d,J=6.8Hz,3H).

MS(ESI)m/z:687.35[M+H]⁺

### SCA(OtBu)-Glu(OtBu)-Val-Cit-OH

¹H NMR(400 MHz,DMSO-d₆)δ12.70(brs,1H),8.21(d,J=7.2Hz,1H),8.05(d,J=8.0Hz,1H),7.6 8(d,J=8.8Hz,1H),5.96(brs,1H),5.25(brs,2H),4.35-4.30(m,1H),4.21-4.18(m,1H),4.15-4.10(m,1H),2.98-2.94(m,2H),2.40-2.28(m,4H),2.24-2.18(m,2H),2.01-1.93(m,1H),1.90-1.81(m,1H),1.73-1.63(m,2H),1.61-1.51(m,1H),1.40-1.31(m,20H),0.86(d,J=6.8Hz,3H),0.83(d,J=6.8Hz,3H).

MS(ESI)m/z:616.30[M+H]⁺

### Ac-Glu(OtBu)-Val-Ala-OH

¹H NMR(400 MHz,DMSO-d₆)δ12.50(brs,1H),8.25(d,J=6.8Hz,1H),8.03(d,J=8.0Hz,1H),7.6 8(d,J=9.2Hz,1H),4.33-4.27(m,1H),4.22-4.16(m,2H),2.23-2.18(m,2H),1.99-1.94(m,1H),1.89-1.80(m,4H),1.73-1.65(m,1H),1.39(s,9H),1.27(d,J=7.2Hz,3H),0.87(d,J=6.8Hz,3H) ,0.83(d,J=6.8Hz,3H).

MS(ESI)m/z:416.20[M+H]⁺

### Example 1: Synthesis of Linker-payload mimic (1-1) Synthesis of Linker-payload mimic (1) Linker-payload mimic (1) was synthesized as follows.

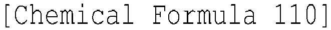

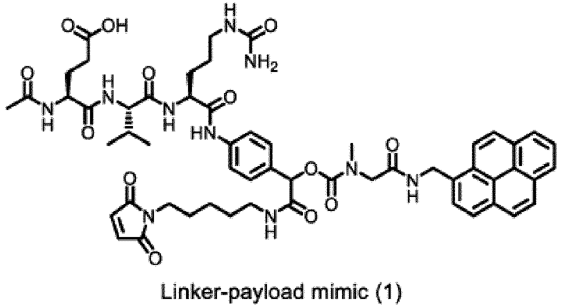

### (1-1-1) Synthesis of alcohol (2)

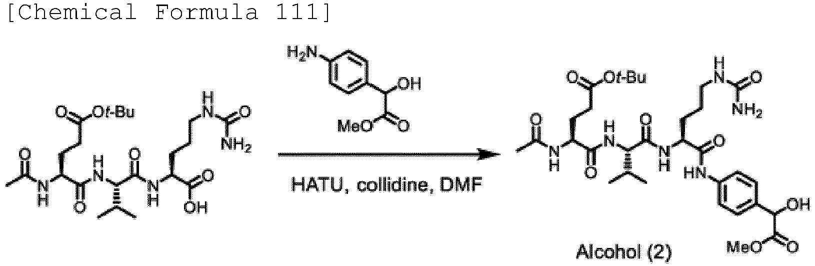

Ac-Glu(OtBu)-Val-Cit-OH (19.9 mg, 39.7 µmol) was dissolved in N,N-dimethylformamide (400 µL), and 1-[bis(dimethylamino) methylene]-1H-1,2,3,-triazolo[4,5-b] pyridinium 3-oxide hexafluorophosphate (18.1 mg, 47.6 µmol) and 2,4,6-trimethylpyridine (6.27 µL, 47.6 µmol) were added thereto. The mixture was stirred at room temperature for ten minutes. Subsequently, methyl 4-aminomandelate (8.63 mg, 47.6 µmol) was added thereto, and the mixture was stirred at room temperature for 21.5 hours, and then purified by reverse phase preparative chromatography. A fraction comprising a product was collected and concentrated under reduced pressure to remove acetonitrile. The residue was freeze-dried to obtain the alcohol (2) (28.5 mg, quant).

¹H NMR(400 MHz,DMSO-d₆)δ9.95(s,1H), 8. 07 (d, J=7.4 Hz,1H),7.99(d,J=8.0 Hz,1H),7.66(d,J=8.4 Hz,1H),7.50(d,J=8.4 Hz,2H),7.25(d,J=8.4 Hz,2H),5.92(brs,1H),5.36(brs,2H),5.01(s,1H),4.34-4.29(m,1H),4.26-4.20(m,1H),4.14-4.10(m,1H),3.53(s,3H),3.00-2.83(m,2H),2.18-2.13(m,2H),1.94-1.89(m,2H),1.84-1.23(m,17H),0.79(d,J=6.8Hz,3H),0.75(d,J=6.8Hz,3H).

MS(ESI)m/z:665.30[M+H]⁺

### (1-1-2) Synthesis of pyrene (3)

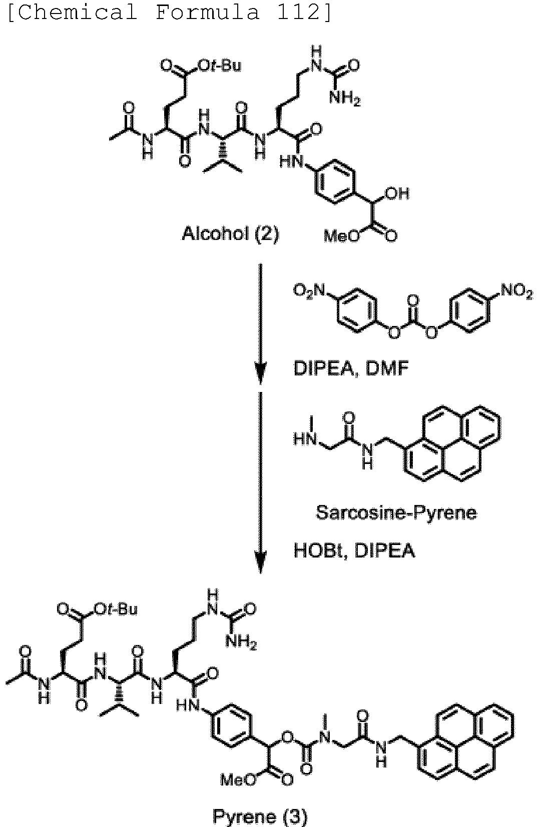

The alcohol (2) (28.5 mg) obtained in (1-1-1) was dissolved in N,N-dimethylformamide (430 µL), and the solution was stirred under ice cooling for five minutes. Thereafter, bis(4-nitrophenyl) carbonate (26.6 mg, 85.7 µmol) and N,N-diisopropylethylamine (11.1 µL, 64.4 µmol) were added thereto, and the mixture was stirred at room temperature for 1.5 hours. As a result of tracking the reaction by LCMS, a remaining raw material was found. Therefore, bis(4-nitrophenyl) carbonate (13.3 mg, 42.9 µmol) and N,N-diisopropylethylamine (5.54 µL, 32.2 µmol) were further added thereto, and the mixture was stirred at room temperature for five hours. Thereafter, the mixture was ice-cooled. Sarcosin-Pyrene (64.9 mg, 215 µmol), 1-hydroxybenzotriazole (8.7 mg, 64 µmol), and N,N-diisopropylethylamine (57.2 µL, 333 µmol) were added thereto, and the mixture was stirred at room temperature for 16 hours. After the reaction, purification was performed by reverse phase preparative chromatography. A fraction comprising a product was collected and concentrated under reduced pressure to remove acetonitrile. The residue was freeze-dried to obtain the pyrene (3) (26.1 mg, 26.3 µmol).

¹H NMR(400 MHz,DMSO-d₆)δ10.09-10.07(m,1H),8.63-8.60(m,1H),8.33-7.65(m,13H),7.60-7.57(m,2H),7.37-7.32(m,2H),5.94-5.91(m,1H),5.72-5.70(m,1H),5.37(brs,2H),4.98-4.96(m,1H),4.93-4.00(m,4H),3.85-3.75(m,1H),3.56-3.55(m,3H),2.97-2.87(m,5H),2.17-2.13(m,2H),1.93-1.17(m,19H),0.80-0.73(m,6H).

MS(ESI)m/z:993.40[M+H]⁺

### (1-1-3) Synthesis of pyrene (4)

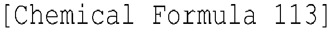

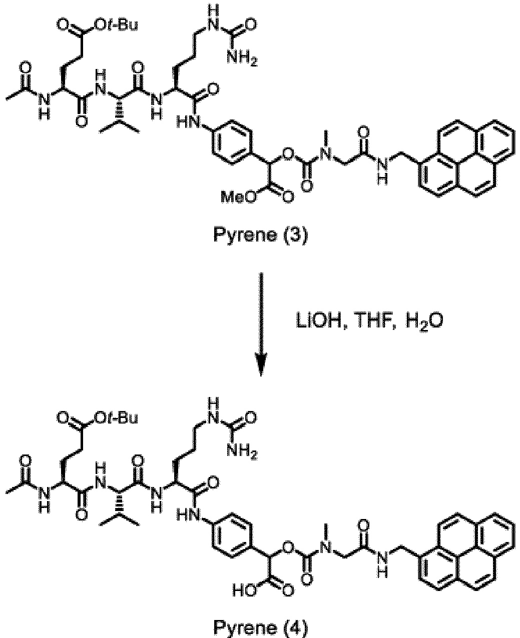

Pyrene (3) (10.8 mg, 10.9 µmol) was dissolved in tetrahydrofuran (700 µL) and water (400 µL), and the solution was stirred under ice cooling for five minutes. Thereafter, a 1 M lithium hydroxide aqueous solution (109 µL, 109 µmol) was added thereto, and the mixture was stirred at room temperature for one hour. After completion of the reaction, the pH was adjusted to about 6 using 0.1 M hydrochloric acid, and purification was performed by reverse phase preparative chromatography. A fraction comprising a product was collected and concentrated under reduced pressure to remove acetonitrile. The residue was freeze-dried to obtain the pyrene (4) (4.5 mg, 4.6 µmol).

¹H NMR(400 MHz,DMSO-d₆)δ13.05(brs,1H),10.08-10.05(m,1H),8.62-8.59(m,1H),8.33-7.56(m,15H),7.37-7.35(m,2H),5.92(brs,1H),5.61-5.60(m,1H),5.36(brs,2H),4.98-4.03(m,5H),3.88-3.74(m,1H),2.99-2.83(m,5H),2.15-2.13(m,2H),1.93-1.14(m,19H),0.84-0.73(m,6H).

MS(ESI)m/z:979.40[M+H]⁺

### (1-1-4) Synthesis of pyrene (5)

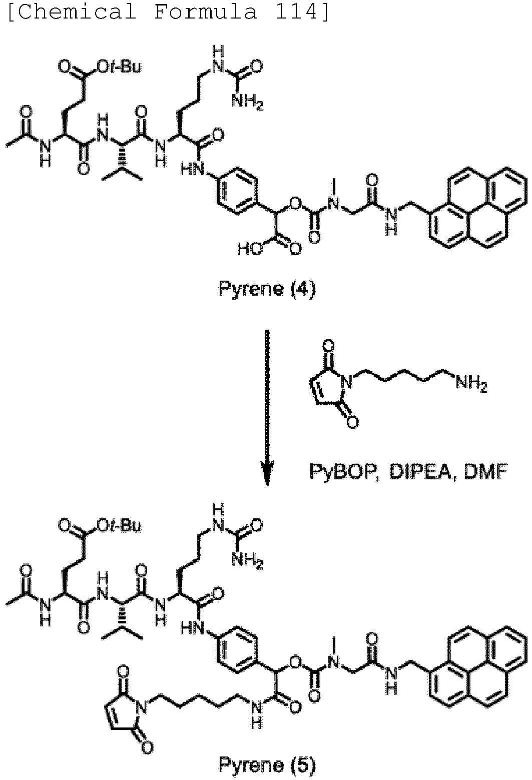

Pyrene (4) (3.7 mg, 3.8 µmol) was dissolved in N,N-dimethylformamide (400 µL). Thereafter, the solution was ice-cooled, and N,N-diisopropylethylamine (1.9 µL, 11 µmol) and 1H-benzotriazol-1-yloxy tripyrrolidinophosphonium hexafluorophosphate (2.9 mg, 5.6 µmol) were added thereto. Next, N-(5-aminopentyl) maleimide hydrochloride (1.3 mg, 5.7 µmol) was added thereto, the temperature was returned to room temperature, and the mixture was stirred for two hours. After completion of the reaction, purification was performed by reverse phase preparative chromatography. A fraction comprising a product was collected and concentrated under reduced pressure to remove acetonitrile. Thereafter, the resulting residue was freeze-dried to obtain pyrene (5) (1.3 mg, 1.1 µmol).

¹H NMR(400 MHz,DMSO-d₆)δ10.02-9.99(m,1H),8.85-7.88(m,14H),7.67-7.65(m,1H),7.60-7.51(m,2H),7.33-7.27(m,2H),6.91-6.87(m,2H),5.92-5.91(m,1H),5.63-5.62(m,1H),5.36(brs,2H), 5.07-4.92(m,2H),4.35-3.76(m,5H),3.18-3.14(m,1H),2.99-2.83(m,7H),2.17-2.13(m,2H),1.95-1.89(m,1H),1.85-1.72(m,4H),1.66-1.45(m,3H),1.40-1.17(m,15H),1.05-1.01(m,2H),0.83-0.73(m,6H).

MS(ESI)m/z:1143.45[M+H]⁺

### (1-1-5) Synthesis of Linker-payload mimic (1)

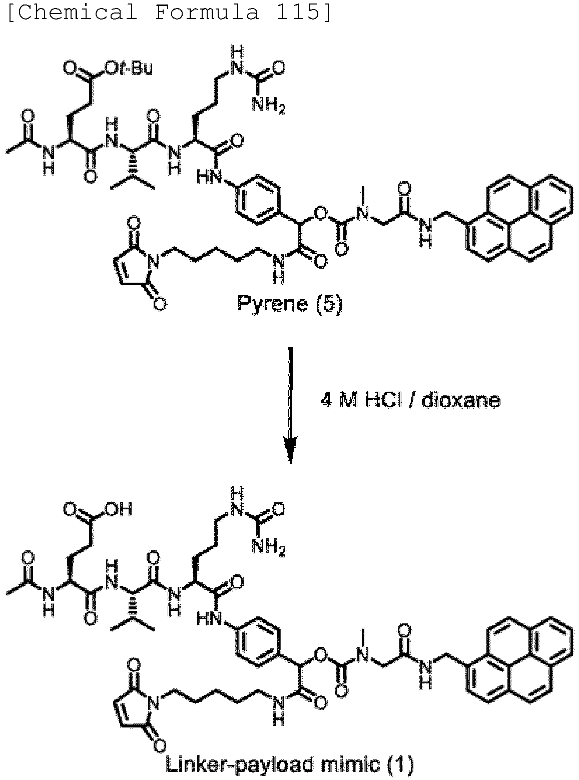

To pyrene (5) (2.2 mg, 1.9 µmol), 1,4-dioxane (380 µL) and a 4 M hydrogen chloride/dioxane solution (95 µL, 380 µmol) were sequentially added, and the mixture was stirred at room temperature for four hours. After ice cooling, N,N-diisopropylethylamine (71.8 µL, 418 µmol) was added thereto, and the mixture was stirred at room temperature for 10 minutes. The reaction solution was purified by reverse phase preparative chromatography, a fraction comprising a product was collected and concentrated under reduced pressure to remove acetonitrile. Thereafter, the residue was freeze-dried to obtain Linker-payload mimic (1) (2.1 mg, 1.9 µmol).

¹H NMR(400 MHz,DMSO-d₆)δ12.06(brs,1H),10.03-10.00(m,1H),8.84-7.88(m,14H),7.67-7.64(m,1H),7.56-7.51(m,2H),7.33-7.27(m,2H),6.90-6.87(m,2H),5.92-5.90(m,1H),5.63-5.61(m,1H),5.36(brs,2H),5.08-4.96(m,2H),4.35-3.76(m,5H),3.18-3.14(m,1H),2.97-2.83(m,7H),2.20-2.16(m,2H),1.93-1.88(m,1H),1.81-1.78(m,4H),1.69-1.53(m,3H),1.35-1.17(m,6H),1.08-1.01(m,2H),0.81-0.73(m,6H).

MS(ESI)m/z:1087.45[M+H]⁺

### (1-2) Synthesis of Linker-payload mimic (6) Linker-payload mimic (6) was synthesized as follows.

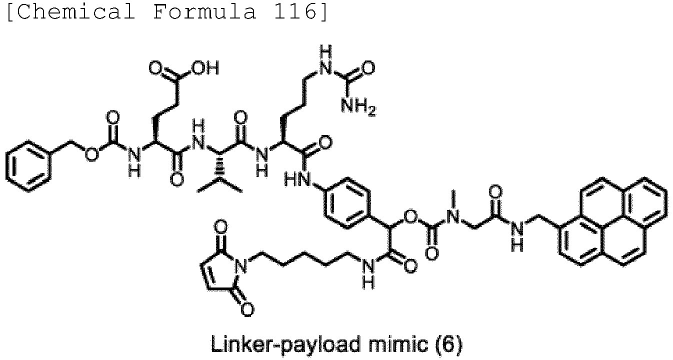

### (1-2-1) Synthesis of alcohol (7)

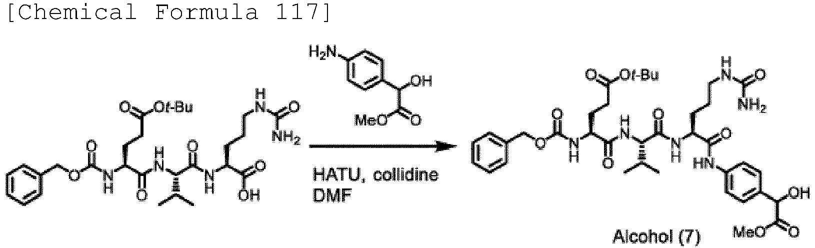

Z-Glu(t-Bu)-Val-Cit-OH (50.0 mg, 84.3 µmol) was dissolved in N,N-dimethylformamide (1.5 mL), and 1-[bis(dimethylamino) methylene]-1H-1,2,3,-triazolo[4,5-b] pyridinium 3-oxide hexafluorophosphate (38.4 mg,101 µmol) and 2,4,6-trimethylpyridine (13.3 µL, 101 µmol) were added thereto. The mixture was stirred at room temperature for ten minutes. Subsequently, methyl 4-aminomandelate (18.3 mg, 101 µmol) was added thereto, and the mixture was stirred at room temperature for 16 hours, and then purified by reverse phase preparative chromatography. A fraction comprising a product was collected and concentrated under reduced pressure to remove acetonitrile. The residue was freeze-dried to obtain the alcohol (7) (49.1 mg, 64.9 µmol).

¹H NMR(400 MHz,DMSO-d₆)δ10.04-9.95(m,1H)8.40-7.28(m,12H),6.00-5.97(m,1H),5.43(brs,2H),5.08-4.97(m,3H),4.43-4.37(m,1H),4.24-4.20(m,1H),4.16-4.05(m,1H),3.60-3.59(m,3H),3.04-2.91(m,2H),2.26-2.20(m,2H),2.03-1.22(m,16H),0.88-0.78(m,6H).

MS(ESI)m/z:757.30[M+H]⁺

### (1-2-2) Synthesis of pyrene (8)

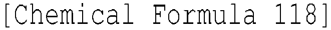

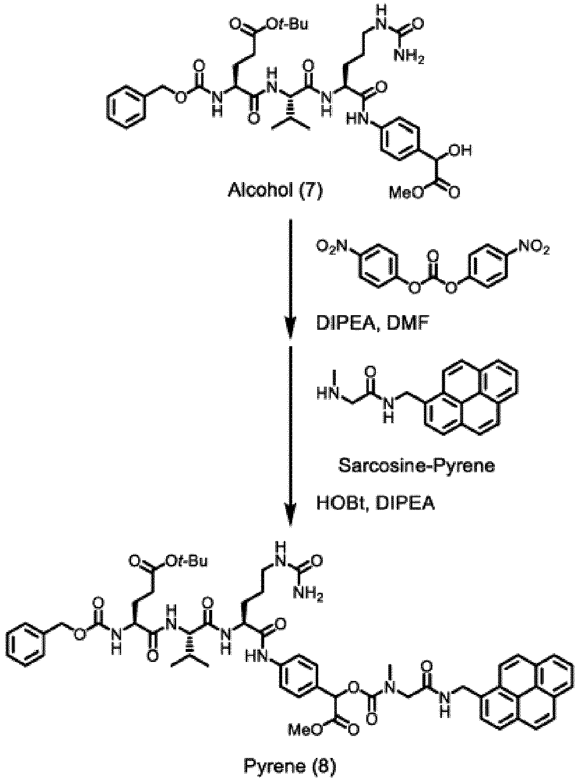

Alcohol (7) (44.6 mg, 58.9 µmol) was dissolved in N,N-dimethylformamide (650 µL), and the solution was stirred under ice cooling for five minutes. Thereafter, bis(4-nitrophenyl) carbonate (53.8 mg, 177 µmol) and N,N-diisopropylethylamine (22.5 µL, 133 µmol) were added thereto, and the mixture was stirred at room temperature for four hours. Thereafter, the mixture was ice-cooled. Sarcosin-Pyrene (89.1 mg, 295 µmol), 1-hydroxybenzotriazole (11.9 mg, 88.4 µmol), and N,N-diisopropylethylamine (77.7 µL, 457 µmol) were added thereto, and the mixture was stirred at room temperature for 18 hours. After the reaction, purification was performed by reverse phase preparative chromatography. A fraction comprising a product was collected and concentrated under reduced pressure to remove acetonitrile. The residue was freeze-dried to obtain the pyrene (8) (49.2 mg, 45.3 µmol).

¹H NMR(400MHz,DMSO-d6)δ10.11-9.97(m,1H),8.64-8.60(m,1H),8.36-7.95(m,11H),7.82-7.74(m,1H),7.65-7.57(m,2H),7.49-7.19(m,8H),5.91-5.90(m,1H),5.72-5.70(m,1H),5.36(brs,2H),4.98-4.86(m,4H),4.41-4.00(m,3H),3.85-3.75(m,1H),3.56-3.54(m,3H),3.00-2.82(m,5H),2.19-2.12(m,2H),1.92-1.17(m,16H),0.80-0.73(m,6H).

MS(ESI)m/z:1085.45[M+H]⁺

### (1-2-3) Synthesis of pyrene (9)

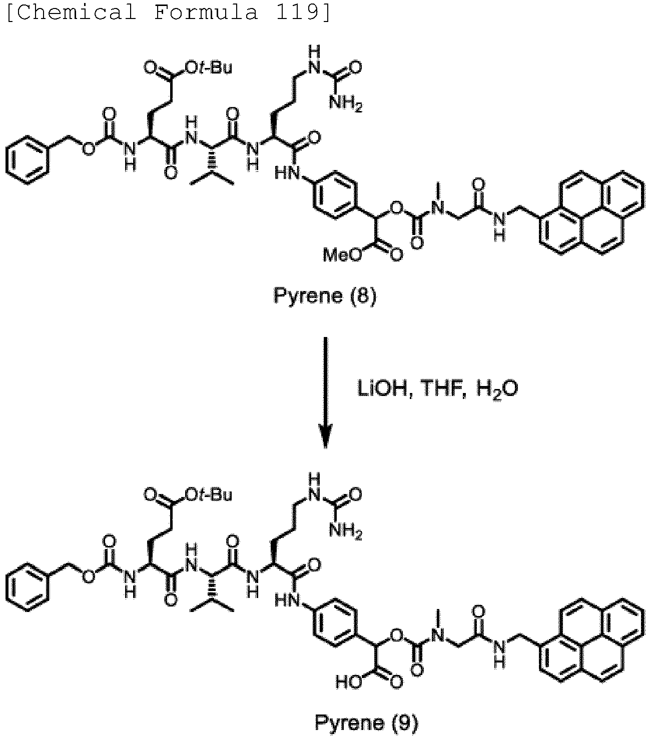

Pyrene (8) (44.8 mg, 41.3 µmol) was dissolved in tetrahydrofuran (3.75 mL) and water (1.25 mL), and the solution was stirred under ice cooling for five minutes. Thereafter, lithium hydroxide monohydrate (8.7 mg, 210 µmol) was added thereto, and the mixture was stirred at room temperature for four hours. After completion of the reaction, the pH was adjusted to about 6 using 0.1 M hydrochloric acid, and purification was performed by reverse phase preparative chromatography. A fraction comprising a product was collected and concentrated under reduced pressure to remove acetonitrile. The residue was freeze-dried to obtain the pyrene (9) (18.4 mg, 17.2 µmol).

¹H NMR(400 MHz,DMSO-d₆)δ13.02(brs,1H),10.09-9.97(m,1H),8.62-8.59(m,1H),8.32-7.78(m,12H),7.63-7.44(m,3H),7.37-7.35(m,2H),7.29-7.20(m,5H),5.91(brs,1H),5.61-5.60(m,1H),5.36(brs,2H),4.98-4.86(m,4H),4.45-4.30(m,1H),4.24-4.15(m,1H),4.07-4.02(m,1H),3.84-3.74(m,1H),2.97-2.82(m,5H),2.19-2.12(m,2H),1.93-1.11(m,16H),0.80-0.72(m,6H).

MS(ESI)m/z:1071.45[M+H]⁺

### (1-2-4) Synthesis of pyrene (10)

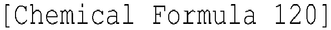

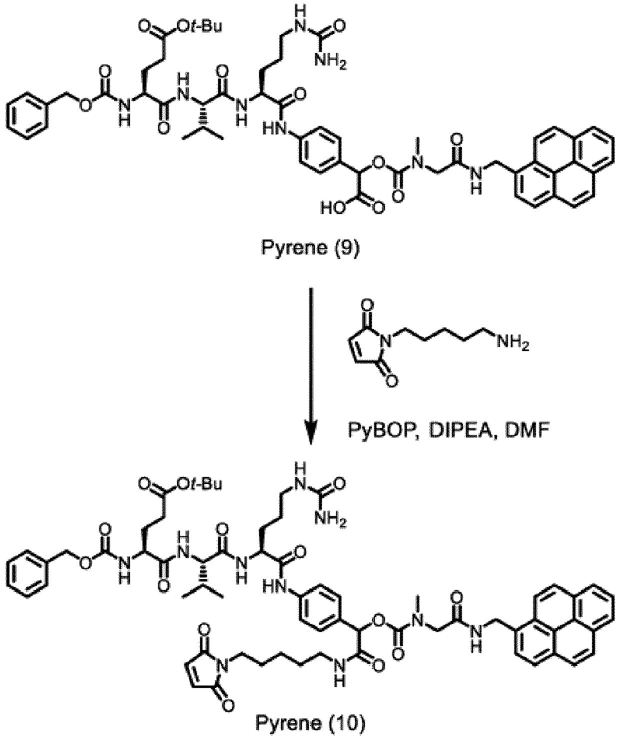

Pyrene (9) (15.6 mg, 14.6 µmol) was dissolved in N,N-dimethylformamide (1.0 mL). Thereafter, the solution was ice-cooled, and N,N-diisopropylethylamine (5.0 µL, 29 µmol) and 1H-benzotriazol-1-yloxy tripyrrolidinophosphonium hexafluorophosphate (11.4 mg, 21.9 µmol) were added thereto. Next, N-(5-aminopentyl) maleimide hydrochloride (4.8 mg, 22 µmol) was added thereto, the temperature was returned to room temperature, and the mixture was stirred for three hours. After completion of the reaction, purification was performed by reverse phase preparative chromatography. A fraction comprising a product was collected and concentrated under reduced pressure to remove acetonitrile. Thereafter, the resulting residue was freeze-dried to obtain pyrene (10) (15.4 mg, 12.5 µmol).

¹H NMR(400 MHz,DMSO-d₆)δ10.11-10.01(m,1H),8.91-8.63(m,1H),8.40-7.96(m,12H),7.72-7.27(m,11H),6.97-6.94(m,2H),5.99(brs,1H),5.71-5.69(m,1H),5.43(brs,2H),5.14-4.96(m,4H),4.49-4.40(m,1H),4.30-4.23(m,1H),4.16-3.83(m,3H),3.25-3.22(m,1H),3.02-2.90(m,7H),2.26-2.22(m,2H),2.01-1.98(m,1H),1.92-1.84(m,1H),1.77-1.66(m,2H),1.65-1.04(m,16H),1.12-1.04(m,2H),0.88-0.80(m,6H).

MS(ESI)m/z:1235.50[M+H]⁺

### (1-2-5) Synthesis of Linker-payload mimic (6)

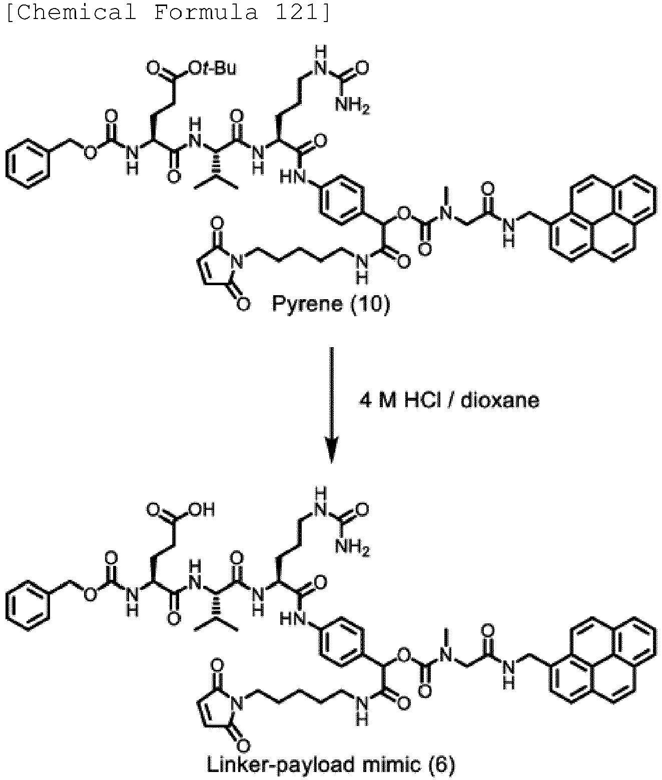

To pyrene (10) (12.1 mg, 9.79 µmol), 1,4-dioxane (2.0 mL) and a 4 M hydrogen chloride/dioxane solution (490 µL, 1.96 mmol) were sequentially added, and the mixture was stirred at room temperature for four hours. After ice cooling, N,N-diisopropylethylamine (366 µL, 2.15 mmol) was added thereto, and the mixture was stirred at room temperature for 10 minutes. The reaction solution was purified by reverse phase preparative chromatography, a fraction comprising a product was collected and concentrated under reduced pressure to remove acetonitrile. Thereafter, the residue was freeze-dried to obtain Linker-payload mimic (6) (6.0 mg, 5.1 µmol).

¹H NMR(400 MHz,DMSO-d₆)δ12.05(brs,1H)10.04-9.94(m,1H),8.84-8.57(m,1H),8.32-7.89(m,12H),7.72-7.20(m,11H),6.90-6.86(m,2H),5.90(brs,1H),5.64-5.62(m,1H),5.36(brs,2H),5.07-4.88(m,4H),4.42-4.29(m,1H),4.21-4.15(m,1H),4.08-3.76(m,3H),3.18-3.14(m,1H),2.95-2.82(m,7H),2.21-2.16(m,2H),1.92-0.97(m,13H),0.82-0.74(m,6H).

MS(ESI)m/z:1179.50[M+H]⁺

### (1-3) Synthesis of Linker-payload mimic (11) Linker-payload mimic (11) was synthesized as follows.

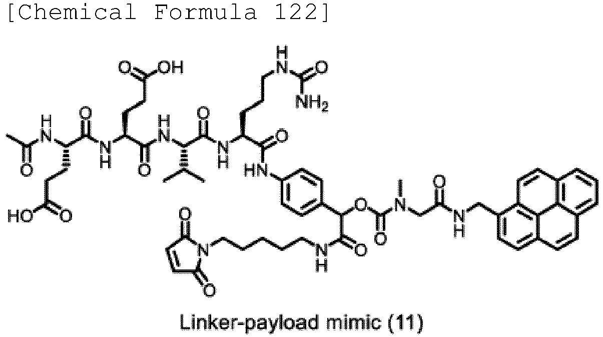

### (1-3-1) Synthesis of alcohol (12)

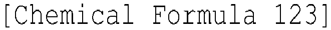

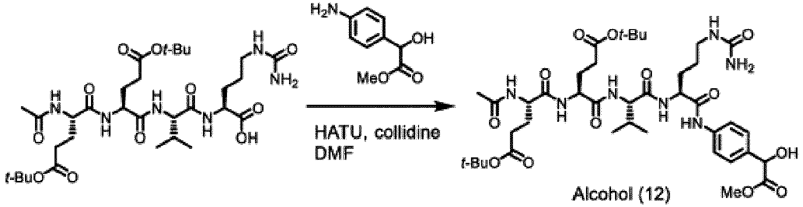

Ac-Glu(t-Bu)-Glu(t-Bu)-Val-Cit-OH (50.0 mg, 72.8 µmol) was dissolved in N,N-dimethylformamide (800 µL), and 1-[bis(dimethylamino) methylene]-1H-1,2,3,-triazolo[4,5-b] pyridinium 3-oxide hexafluorophosphate (33.2 mg, 87.4 µmol) and 2,4,6-trimethylpyridine (11.5 µL, 87.4 µmol) were added thereto. The mixture was stirred at room temperature for ten minutes. Subsequently, methyl 4-aminomandelate (15.8 mg, 87.4 µmol) was added thereto, and the mixture was stirred at room temperature for 16 hours, and then purified by reverse phase preparative chromatography. A fraction comprising a product was collected and concentrated under reduced pressure to remove acetonitrile. The residue was freeze-dried to obtain the alcohol (12) (54.0 mg, 63.5 µmol).

¹H NMR(400 MHz,DMSO-d₆)δ10.00(s,1H),8.26-7.88(m,3H),7.68-7.60(m,1H),7.57(d,J=8.4 Hz,2H),7.32(d,J=8.4 Hz,2H),6.00-5.97(m,1H),5.43(brs,2H),5.08(s,1H),4.40-4.37(m,1H),4.32-4.19(m,3H),3.60(s,3H),3.09-2.90(m,2H),2.25-2.18(m,4H),2.03-1.53(m,10H),1.46-1.36(m,20H),0.86(d,J=6.8Hz,3H),0.82(d,J=6.8Hz,3H).

MS(ESI)m/z:850.40[M+H]⁺

### (1-3-2) Synthesis of pyrene (13)

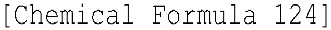

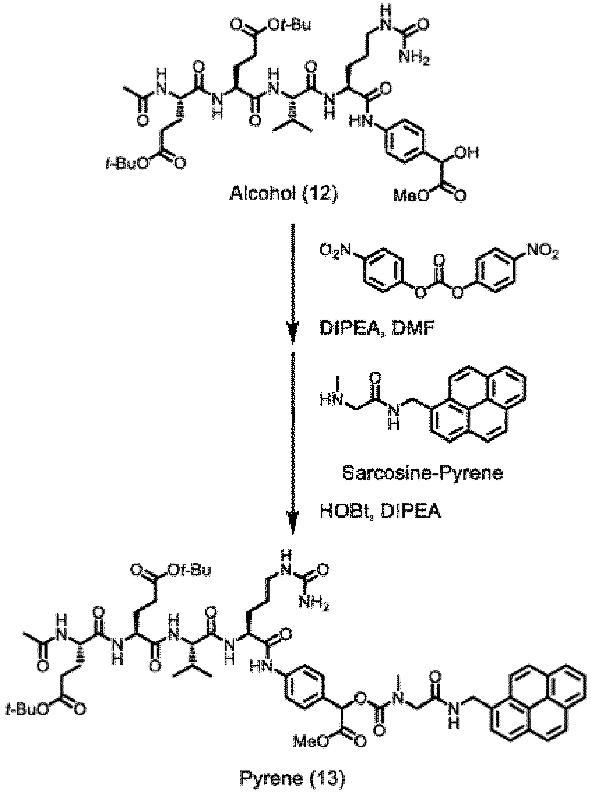

Alcohol (12) (50.3 mg, 59.2 µmol) was dissolved in N,N-dimethylformamide (650 µL), and the solution was stirred under ice cooling for five minutes. Thereafter, bis(4-nitrophenyl) carbonate (54.0 mg, 178 µmol) and N,N-diisopropylethylamine (22.7 µL, 133 µmol) were added thereto, and the mixture was stirred at room temperature for five hours. Thereafter, the mixture was ice-cooled. Sarcosin-Pyrene (89.5 mg, 296 µmol), 1-hydroxybenzotriazole (12.0 mg, 88.8 µmol), and N,N-diisopropylethylamine (78.1 µL, 459 µmol) were added thereto, and the mixture was stirred at room temperature for 18 hours. After the reaction, purification was performed by reverse phase preparative chromatography. A fraction comprising a product was collected and concentrated under reduced pressure to remove acetonitrile. The residue was freeze-dried to obtain the pyrene (13) (34.0 mg, 28.9 µmol).

¹H NMR(400 MHz,DMSO-d₆)δ10.14-10.12(m,1H),8.69-8.67(m,1H),8.40-7.82(m,13H),7.76-7.72(m,1H),7.68-7.65(m,2H),7.44-7.39(m,2H),5.99(brs,1H),5.79(d,J=6.4Hz,1H),5.44(brs,2H),5.0 5-4.97(m,2H),4.44-4.08(m,4H),3.93-3.80(m,1H),3.63-3.62(m,3H),3.05-2.92(m,5H),2.25-2.14(m,4H),2.00-1.28(m,30H),0.87-0.80(m,6H).

MS(ESI)m/z:1178.50[M+H]⁺

### (1-3-3) Synthesis of pyrene (14)

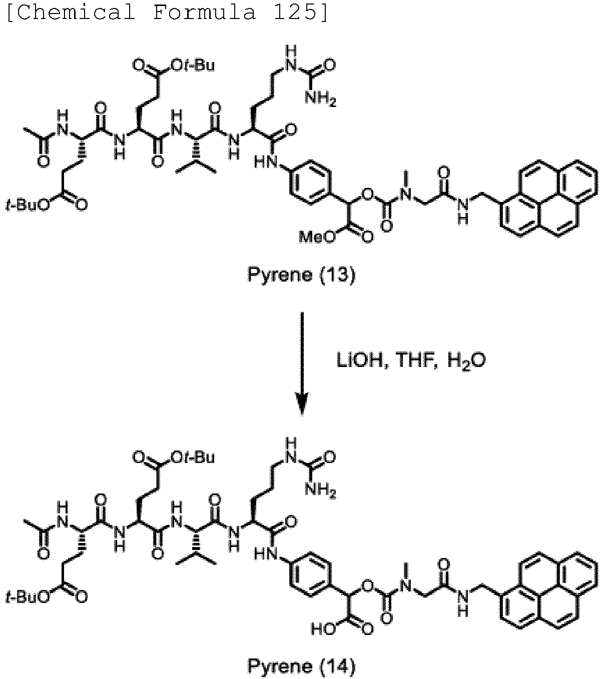

Pyrene (13) (30.7 mg, 26.1 µmol) was dissolved in tetrahydrofuran (2.25 mL) and water (0.75 mL), and the solution was stirred under ice cooling for five minutes. Thereafter, lithium hydroxide monohydrate (5.5 mg, 0.13 mmol) was added thereto, and the mixture was stirred at room temperature for four hours. After completion of the reaction, the pH was adjusted to about 6 using 0.1 M hydrochloric acid, and purification was performed by reverse phase preparative chromatography. A fraction comprising a product was collected and concentrated under reduced pressure to remove acetonitrile. The residue was freeze-dried to obtain the pyrene (14) (17.2 mg, 14.8 µmol).

¹H NMR(400 MHz,DMSO-d₆)δ13.06(brs,1H),10.13-10.10(m,1H),8.69-8.66(m,1H),8.40-7.85(m,13H),7.77-7.73(m,1H),7.67-7.64(m,2H),7.44-7.42(m,2H),5.99(brs,1H),5.68-5.67(m,1H),5.44(brs,2H),5.05-4.96(m,2H),4.46-4.10(m,4H),3.92-3.81(m,1H),3.05-2.90(m,5H),2.25-2.14(m,4H),2.03-1.29(m,30H),0.88-0.80(m,6H).

MS(ESI)m/z:1164.55[M+H]⁺

### (1-3-4) Synthesis of pyrene (15)

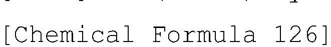

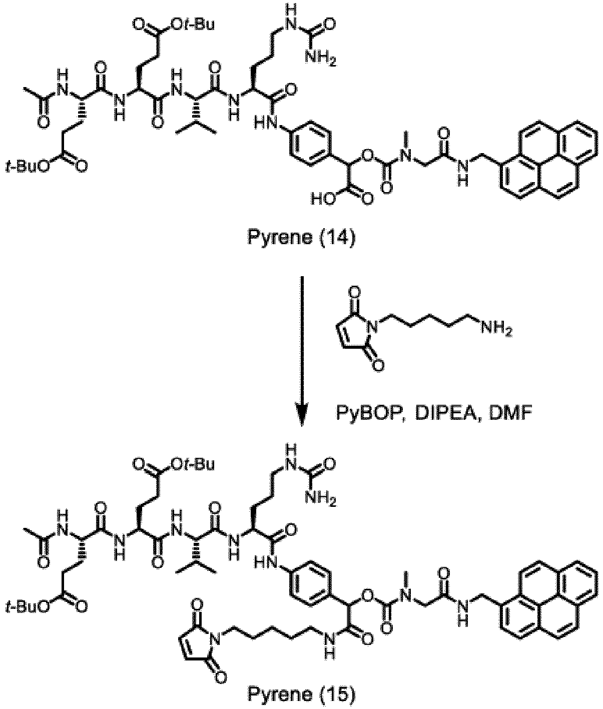

Pyrene (14) (14.7 mg, 12.6 µmol) was dissolved in N,N-dimethylformamide (1.0 mL). Thereafter, the solution was ice-cooled, and N,N-diisopropylethylamine (4.29 µL, 25.2 µmol) and 1H-benzotriazol-1-yloxy tripyrrolidinophosphonium hexafluorophosphate (9.8 mg, 19 µmol) were added thereto. Next, N-(5-aminopentyl) maleimide hydrochloride (4.1 mg, 19 µmol) was added thereto, the temperature was returned to room temperature, and the mixture was stirred for 3.5 hours. After completion of the reaction, purification was performed by reverse phase preparative chromatography. A fraction comprising a product was collected and concentrated under reduced pressure to remove acetonitrile. Thereafter, the resulting residue was freeze-dried to obtain pyrene (15) (7.0 mg, 9.2 µmol).

¹H NMR(400 MHz,DMSO-d₆)δ10.08-10.05(m,1H),8.92-7.95(m,15H),7.75-7.73(m,1H),7.63-7.59(m,2H),7.40-7.34(m,2H),6.97-6.94(m,2H),6.00-5.98(m,1H),5.70-5.69(m,1H),5.43(brs,2H),5.14-5.01(m,2H),4.45-4.38(m,1H),4.32-3.83(m,5H),3.25-3.20(m,1H),3.10-2.90(m,7H),2.25-2.18(m,4H),2.08-1.24(m,34H),1.12-1.04(m,2H),0.88-0.81(m,6H).

MS(ESI)m/z:1328.60[M+H]⁺

### (1-3-5) Synthesis of Linker-payload mimic (11)

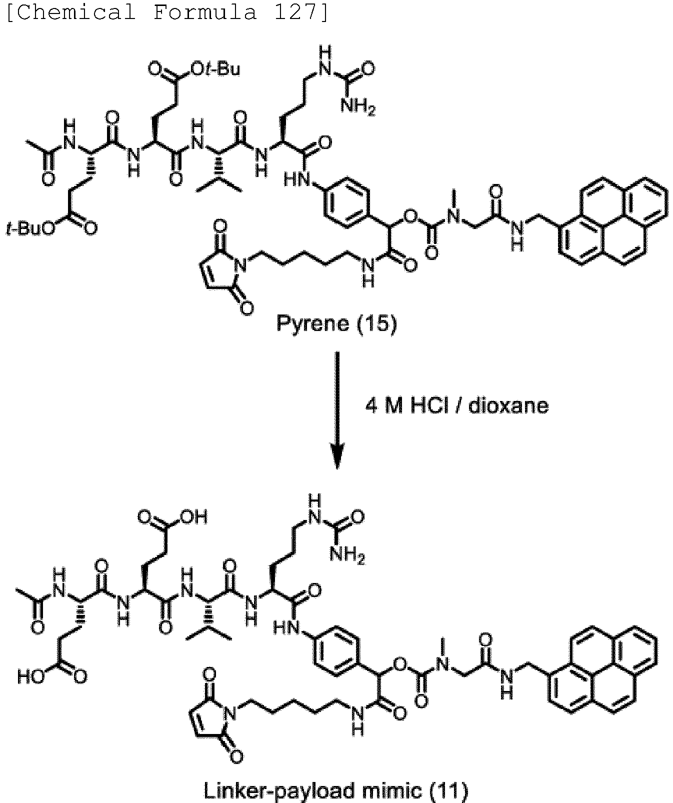

To pyrene (15) (6.1 mg, 4.6 µmol), 1,4-dioxane (920 µL) and a 4 M hydrogen chloride/dioxane solution (230 µL, 918 µmol) were sequentially added, and the mixture was stirred at room temperature for four hours. After ice cooling, N,N-diisopropylethylamine (172 µL, 1.10 mmol) was added thereto, and the mixture was stirred at room temperature for 10 minutes. The reaction solution was purified by reverse phase preparative chromatography, a fraction comprising a product was collected and concentrated under reduced pressure to remove acetonitrile. Thereafter, the residue was freeze-dried to obtain Linker-payload mimic (11) (3.7 mg, 3.0 µmol).

¹H NMR(400 MHz,DMSO-d₆)δ12.04(brs,2H),10.01-9.98(m,1H),8.83-7.89(m,15H),7.71-7.69(m,1H),7.56-7.51(m,2H),7.33-7.26(m,2H),6.90-6.87(m,2H),5.91-5.90(m,1H),5.64-5.62(m,1H),5.36(brs,2H),5.08-4.92(m,2H),4.35-4.32(m,1H),4.25-3.76(m,5H),3.18-3.14(m,1H),2.99-2.82(m,7H),2.21-2.15(m,4H),1.93-1.17(m,16H),1.05-1.01(m,2H),0.82-0.74(m,6H).

MS(ESI)m/z:1216.45[M+H]⁺

### (1-4) Synthesis of Linker-payload mimic (16) Linker-payload mimic (16) was synthesized as follows.

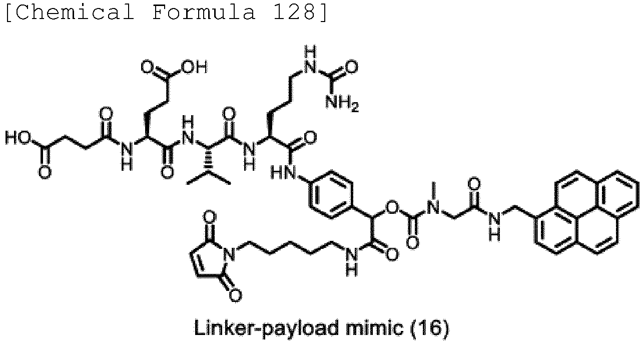

### (1-4-1) Synthesis of alcohol (17)

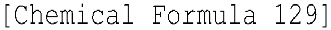

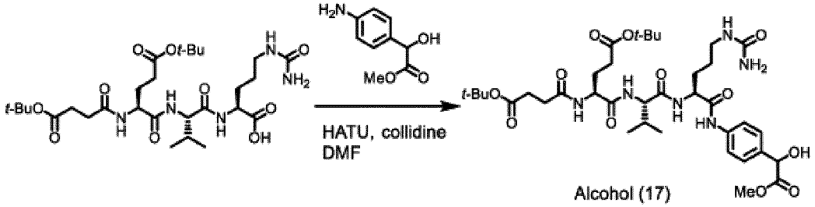

SCA(t-Bu)-Glu(t-Bu)-Val-Cit-OH (50.0 mg, 81.2 µmol) was dissolved in N,N-dimethylformamide (890 µL), and 1-[bis(dimethylamino) methylene]-1H-1,2,3,-triazolo[4,5-b] pyridinium 3-oxide hexafluorophosphate (37.0 mg, 97.4 µmol) and 2,4,6-trimethylpyridine (12.8 µL, 97.4 µmol) were added thereto. The mixture was stirred at room temperature for ten minutes. Subsequently, methyl 4-aminomandelate (17.6 mg, 97.4 µmol) was added thereto, and the mixture was stirred at room temperature for 16 hours, and then purified by reverse phase preparative chromatography. A fraction comprising a product was collected and concentrated under reduced pressure to remove acetonitrile. The residue was freeze-dried to obtain the alcohol (17) (60.4 mg, 77.5 µmol).

¹H NMR(400 MHz,DMSO-d₆)δ10.01(s,1H),8.13(d,J=7.2 Hz,1H),8.07(d,J=8.4 Hz,1H),7.73(d,J=8.8 Hz,1H),7.57(d,J=8.4 Hz,2H),7.32(d,J=8.4 Hz,2H),5.99(brs,1H),5.43(brs,2H),5.08(s,1H),4.41-4.30(m,2H),4.21-4.17(m,1H),3.60(s,3H),3.04-2.95(m,2H),2.44-2.15(m,6H),2.03-1.95(m,1H),1.92-1.83(m,1H),1.74-1.58(m,3H),1.46-1.34(m,20H),0.86(d,J=6.8Hz,3H),0.83(d,J=6.8Hz,3H).

MS(ESI)m/z:779.40[M+H]⁺

### (1-4-2) Synthesis of pyrene (18)

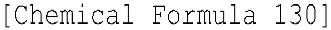

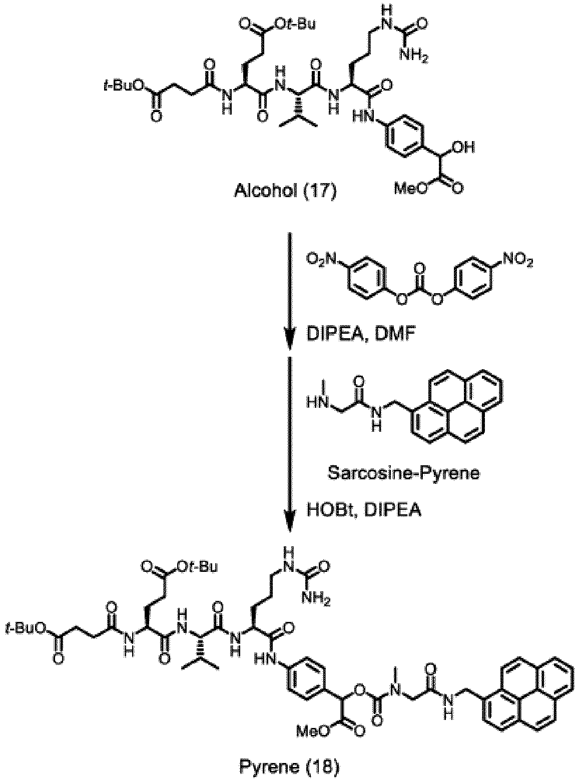

Alcohol (17) (58.0 mg, 74.5 µmol) was dissolved in N,N-dimethylformamide (820 µL), and the solution was stirred under ice cooling for five minutes. Thereafter, bis(4-nitrophenyl) carbonate (68.0 mg, 223 µmol) and N,N-diisopropylethylamine (28.5 µL, 168 µmol) were added thereto, and the mixture was stirred at room temperature for six hours. Thereafter, the mixture was ice-cooled. Sarcosin-Pyrene (113 mg, 373 µmol), 1-hydroxybenzotriazole (15.1 mg, 112 µmol), and N,N-diisopropylethylamine (98.3 µL, 578 µmol) were added thereto, and the mixture was stirred at room temperature for 17 hours. After the reaction, purification was performed by reverse phase preparative chromatography. A fraction comprising a product was collected and concentrated under reduced pressure to remove acetonitrile. The residue was freeze-dried to obtain the pyrene (18) (37.1 mg, 33.5 µmol).

¹H NMR(400 MHz,DMSO-d₆)δ10.15-10.13(m,1H),8.70-8.67(m,1H),8.40-8.03(m,11H),7.90-7.86(m,1H),7.75-7.72(m,1H),7.68-7.64(m,2H),7.44-7.39(m,2H),5.99(brs,1H),5.79(d,J=6.8Hz,1H),5.44(brs,2H),5.0 5-4.98(m,2H),4.40-4.08(m,3H),3.93-3.80(m,1H),3.63-3.62(m,3H),3.05-2.90(m,5H),2.44-2.15(m,6H),2.02-1.98(m,1H),1.91-1.85(m,1H),1.75-1.55(m,3H),1.50-1.30(m,20H),0.88-0.80(m,6H).

MS(ESI)m/z:1107.55[M+H]⁺

### (1-4-3) Synthesis of pyrene (19)

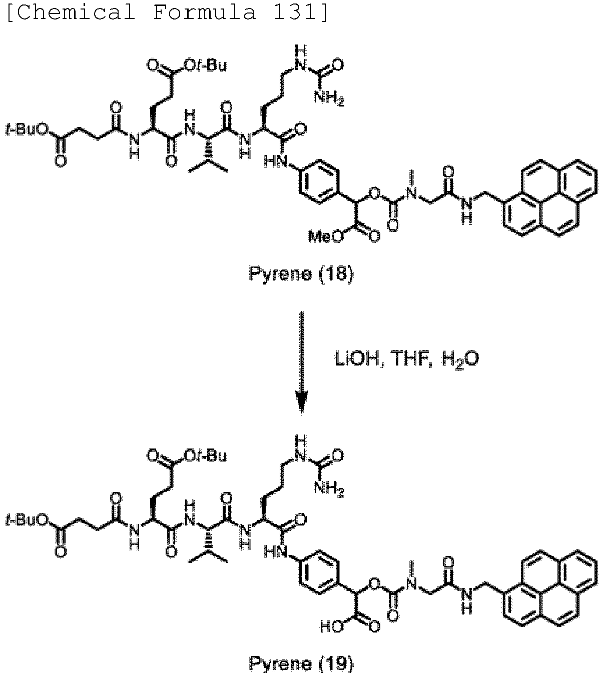

Pyrene (18) (17.4 mg, 15.7 µmol) was dissolved in tetrahydrofuran (675 µL) and water (225 µL), and the solution was stirred under ice cooling for five minutes. Thereafter, a 1 M lithium hydroxide aqueous solution (86.4 µL, 86.4 µmol) was added thereto, and the mixture was stirred under ice cooling for five hours. After completion of the reaction, the pH was adjusted to about 6 using 0.1 M hydrochloric acid, and purification was performed by reverse phase preparative chromatography. A fraction comprising a product was collected and concentrated under reduced pressure to remove acetonitrile. The residue was freeze-dried to obtain the pyrene (19) (17.2 mg, 15.7 µmol).

¹H NMR(400 MHz,DMSO-d₆)δ13.06(brs,1H),10.14-10.11(m,1H),8.68-8.66(m,1H),8.40-8.01(m,11H),7.89-7.85(m,1H),7.75-7.72(m,1H),7.67-7.64(m,2H),7.44-7.41(m,2H),5.99(brs,1H),5.68-5.67(m,1H),5.44(brs,2H),5.05-4.93(m,2H),4.44-4.10(m,3H),3.92-3.81(m,1H),3.05-2.94(m,5H),2.43-2.15(m,6H),2.02-1.97(m,1H),1.88-1.85(m,1H),1.77-1.66(m,3H),1.61-1.30(m,20H),0.88-0.80(m,6H).

MS(ESI)m/z:1093.50[M+H]⁺

### (1-4-4) Synthesis of pyrene (20)

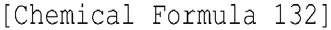

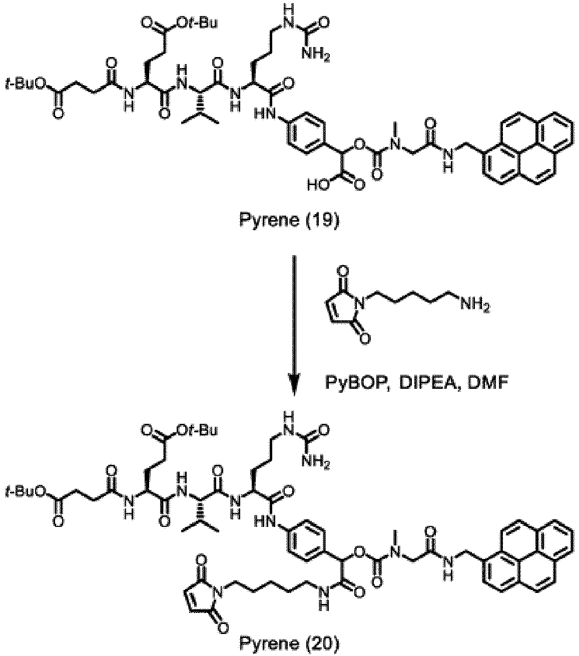

Pyrene (19) (16.3 mg, 14.9 µmol) was dissolved in N,N-dimethylformamide (1.0 mL). Thereafter, the solution was ice-cooled, and N,N-diisopropylethylamine (5.1 µL, 30 µmol) and 1H-benzotriazol-1-yloxy tripyrrolidinophosphonium hexafluorophosphate (11.7 mg, 22.4 µmol) were added thereto. Next, N-(5-aminopentyl) maleimide hydrochloride (4.9 mg, 22 µmol) was added thereto, the temperature was returned to room temperature, and the mixture was stirred for three hours. After completion of the reaction, purification was performed by reverse phase preparative chromatography. A fraction comprising a product was collected and concentrated under reduced pressure to remove acetonitrile. Thereafter, the resulting residue was freeze-dried to obtain pyrene (20) (12.1 mg, 9.62 µmol).

¹H NMR(400 MHz,DMSO-d₆)δ10.01-9.98(m,1H),8.85-7.88(m,14H),7.68-7.65(m,1H),7.56-7.51(m,2H),7.33-7.27(m,2H),6.90-6.87(m,2H),5.92-5.90(m,1H),5.63-5.62(m,1H),5.36(brs,2H),5.08-4.92(m,2H),4.36-3.76(m,5H),3.18-3.14(m,1H),3.00-2.83(m,7H),2.36-2.08(m,6H),1.95-1.89(m,1H),1.84-1.78(m,1H),1.68-1.50(m,3H),1.42-1.17(m,24H),1.07-0.97(m,2H),0.81-0.74(m,6H).

MS(ESI)m/z:1257.55[M+H]⁺

### (1-4-5) Synthesis of Linker-payload mimic (16)

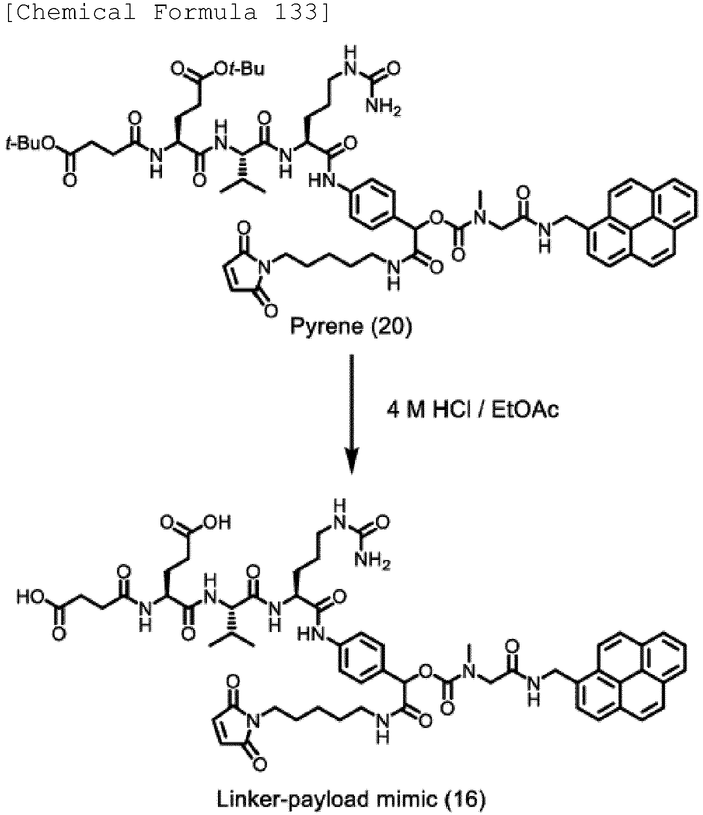

To pyrene (20) (10.5 mg, 8.35 µmol), ethyl acetate (1.7 mL) and 4 M hydrogen chloride/ethyl acetate (2.09 mL, 8.36 mmol) were sequentially added, and the mixture was stirred at room temperature for 4.5 hours. After ice cooling, N,N-diisopropylethylamine (781 µL, 4.59 mmol) was added thereto, and the mixture was stirred at room temperature for 10 minutes. The reaction solution was purified by reverse phase preparative chromatography, a fraction comprising a product was collected and concentrated under reduced pressure to remove acetonitrile. Thereafter, the residue was freeze-dried to obtain Linker-payload mimic (16) (7.5 mg, 6.6 µmol).

¹H NMR(400 MHz,DMSO-d₆)δ12.02(brs,2H),10.04-9.98(m,1H),8.85-7.89(m,14H),7.65-7.62(m,1H),7.56-7.52(m,2H),7.33-7.27(m,2H),6.90-6.87(m,2H),5.92(brs,1H),5.63-5.62(m,1H),5.37(brs,2H),5.08-4.93(m,2H),4.36-3.76(m,5H),3.18-3.14(m,1H),3.02-2.80(m,7H),2.38-2.16(m,6H),1.96-1.77(m,2H),1.70-1.17(m,9H),1.06-0.98(m,2H),0.81-0.74(m,6H).

MS(ESI)m/z:1145.45[M+H]⁺

### (1-5) Synthesis of Linker-payload mimic (21) Linker-payload mimic (21) was synthesized as follows.

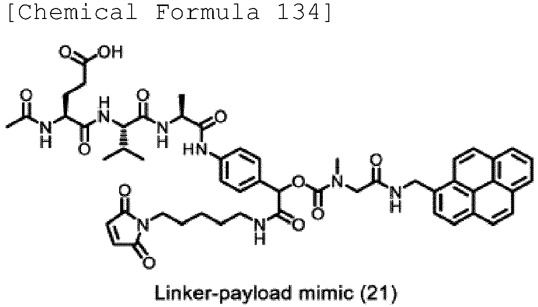

### (1-5-1) Synthesis of alcohol (22)

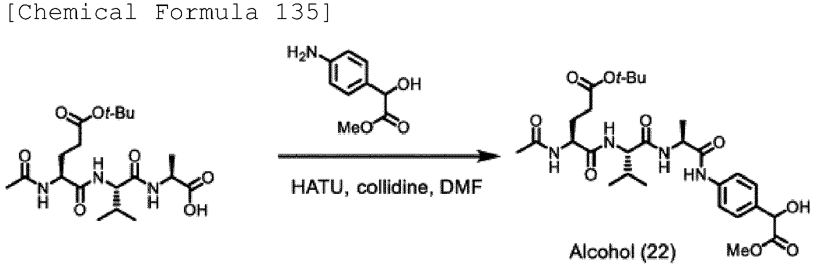

Ac-Glu(OtBu)-Val-Ala-OH (20.9 mg, 50.3 µmol) was dissolved in N,N-dimethylformamide (700 µL), and 1-[bis(dimethylamino) methylene]-1H-1,2,3,-triazolo[4,5-b] pyridinium 3-oxide hexafluorophosphate (29.0 mg, 76.3 µmol) and 2,4,6-trimethylpyridine (10.1 µL, 76.7 µmol) were added thereto. The mixture was stirred at room temperature for 12 minutes. Subsequently, methyl 4-aminomandelate (11.0 mg, 60.7 µmol) was added thereto, and the mixture was stirred at room temperature for 18 hours, and then purified by reverse phase preparative chromatography. A fraction comprising a product was collected and concentrated under reduced pressure to remove acetonitrile. The residue was freeze-dried to obtain the alcohol (22) (20.3 mg, 35.1 µmol).

¹H NMR(400 MHz,DMSO-d₆)δ9.98-9.89(m,1H),8.32-8.18(m,1H),8.07-7.85(m,1H),7.73-7.59(m,1H),7.55(d,J=8.4 Hz,2H),7.32(d,J=8.4 Hz,2H),5.08(s,1H),4.45-4.34(m,1H),4.32-4.27(m,1H),4.20-4.09(m,1H),3.59(s,3H),2.24-2.20(m,2H),2.01-1.96(m,1H),1.90-1.80(m,4H),1.72-1.67(m,1H),1.39-1.36(m,9H),1.30(d,J=7.2Hz,3H),0.86(d,J=6.8Hz,3H),0.82(d,J=6 .8Hz,3H).

MS(ESI)m/z:579.30[M+H]⁺

### (1-5-2) Synthesis of pyrene (23)

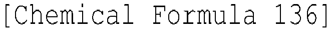

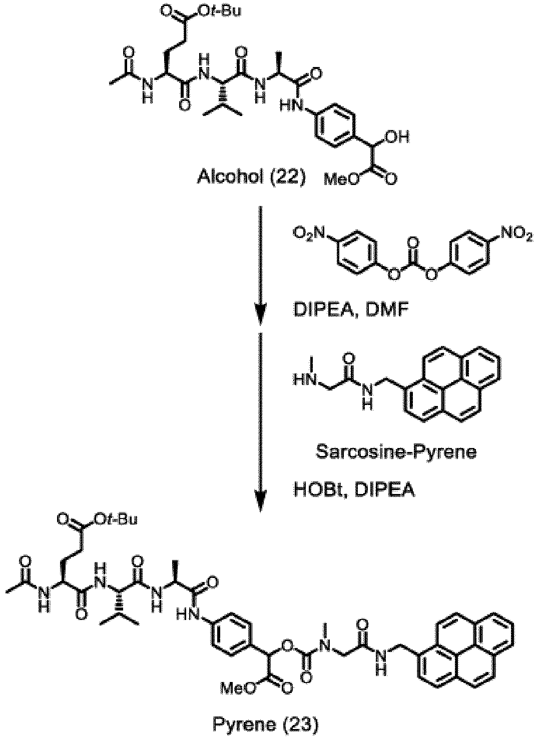

Alcohol (22) (17.5 mg,30.2µmol) was dissolved in N,N-dimethylformamide (174 µL), and the solution was stirred under ice cooling for five minutes. Thereafter, bis(4-nitrophenyl) carbonate (18.9 mg, 60.8 µmol) and N,N-diisopropylethylamine (7.80 µL, 45.4 µmol) were added thereto, and the mixture was stirred at room temperature for 30 minutes. As a result of confirmation by LCMS, a remaining raw material was found. Therefore, bis(4-nitrophenyl) carbonate (9.5 mg, 31 µmol) and N,N-diisopropylethylamine (3.90 µL, 22.7 µmol) were added thereto, and the mixture was stirred at room temperature for one hour. Thereafter, the mixture was ice-cooled. Sarcosin-Pyrene (36.2 mg, 120 µmol), 1-hydroxybenzotriazole (6.3 mg, 47 µmol), and N,N-diisopropylethylamine (40.3 µL, 235 µmol) were added thereto, and the mixture was stirred at room temperature for one hour. After the reaction, purification was performed by reverse phase preparative chromatography. A fraction comprising a product was collected and concentrated under reduced pressure to remove acetonitrile. The residue was freeze-dried to obtain the pyrene (23) (12.9 mg, 14.2 µmol).

¹H NMR(400 MHz,DMSO-d₆)δ10.05-10.02(m,1H)8.61-8.60(m,1H),8.30-7.78(m,12H),7.67-7.64(m,1H),7.57(d,J=8.8Hz,2H),7.37-7.33(m,2H),5.72-5.70(m,1H),4.98-4.89(m,2H),4.34-4.00(m,3H),3.85-3.75(m,1H),3.56-3.55(m,3H),2.93-2.87(m,3H),2.17-2.13(m,2H),1.94-1.91(m,1H),1.85-1.75(m,4H),1.65-1.61(m,1H),1.31-1.23(m,12H),0.80-0.73(m,6H).

MS(ESI)m/z:907.35[M+H]⁺

### (1-5-3) Synthesis of pyrene (24)

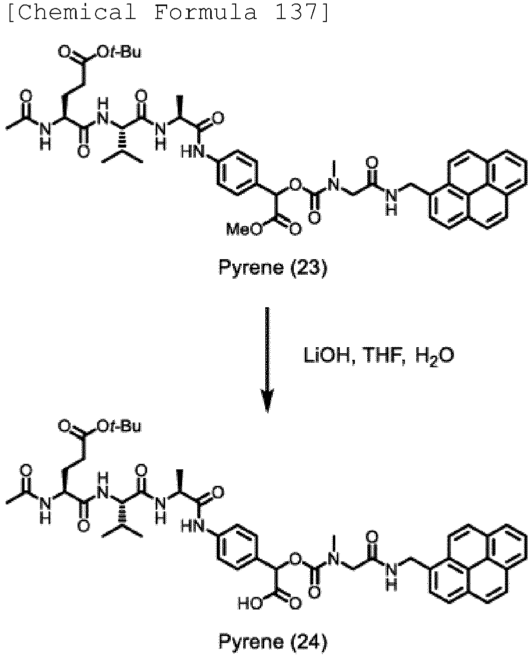

Pyrene (23) (11.8 mg, 13.0 µmol) was dissolved in tetrahydrofuran (800 µL) and water (400 µL), and then ice-cooled. A 1 M lithium hydroxide aqueous solution (65 µL, 65 µmol) was added thereto, and the mixture was stirred for 30 minutes. After completion of the reaction, the pH was adjusted to about 6 using 0.1 M hydrochloric acid, and purification was performed by reverse phase preparative chromatography. A fraction comprising a product was collected and concentrated under reduced pressure to remove acetonitrile. The residue was freeze-dried to obtain the pyrene (24) (8.9 mg, 10.0 µmol).

¹H NMR(400 MHz,DMSO-d₆)δ13.05(brs,1H),10.04-9.91(m,1H),8.63-8.60(m,1H),8.33-7.77(m,12H),7.69-7.55(m,3H),7.37-7.35(m,2H),5.61-5.60(m,1H),4.98-4.86(m,2H),4.26-4.03(m,3H),3.84-3.74(m,1H),2.93-2.87(m,3H),2.18-2.13(m,2H),1.95-1.92(m,1H),1.8 5-1.77(m,4H),1.67-1.62(m,1H),1.31-1.24(m,12H),0.81-0.75(m,6H).

MS(ESI)m/z:893.35[M+H]⁺

### (1-5-4) Synthesis of pyrene (25)

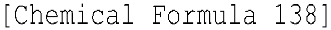

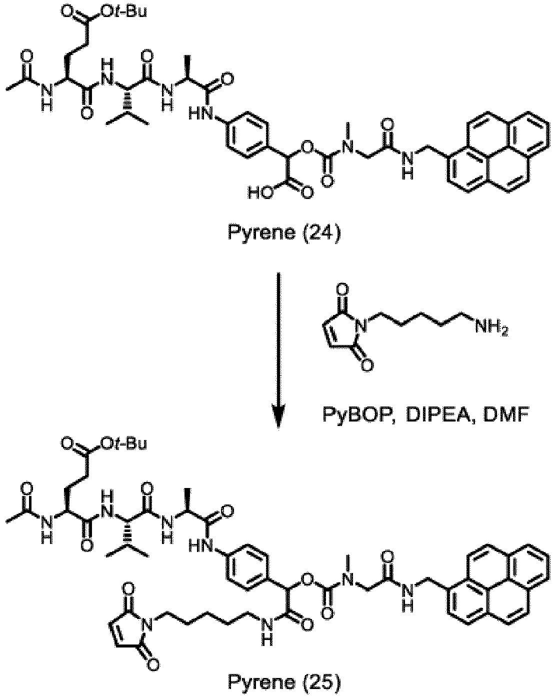

Pyrene (24) (6.7 mg, 7.5 µmol) was dissolved in N,N-dimethylformamide (400 µL). Thereafter, the solution was ice-cooled, and N,N-diisopropylethylamine (3.9 µL, 22 µmol) and 1H-benzotriazol-1-yloxy tripyrrolidinophosphonium hexafluorophosphate (5.9 mg, 11 µmol) were added thereto. Next, N-(5-aminopentyl) maleimide hydrochloride (2.4 mg, 11 µmol) was added thereto, the temperature was returned to room temperature, and the mixture was stirred for one hour. After completion of the reaction, purification was performed by reverse phase preparative chromatography. A fraction comprising a product was collected and concentrated under reduced pressure to remove acetonitrile. Thereafter, the resulting residue was freeze-dried to obtain pyrene (25) (4.1 mg, 3.9 µmol).

¹H NMR(400 MHz,DMSO-d₆)δ9.98-9.95(m,1H),8.83-7.77(m,14H),7.67-7.65(m,1H),7.56-7.50(m,2H),7.34-7.27(m,2H),6.89-6.86(m,2H),5.63-5.61(m,1H),5.06-4.93(m,2H),4.37-3.76(m,5H),3.17-3.14(m,1H),2.95-2.83(m,5H),2.17-2.10(m,2H),1.95-1.89(m,1H),1.85-1.75(m,4H),1.65-1.62(m,1H),1.31-1.17(m,16H),1.02-1.00(m,2H),0.81-0.74(m,6H).

MS(ESI)m/z:1057.45[M+H]⁺

### (1-5-5) Synthesis of Linker-payload mimic (21)

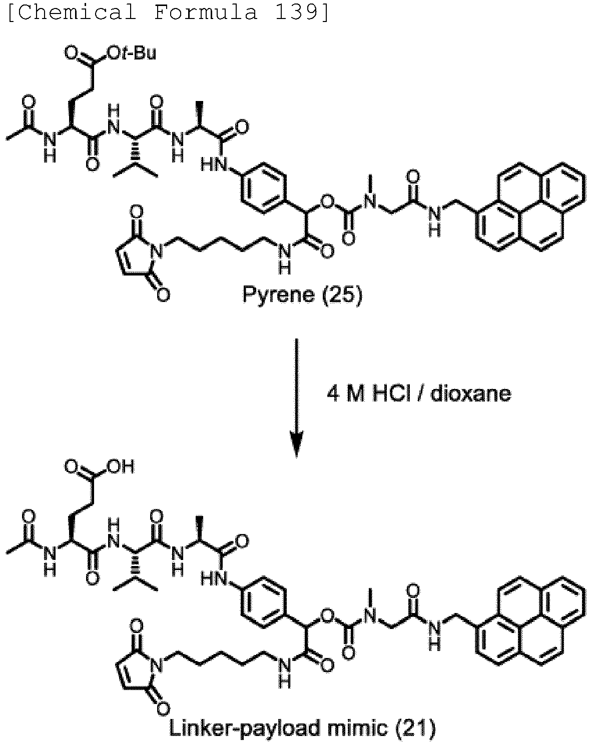

To pyrene (25) (2.4 mg, 2.3 µmol), 1,4-dioxane (454 µL) and a 4 M hydrogen chloride/dioxane solution (568 µL, 2.27 mmol) were sequentially added, and the mixture was stirred at room temperature for four hours. N,N-dimethylformamide (300 µL) was added thereto. Thereafter, N,N-diisopropylethylamine (214 µL, 1.25 mmol) was added thereto under ice cooling, and the mixture was stirred at room temperature for 10 minutes. The reaction solution was purified by reverse phase preparative chromatography, a fraction comprising a product was collected and concentrated under reduced pressure to remove acetonitrile. Thereafter, the residue was freeze-dried to obtain Linker-payload mimic (21) (1.1 mg, 1.1 µmol).

¹H NMR(400 MHz,DMSO-d₆)δ11.99(brs,1H),9.98-9.96(m,1H),8.84-7.88(m,14H),7.66-7.63(m,1H),7.54-7.50(m,2H),7.34-7.27(m,2H),6.90-6.87(m,2H),5.63-5.61(m,1H),5.07-4.93(m,2H),4.36-3.76(m,5H),3.17-3.14(m,lH),2.95-2.83(m,5H),2.20-2.16(m,2H),1.95-1.90(m,1H),1.88-1.76(m,4H),1.67-1.62(m,1H),1.32-1.17(m,7H),1.02-1.01(m,2H),0.81-0.74(m,6H).

MS(ESI)m/z:999.35[M-H]⁻

### Comparative Example 1: Synthesis of Linker-payload

### (1-1) Synthesis of Linker-Payload (26)

Linker-Payload (26) was synthesized as follows.

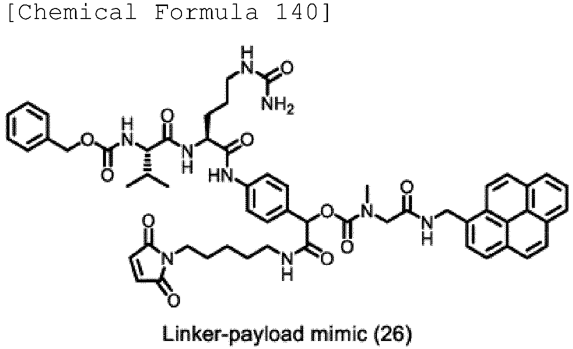

Linker-Payload (26) was synthesized according to the following scheme.

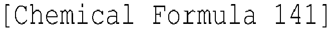

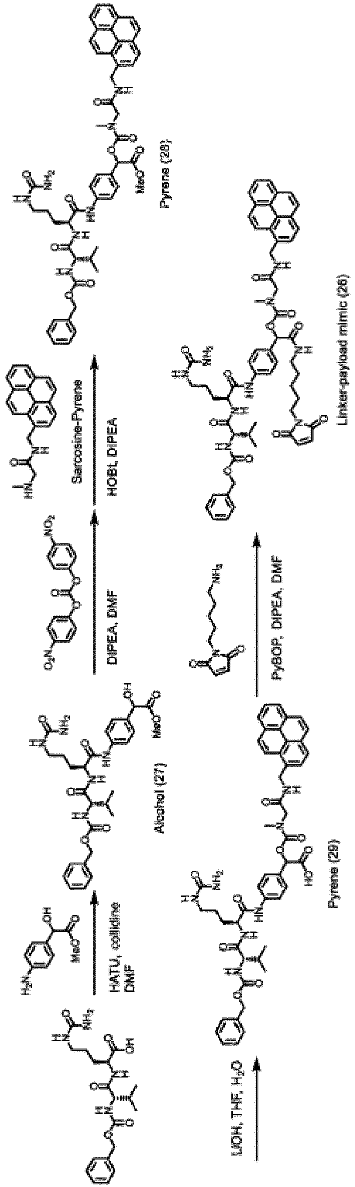

MS analysis results of Linker-Payload (26) were as follows.
MS(ESI)m/z:1050.55[M+H]⁺

### Example 2: Synthesis of ADC mimic

### (2-1) Synthesis of ADC mimic

In the following Comparative Examples and Examples, an antibody derivative (thiol group-introduced trastuzumab) described in Example 81-7 of WO 2019/240287 A1 was used as a thiol group-introduced antibody. This antibody derivative has the following structure in which a thiol group is regioselectively introduced into trastuzumab (humanized IgG1 antibody) via an amino group of a side chain of a lysine residue at position 246 or 248 of an antibody heavy chain (the position of the lysine residue is in accordance with EU numbering).

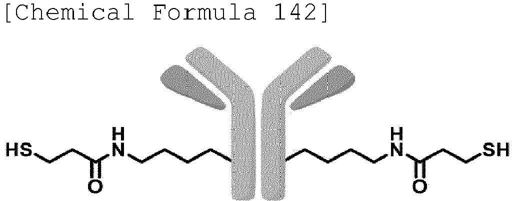

(In the above structure, NH-CH₂-CH₂-CH₂-CH₂- extending from the antibody heavy chain corresponds to a side chain of a lysine residue, and HS-CH₂-CH₂-C(=O) which is a thiol-comprising group is added to an amino group in the side chain of the lysine residue. In the present antibody, modification with another lysine residue was not detected in a peptide mapping method, and therefore position selectivity at position 246 or 248 of the antibody heavy chain is understood to be 100%.)

To a buffer (pH 7.4 PBS buffer) solution (20 µM) of the thiol group-introduced antibody, 10 equivalents of a DMF solution (10 mM) of Linker-payload mimic synthesized in Example 1 was added, and the mixture was allowed to stand at room temperature for two hours, and then purified using NAP-5 Columns (manufactured by GE Healthcare) to obtain an ADC mimic.

ADC mimic 1 having the following structure was synthesized from Linker-payload mimic (1) synthesized in Example 1-1 and the thiol-comprising antibody. ESI-TOFMS analysis was performed. For the reaction product, a peak was observed at 150350 indicating a product with two Linker-payload mimics (1) introduced.

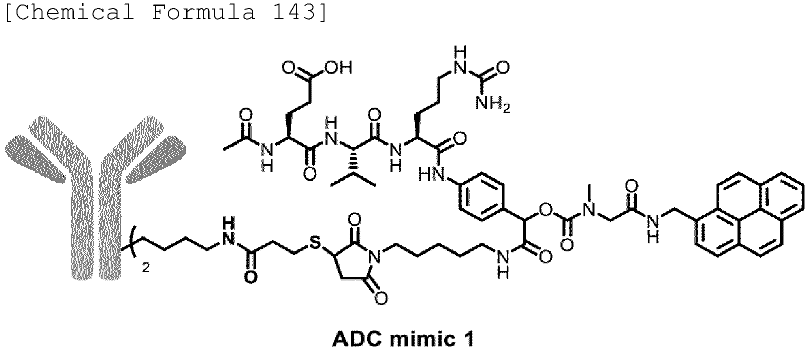

Similarly, ADC mimic 2 having the following structure was synthesized from Linker-payload mimic (6) of Example 1-2 and the thiol-comprising antibody. ESI-TOFMS analysis was performed. For the reaction product, a peak was observed at 150535 indicating a product with two Linker-payload mimics (6) introduced.

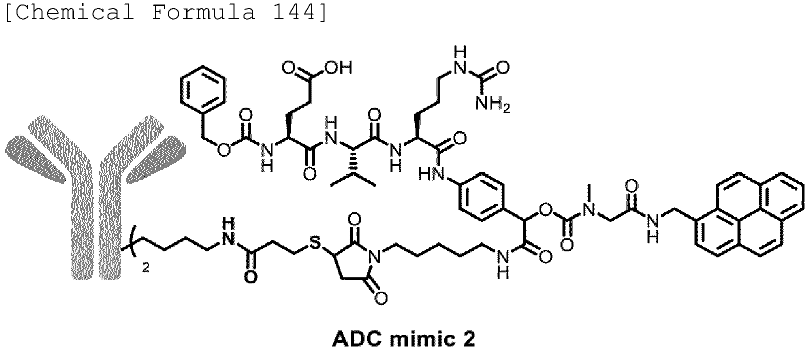

Similarly, ADC mimic 3 having the following structure was synthesized from Linker-payload mimic (11) of Example 1-3 and the thiol-comprising antibody. ESI-TOFMS analysis was performed. For the reaction product, a peak was observed at 150609 indicating a product with two Linker-payload mimics (11) introduced.

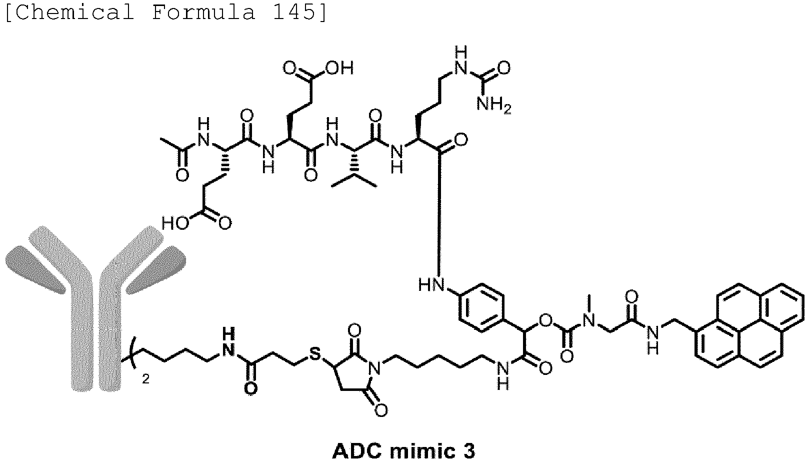

Similarly, ADC mimic 4 having the following structure was synthesized from Linker-payload mimic (16) of Example 1-4 and the thiol-comprising antibody. ESI-TOFMS analysis was performed. For the reaction product, a peak was observed at 150466 indicating a product with two Linker-payload mimics (16) introduced.

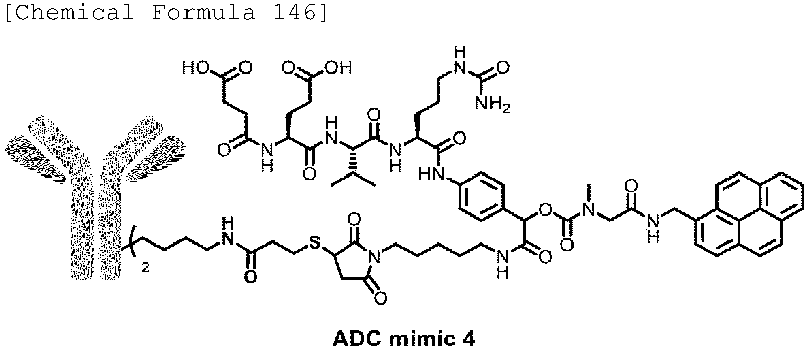

Similarly, ADC mimic 5 having the following structure was synthesized from Linker-payload mimic (26) of Comparative Example 1 and the thiol-comprising antibody. ESI-TOFMS analysis was performed. For the reaction product, a peak was observed at 150276 indicating a product with two Linker-payload mimics (26) introduced.

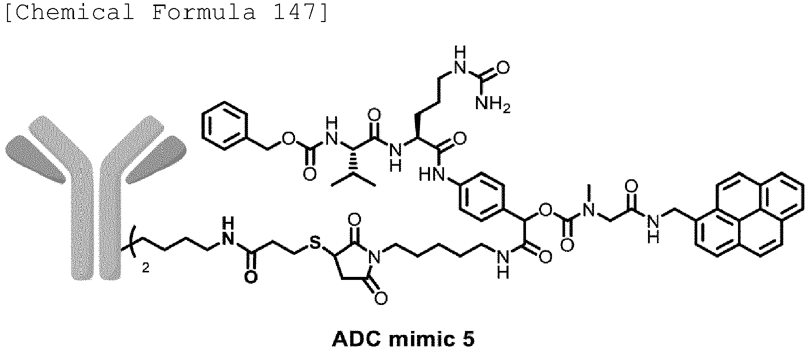

### (2-2) DAR analysis of ADC mimic

The ADC mimic synthesized in Example 2-1 was subjected to ESI-TOFMS analysis according to a previous report (WO 2019/240287 A1) and confirmed to have a DAR of 2.

**[Table 1]**

| Table 1. DAR of ADC mimic | | | |
|---|---|---|---|
| | Linker-payload mimic | Example/ Comparative Example | DAR |
| ADC mimic 1 | Linker-payload mimic (1) | Example 1-1 | 2 |
| ADC mimic 2 | Linker-payload mimic (6) | Example 1-2 | 2 |
| ADC mimic 3 | Linker-payload mimic (11) | Example 1-3 | 2 |
| ADC mimic 4 | Linker-payload mimic (16) | Example 1-4 | 2 |
| ADC mimic 5 | Linker-payload mimic (26) | Comparative Example 1 | 2 |

### Example 3: Evaluation of degrees of hydrophobicity of ADC and ADC mimic by hydrophobic column chromatography (HIC-HPLC)

According to a previous report (Anal. Chem., 2019, 91, 20, 12724-12732), HIC-HPLC analysis was performed. Measurement was performed using the following conditions. Degrees of hydrophobicity of an ADC can be evaluated by retention time of the ADC in HIC chromatogram.

### Measurement system: Chromaster (registered trademark) (manufactured by Hitachi, Ltd.)

Column: Tosoh Biobuthyl NPR 2.5 µm 4.6 × 35 mm column manufactured by Tosoh Bio-Sciences Inc.
Gradient: linear gradient of eluent A/B
Flow rate: 0.8 mL/min
Eluent A: 1.1 M (NH₄)₂SO₄, 25mM Na₂HPO₄/NaH₂PO₄ (pH 6. 0)
Eluent B: 25 mM Na₂HPO₄/NaH₂PO₄ (pH 6.0, 25 v/v% isopropanol added)
Detector: UV (280 nm)

**[Table 2]**

| Table 2. Evaluation of degrees of hydrophobicity of ADC and ADC mimic using HIC-HPLC | | | |
|---|---|---|---|
| | Linker-payload mimic or Linker-payload | Example/ Comparative Example | Retention time |
| ADC mimic 1 | Linker-payload mimic (1) | Example 1-1 | 11.0 minutes |
| ADC mimic 2 | Linker-payload mimic (6) | Example 1-2 | 12.9 minutes |
| ADC mimic 3 | Linker-payload mimic (11) | Example 1-3 | 10.5 minutes |
| ADC mimic 4 | Linker-payload mimic (16) | Example 1-4 | 11.0 minutes |
| ADC mimic 5 | Linker-payload mimic (26) | Comparative Example 1 | 13.6 minutes |

As a result, it has been confirmed that retention times of the ADC mimics synthesized in Examples 1-1, 1-2, 1-3, and 1-4 tend to be early, and it is found that the ADC mimics synthesized in Examples 1-1, 1-2, 1-3, and 1-4 have high degrees of hydrophilicity. Therefore, it has been confirmed that the ADC mimics synthesized in Examples 1-1, 1-2, 1-3, and 1-4 are preferable ADCs because it is considered that the ADC mimics synthesized in Examples 1-1, 1-2, 1-3, and 1-4 have slow plasma clearances and long times during which the ADC mimics remain in the body.

Example 4: Evaluation of aggregation ratios of ADC and ADC mimic by size exclusion chromatography (SEC-HPLC)

SEC-HPLC analysis was performed according to a previous report (Chemistry Select, 2020, 5, 8435-8439). Measurement was performed using the following conditions.

### Measurement system: 1260 HPLC system (manufactured by Agilent)

Column: AdvanceBio SEC 300 Å, 2.7 µm, 4.6 mm × 150 mm, manufactured by Agilent Technologies
Flow rate: 0.25 mL/min
Eluent: 100 mM sodium dihydrogen phosphate/sodium hydrogen phosphate, aqueous solution of 250 mM sodium chloride (pH 6.8), 10% v/v isopropanol
Detector: UV (280 nm)

**[Table 3]**

| Table 3. Evaluation of aggregation ratios of ADC and ADC mimic using SEC-HPLC | | | |
|---|---|---|---|
| | Linker-payload mimic or Linker-payload | Example/ Comparative Example | Aggregation ratio |
| ADC mimic 1 | Linker-payload mimic (1) | Example 1-1 | 1.6% |
| ADC mimic 2 | Linker-payload mimic (6) | Example 1-2 | 2.7% |
| ADC mimic 3 | Linker-payload mimic (11) | Example 1-3 | 1.5% |
| ADC mimic 4 | Linker-payload mimic (16) | Example 1-4 | 2.4% |
| ADC mimic 5 | Linker-payload mimic (26) | Comparative Example 1 | 2.7% |

### Example 5: Evaluation of ADC mimic using enzyme cathepsin B

Cleavabilities for various ADC mimics by cathepsin B were evaluated by analyzing the amount of fluorescent molecules dropped from the ADC mimics as described below.

### (5-1) Cathepsin B cleavability test

A cathepsin B cleavability test was performed as follows according to a previous report (Nature Communications 2018, 9, 2512). An ADC mimic was added to 180 µL of a MES buffer (10 mM MES, 40 µM DTT, pH 5.0) so as to have a concentration of 1 mg/mL, and then 30 µL of the mixture was poured into each of six Eppendorf tubes. To each of three of the six samples, 100 µL of acetonitrile was immediately added at 0°C. The mixture was stirred by vortex, and then centrifuged to obtain a precipitate. The resulting supernatant solution was collected and subjected to HPLC analysis. The remaining three samples were incubated at 37°C for six hours. To each of the samples, 100 µL of acetonitrile was added. The mixture was stirred by vortex, and then centrifuged to obtain a precipitate. The resulting supernatant solution was collected and subjected to HPLC analysis.

### (5-2) Analysis of amount of dropped fluorescent molecules using HPLC analysis

For the measurement, the amount of fluorescent molecules dropped from an ADC mimic was measured using a liquid chromatography/fluorescence detection method. The three samples to which acetonitrile was immediately added at 0°C in Example 7-1 were taken as 0 hour samples, and the three samples incubated at 37°C for six hours as described in Example 7-1 were taken as 6 hour samples. A difference in fluorescence intensity between the 6 hour samples and the 0 hour samples was analyzed.

Separately, a correlation between the area of a fluorescence intensity area by HPLC and a concentration was calculated using Pyrene. The difference in fluorescence intensity between the ADC mimics was converted into a concentration using the calculation formula. When the concentration at 0 hour was set to 100%, a ratio of the above-described difference in fluorescence intensity was calculated as a dropping ratio.

**[Table 4]**

| Table 4. Evaluation of Cathepsin B cleavability of ADC mimic | | | |
|---|---|---|---|
| | Linker-payload mimic | Example/ Comparative Example | Dropping ratio of fluorescent molecules in six hours |
| ADC mimic 1 | Linker-payload mimic (1) | Example 1-1 | 200% |
| ADC mimic 2 | Linker-payload mimic (6) | Example 1-2 | 200% |
| ADC mimic 3 | Linker-payload mimic (11) | Example 1-3 | 200% |
| ADC mimic 4 | Linker-payload mimic (16) | Example 1-4 | 79% |
| ADC mimic 5 | Linker-payload mimic (26) | Comparative Example 1 | 200% |

As presented in Table 4, the synthesized ADC mimic was found to have sufficient cathepsin B cleavage.

### Example 6: Evaluation of ADC mimic using mouse plasma

### (6-1) Test for stability of ADC mimic in plasma

An ADC mimic was added to 500 µL of mouse plasma (manufactured by Charles River) so as to have a concentration of 0.1 mg/mL, and then the mixture was subjected to sterile filtration. 50 µL of this solution was poured into each of six Eppendorf tubes. Three of the six samples were stored in an incubator set at 37°C for four days. The remaining three samples were stored in a freezer at -80°C for four days similarly. To each of the samples, 100 µL of acetonitrile was added. The mixture was stirred by vortex, and then centrifuged to obtain a precipitate. The resulting supernatant solution was collected and subjected to HPLC analysis.

### (6-2) Analysis of amount of dropped fluorescent molecules using HPLC analysis

For the measurement, the amount of fluorescent molecules dropped from an ADC mimic was measured using a liquid chromatography/fluorescence detection method. The three samples stored in a freezer in Example 9-1 were taken as Day = 0 samples, and the three samples stored at 37°C in Example 9-1 were taken as Day = 4 samples. A difference in fluorescence intensity between Day = 4 samples and Day = 0 samples was analyzed.

### A dropping ratio of the fluorescent molecules was calculated according to Example 5-2.

Regarding the results, as illustrated in the following Table, a dropping ratio of the fluorescent molecules was evaluated.

**[Table 5]**

| Table 5. Results of test for stability of ADC mimic in plasma | | | |
|---|---|---|---|
| | Linker-payload mimic | Example/ Comparative Example | Dropping ratio of payload at Day=4 |
| ADC mimic 1 | Linker-payload mimic (1) | Example 1-1 | 7% |
| ADC mimic 2 | Linker-payload mimic (6) | Example 1-2 | 15% |
| ADC mimic 3 | Linker-payload mimic (11) | Example 1-3 | 5% |
| ADC mimic 4 | Linker-payload mimic (16) | Example 1-4 | 3% |
| ADC mimic 5 | Linker-payload mimic (26) | Comparative Example 1 | 52% |

As a result, the ADC mimics synthesized in Examples 1-1 and 1-2 exhibited stability of 3 times or more, and the ADC mimics synthesized in Examples 1-3 and 1-4 exhibited stability of 10 times or more, as compared with the ADC mimic synthesized in Comparative Example 1.

### Example 7: Synthesis of ADC mimic

### Example 7-1: Synthesis of ADC mimic 6

To a 3 mg/mL PBSE buffer (50 mM phosphate buffered saline (PBS), 10 mM EDTA, pH = 7.4) of anti-HER2 IgG antibody trastuzumab (Chugai Pharmaceutical), a PBSE solution (2 mM) of a reducing agent (TCEP) was added in an amount of 8 equivalents to the antibody, and the mixture was incubated at 37°C for one hour. To the resulting reaction mixture, dimethylacetamide (DMA) (6% v/v) and a 10 mM DMA solution of Linker-payload mimic (1) (20 equivalents to the antibody) were sequentially added and stirred gently at 20°C for one hour. An excess amount of N-acetylcysteine was added to the reaction mixture, and the mixture was stirred at 25°C for 20 minutes. The final mixture was purified using a NAP-5 desalting column (manufactured by GE Healthcare) and eluted with a formulation buffer (20 mM histidine, comprising 5 wt% trehalose, pH 5.2) to obtain ADC mimic 6.

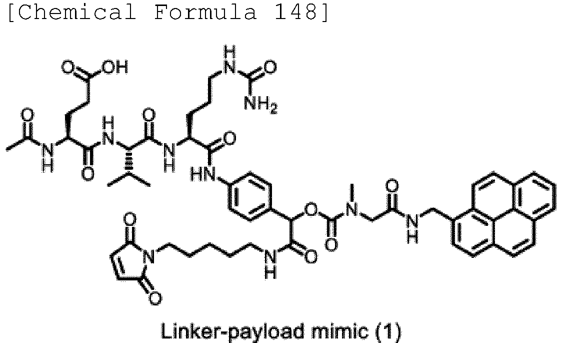

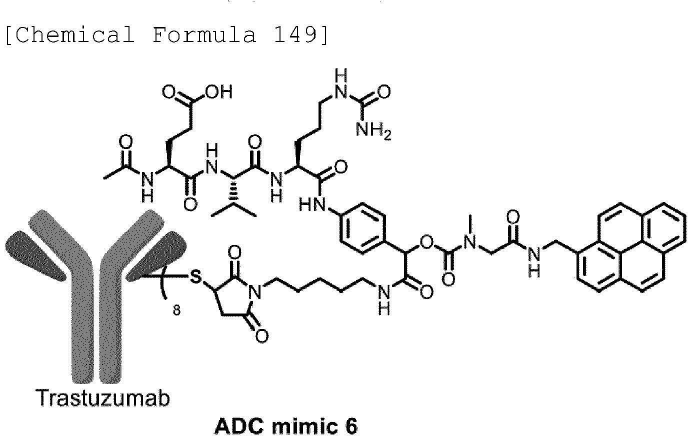

### Example 7-2: Synthesis of ADC mimic 7

According to Example 7-1, ADC mimic 7 was obtained from Linker-payload mimic (11).

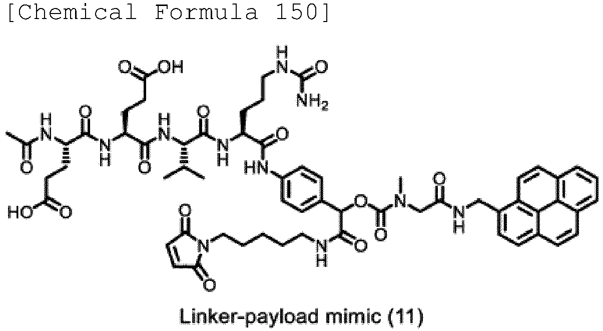

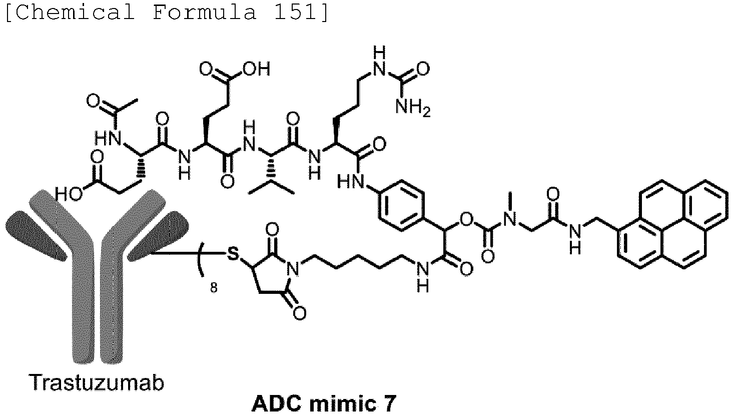

### Example 7-3: Synthesis of ADC mimic 8

According to Example 7-1, ADC mimic 8 was obtained from Linker-payload mimic (21).

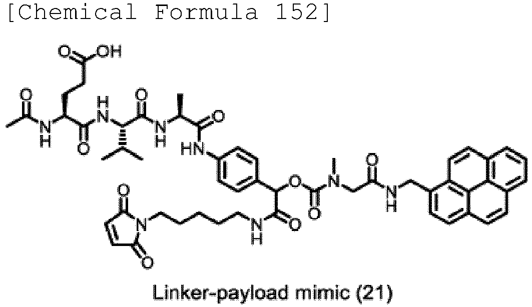

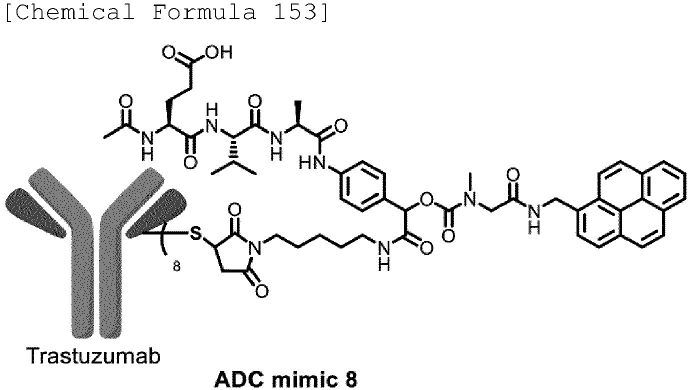

### Example 7-4: Synthesis of ADC mimic 9

According to Example 7-1, Linker-payload mimic (1) was caused to react with anti-CD20 IgG antibody rituximab (Roche) to obtain ADC mimic 9.

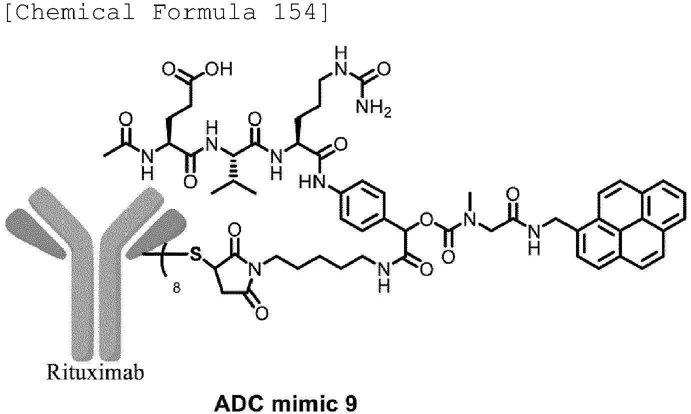

### Example 7-5: Synthesis of ADC mimic 10

According to Example 7-1, Linker-payload mimic (11) was caused to react with anti-CD20 IgG antibody rituximab (Roche) to obtain ADC mimic 10.

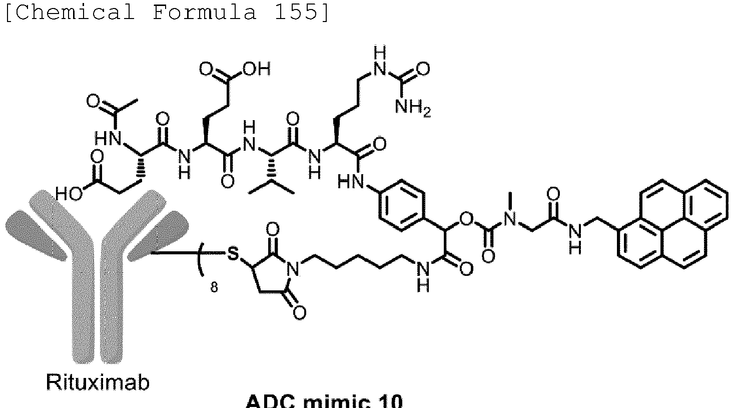

### Example 7-6: Synthesis of ADC mimic 11

According to Example 7-1, Linker-payload mimic (21) was caused to react with anti-CD20 IgG antibody rituximab (Roche) to obtain ADC mimic 11.

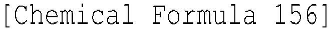

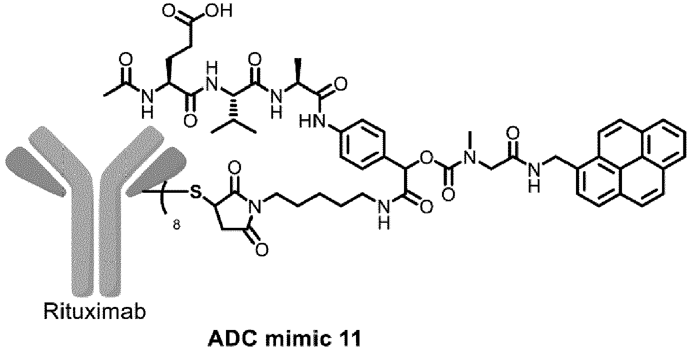

### Example 7-7: Synthesis of ADC mimic 12

According to Example 7-1, Linker-payload mimic (1) was caused to react with anti-TNF-α IgG antibody infliximab (Centocor) to obtain ADC mimic 12.

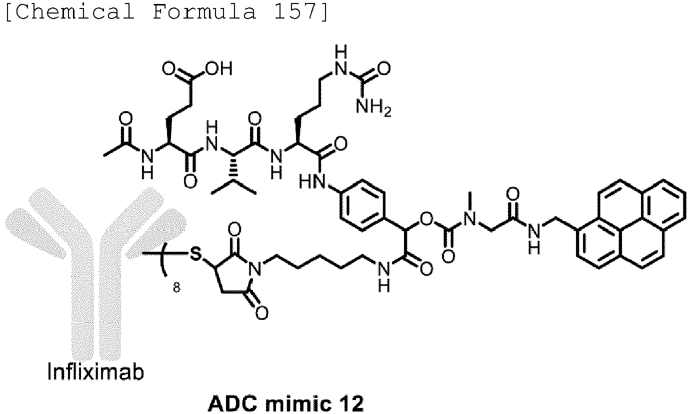

### Example 7-8: Synthesis of ADC mimic 13

According to Example 7-1, Linker-payload mimic (11) was caused to react with anti-TNF-α IgG antibody infliximab (Centocor) to obtain ADC mimic 13.

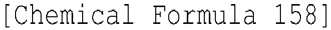

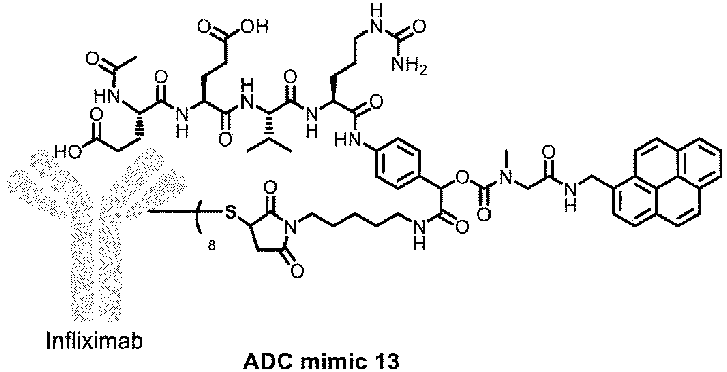

### Example 7-9: Synthesis of ADC mimic 14

According to Example 7-1, Linker-payload mimic (21) was caused to react with anti-TNF-α IgG antibody infliximab (Centocor) to obtain ADC mimic 14.

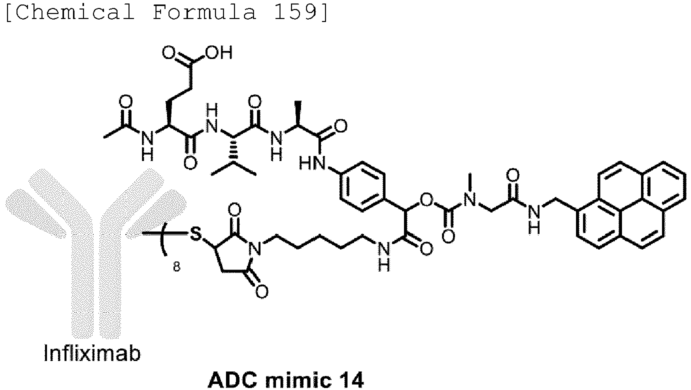

### Comparative Example 2: Synthesis of ADC mimic

### Comparative Example 2-1: Synthesis of Linker-payload mimic (30)

Linker-payload mimic (30) was synthesized as follows. Linker-payload mimic (30) was synthesized in one step from commercially available MC-VC-PAB-PNP (CAS No: 159857-81-5) and known Sarcosine-pyrene (WO 2018218004 A1).

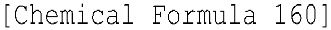

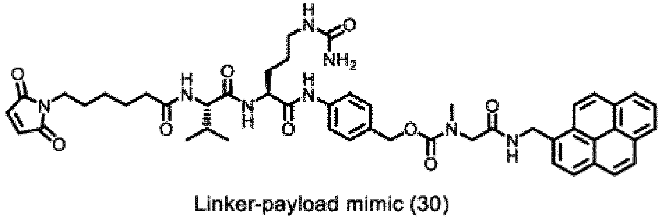

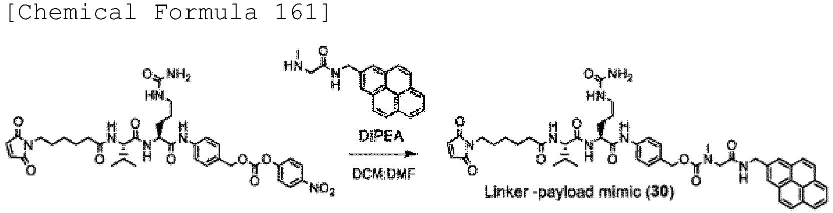

Commercially available MC-VC-PAB-PNP (CAS No: 159857-81-5) (15.5 mg, 0.021 mmol) was dissolved in dichloromethane (1 mL), and a dimethylformamide solution (0.5 mL) of N,N-diisopropylethylamine (0.025 mL, 0.142 mmol) and known Sarcosine-pyrene (WO 2018218004 A1) (7.6 mg, 0.025 mmol) was added thereto. The mixture was stirred for 17 hours. After purification by reverse phase preparative chromatography, a fraction comprising a product was collected and concentrated under reduced pressure to remove acetonitrile. The residue was freeze-dried to obtain Linker-payload mimic (30) (7.3 mg, 0.008 mmol).
¹H NMR(400 MHz,DMSO-d6)δ9.98(s,1H),8.34(d,J=9.2Hz,2H),8.32-8.23(m,4H),8.16(s,2H),8.10-8.00(m,4H),7.80(d,J=8.8Hz,1H),7.59(d,J=8.4Hz,2H),7.31(d,J=8 .0Hz,2H),6.99(s,2H),5.96(m,1H),5.40(s,2H),5.01(s,2H),4.95(d ,J=6.0Hz,2H),4.38(m,1H),4.19(m,1H),3.03-2.92(m,3H),2.67(m,1H),2.33(m,1H),2.20-2.07(m,2H),1.97(m,1H),1.67(m,1H),1.59(m,1H),1.51-1.45(m,6H),1.26-1.15(m,3H),0.83(dd,J=12.8,6.8Hz,6H)
MS(ESI)m/z:901.45[M+H]⁺

### Comparative Example 2-2: Synthesis of ADC mimics 15 to 17

According to Example 7-1, Linker-payload mimic (30) was caused to react to obtain ADC mimic 15 from trastuzumab, ADC mimic 16 from rituximab, and ADC mimic 17 from infliximab.

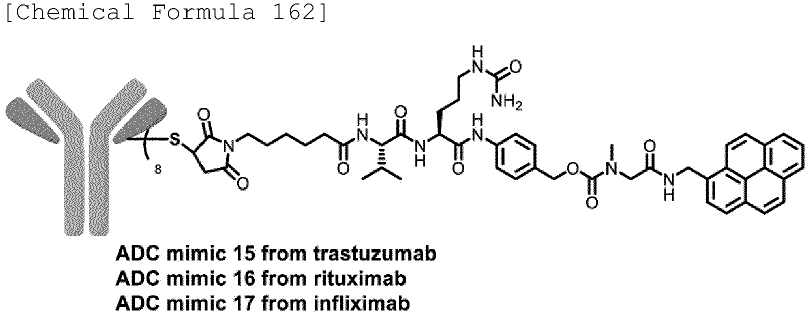

### Comparative Example 2-3: Synthesis of Linker-payload mimic (31)

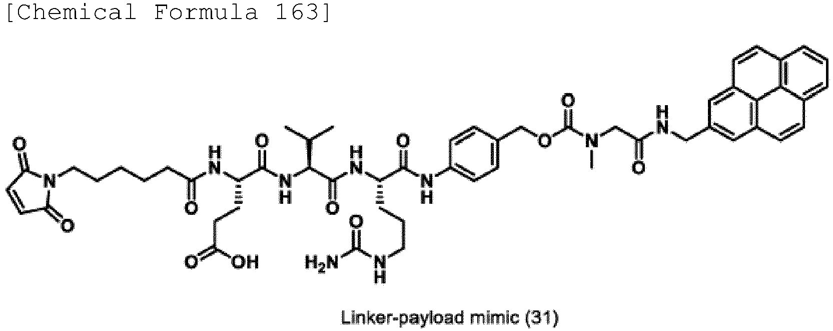

### Comparative Example 2-3-1: Synthesis of Pyrene (32)

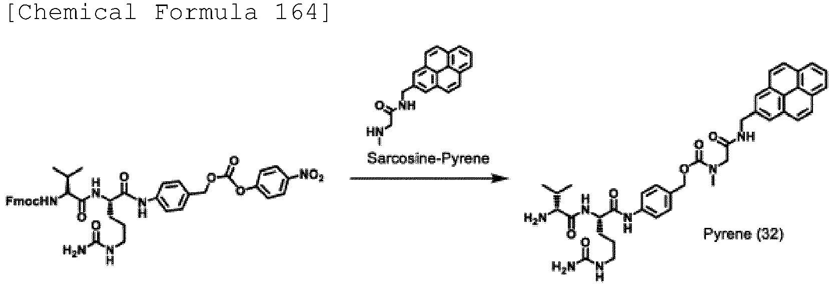

Fmoc-Val-Cit-PAB-PNP (CAS No: 863971-53-2, 121.2 mg, 0.15 mmol) was dissolved in N,N-dimethylformamide (5 mL), and well-known (described in WO 2018218004 A1) sarcosine-pyrene (59.2 mg, 0.196 mmol), N,N-diisopropylethylamine (39 µL, 0.227 mmol), and 4-dimethylaminopyridine (3.7 mg, 0.03 mmol) were added thereto. The mixture was stirred at room temperature for two hours, and then diethylamine (2 mL, 18.95 mmol) was added thereto, and the mixture was stirred at room temperature for 1.5 hours. The mixture was concentrated under reduced pressure, and then purified by reverse phase preparative chromatography. A fraction comprising a product was collected and concentrated under reduced pressure to remove acetonitrile. The residue was freeze-dried to obtain the pyrene (32) (73.7 mg, 0.104 mmol).

¹H NMR(400MHz,DMSO-d6)δ10.21(s,1H),8.68(s,1H),8.43-7.93(m,12H),7.60(t,J=7.1Hz,2H),7.31(m,2H),6.04(s,1H),5.48(s ,2H),5.02(d,J=16.1Hz,4H),4.54(s,1H),3.96(s,2H),3.66(s,2H),2 .92(d,J=6.1Hz,3H),2.08(q,J=6.6Hz,1H),1.80-1.56(m,2H),1.46(s,2H),1.21-1.13(m,1H),0.94(dt,J=6.8,3.0Hz,6H).

MS(ESI)m/z:708.80[M+H]⁺

### Comparative Example 2-3-2: Synthesis of Pyrene (33)

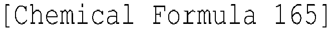

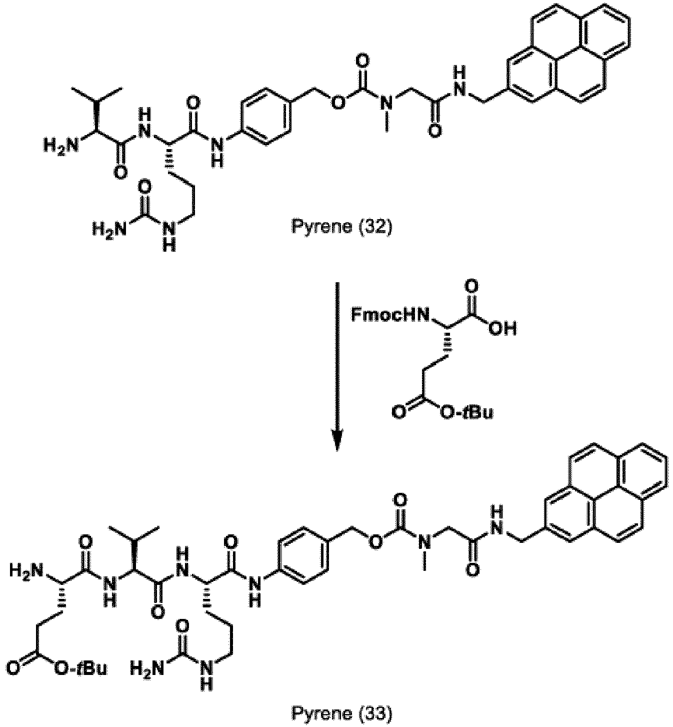

Fmoc-Glu (OtBu)-OH•H₂O (11.1 mg, 0.025 mmol) was dissolved in dimethylformamide (1 mL), and pyrene (32) (17.3 mg, 0.024 mmol), 1-hydroxy-7 azabenzotriazole (5.1 mg, 0.037 mmol), 1-(3-dimethylaminopropyl)-3-ethylcarbodiimide hydrochloride (7.3 mg, 0.038 mmol), and triethylamine (7.1 µL, 0.51 mmol) were added thereto. The mixture was stirred at room temperature for 2.5 hours, and then diethylamine (0.2 mL, 1.91 mmol) was added thereto. The mixture was stirred at room temperature for 1.5 hours. The mixture was concentrated under reduced pressure, and then purified by reverse phase preparative chromatography. A fraction comprising a product was collected and concentrated under reduced pressure to remove acetonitrile. The residue was freeze-dried to obtain pyrene (33) (15.6 mg, 0.017 mmol).

¹H NMR(400MHz,Chloroform-d)δ10.87(s,1H),8.68(m,1H),8.41-7.97(m,13H),7.60-7.58(m,2H),7.30(m,2H),6.00(t,J=6.2Hz,1H),5.45(s,2H),5.05-4.99(m,4H),4.46(m,1H),4.26(m,1H),3.96(s,2H),3.88(m,1H),3.07 (m,1H),2.96(m,1H),2.93(d,J=5.6Hz,3H),2.68(t,J=1.8Hz,1H),2.3 4-2.30(m,3H),2.03(m,1H),1.93-1.90 (m, 2H), 1.30 (s, 9H), 1.15 (s, 1H), 0.92-0.86 (m, 6H) .

MS(ESI)m/z:893.45[M+H]⁺

### Comparative Example 2-3-3: Synthesis of Pyrene (34)

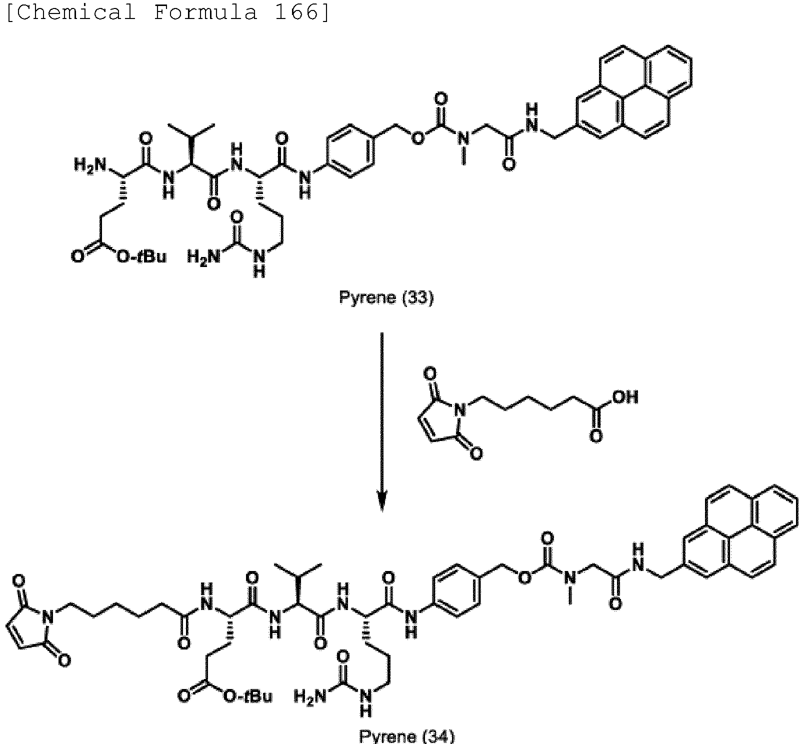

Pyrene (33) (15.6 mg, 0.017 mmol) was dissolved in dimethylformamide (1.5 mL), and 6-maleimidohexanoic acid (3.7 mg, 0.018 mmol), 1-hydroxy-7 azabenzotriazole (3.5 mg, 0.025 mmol), 1-(3-dimethylaminopropyl)-3-ethylcarbodiimide hydrochloride (4.8 mg, 0.025 mmol), and triethylamine (4.8 µL, 0.34 mmol) were added thereto. The mixture was stirred at room temperature for three hours, and then 6-maleimidohexanoic acid (1.8 mg, 0.009 mmol), 1-(3-dimethylaminopropyl)-3 ethylcarbodiimide hydrochloride (2.3 mg, 0.012 mmol), and triethylamine (2.4 µL, 0.17 mmol) were added thereto. The mixture was stirred at room temperature for 1.5 hours. Thereafter, 4-dimethylaminopyridine (0.5 mg, 0.004 mmol) was added thereto, and the mixture was further stirred for 2.5 hours. The mixture was concentrated under reduced pressure, and then purified by column chromatography (dichloromethane : methanol = 9 : 1). A fraction comprising a product was collected and concentrated under a reduced pressure to obtain the pyrene (34) (8.0 mg, 0.007 mmol).
¹H NMR(400MHz,DMSO-d6)δ10.02(s,1H),8.65(m,1H),8.40-7.96(m,9H),7.69-7.53(m,3H),7.28(m,2H),7.00-6.98(m,2H),5.97(m,1H),5.41(s,2H),5.04-4.98(m,4H),4.56(t,5.4Hz,1H),4.41-4,27(m,2H),4,21-4.15(m,2H),3.95(s,2H),2.91(d,J=5.6Hz,3H),2.22-2.15(m,2H),2.10-2.07(m,2H),1.69(m,1H),1.59(m,1H),1.50-1.44(m,6H),1.46(d,J=2.0Hz,9H),1.35-1.15(m,4H),0.82(dd,J=14.8,6.8Hz,6H)
MS(ESI)m/z:1086.60[M+H]⁺

### Comparative Example 2-3-4: Synthesis of Linker-payload mimic (31)

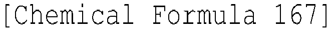

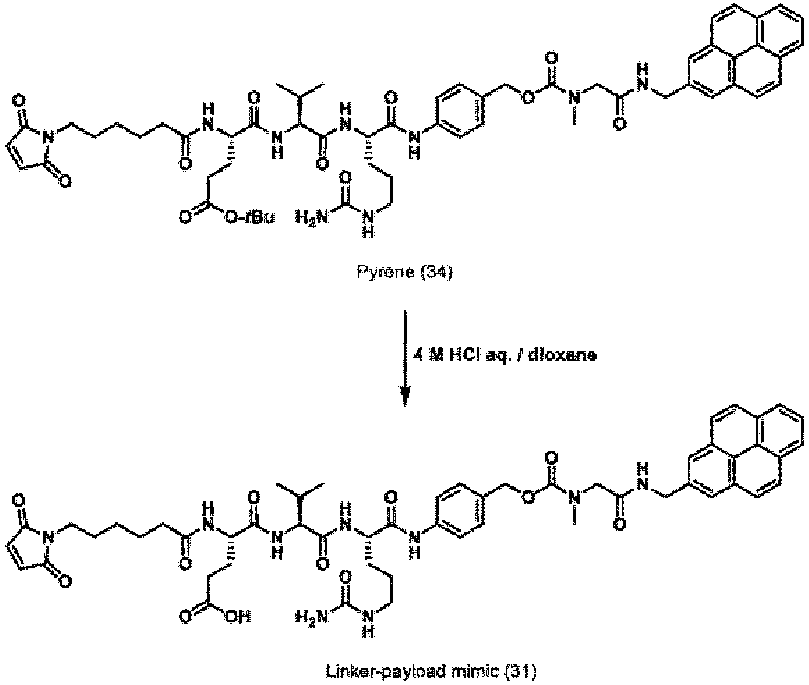

Pyrene (4) (9.7 mg, 0.0089 mmol) was dissolved in 1,4-dioxane (1 mL) and a hydrogen chloride/1,4-dioxane solution (1 mL), and the solution was stirred in an ice water bath for two hours. Dimethylformamide (1 mL) was added thereto to raise the temperature to room temperature, and then the mixture was concentrated under reduced pressure. Thereafter, purification was performed by reverse phase preparative chromatography. A fraction comprising a product was collected and concentrated under reduced pressure to remove acetonitrile. The residue was freeze-dried to obtain Linker-payload mimic (31) (2.0 mg, 0.002 mmol).

¹H NMR(400MHz,DMSO-d6)δ10.03(s,1H),8.67(m,1H),8.40-7.96(m,9H),7.69-7.57(m,3H),7.28(m,2H),6.98(s,2H),5.98(m,1H),5.87(m,1H),5.42 (s,2H),5.05-4.97(m,4H),4.4(m,1H),4,29(m,1H),4.19(m,1H),3.95(s,2H),2.90( d,J=5.2Hz,3H),2.22-2.17(m,2H),2.11-2.09(m,2H),1.71-1.59(m,3H),1.49-1.44(m,6H),1.30-1.15(m,4H),0.82(dd,J=15.0,6.6Hz,6H)
MS(ESI)m/z:1030.50[M+H]⁺

### Comparative Example 2-4: Synthesis of ADC mimics 18 to 20

According to Example 7-1, Linker-payload mimic (31) was caused to react to obtain ADC mimic 18 from trastuzumab, ADC mimic 19 from rituximab, and ADC mimic 20 from infliximab.

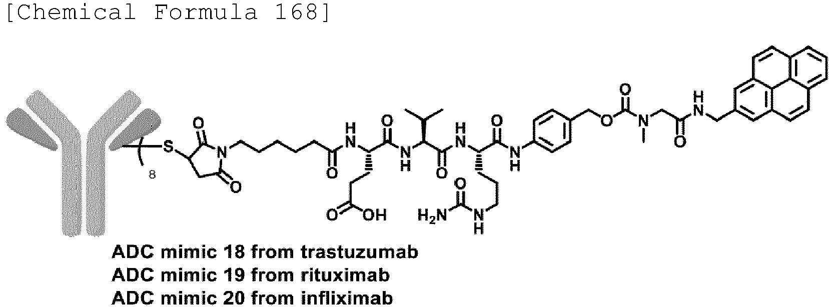

### Example 8: DAR analysis of ADC mimic

For DAR analysis of the ADC mimics synthesized in Example 7 and Comparative Example 2, HIC-HPLC analysis was performed according to a previous report (Anal. Chem., 2019, 91, 20, 12724-12732). Measurement was performed using the following conditions.

### Measurement system: Chromaster (registered trademark) (manufactured by Hitachi, Ltd.)

Column: Tosoh Biobuthyl NPR 2.5 µm 4.6 × 35 mm column manufactured by Tosoh Bio-Sciences Inc.
Gradient: linear gradient of eluent A/B
Flow rate: 0.8 mL/min
Eluent A: 1.1 M (NH₄)₂SO₄,25mM Na₂HPO₄/NaH₂PO₄ (pH 6.0)
Eluent B: 25 mM Na₂HPO₄/NaH₂PO₄ (pH 6.0, 25 v/v% isopropanol added)
Detector: UV (280 nm)

**[Table 6]**

| Table 6. DAR of ADC mimic | | | |
|---|---|---|---|
| | Linker-payload mimic | Example/ Comparative Example | DAR |
| ADC mimic 6 | Linker-payload mimic (1) | Example 7-1 | 8 |
| ADC mimic 7 | Linker-payload mimic (11) | Example 7-2 | 8 |
| ADC mimic 8 | Linker-payload mimic (21) | Example 7-3 | 8 |
| ADC mimic 9 | Linker-payload mimic (1) | Example 7-4 | 8 |
| ADC mimic 10 | Linker-payload mimic (11) | Example 7-5 | 8 |
| ADC mimic 11 | Linker-payload mimic (21) | Example 7-6 | 8 |
| ADC mimic 12 | Linker-payload mimic (1) | Example 7-7 | 8 |
| ADC mimic 13 | Linker-payload mimic (11) | Example 7-8 | 8 |
| ADC mimic 14 | Linker-payload mimic (21) | Example 7-9 | 8 |
| ADC mimic 15 | Linker-payload mimic (30) | Comparative Example 2-2 | 8 |
| ADC mimic 16 | Linker-payload mimic (30) | Comparative Example 2-2 | 8 |
| ADC mimic 17 | Linker-payload mimic (30) | Comparative Example 2-2 | 8 |
| ADC mimic 18 | Linker-payload mimic (31) | Comparative Example 2-4 | 8 |
| ADC mimic 19 | Linker-payload mimic (31) | Comparative Example 2-4 | 8 |
| ADC mimic 20 | Linker-payload mimic (31) | Comparative Example 2-4 | 8 |

### Example 9: Evaluation of degree of hydrophobicity of ADC mimic by hydrophobic column chromatography (HIC-HPLC)

HIC-HPLC analysis was performed using the condition of Example 8. Measurement was performed using the following conditions. A degree of hydrophobicity of ADC mimic can be evaluated by retention time of ADC in HIC chromatogram.

**[Table 7]**

| Table 7. Evaluation of degree of hydrophobicity of trastuzumab-based ADC mimic using HIC-HPLC | | | |
|---|---|---|---|
| | Linker-payload mimic | Example/ Comparative Example | Retention time |
| ADC mimic 6 | Linker-payload mimic (1) | Example 7-1 | 11.6 minutes |
| ADC mimic 7 | Linker-payload mimic (11) | Example 7-2 | 10.9 minutes |
| ADC mimic 8 | Linker-payload mimic (21) | Example 7-3 | 11.9 minutes |
| ADC mimic 15 | Linker-payload mimic (30) | Comparative Example 2-1 | 16.5 minutes |
| ADC mimic 18 | Linker-payload mimic (31) | Comparative Example 2-4 | 16.2 minutes |

It is found that ADC mimics 6, 7, and 8, which are exo-type ADCs, have a shorter retention time in the HIC chromatogram, and are more hydrophilic ADC mimics than ADC mimics 15 and 18, which are linear ADCs.

**[Table 8]**

| Table 8. Evaluation of degree of hydrophobicity of rituximab-based ADC mimic using HIC-HPLC | | | |
|---|---|---|---|
| | Linker-payload mimic | Example/ Comparative Example | Retention time |
| ADC mimic 9 | Linker-payload mimic (1) | Example 7-4 | 11.6 minutes |
| ADC mimic 10 | Linker-payload mimic (11) | Example 7-5 | 11.3 minutes |
| ADC mimic 11 | Linker-payload mimic (21) | Example 7-6 | 11.6 minutes |
| ADC mimic 16 | Linker-payload mimic (30) | Comparative Example 2-1 | 16.4 minutes |
| ADC mimic 19 | Linker-payload mimic (31) | Comparative Example 2-4 | 16.0 minutes |

It is found that ADC mimics 9, 10, and 11, which are exo-type ADCs, have a shorter retention time in the HIC chromatogram, and are more hydrophilic ADC mimics than ADC mimics 16 and 19, which are linear ADCs.

**[Table 9]**

| Table 9. Evaluation of degree of hydrophobicity of infliximab-based ADC mimic using HIC-HPLC | | | |
|---|---|---|---|
| | Linker-payload mimic | Example/ Comparative Example | Retention time |
| ADC mimic 12 | Linker-payload mimic (1) | Example 7-7 | 11.9 minutes |
| ADC mimic 13 | Linker-payload mimic (11) | Example 7-8 | 11.3 minutes |
| ADC mimic 14 | Linker-payload mimic (21) | Example 7-9 | 11.8 minutes |
| ADC mimic 17 | Linker-payload mimic (30) | Comparative Example 2-1 | 16.4 minutes |
| ADC mimic 20 | Linker-payload mimic (31) | Comparative Example 2-4 | 16.2 minutes |

It is found that ADC mimics 12, 13, and 14, which are exo-type ADCs, have a shorter retention time in the HIC chromatogram, and are more hydrophilic ADC mimics than ADC mimics 17 and 20, which are linear ADCs.

As a result, it has been confirmed that retention times of the ADC mimics synthesized in Examples 7-1, 7-2, 7-3, 7-4, 7-5, 7-6, 7-7, 7-8, and 7-9 tend to be short, and it is found that the ADC mimics synthesized in Examples 7-1, 7-2, 7-3, 7-4, 7-5, 7-6, 7-7, 7-8, and 7-9 have high degrees of hydrophilicity. Therefore, it has been confirmed that the ADC mimics synthesized in Examples 7-1, 7-2, 7-3, 7-4, 7-5, 7-6, 7-7, 7-8, and 7-9 are preferable ADCs because it is considered that the ADC mimics synthesized in Examples 7-1, 7-2, 7-3, 7-4, 7-5, 7-6, 7-7, 7-8, and 7-9 have slow plasma clearances and long times during which the ADC mimics remain in a body.

### Example 10: Evaluation of aggregation ratios of ADC and ADC mimic by size exclusion chromatography (SEC-HPLC)

SEC-HPLC analysis was performed according to a previous report (Chemistry Select, 2020, 5, 8435-8439). Measurement was performed using the following conditions.

### Measurement system: 1260 HPLC system (manufactured by Agilent)

Column: AdvanceBio SEC 300 Å, 2.7 µm, 4.6 mm × 150 mm, manufactured by Agilent Technologies
Flow rate: 0.25 mL/min
Eluent: 100 mM sodium dihydrogen phosphate/sodium hydrogen phosphate, aqueous solution of 250 mM sodium chloride (pH 6.8), 10% v/v isopropanol
Detector: UV (280 nm)

**[Table 10]**

| Table 10. Evaluation of aggregation ratio of trastuzumab-based ADC mimic using SEC-HPLC | | | |
|---|---|---|---|
| | Linker-payload mimic | Example/ Comparative Example | Aggregation ratio |
| ADC mimic 6 | Linker-payload mimic (1) | Example 7-1 | 0.7% |
| ADC mimic 7 | Linker-payload mimic (11) | Example 7-2 | 0.8% |
| ADC mimic 8 | Linker-payload mimic (21) | Example 7-3 | 0.4% |
| ADC mimic 15 | Linker-payload mimic (30) | Comparative Example 2-1 | 100% |
| ADC mimic 18 | Linker-payload mimic (31) | Comparative Example 2-4 | 20% |

**[Table 11]**

| Table 11. Evaluation of aggregation ratio of rituximab-based ADC mimic using SEC-HPLC | | | |
|---|---|---|---|
| | Linker-payload mimic | Example/ Comparative Example | Aggregation ratio |
| ADC mimic 9 | Linker-payload mimic (1) | Example 7-4 | 0.6% |
| ADC mimic 10 | Linker-payload mimic (11) | Example 7-5 | 1.1% |
| ADC mimic 11 | Linker-payload mimic (21) | Example 7-6 | 1.4% |
| ADC mimic 16 | Linker-payload mimic (30) | Comparative Example 2-1 | 100% |
| ADC mimic 19 | Linker-payload mimic (31) | Comparative Example 2-4 | 12% |

**[Table 12]**

| Table 12. Evaluation of aggregation ratio of intliximab-based ADC mimic using SEC-HPLC | | | |
|---|---|---|---|
| | Linker-payload mimic | Example/ Comparative Example | Aggregation ratio |
| ADC mimic 12 | Linker-payload mimic (1) | Example 7-7 | 2.1% |
| ADC mimic 13 | Linker-payload mimic (11) | Example 7-8 | 1.9% |
| ADC mimic 14 | Linker-payload mimic (21) | Example 7-9 | 0.3% |
| ADC mimic 17 | Linker-payload mimic (30) | Comparative Example 2-1 | 100% |
| ADC mimic 20 | Linker-payload mimic (31) | Comparative Example 2-4 | 16% |

As a result, it has been confirmed that retention times of the ADC mimics synthesized in Examples 7-1, 7-2, 7-3, 7-4, 7-5, 7-6, 7-7, 7-8, and 7-9 tend to have low aggregation ratios, and it is found that the ADC mimics synthesized in Examples 7-1, 7-2, 7-3, 7-4, 7-5, 7-6, 7-7, 7-8, and 7-9 have higher stability. Therefore, it has been confirmed that the ADC mimics synthesized in Examples 7-1, 7-2, 7-3, 7-4, 7-5, 7-6, 7-7, 7-8, and 7-9 are preferable ADCs.

### Example 11: Evaluation of ADC mimic using enzyme cathepsin B

Cleavabilities for various ADC mimics by cathepsin B were evaluated by analyzing the amount of fluorescent molecules dropped from the ADC mimics as described below.

### Example 11-1: Cathepsin B Cleavability Test

A cathepsin B cleavability test was performed as follows according to a previous report (Nature Communications 2018, 9, 2512). ADC mimic 7 was added to 180 µL of a MES buffer (10 mM MES, 40 µM DTT, pH 5.0) so as to have a concentration of 1 mg/mL, and then 30 µL of the mixture was poured into each of six Eppendorf tubes. To each of three of the six samples, 100 µL of acetonitrile was immediately added at 0°C. The mixture was stirred by vortex, and then centrifuged to obtain a precipitate. The resulting supernatant solution was collected and subjected to HPLC analysis. The remaining three samples were incubated at 37°C for six hours. To each of the samples, 100 µL of acetonitrile was added. The mixture was stirred by vortex, and then centrifuged to obtain a precipitate. The resulting supernatant solution was collected and subjected to HPLC analysis.

### Example 11-2: Analysis of amount of dropped fluorescent molecules using HPLC analysis

For the measurement, the amount of fluorescent molecules dropped from ADC mimic 7 was measured using a liquid chromatography/fluorescence detection method. The three samples to which acetonitrile was immediately added at 0°C in Example 11-1 were taken as 0 hour samples, and the three samples incubated at 37°C for six hours as described in Example 11-1 were taken as 6 hour samples. A difference in fluorescence intensity between the 6 hour samples and the 0 hour samples was analyzed.

Separately, a correlation between the area of a fluorescence intensity area by HPLC and a concentration was calculated using Pyrene. The difference in fluorescence intensity between the ADC mimics was converted into a concentration using the calculation formula. When the concentration at 0 hour was set to 100%, a ratio of the above-described difference in fluorescence intensity was calculated as a dropping ratio. The dropping ratio of fluorescent molecules from ADC mimic 7 in six hours was 400%, indicating that ADC mimic 7 had sufficient cathepsin B cleavage.

### Example 12: Synthesis of Linker-payload

### (12-1) Synthesis of Linker-payload (35)

Linker-payload (35) was synthesized as follows.

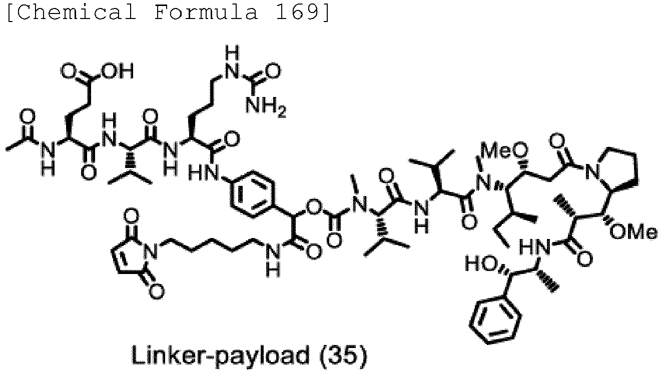

### (12-1-1) Synthesis of carbonate (36)

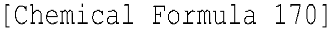

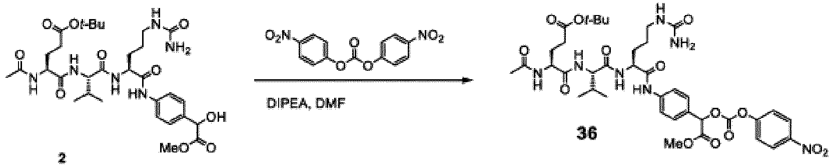

Alcohol (2) (105 mg, 0.158 mmol) obtained in (1-1-1) was dissolved in N,N-dimethylformamide (2 mL), and the solution was stirred under ice cooling for five minutes. Thereafter, bis(4-nitrophenyl) carbonate (100 mg, 0.329 mmol) and N,N-diisopropylethylamine (83 µL, 0.48 mmol) were added thereto, and the mixture was stirred under a nitrogen atmosphere at room temperature for 19.5 hours. N,N-dimethylformamide was removed by an evaporator. Thereafter, ethyl acetate (3 mL) was added to the obtained crude product to dissolve the crude product therein, and then diethyl ether (3 mL) was added thereto. The obtained solution was subjected to filtration to remove a residue. Thereafter, an organic solvent was removed by a vacuum pump to obtain carbonate (36) (102 mg, 0.123 mmol).

MS(ESI) m/z:830.1[M+H]⁺,852.1[M+Na]⁺

### (12-1-2) Synthesis of compound (37)

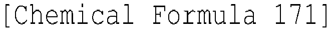

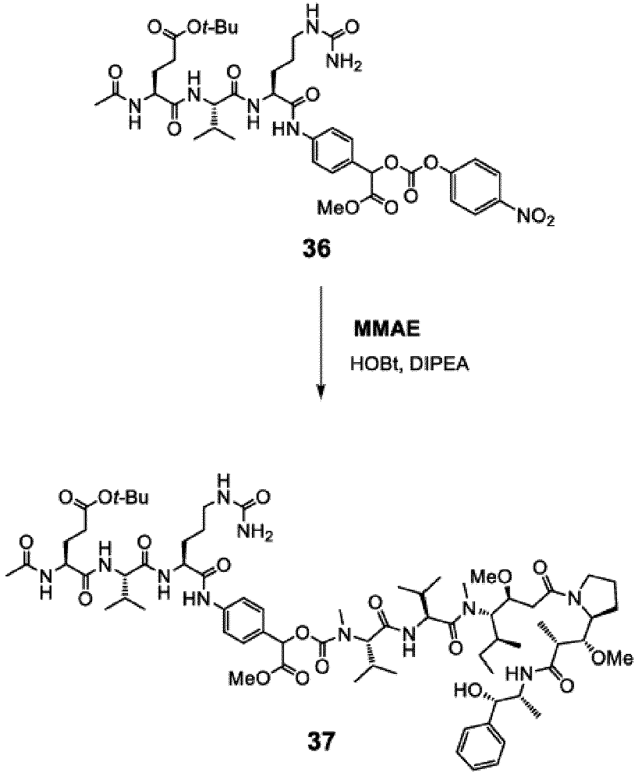

Compound (36) (69 mg, 0.083 mmol) obtained in (12-1-1) was dissolved in N,N-dimethylformamide (3.5 mL), and 1-hydroxybenzotriazole (16 mg, 0.10 mmol) and commercially available monomethylauristatin E (MMAE, 61 mg, 0.085 mmol) were added thereto at room temperature. Subsequently, diisopropylethylamine (29 µL, 0.17 mmol) was added thereto, and then the mixture was stirred at room temperature under a nitrogen atmosphere for 22.5 hours. The organic solvent was removed by an evaporator. Thereafter, a solution of acetonitrile : water = 1 : 1 was added to the residue, and the mixture was purified by reverse phase preparative chromatography. A fraction comprising a product was collected and concentrated under reduced pressure to remove acetonitrile. The residue was freeze-dried to obtain compound 37 (79 mg, 0.056 mmol).
MS(ESI) m/z:1408.9[M+H]⁺

### (12-1-3) Synthesis of compound (38)

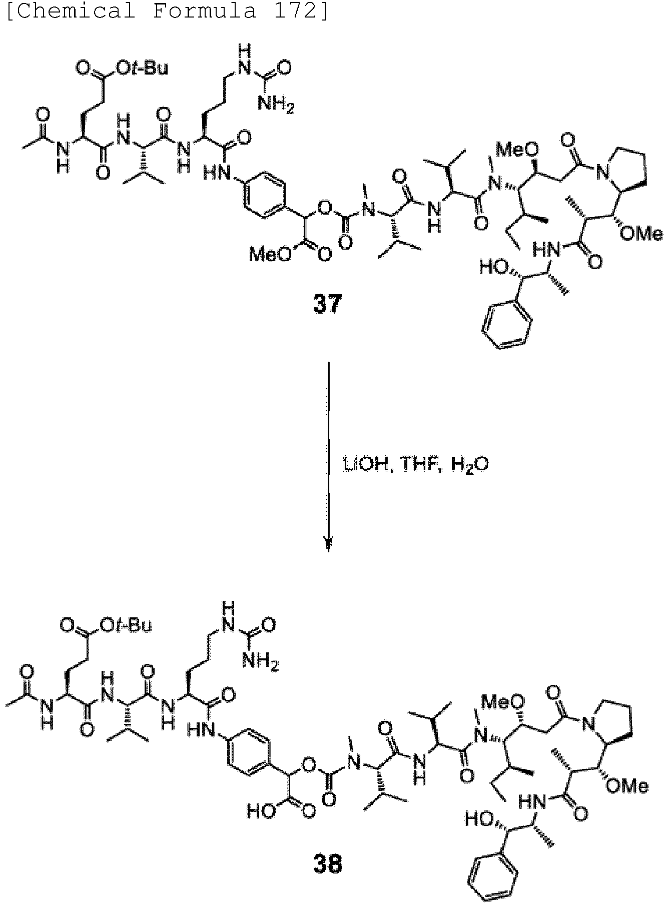

Compound (37) (97 mg, 0.069 mmol) obtained in (12-1-2) was dissolved in tetrahydrofuran (7 mL) and water (2 mL). Lithium hydroxide (1.0 M, 1.4 mL, 1.4 mmol) was added thereto under ice cooling, and the mixture was stirred for one hour as it was. Hydrochloric acid was added to the reaction solution to adjust the pH to 6. Thereafter, acetonitrile : water = 1 : 1 was added thereto, and the mixture was purified by reverse phase preparative chromatography. A fraction comprising a product was collected and concentrated under reduced pressure to remove acetonitrile. The residue was freeze-dried to obtain compound 38 (70 mg, 0.050 mmol).

MS(ESI) m/z:1394.7[M+H]⁺

### (12-1-4) Synthesis of compound (39)

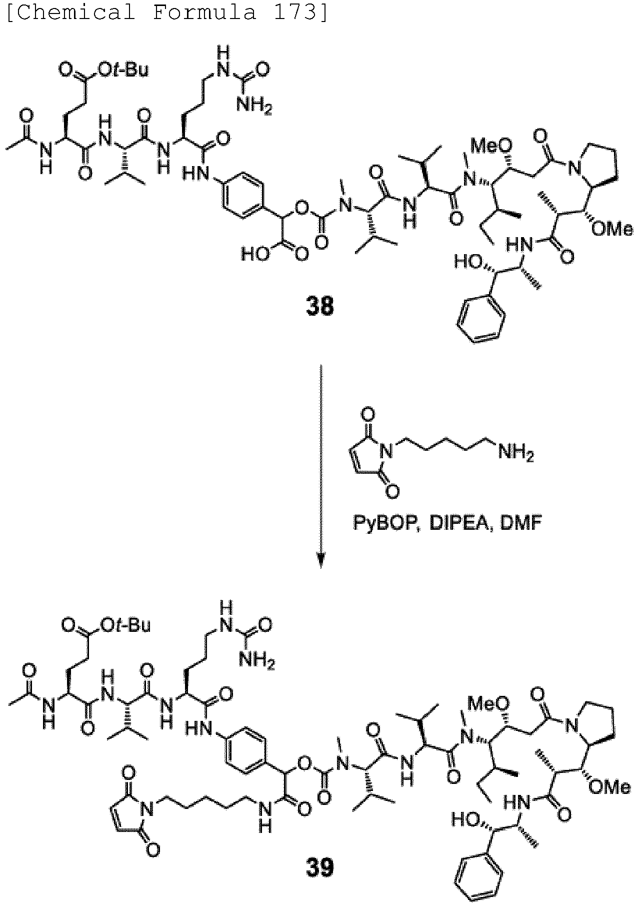

Compound (38) (34 mg, 0.024 mmol) obtained in (12-1-3) was dissolved in N,N-dimethylformamide (3 mL). Thereafter, the solution was ice-cooled, and N,N-diisopropylethylamine (25 µL, 0.14 mmol) and 1H-benzotriazol-1-yloxy tripyrrolidinophosphonium hexafluorophosphate (20 mg, 0.038 mmol) were added thereto. Next, N-(5-aminopentyl) maleimide hydrochloride (8.7 mg, 0.040 mmol) was added thereto, the temperature was returned to room temperature, and the mixture was stirred for 20 hours. After completion of the reaction, purification was performed by reverse phase preparative chromatography. A fraction comprising a product was collected and concentrated under reduced pressure to remove acetonitrile. Thereafter, the resulting residue was freeze-dried to obtain compound (39) (29 mg, 0.019 mmol).

MS(ESI) m/z:1558.9[M+H]⁺

### (12-1-5) Synthesis of Linker-payload (35)

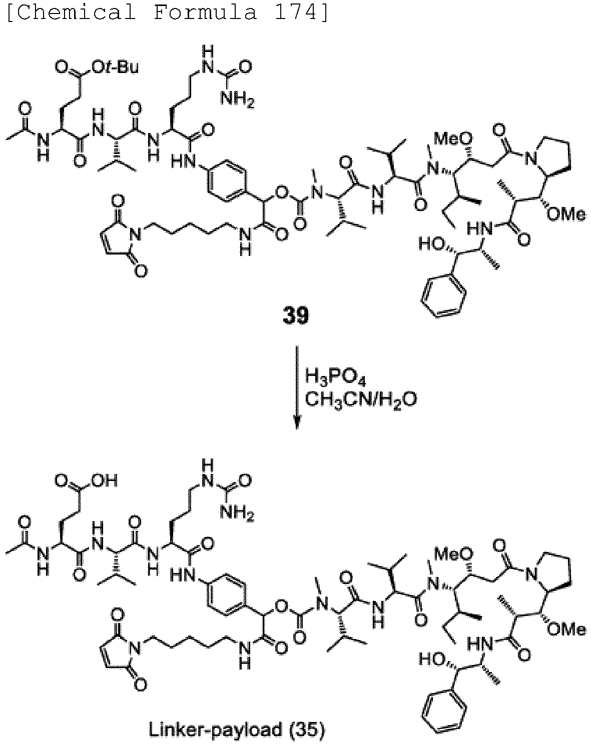

To compound (39) (29 mg, 0.019 mmol) obtained in (12-1-4), acetonitrile (500 µL) and an 85 wt% phosphoric acid aqueous solution (0.50 mL, 7.3 mmol) were sequentially added, and the mixture was stirred at room temperature for three hours. After completion of the reaction, water (2 mL) was added thereto. The reaction solution was purified by reverse phase preparative chromatography, a fraction comprising a product was collected and concentrated under reduced pressure to remove acetonitrile. Thereafter, the residue was freeze-dried to obtain Linker-payload (35) (18 mg, 0.012 mmol).

MS(ESI) m/z:1502.9[M+H]⁺

### (12-2) Synthesis of Linker-payload (40) Linker-payload (40) was synthesized as follows.

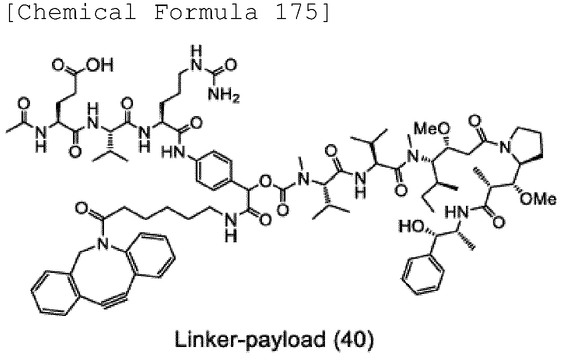

### (12-2-1) Synthesis of compound (41)

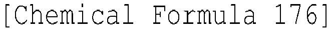

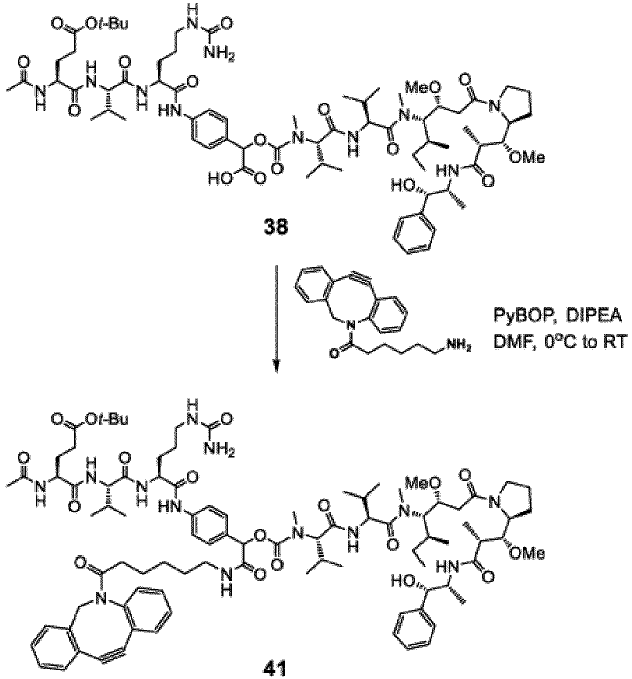

Compound (38) (10 mg, 0.0072 mmol) obtained in (12-1-3) was dissolved in N,N-dimethylformamide (1 mL). Thereafter, the solution was ice-cooled, and N,N-diisopropylethylamine (10 µL, 0.057 mmol) and 1H-benzotriazol-1-yloxy tripyrrolidinophosphonium hexafluorophosphate (7.0 mg, 0.013 mmol) were added thereto. Next, an N,N-dimethylformamide (0.5 mL) solution of DBCO-hexylamine (4.7 mg, 0.015 mmol) was added thereto, the temperature was returned to room temperature, and the mixture was stirred for 20 hours. After completion of the reaction, purification was performed by reverse phase preparative chromatography. A fraction comprising a product was collected and concentrated under reduced pressure to remove acetonitrile. Thereafter, the resulting residue was freeze-dried to obtain compound (41) (5.8 mg, 0.0034 mmol).

MS(ESI) m/z:1696.0[M+H]⁺

### (12-2-2) Synthesis of Linker-payload (40)

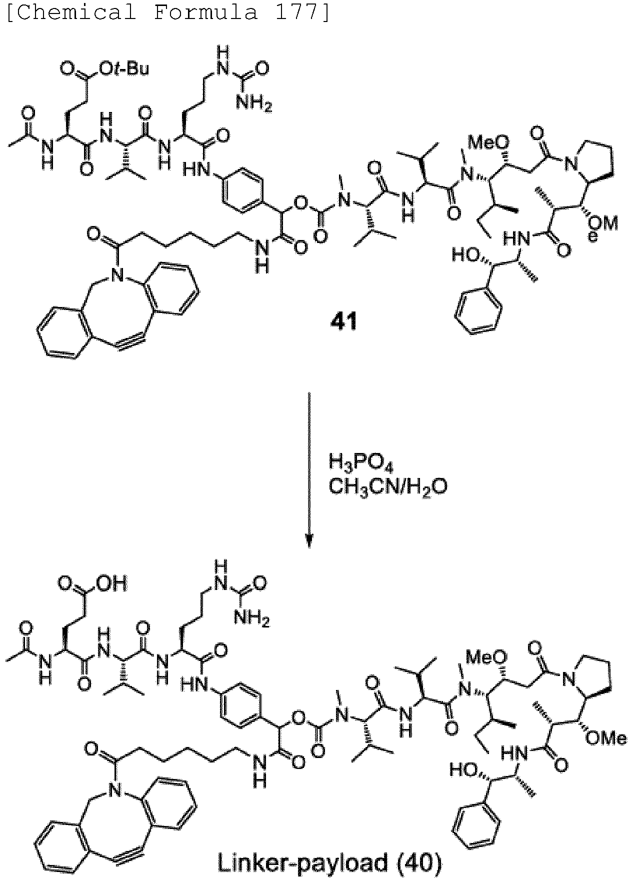

To compound (41) (13 mg, 0.0077 mmol) obtained in (12-2-1), acetonitrile (500 µL) and an 85 wt% phosphoric acid aqueous solution (0.50 mL, 7.3 mmol) were sequentially added, and the mixture was stirred at room temperature for four hours. After completion of the reaction, water (2 mL) was added thereto. The reaction solution was purified by reverse phase preparative chromatography, a fraction comprising a product was collected and concentrated under reduced pressure to remove acetonitrile. Thereafter, the residue was freeze-dried to obtain Linker-payload (40) (7.4 mg, 0.0045 mmol).

MS(ESI) m/z:1638.9[M+H]⁺

### (12-3) Synthesis of Linker-payload (42)

Linker-payload (42) was synthesized as follows.

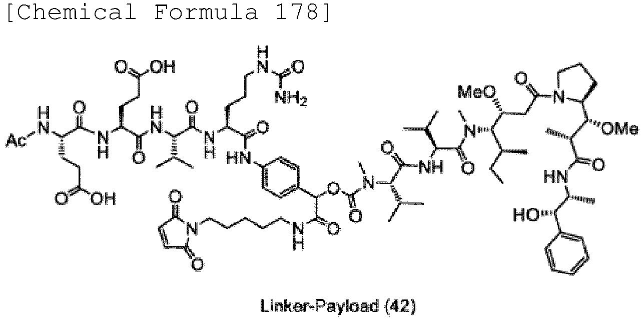

### (12-3-1) Synthesis of carbonate (43)

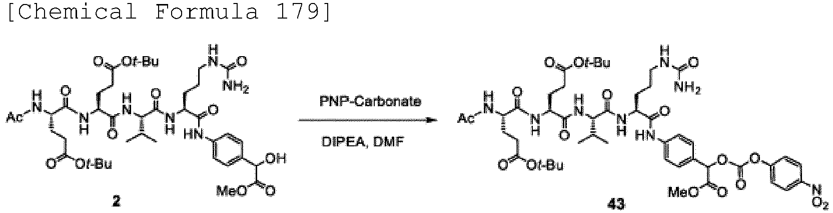

Alcohol (2) (140 mg, 0.165 mmol) obtained in (1-1-1) was dissolved in N,N-dimethylformamide (4 mL), and the solution was stirred under ice cooling for five minutes. Thereafter, bis(4-nitrophenyl) carbonate (108 mg, 0.355 mmol) and N,N-diisopropylethylamine (100 µL, 0.574 mmol) were added thereto, and the mixture was stirred under a nitrogen atmosphere at room temperature for 18 hours. The organic solvent was removed by an evaporator. Thereafter, a solution of acetonitrile : water = 1 : 1 was added to the residue, and the mixture was purified by reverse phase preparative chromatography. A fraction comprising a product was collected and concentrated under reduced pressure to remove acetonitrile. The residue was freeze-dried to obtain compound 43 (130 mg, 0.128 mmol).

MS(ESI) m/z:1015.6[M+H]⁺

### (12-3-2) Synthesis of compound (44)

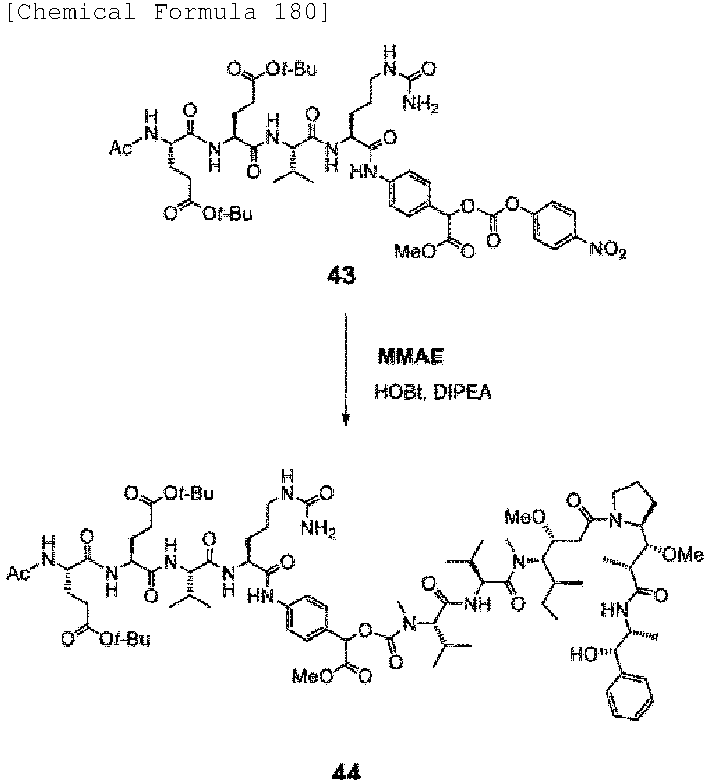

Compound (43) (85 mg, 0.084 mmol) obtained in (12-3-1) was dissolved in N,N-dimethylformamide (2 mL), and 1-hydroxybenzotriazole (20 mg, 0.13 mmol) and commercially available monomethylauristatin E (MMAE, 63 mg, 0.088 mmol) were added thereto at room temperature. Subsequently, diisopropylethylamine (75 µL, 0.43 mmol) was added thereto, and then the mixture was stirred at room temperature under a nitrogen atmosphere for 23 hours. The organic solvent was removed by an evaporator. Thereafter, a solution of acetonitrile : water = 1 : 1 was added to the residue, and the mixture was purified by reverse phase preparative chromatography. A fraction comprising a product was collected and concentrated under reduced pressure to remove acetonitrile. The residue was freeze-dried to obtain compound 44 (94 mg, 0.059 mmol).

MS(ESI) m/z:1593.6[M+H]⁺

### (12-3-3) Synthesis of compound (45)

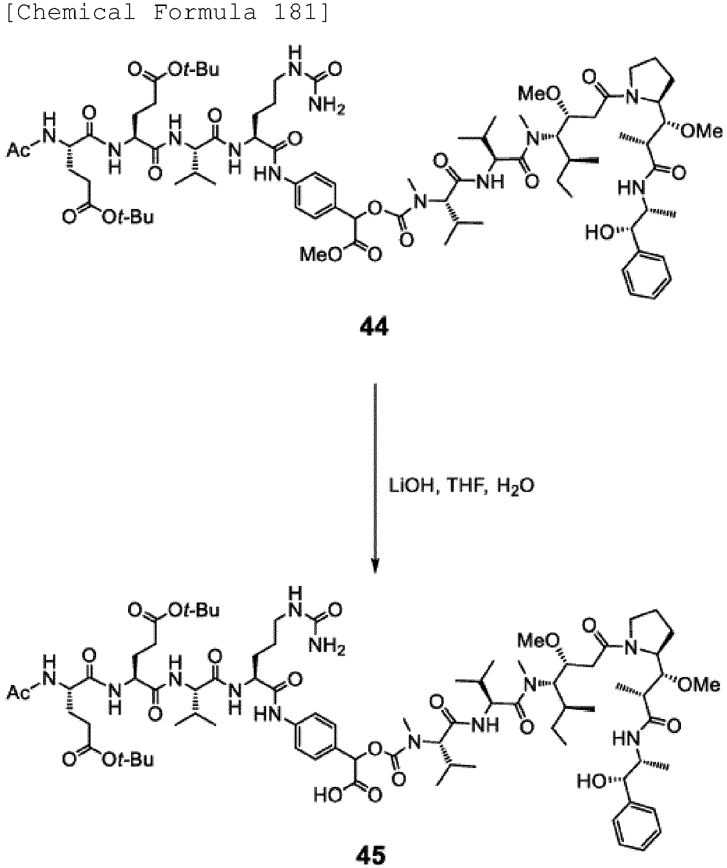

Compound (44) (94 mg, 0.059 mmol) obtained in (12-3-2) was dissolved in tetrahydrofuran (5 mL) and water (2 mL), and lithium hydroxide (1.0 M, 0.6 mL, 0.6 mmol) was added thereto under ice cooling, and the mixture was stirred for one hour as it was. Hydrochloric acid was added to the reaction solution to adjust the pH to 5. Thereafter, acetonitrile : water = 1 : 1 was added thereto, and the mixture was purified by reverse phase preparative chromatography. A fraction comprising a product was collected and concentrated under reduced pressure to remove acetonitrile. The residue was freeze-dried to obtain Compound 45 (77 mg, 0.049 mmol).

MS(ESI) m/z:1579.7[M+H]⁺

### (12-3-4) Synthesis of compound (46)

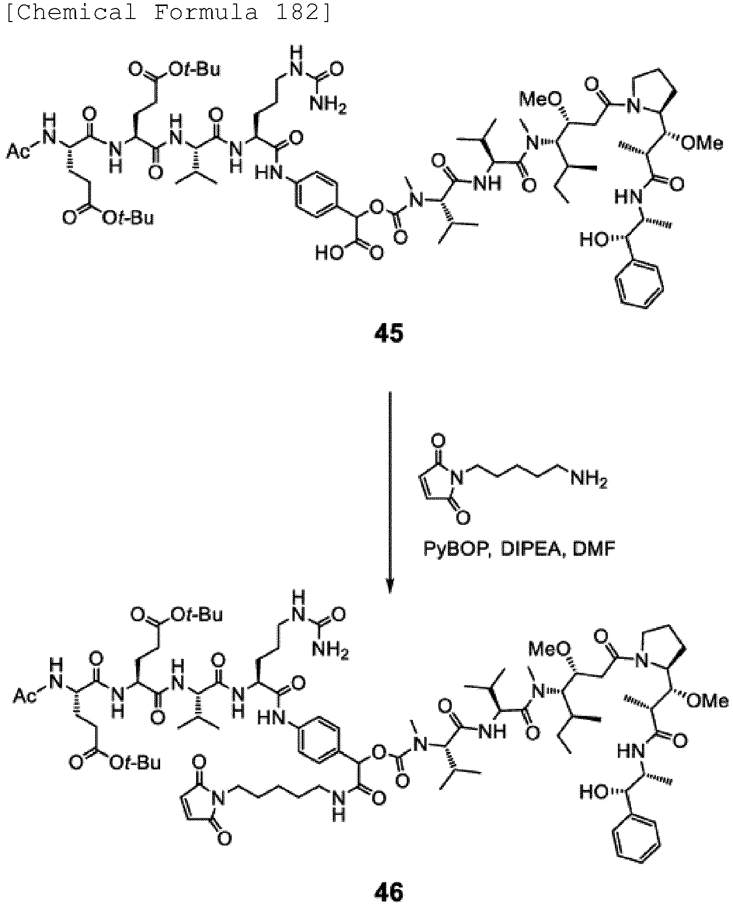

Compound (45) (77 mg, 0.049 mmol) obtained in (12-3-3) was dissolved in N,N-dimethylformamide (3 mL). Thereafter, the solution was ice-cooled, and N,N-diisopropylethylamine (50 µL, 0.29 mmol) and 1H-benzotriazol-1-yloxy tripyrrolidinophosphonium hexafluorophosphate (87 mg, 0.17 mmol) were added thereto. Next, N-(5-aminopentyl) maleimide hydrochloride (35 mg, 0.16 mmol) was added thereto, the temperature was returned to room temperature, and the mixture was stirred for 20 hours. After completion of the reaction, purification was performed by reverse phase preparative chromatography. A fraction comprising a product was collected and concentrated under reduced pressure to remove acetonitrile. Thereafter, the resulting residue was freeze-dried to obtain compound (46) (65 mg, 0.037 mmol).

MS(ESI) m/z:1744.7[M+H]⁺

### (12-3-5) Synthesis of Linker-payload (42)

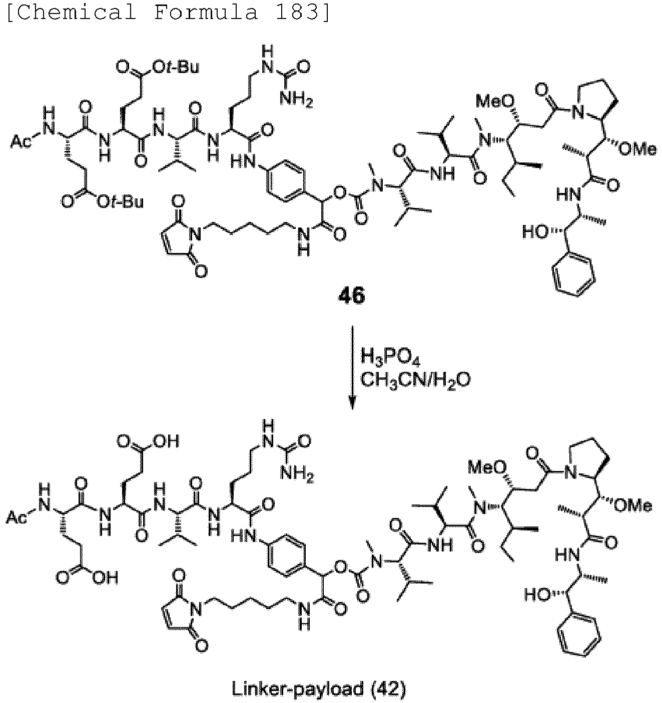

To compound (46) (65 mg, 0.037 mmol) obtained in (12-3-4), acetonitrile (2 mL) and an 85 wt% phosphoric acid aqueous solution (1.00 mL, 14.6 mmol) were sequentially added, and the mixture was stirred at room temperature for six hours. After completion of the reaction, water (2 mL) was added thereto. The reaction solution was purified by reverse phase preparative chromatography, a fraction comprising a product was collected and concentrated under reduced pressure to remove acetonitrile. Thereafter, the residue was freeze-dried to obtain Linker-payload (42) (49 mg, 0.030 mmol).

MS(ESI) m/z:1631.6[M+H]⁺

### (12-4) Synthesis of Linker-payload (47)

Linker-payload (47) was synthesized as follows.

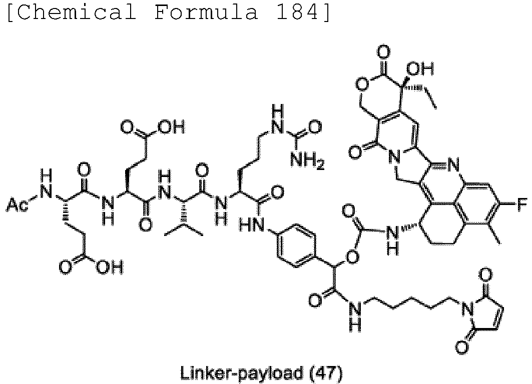

### (12-4-1) Synthesis of compound (48)

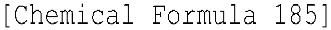

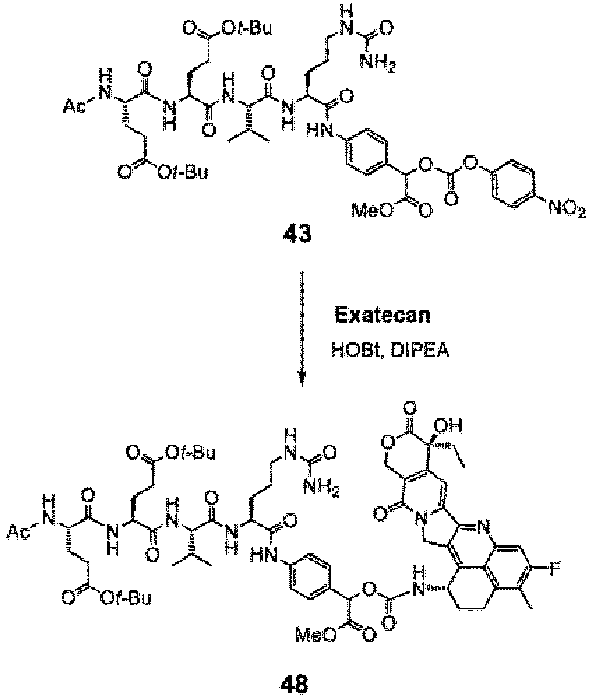

Compound (43) (67 mg, 0.066 mmol) obtained in (12-3-1) was dissolved in N,N-dimethylformamide (2 mL), and 1-hydroxybenzotriazole (17 mg, 0.11 mmol) and commercially available Exatecan mesylate (CAS: 169869-90-3, 35mg, 0.066mmol) were added thereto at room temperature. Subsequently, diisopropylethylamine (50 µL, 0.29 mmol) was added thereto, and then the mixture was stirred at room temperature under a nitrogen atmosphere for four hours. The organic solvent was removed by an evaporator. Thereafter, a solution of acetonitrile : water = 1 : 1 was added to the residue, and the mixture was purified by reverse phase preparative chromatography. A fraction comprising a product was collected and concentrated under reduced pressure to remove acetonitrile. The residue was freeze-dried to obtain compound 48 (57 mg, 0.043 mmol).

MS(ESI) m/z:1311.7[M+H]⁺

### (12-4-2) Synthesis of compound (49)

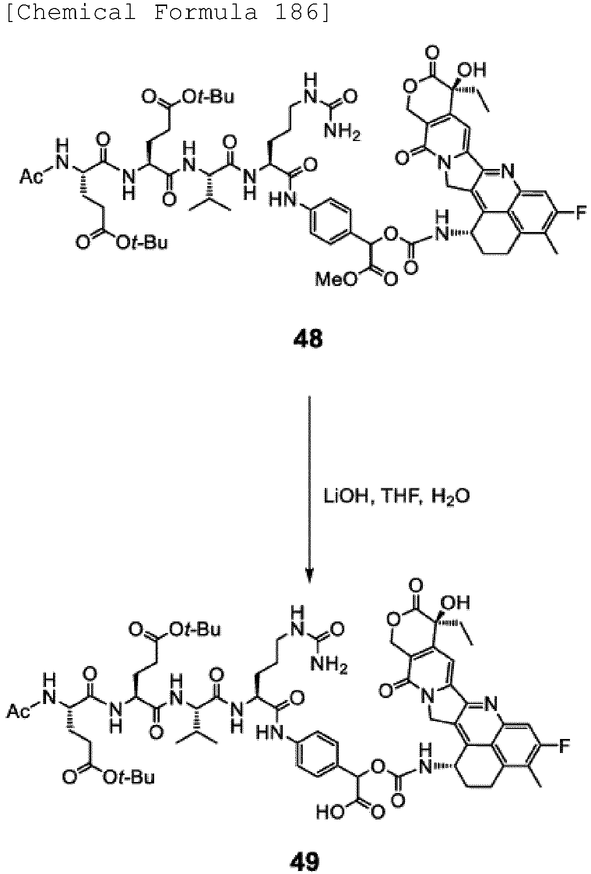

Compound (48) (57 mg, 0.043 mmol) obtained in (12-4-1) was dissolved in tetrahydrofuran (3 mL) and water (1.5 mL), and lithium hydroxide (1.0 M, 0.5 mL, 0.5 mmol) was added thereto under ice cooling, and the mixture was stirred for one hour as it was. Hydrochloric acid was added to the reaction solution to adjust the pH to 5. Thereafter, acetonitrile : water = 1 : 1 was added thereto, and the mixture was purified by reverse phase preparative chromatography. A fraction comprising a product was collected and concentrated under reduced pressure to remove acetonitrile. The residue was freeze-dried to obtain compound 49 (45 mg, 0.035 mmol).

MS(ESI) m/z:1297.6[M+H]⁺

### (12-4-3) Synthesis of compound (50)

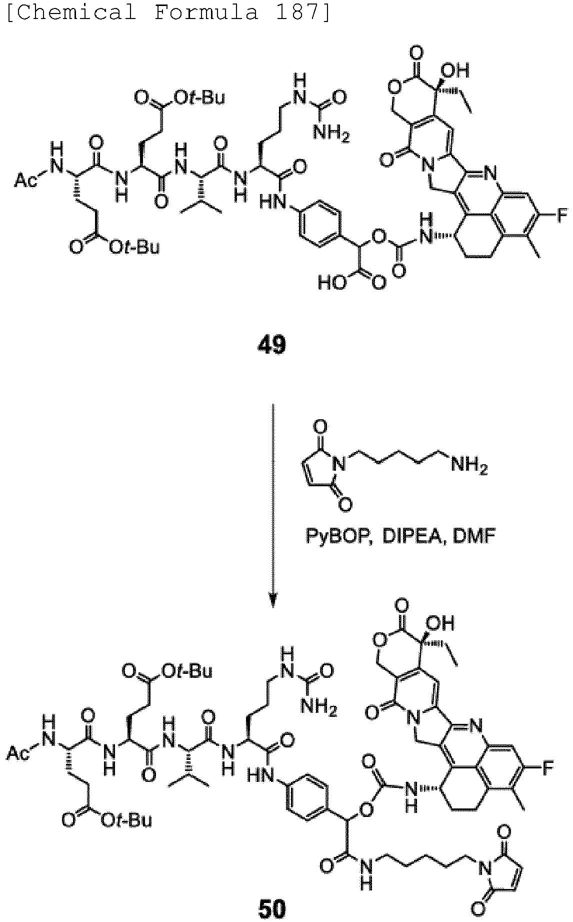

Compound (49) (45 mg, 0.035 mmol) obtained in (12-4-2) was dissolved in N,N-dimethylformamide (3 mL). Thereafter, the solution was ice-cooled, and N,N-diisopropylethylamine (30 µL, 0.17 mmol) and 1H-benzotriazol-1-yloxy tripyrrolidinophosphonium hexafluorophosphate (55 mg, 0.11 mmol) were added thereto. Next, N-(5-aminopentyl) maleimide hydrochloride (22 mg, 0.10 mmol) was added thereto, the temperature was returned to room temperature, and the mixture was stirred for 18 hours. After completion of the reaction, purification was performed by reverse phase preparative chromatography. A fraction comprising a product was collected and concentrated under reduced pressure to remove acetonitrile. Thereafter, the resulting residue was freeze-dried to obtain compound (50) (22 mg, 0.015 mmol).

MS(ESI) m/z:1462.7[M+H]⁺

### (12-3-5) Synthesis of Linker-payload (47)

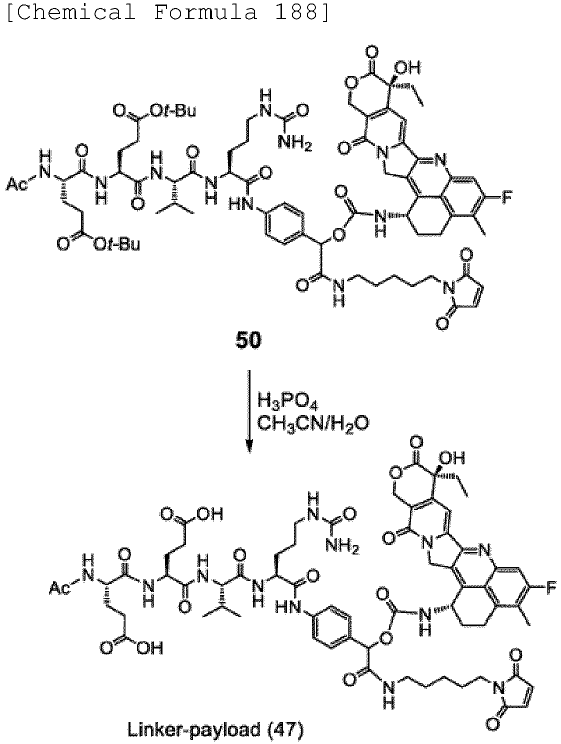

To compound (50) (22 mg, 0.015 mmol) obtained in (12-4-3), acetonitrile (1 mL) and an 85 wt% phosphoric acid aqueous solution (1.0 mL, 14.6 mmol) were sequentially added, and the mixture was stirred at room temperature for 1.5 hours. After completion of the reaction, water (1 mL) was added thereto. The reaction solution was purified by reverse phase preparative chromatography, a fraction comprising a product was collected and concentrated under reduced pressure to remove acetonitrile. Thereafter, the residue was freeze-dried to obtain Linker-payload (47) (18.8 mg, 0.0139 mmol).

¹H NMR(300MHz;DMSO-d6) δ 10.04(s,1H),8.10-8.05(m,4H),7.80-7.77(m,2H),7.59-7.55(m,2H),7.35-7.30(m,3H),6.99(d,J=8.8Hz,2H),6.54-6.53(m,1H),6.01(brs,1H),5.74(d,J=9.0Hz,1H),5.43(brs,4H),5.3 3-5.28(m,2H),4.29-4.15(m,4H),3.19-2.98(m5H),2.43-2.38(m,5H),2.27-2.20(m,7H),1.95-1.83(m,8H),1.73-1.62(m,4H),1.42-1.30(m,7H),1.23-1.13(m,3H),0.84(m,9H).

MS(ESI) m/z:1349.2[M+H]⁺

### (12-5) Synthesis of Linker-payload (125)

Linker-payload (125) described below was synthesized in a similar manner to the synthesis of Linker-payload mimic (120) using MMAE in place of sarcosine-pyrene.

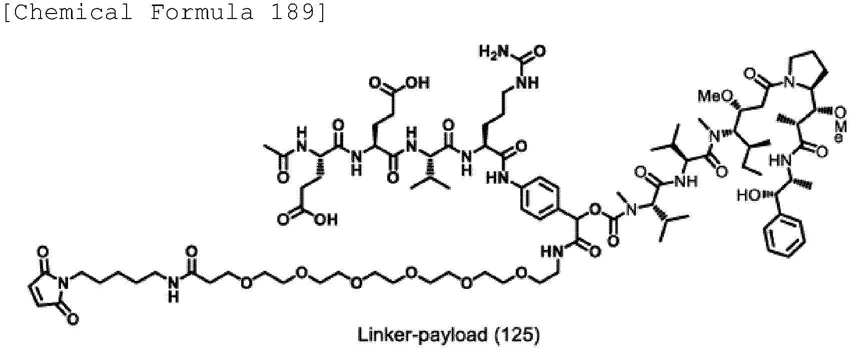

MS(ESI)m/z:1968.14[M+H]⁺

### (12-6) Synthesis of Linker-payload (126)

Linker-payload (126) described below was synthesized in a similar manner to the synthesis of Linker-payload mimic (35) using Exatecan mesylate in place of MMAE.

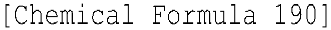

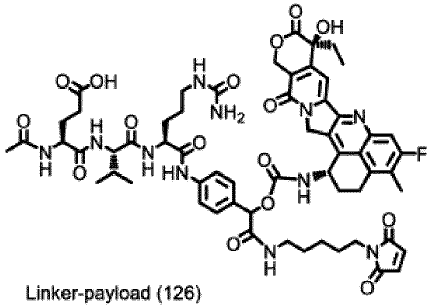

MS(ESI)m/z:1220.50[M+H]⁺

### Example 13: Synthesis of ADC

### (13-1) Synthesis of ADC1

To a 3 mg/mL PBSE buffer (50 mM phosphate buffered saline (PBS), 10 mM EDTA, pH = 7.4) of anti-HER2 IgG antibody trastuzumab (Chugai Pharmaceutical), a PBSE solution (2 mM) of a reducing agent (TCEP) was added in an amount of 8 equivalents to the antibody, and the mixture was incubated at 37°C for one hour. To the resulting reaction mixture, dimethylacetamide (DMA) (6% v/v) and a 10 mM DMA solution of Linker-payload mimic (35) (20 equivalents to the antibody) were sequentially added and stirred gently at 20°C for one hour. An excess amount of N-acetylcysteine was added to the reaction mixture, and the mixture was stirred at 25°C for 20 minutes. The final mixture was purified using a NAP-5 desalting column (manufactured by GE Healthcare) and eluted with a formulation buffer (20 mM histidine, comprising 5 wt% trehalose, pH 5.2) to obtain ADC mimic 1.

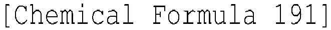

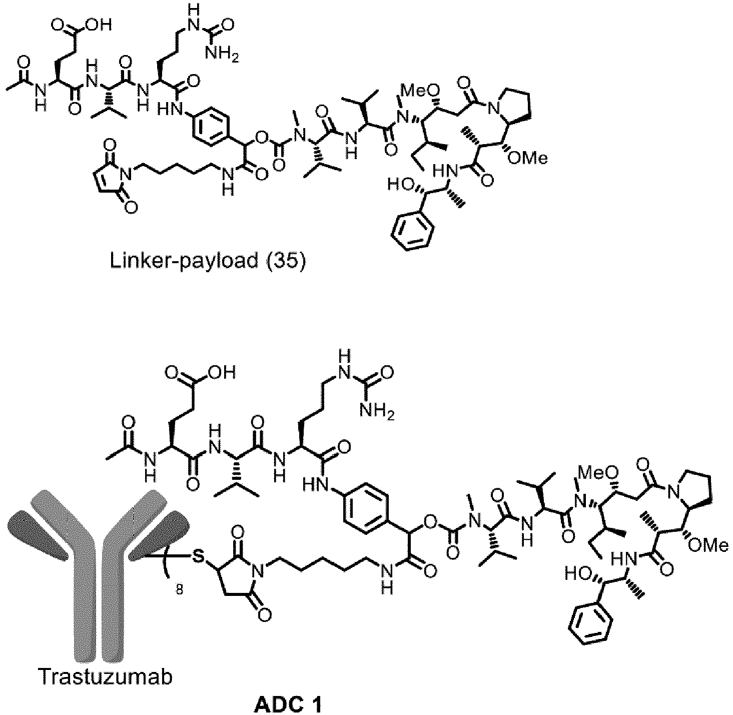

### (13-2) Synthesis of ADC2

According to (13-1), ADC2 was obtained from Linker-payload (42).

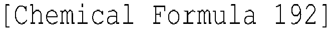

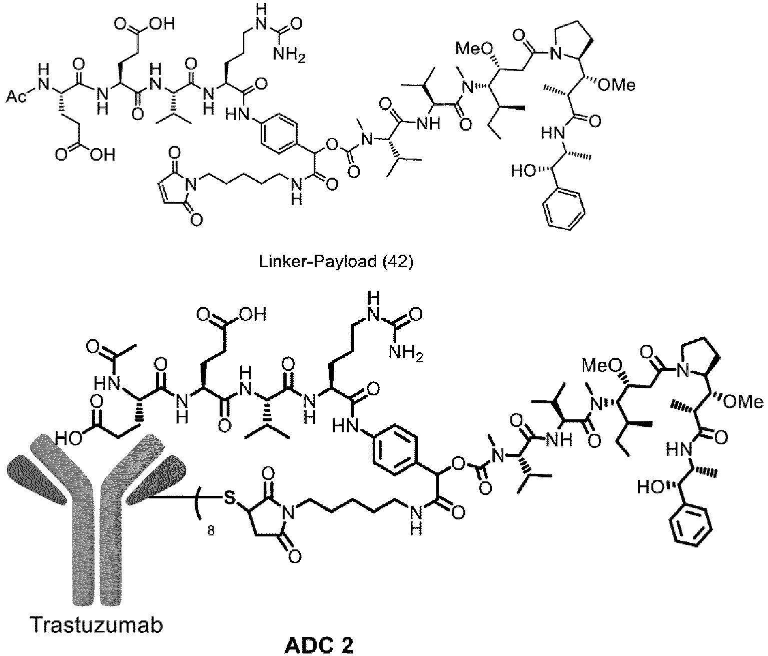

### (13-3) Synthesis of ADC3

According to (13-1), ADC3 was obtained from Linker-payload (47).

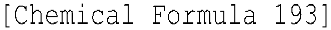

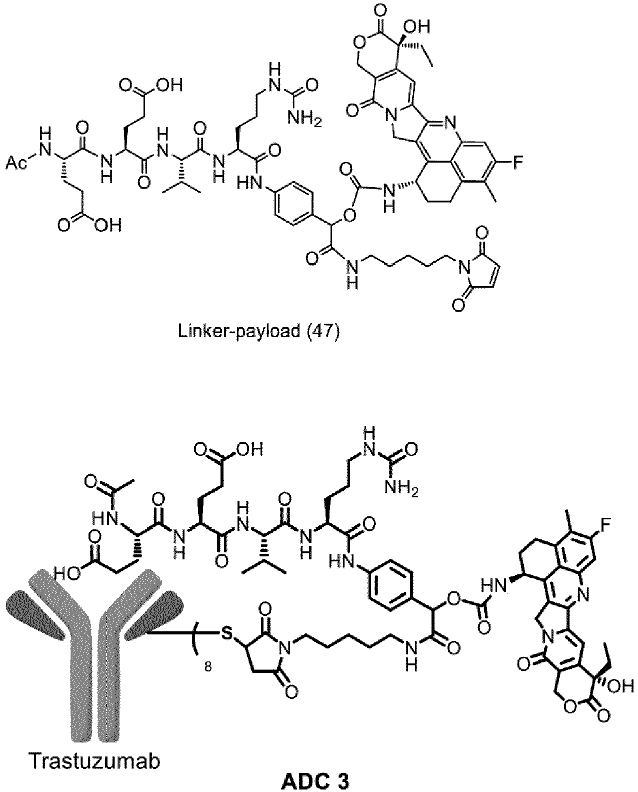

### (13-4) Synthesis of ADC9

According to (13-1), ADC9 was obtained from Linker-payload (125).

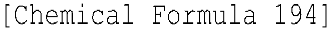

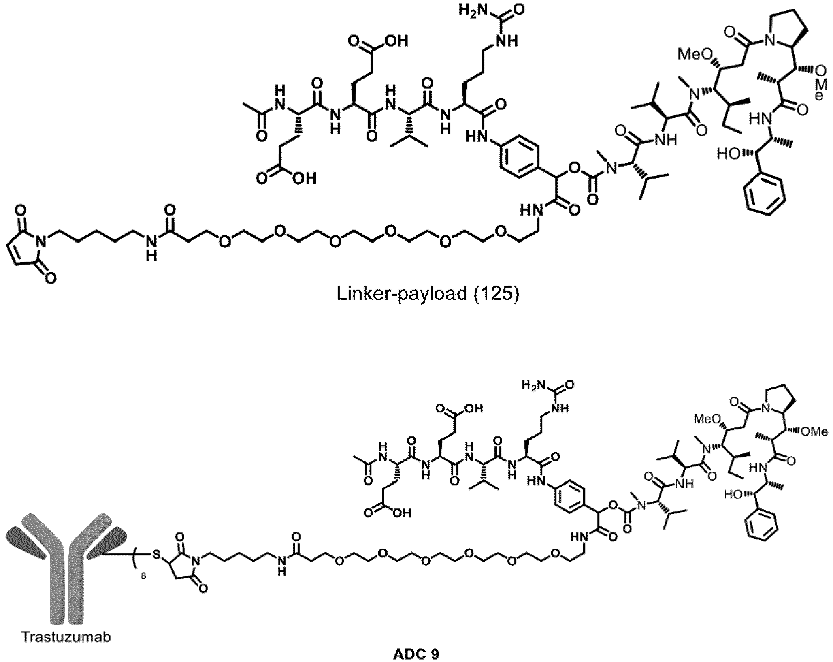

### (13-5) Synthesis of ADC10

According to (13-1), ADC10 was obtained from Linker-payload (126).

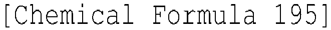

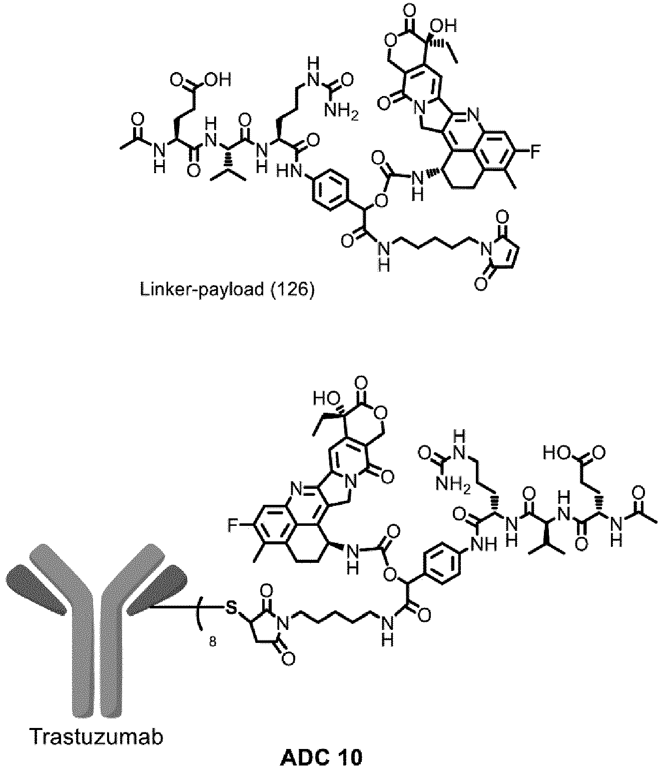

### Example 14: HIC-HPLC Analysis of ADC

HIC-HPLC analysis was performed using the condition of Example 8.

**[Table 13]**

| Table 13. DAR of ADC | | | |
|---|---|---|---|
| | Linker-payload | Example | DAR |
| ADC 1 | Linker-payload (35) | Example 13-1 | 8 |
| ADC 2 | Linker-payload (42) | Example 13-2 | 8 |
| ADC 3 | Linker-payload (47) | Example 13-3 | 8 |
| ADC 9 | Linker-payload (125) | Example 13-4 | 8 |
| ADC 10 | Linker-payload (126) | Example 13-5 | 8 |

Subsequently, hydrophobicity of an ADC was evaluated using HIC-HPLC. Measurement was performed according to Example 9. A degree of hydrophobicity of an ADC can be evaluated by retention time of ADC in HIC chromatogram. Trastuzumab, which is a raw material antibody, was used for comparison.

**[Table 14]**

| Table 14. Evaluation of degree of hydrophobicity of trastuzumab-based ADC using HIC-HPLC | | | |
|---|---|---|---|
| | Linker-payload | Example | Retention time |
| ADC 1 | Linker-payload (35) | 13-1 | 12.3 minutes |
| ADC 2 | Linker-payload (42) | 13-2 | 11.1 minutes |
| ADC 3 | Linker-payload (47) | 13-3 | 9.1 minutes |
| Trastuzumab | None | None | 8.2 minutes |

It is found that ADCs 1, 2, and 3, which are exo-type ADCs, have retention times in HIC chromatogram comparable to those of the raw material antibody, and are more hydrophilic ADCs.

### Example 15: Evaluation of aggregation ratio of ADC by size exclusion chromatography (SEC-HPLC)

SEC-HPLC analysis was performed according to Example 10.

**[Table 15]**

| Table 15. Evaluation of aggregation ratio of trastuzumab-based ADC mimic using SEC-HPLC | | | |
|---|---|---|---|
| | Linker-payload | Example | Aggregation ratio |
| ADC 1 | Linker-payload (35) | 13-1 | 0.8% |
| ADC 2 | Linker-payload (42) | 13-2 | 0.6% |
| ADC 3 | Linker-payload (47) | 13-3 | 0.5% |
| Trastuzumab | None | None | 0.5% |

As a result, it has been confirmed that the ADCs synthesized in Examples 13-1, 13-2, and 13-3 tend to have low aggregation ratios, and it is found that the ADCs synthesized in Examples 13-1, 13-2, and 13-3 are more stable. Therefore, it was confirmed that the ADC synthesized in Example 13 was a preferred ADC.

### Example 16: Synthesis of ADC

### (16-1) Synthesis of ADC4

In the following Comparative Examples and Examples, an antibody derivative (thiol group-introduced trastuzumab) described in Example 81-7 of WO 2019/240287 A1 was used as a thiol group-introduced antibody. This antibody derivative has the following structure in which a thiol group is regioselectively introduced into trastuzumab (humanized IgG1 antibody) via an amino group of a side chain of a lysine residue at position 246 or 248 of an antibody heavy chain (the position of the lysine residue is in accordance with EU numbering).

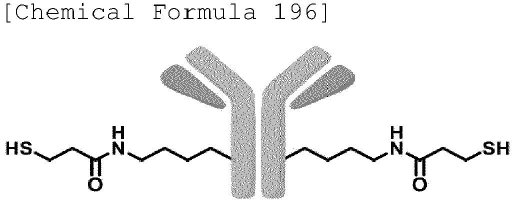

(In the above structure, NH-CH₂-CH₂-CH₂-CH₂- extending from the antibody heavy chain corresponds to a side chain of a lysine residue, and HS-CH₂-CH₂-C(=O) which is a thiol-comprising group is added to an amino group in the side chain of the lysine residue. In the present antibody, modification with another lysine residue was not detected in a peptide mapping method, and therefore position selectivity at position 246 or 248 of the antibody heavy chain is understood to be 1000.)

To a buffer (pH 7.4 PBS buffer) solution (20 µM) of the thiol group-introduced antibody, 10 equivalents of a DMF solution (10 mM) of Linker-payload (35) synthesized in Example 13-1 was added, and the mixture was allowed to stand at room temperature for two hours, and then purified using NAP-5 Columns (manufactured by GE Healthcare) to obtain ADC 4. ESI-TOFMS analysis was performed. For the reaction product, a peak was observed at 151414 indicating a product with two Linker-payloads (35) introduced.

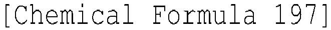

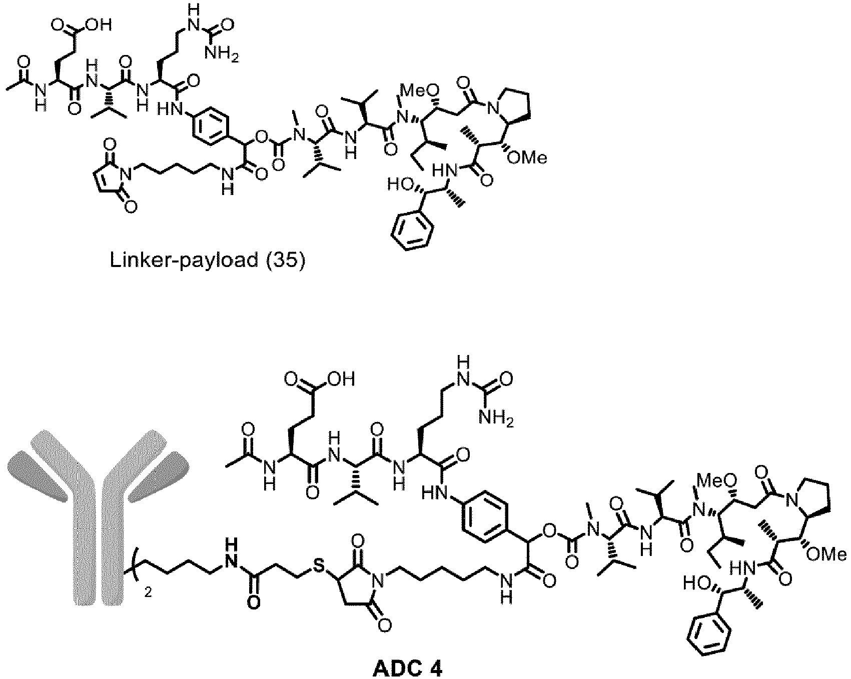

### (16-2) Synthesis of ADC5

According to (16-1), ADC5 was obtained from Linker-payload (42). ESI-TOFMS analysis was performed. For the reaction product, a peak was observed at 151673 indicating a product with two Linker-payloads (42) introduced.

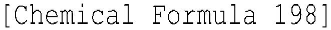

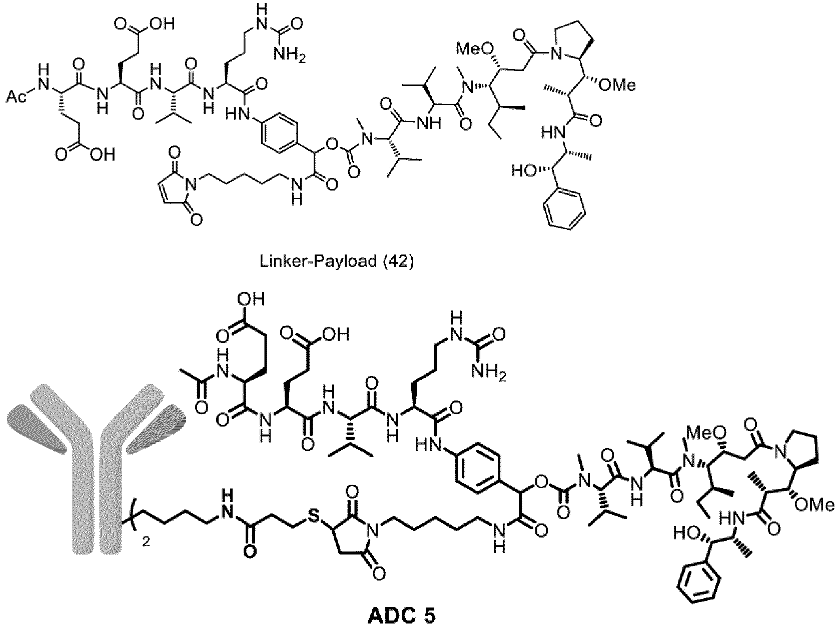

### (16-3) Synthesis of ADC6

According to (16-1), ADC6 was obtained from Linker-payload (47). ESI-TOFMS analysis was performed. For the reaction product, a peak was observed at 151109 indicating a product with two Linker-payloads (47) introduced.

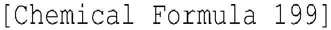

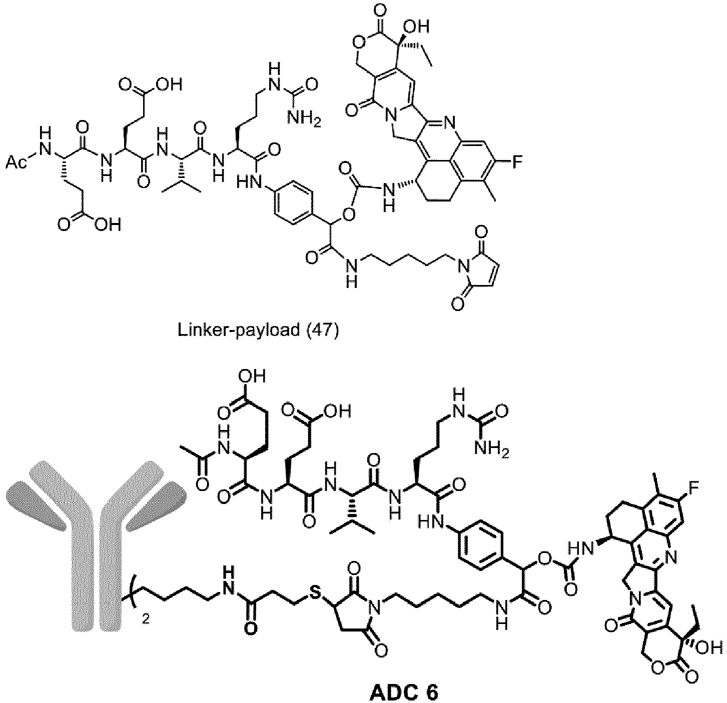

### (16-4) Synthesis of ADC6

According to (16-1), ADC8 was obtained from Linker-payload (125). ESI-TOFMS analysis was performed. For the reaction product, a peak was observed at 150524 indicating a product with two Linker-payloads (125) introduced.

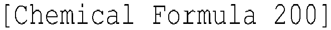

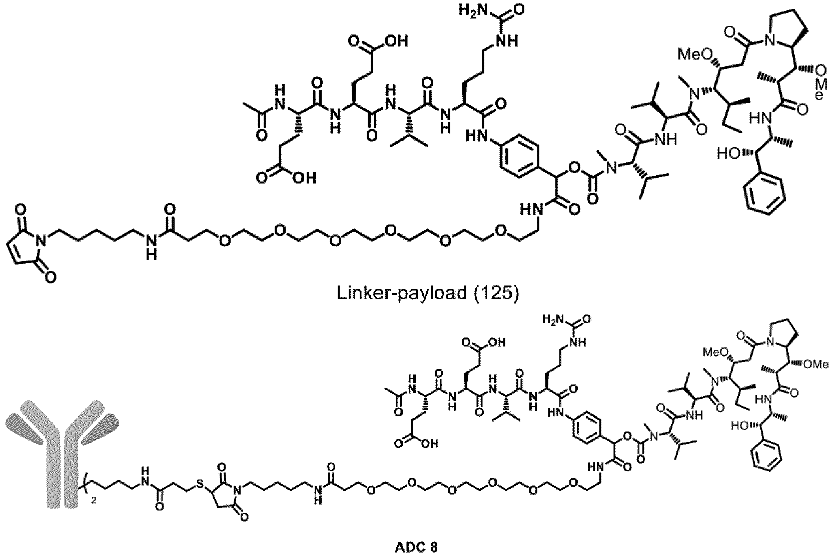

### (16-5) Synthesis of ADC11

According to (16-1), ADC11 was obtained from Linker-payload (126). ESI-TOFMS analysis was performed. For the reaction product, a peak was observed at 150615 indicating a product with two Linker-payloads (126) introduced.

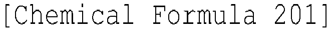

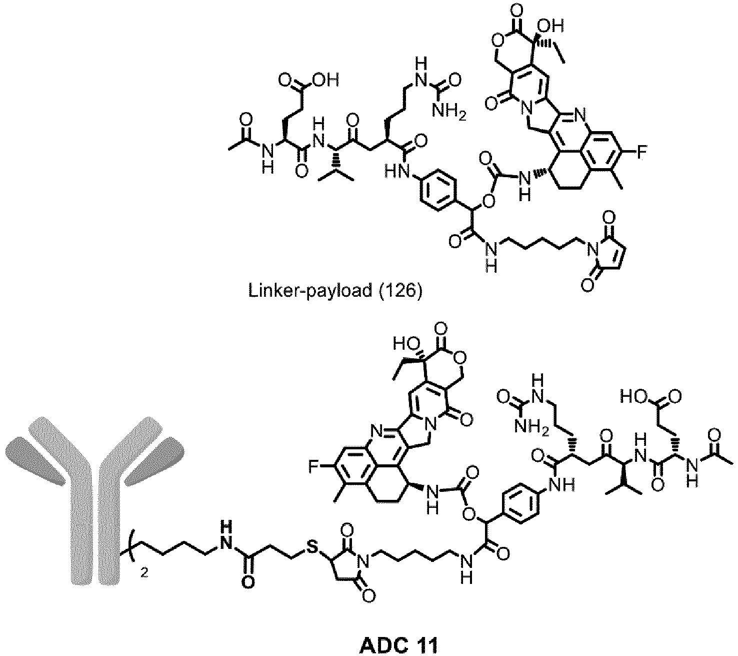

### Example 17: HIC-HPLC Analysis of ADC

HIC-HPLC analysis was performed using the condition of Example 8.

**[Table 16]**

| Table 16. DAR of ADC | | | |
|---|---|---|---|
| | Linker-payload | Example | DAR |
| ADC 4 | Linker-payload (35) | 16-1 | 2 |
| ADC 5 | Linker-payload (42) | 16-2 | 2 |
| ADC 6 | Linker-payload (47) | 16-3 | 2 |

Subsequently, hydrophobicity of an ADC was evaluated using HIC-HPLC. Measurement was performed according to Example 9. A degree of hydrophobicity of an ADC can be evaluated by retention time of ADC in HIC chromatogram. Trastuzumab, which is a raw material antibody, was used for comparison.

**[Table 17]**

| Table 17. Evaluation of degree of hydrophobicity of trastuzumab-based ADC using HIC-HPLC | | | |
|---|---|---|---|
| | Linker-payload | Example | Retention time |
| ADC 4 | Linker-payload (35) | 16-1 | 11.2 minutes |
| ADC 5 | Linker-payload (42) | 16-2 | 10.7 minutes |
| ADC 6 | Linker-payload (47) | 16-3 | 10.4 minutes |
| Trastuzumab | None | None | 8.2 minutes |

It is found that ADCs 4, 5, and 6, which are exo-type ADCs, have retention times in HIC chromatogram comparable to those of the raw material antibody, and are more hydrophilic ADCs.

### Example 18: Evaluation of aggregation ratio of ADC by size exclusion chromatography (SEC-HPLC)

SEC-HPLC analysis was performed according to Example 10.

**[Table 18]**

| Table 18. Evaluation of aggregation ratio of trastuzumab-based ADC mimic using SEC-HPLC | | | |
|---|---|---|---|
| | Linker-payload | Example | Aggregation ratio |
| ADC 4 | Linker-payload (35) | 16-1 | 0.6% |
| ADC 5 | Linker-payload (42) | 16-2 | 0.8% |
| ADC 6 | Linker-payload (47) | 16-3 | 0.5% |
| Trastuzumab | None | None | 0.5% |

As a result, it has been confirmed that the ADCs synthesized in (16-1), (16-2) and (16-3) tend to have low aggregation ratios, and it is found that the ADCs synthesized in (16-1), (16-2) and (16-3) are more stable. Therefore, it was confirmed that the ADC synthesized in Example 16 was a preferred ADC.

### Example 19: Synthesis of Linker-payload mimic

### (19-1) Synthesis of Linker-payload mimic (56)

Linker-payload mimic (56) was synthesized as follows.

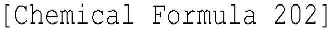

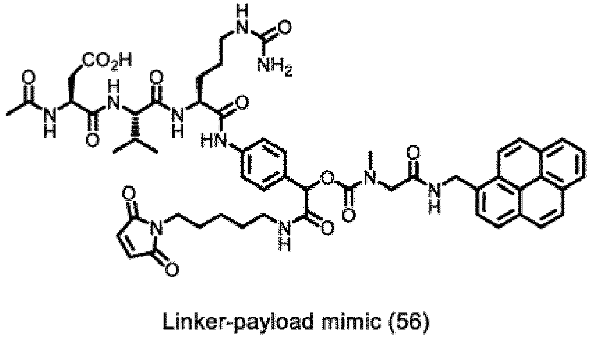

### (19-1-1) Synthesis of alcohol (57)

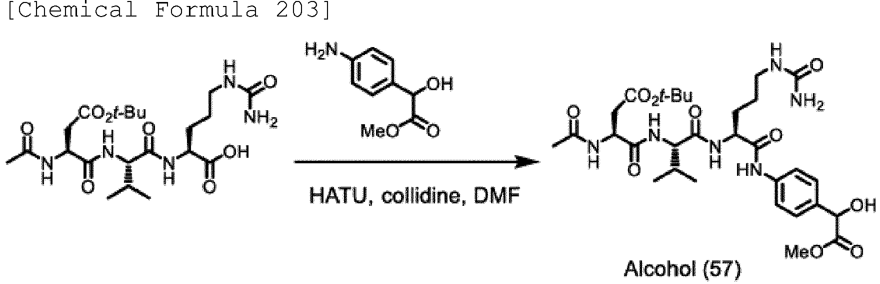

Ac-Asp(OtBu)-Val-Cit-OH (51.7 mg, 103 µmol) was dissolved in N,N-dimethylformamide (520 µL), and 1-[bis(dimethylamino) methylene]-1H-1,2,3,-triazolo[4,5-b] pyridinium 3-oxide hexafluorophosphate (46.9 mg, 123 µmol) and 2,4,6-trimethylpyridine (15.9 µL, 123 µmol) were added thereto. The mixture was stirred at room temperature for ten minutes. Subsequently, methyl 4-aminomandelate (22.3 mg, 123µmol was added thereto, and the mixture was stirred at room temperature for 19 hours, and then purified by reverse phase preparative chromatography. A fraction comprising a product was collected and concentrated under reduced pressure to remove acetonitrile. The residue was freeze-dried to obtain the alcohol (57) (56.3 mg, 86.5 µmol).

¹H NMR(400 MHz,DMSO-d₆)δ9.99(s,1H),8.27(d,J=8.4Hz,1H),8.17(d,J=8.4Hz,1H),7.59(d ,J=8.8Hz,1H),7.56(d,J=8.8Hz,2H),7.31(d,J=8.8Hz,2H),5.98(brs ,1H),5.41(brs,2H),5.08(s,1H),4.64-4.59(m,1H),4.39-4.34(m,1H),4.22-4.18(m,1H),3.59(s,3H),3.01-2.94(m,2H),2.69-2.63(m,1H),2.44-2.38(m,1H),2.00-1.95(m,1H),1.83(s,3H),1.69-1.66(m,1H),1.62-1.53(m,1H),1.43-1.34(m,11H),0.84(d,J=6.8Hz,3H),0.79(d,J=6.8Hz,3H).
MS(ESI)m/z:651.35[M+H]⁺

### (19-1-2) Synthesis of pyrene (58)

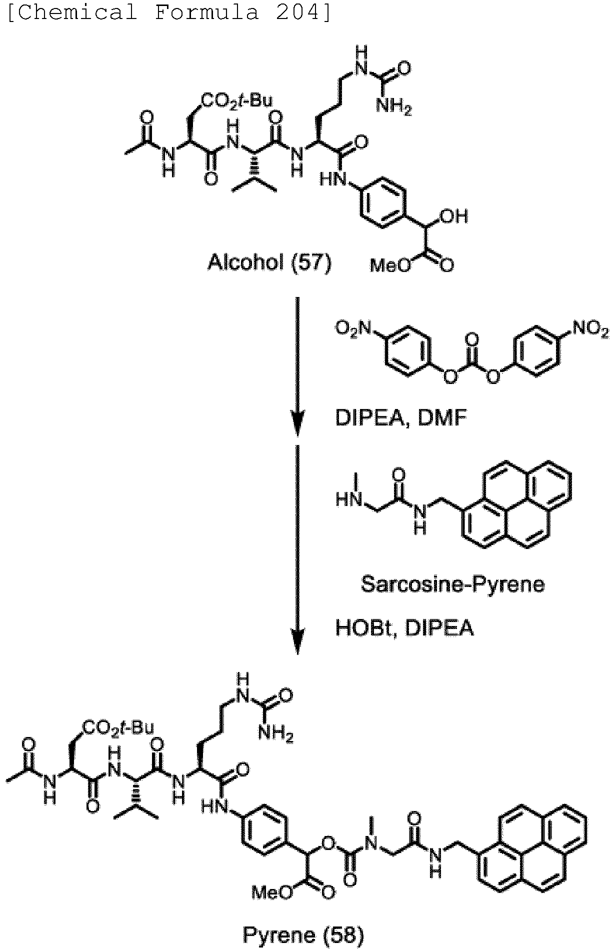

Alcohol (57) (55.0 mg, 84.5 µmol) was dissolved in N,N-dimethylformamide (423 µL), and the solution was stirred under ice cooling for five minutes. Thereafter, bis(4-nitrophenyl) carbonate (77.1 mg, 254 µmol) and N,N-diisopropylethylamine (32.3 µL, 190 µmol) were added thereto, and the mixture was stirred at room temperature for two hours. Thereafter, the mixture was ice-cooled. Sarcosin-Pyrene (76.7 mg, 254 µmol), 1-hydroxybenzotriazole (17.1 mg, 127 µmol), and N,N-diisopropylethylamine (53.9 µL, 317 µmol) were added thereto, and the mixture was stirred at room temperature for 1.5 hours. After the reaction, purification was performed by reverse phase preparative chromatography. A fraction comprising a product was collected and concentrated under reduced pressure to remove acetonitrile. The residue was freeze-dried to obtain the pyrene (58) (60.9 mg, 62.2 µmol).

¹H NMR(400 MHz,DMSO-d₆)δ10.14-10.11(m,1H),8.68-8.66(m,1H),8.39-8.01(m,10H),7.89-7.85(m,1H),7.67-7.58(m,3H),7.44-7.39(m,2H),5.98(brs,1H),5.79-5.77(m,1H),5.42(brs,2H),5.04-4.97(m,2H),4.65-4.59(m,1H),4.39-4.36(m,1H),4.28-4.07(m,2H),3.92-3.82(m,lH),3.62-3.61(m,3H),2.99-2.91(m,5H),2.69-2.63(m,1H),2.44-2.38(m,1H),2.01-1.97(m,1H),1.83(s,3H),1.70-1.60(m,2H),1.44-1.30(m,11H),0.86-0.77(m,6H).

MS(ESI)m/z:979.45[M+H]⁺

### (19-1-3) Synthesis of pyrene (59)

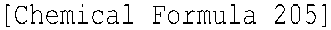

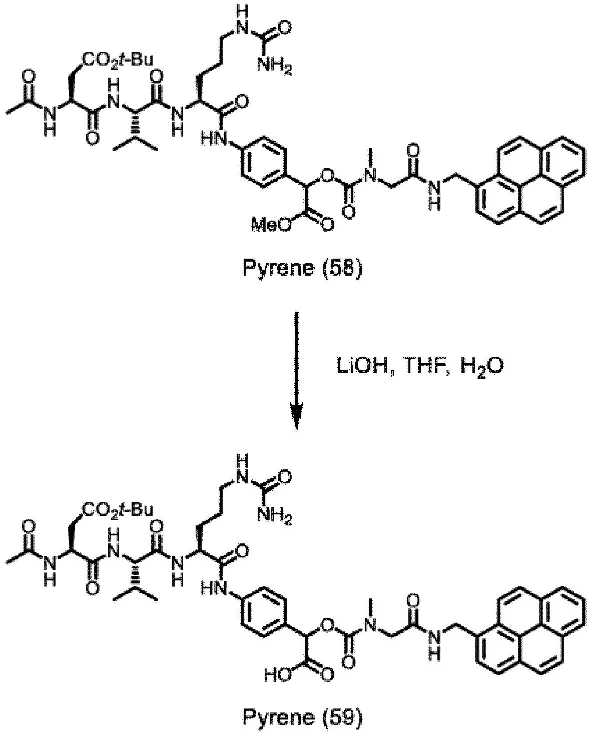

Pyrene (58) (24.9 mg, 25.4 µmol) was dissolved in tetrahydrofuran (1.88 mL) and water (625 µL), and the solution was stirred under ice cooling for five minutes. Thereafter, a 1 M lithium hydroxide aqueous solution (30.5 µL, 30.5 µmol) was added thereto, and the mixture was stirred at room temperature for 50 minutes. After completion of the reaction, the pH was adjusted to about 6 using 0.1 M hydrochloric acid, and purification was performed by reverse phase preparative chromatography. A fraction comprising a product was collected and concentrated under reduced pressure to remove acetonitrile. The residue was freeze-dried to obtain the pyrene (59) (8.3 mg, 8.6 µmol).

¹H NMR(400 MHz,DMSO-d₆) δ13.06(brs,1H),10.13-10.09(m,1H),8.68-8.65(m,1H),8.39-8.00(m,10H),7.88-7.84(m,1H),7.67-7.59(m,3H),7.44-7.41(m,2H),5.98(brs,1H),5.68-5.67(m,1H),5.42(brs,2H),5.04-4.93(m,2H),4.64-4.61(m,1H),4.41-4.35(m,1H),4.32-4.09(m,2H),3.91-3.80(m,1H),2.99-2.91(m,5H),2.70-2.64(m,1H),2.44-2.38(m,1H),2.01-1.98(m,1H),1.83(s,3H),1.70-1.57(m,2H),1.45-1.30(m,11H),0.86-0.77(m,6H).

MS(ESI)m/z:965.45[M+H]⁺

### (19-1-4) Synthesis of pyrene (60)

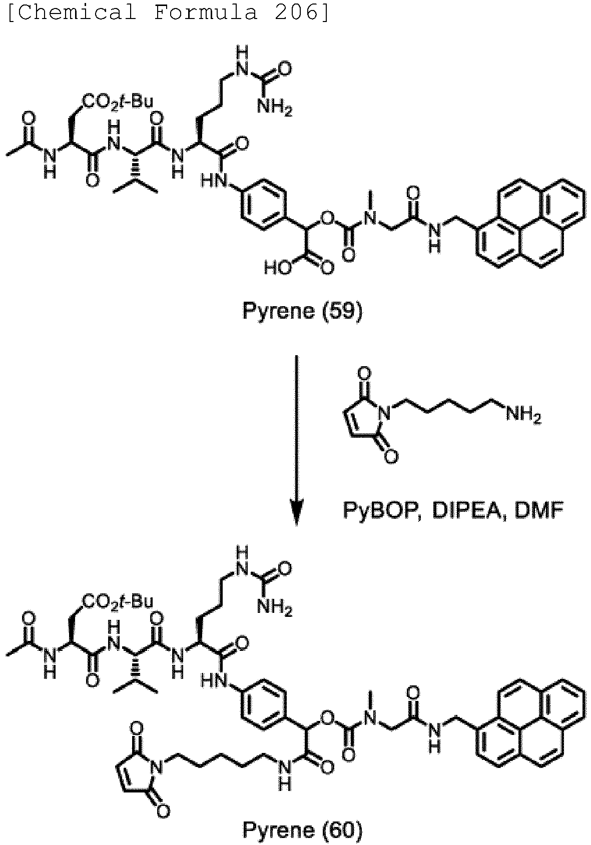

Pyrene (59) (7.2 mg, 7.5 µmol) was dissolved in N,N-dimethylformamide (150 µL). Thereafter, the solution was ice-cooled, and N,N-diisopropylethylamine (2.5 µL, 14.9 µmol) and 1H-benzotriazol-1-yloxy tripyrrolidinophosphonium hexafluorophosphate (5.8 mg, 11.2 µmol) were added thereto. Next, N-(5-aminopentyl) maleimide hydrochloride (2.4 mg, 11.2 µmol) was added thereto, the temperature was returned to room temperature, and the mixture was stirred for one hour. After completion of the reaction, purification was performed by reverse phase preparative chromatography. A fraction comprising a product was collected and concentrated under reduced pressure to remove acetonitrile. Thereafter, the resulting residue was freeze-dried to obtain pyrene (60) (5.7 mg, 5.1 µmol).

¹H NMR(400 MHz,DMSO-d₆)δ10.07-10.04(m,1H),8.91-8.88(m,1H),8.65-7.95(m,12H),7.62-7.58(m,3H),7.40-7.36(m,2H),6.96-6.94(m,2H),5.97(brs,1H),5.70-5.68(m,1H),5.41(brs,2H),5.14-5.00(m,2H),4.65-4.61(m,1H),4.42-4.37(m,1H),4.25-4.19(m,1H),4.15-3.82(m,2H),3.30-3.21(m,2H),3.04-2.89(m,7H),2.70-2.64(m,1H),2.44-2.38(m,1H),2.01-1.97(m,1H),1.83(s,3H),1.75-1.60(m,2H),1.48-1.24(m,15H),1.12-1.03(m,2H),0.86-0.78(m,6H).

MS(ESI)m/z:1129.50[M+H]⁺

### (19-1-5) Synthesis of Linker-payload mimic (56)

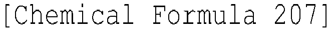

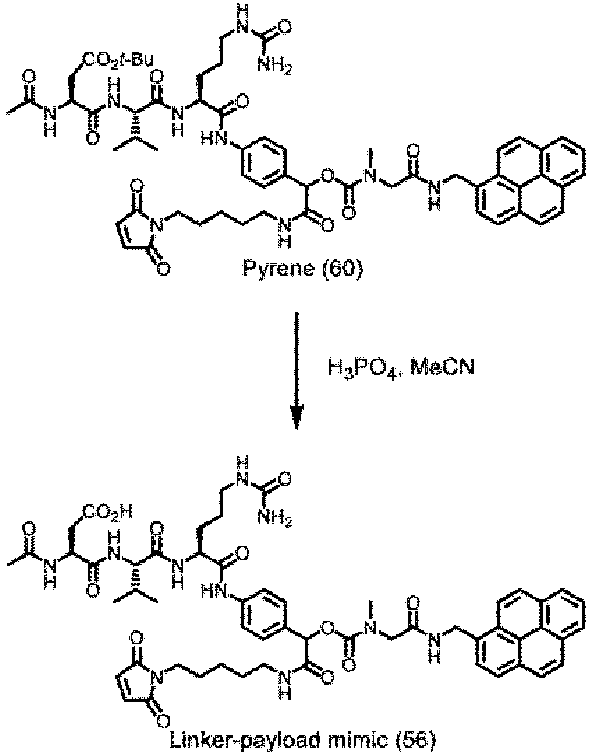

To pyrene (60) (4.7 mg, 4.2 µmol), acetonitrile (208 µL) was added, an 85% phosphoric acid solution (72.4 µL, 1.25 mmol) was added thereto under ice cooling, and the mixture was stirred at room temperature for three hours. After completion of the reaction, purification was performed by reverse phase preparative chromatography. A fraction comprising a product was collected and concentrated under reduced pressure to remove acetonitrile. Thereafter, the residue was freeze-dried to obtain Linker-payload mimic (56) (3.1 mg, 2.9 µmol).

¹H NMR(400 MHz,DMSO-d₆)δ12.38(brs,1H),10.05-10.01(m,1H),8.91-8.88(m,1H),8.65-7.94(m,12H),7.63-7.58(m,3H),7.40-7.34(m,2H),6.96-6.93(m,2H),6.00(brs,1H),5.70-5.68(m,1H),5.44(brs,2H),5.15-4.99(m,2H),4.64-4.60(m,1H),4.43-4.37(m,1H),4.25-4.22(m,1H),4.15-3.82(m,2H),3.30-3.21(m,2H),3.04-2.89(m,7H),2.73-2.69(m,1H),2.46-2.44(m,1H),2.04-1.96(m,1H),1.84-1.83(m,3H),1.77-1.59(m,2H),1.44-1.04(m,8H),0.86-0.77(m,6H).

MS(ESI)m/z:1073.50[M+H]⁺

### (19-3) Synthesis of Linker-payload mimic (66)

Linker-payload mimic (1) was synthesized as follows.

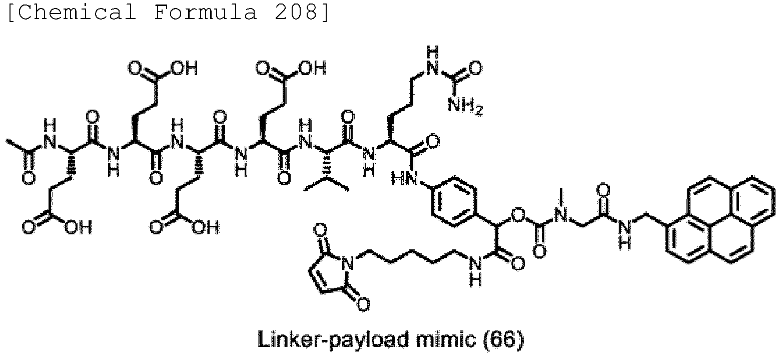

### (19-3-1) Synthesis of alcohol (67)

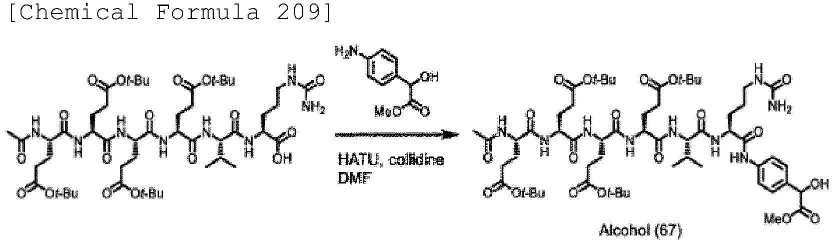

Ac-Glu(OtBu)-Glu(OtBu)-Glu(OtBu)-Glu(OtBu)-Val-Cit-OH (50.2 mg, 47.3 µmol) was dissolved in N,N-dimethylformamide (237 µL), and 1-[bis(dimethylamino) methylene]-1H-1,2,3,-triazolo[4,5-b] pyridinium 3-oxide hexafluorophosphate (21.6 mg, 56.8 µmol) and 2,4,6-trimethylpyridine (7.48 µL, 56.8 µmol) were added thereto. The mixture was stirred at room temperature for ten minutes. Subsequently, methyl 4-aminomandelate (10.3 mg, 56.8 µmol) was added thereto, and the mixture was stirred at room temperature for 20 hours, and then purified by reverse phase preparative chromatography. A fraction comprising a product was collected and concentrated under reduced pressure to remove acetonitrile. The residue was freeze-dried to obtain the alcohol (67) (47.7 mg, 39.1 µmol).

MS(ESI)m/z:1220.65[M+H]⁺

### (19-3-2) Synthesis of pyrene (68)

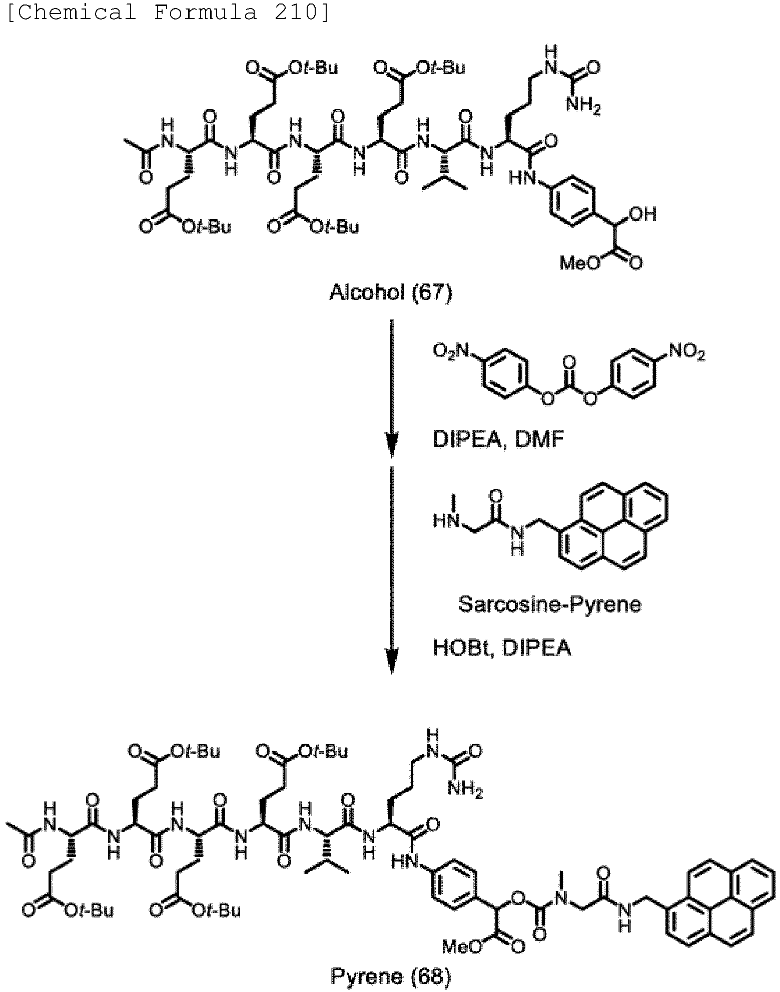

Alcohol (67) (46.3 mg, 37.9 µmol) obtained in (1-1-1) was dissolved in N,N-dimethylformamide (380 µL), and the solution was stirred under ice cooling for five minutes. Thereafter, bis(4-nitrophenyl) carbonate (34.6 mg, 114 µmol) and N,N-diisopropylethylamine (14.5 µL, 85.3 µmol) were added thereto, and the mixture was stirred at room temperature for two hours. Thereafter, the mixture was ice-cooled. Sarcosin-Pyrene (34.5 mg, 114 µmol), 1-hydroxybenzotriazole (7.7 mg, 56.9 µmol), and N,N-diisopropylethylamine (24.2 µL, 142 µmol) were added thereto, and the mixture was stirred at room temperature for two hours. After the reaction, purification was performed by reverse phase preparative chromatography. A fraction comprising a product was collected and concentrated under reduced pressure to remove acetonitrile. The residue was freeze-dried to obtain the pyrene (68) (37.1 mg, 24.0 µmol).

MS(ESI)m/z:775.30[M+H]⁺

### (19-3-3) Synthesis of pyrene (69)

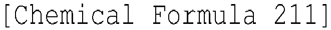

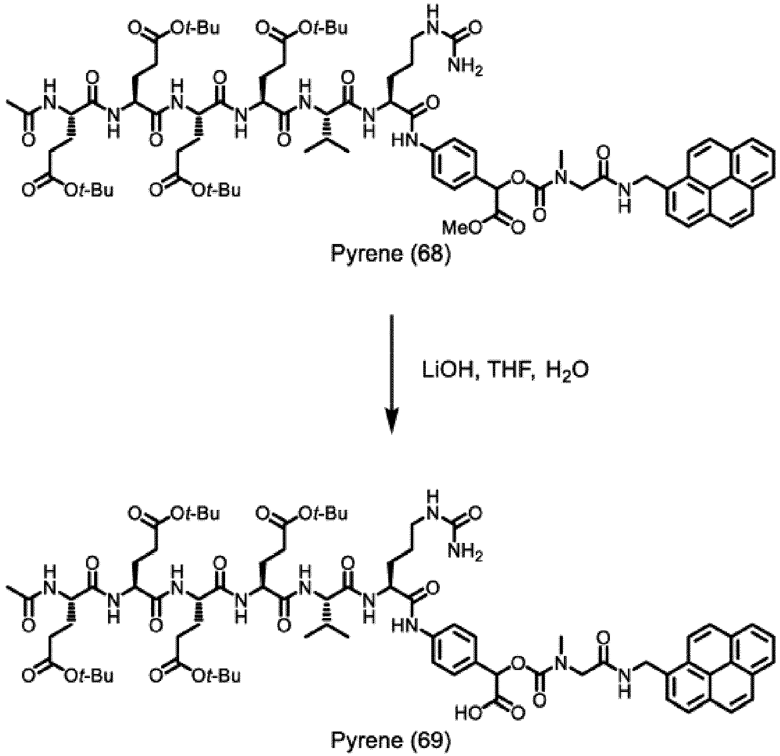

Pyrene (68) (20.3 mg, 13.1 µmol) was dissolved in tetrahydrofuran (983 µL) and water (327 µL), and the solution was stirred under ice cooling for five minutes. Thereafter, a 1 M lithium hydroxide aqueous solution (31.4 µL, 31.4 µmol) was added thereto, and the mixture was stirred at room temperature for 2.5 hours. After completion of the reaction, the pH was adjusted to about 6 using 1 M hydrochloric acid, and purification was performed by reverse phase preparative chromatography. A fraction comprising a product was collected and concentrated under reduced pressure to remove acetonitrile. The residue was freeze-dried to obtain the pyrene (69) (16.7 mg, 10.9 µmol).

MS(ESI)m/z:1534.85[M+H]⁺

### (19-3-4) Synthesis of pyrene (70)

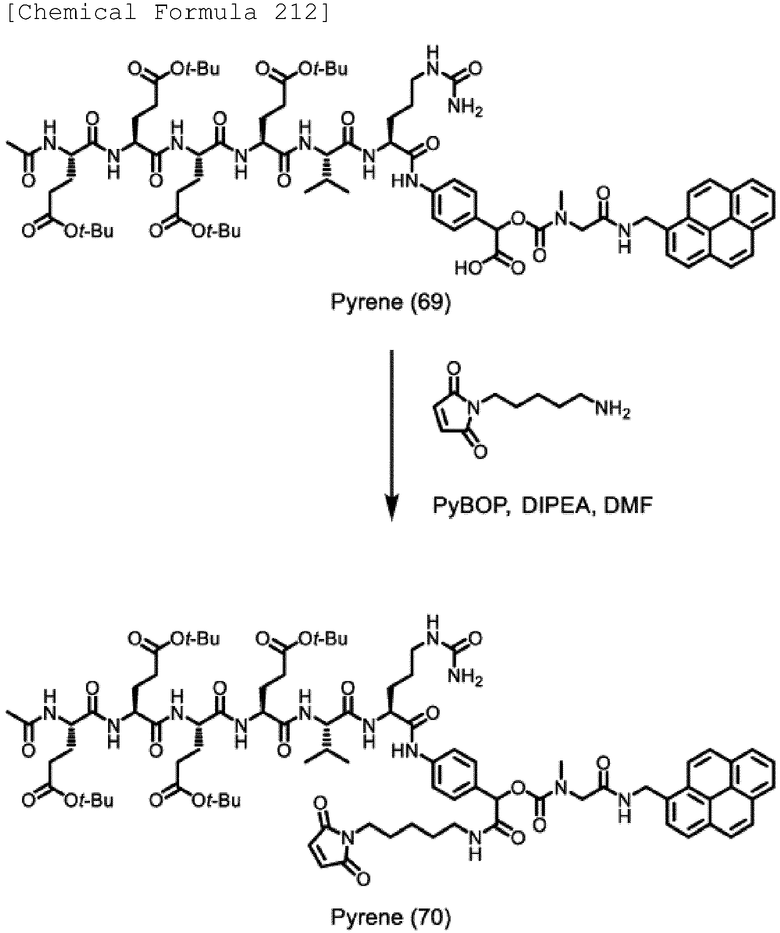

Pyrene (69) (14.9 mg, 9.71 µmol) was dissolved in N,N-dimethylformamide (486 µL). Thereafter, the solution was ice-cooled, and N,N-diisopropylethylamine (3.3 µL, 19.4 µmol) and 1H-benzotriazol-1-yloxy tripyrrolidinophosphonium hexafluorophosphate (7.6 mg, 14.6 µmol) were added thereto. Next, N-(5-aminopentyl) maleimide hydrochloride (3.2 mg, 14.6 µmol) was added thereto, the temperature was returned to room temperature, and the mixture was stirred for one hour. After completion of the reaction, purification was performed by reverse phase preparative chromatography. A fraction comprising a product was collected and concentrated under reduced pressure to remove acetonitrile. Thereafter, the resulting residue was freeze-dried to obtain pyrene (70) (13.1 mg, 7.71 µmol).

MS(ESI)m/z:1698.90[M+H]⁺

### (19-3-5) Synthesis of Linker-payload mimic (66)

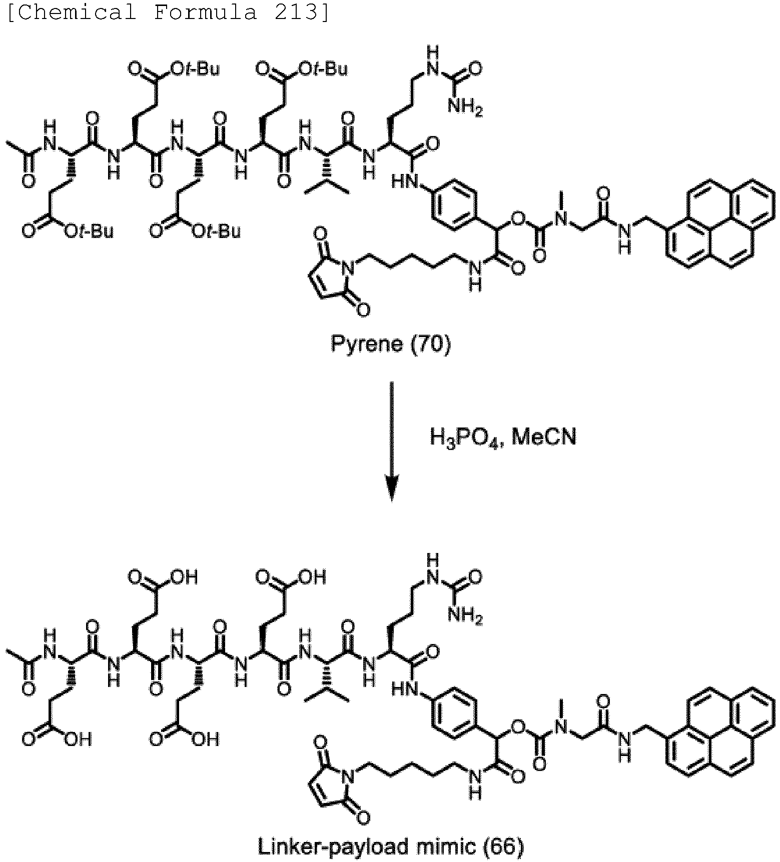

To pyrene (70) (5.25 mg, 3.09 µmol), acetonitrile (309 µL) was added, an 85% phosphoric acid solution (53.8 µL, µ927mol) was added thereto under ice cooling, and the mixture was stirred at room temperature for 25 hours. After completion of the reaction, purification was performed by reverse phase preparative chromatography. A fraction comprising a product was collected and concentrated under reduced pressure to remove acetonitrile. Thereafter, the residue was freeze-dried to obtain Linker-payload mimic (66) (3.7 mg, 2.51 µmol).

MS(ESI)m/z:1474.00[M+H]⁺

### (19-3) Synthesis of Linker-payload mimic (11)

Linker-payload mimic (11) was synthesized by a route different from (1-3) as described below.

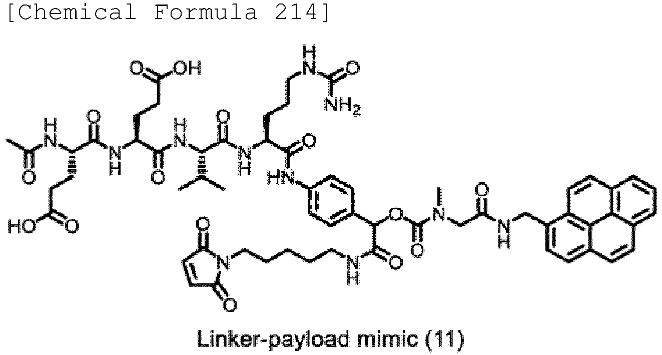

### (19-3-1) Synthesis of alcohol (96)

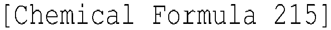

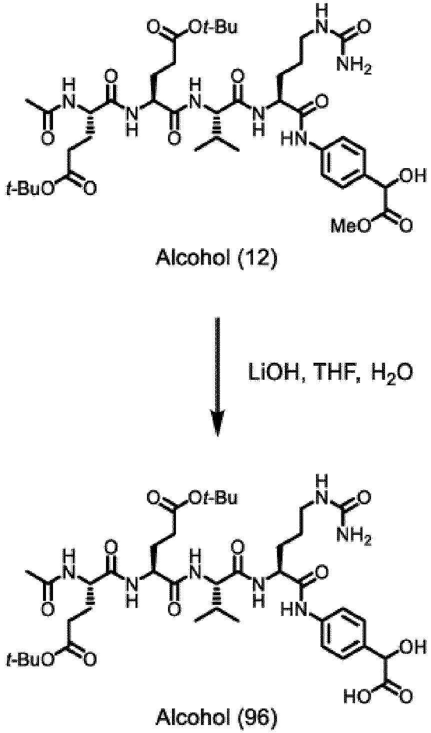

Alcohol (12) (80.0 mg, 94.1 µmol) obtained in Example (1-3-1) was dissolved in tetrahydrofuran (7.0 mL) and water (2.35 mL), and the solution was stirred under ice cooling for five minutes. Thereafter, a 1 M lithium hydroxide aqueous solution (226 µL, 226 µmol) was added thereto, and the mixture was stirred at room temperature for two hours. After completion of the reaction, the pH was adjusted to about 6 using 1 M hydrochloric acid, and purification was performed by reverse phase preparative chromatography. A fraction comprising a product was collected and concentrated under reduced pressure to remove acetonitrile. The residue was freeze-dried to obtain the alcohol (96) (61.5 mg, 73.6 µmol).

MS(ESI)m/z:836.40[M+H]⁺

### (19-3-2) Synthesis of alcohol (97)

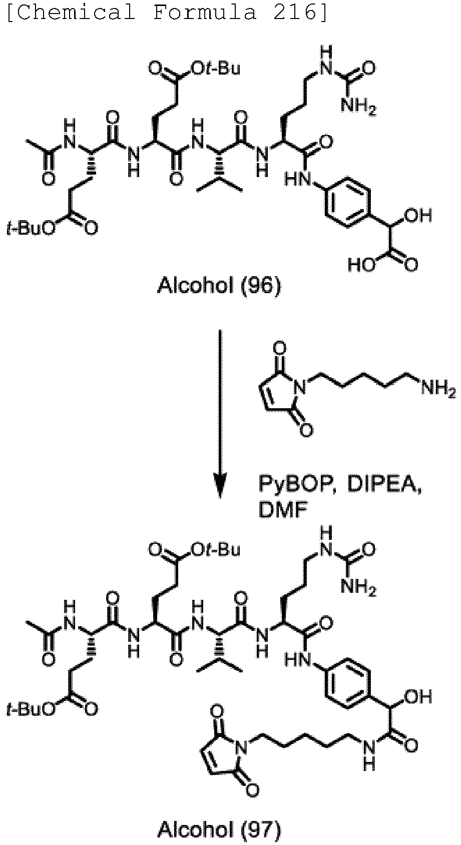

Alcohol (96) (60.5 mg, 72.4 µmol) was dissolved in N,N-dimethylformamide (3.6 mL). Thereafter, the solution was ice-cooled, and N,N-diisopropylethylamine (25 µL, 145 µmol) and 1H-benzotriazol-1-yloxy tripyrrolidinophosphonium hexafluorophosphate (56.5 mg, 109 µmol) were added thereto. Next, N-(5-aminopentyl) maleimide hydrochloride (23.7 mg, 109 µmol) was added thereto, the temperature was returned to room temperature, and the mixture was stirred for three hours. After completion of the reaction, purification was performed by reverse phase preparative chromatography. A fraction comprising a product was collected and concentrated under reduced pressure to remove acetonitrile. Thereafter, the resulting residue was freeze-dried to obtain alcohol (97) (68.0 mg, 72.4 µmol).

MS(ESI)m/z:1000.50[M+H]⁺

### (19-3-3) Synthesis of compound (98)

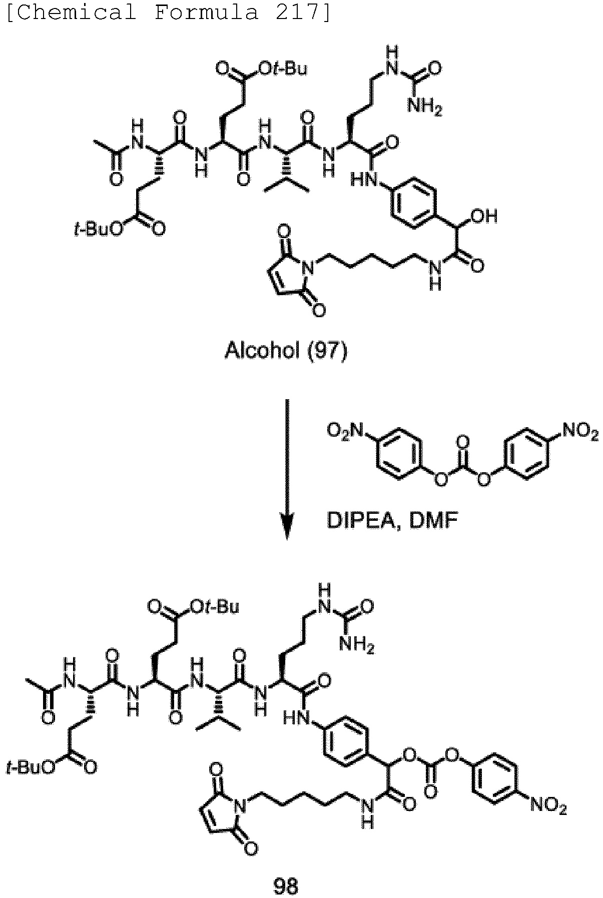

Alcohol (97) (10.0 mg, 10.0 µmol) was dissolved in N,N-dimethylformamide (0.1 mL). Thereafter, the solution was ice-cooled, and bis(4-nitrophenyl) carbonate (30.4 mg, 100 µmol) and N,N-diisopropylethylamine (3.8 µL, 22.5 µmol) were added thereto, and the mixture was stirred at room temperature for three hours. After the reaction, purification was performed by normal phase preparative chromatography. A fraction comprising a product was collected and concentrated under reduced pressure to remove acetonitrile. The residue was freeze-dried to obtain the compound (98) (5.6 mg, 4.8 µmol).

MS(ESI)m/z:1163.50[M+H]⁺

### (19-3-4) Synthesis of pyrene (15)

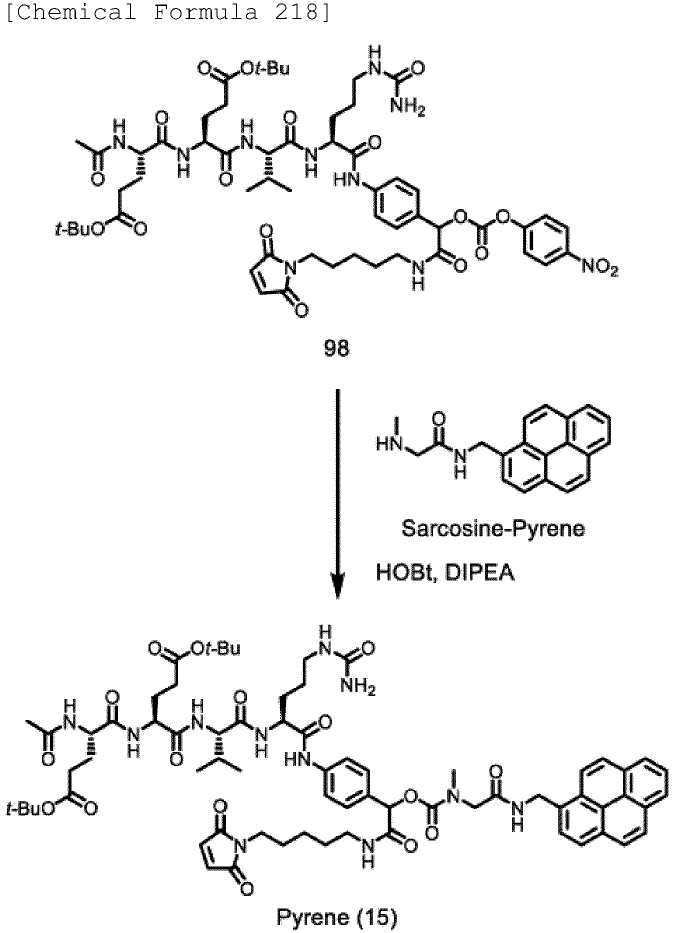

Compound (98) (10.0 mg, 8.6 µmol) was dissolved in N,N-dimethylformamide (86 µL). Thereafter, the solution was ice-cooled, and Sarcosin-Pyrene (2.2 mg, 7.2 µmol), 1-hydroxybenzotriazole (1.5 mg, 11 µmol), and N,N-diisopropylethylamine (1.8 µL, 11 µmol) were added thereto, and the mixture was stirred at room temperature for four hours. After the reaction, purification was performed by normal phase preparative chromatography. A fraction comprising a product was collected and concentrated under reduced pressure to remove acetonitrile. The residue was freeze-dried to obtain the pyrene (15) (3.0 mg, 2.3 µmol).

MS(ESI)m/z:1328.60[M+H]⁺

### (19-3-5) Synthesis of Linker-payload mimic (11)

Linker-payload mimic (11) was synthesized in a similar manner to Example (1-3-5).

### (19-4) Synthesis of Linker-payload mimic (120)

Linker-payload mimic (120) was synthesized as follows.

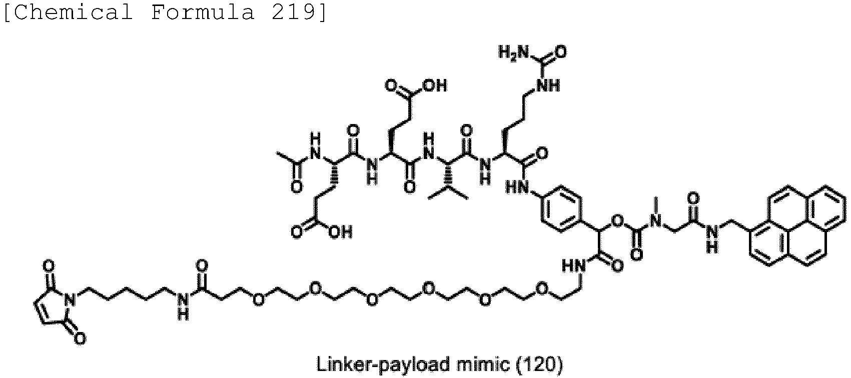

### (19-4-1) Synthesis of compound (122)

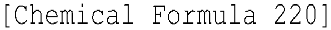

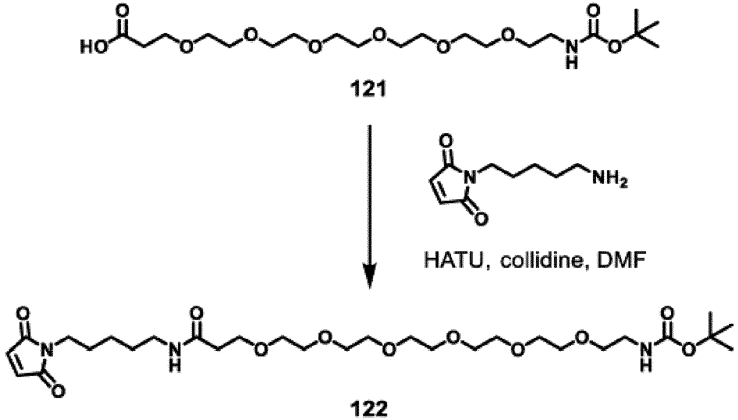

21-[(tert-butoxycarbonyl) amino]-4,7,10,13,16,19-hexaoxaheneicosanoic acid (121) (70.0 mg, 154 µmol) was dissolved in N,N-dimethylformamide (7.72 mL). Thereafter, the solution was ice-cooled, and N,N-diisopropylethylamine (52.0 µL, 309 µmol) and 1H-benzotriazol-1-yloxy tripyrrolidinophosphonium hexafluorophosphate (120 mg, 232 µmol) were added thereto. Next, N-(5-aminopentyl) maleimide hydrochloride (50.6 mg, 232 µmol) was added thereto, the temperature was returned to room temperature, and the mixture was stirred for four hours. After completion of the reaction, purification was performed by reverse phase preparative chromatography. A fraction comprising a product was collected and concentrated under reduced pressure to remove acetonitrile. Thereafter, the resulting residue was freeze-dried to obtain compound (122) (83.4 mg, 135 µmol).

MS(ESI)m/z:618.50[M+H]⁺

### (19-4-2) Synthesis of compound (123)

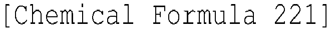

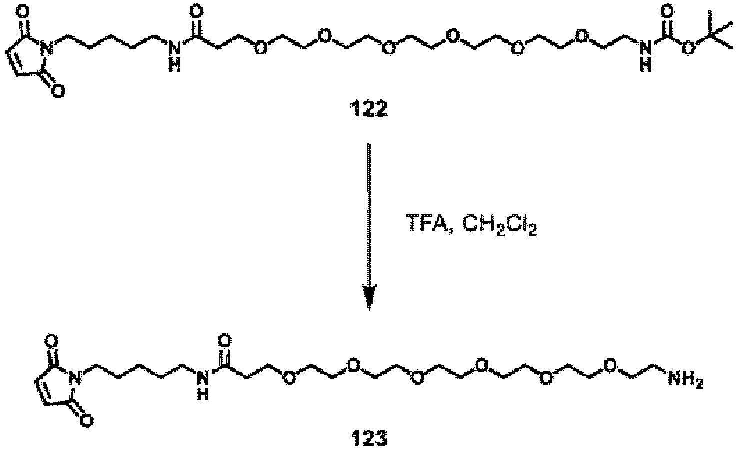

Compound (122) (82.0 mg, 133 µmol) was dissolved in dichloromethane (13.3 mL) and trifluoroacetic acid (6.64 mL). The mixture was stirred at room temperature for 30 minutes. After completion of the reaction, dichloromethane and trifluoroacetic acid were removed by concentration under reduced pressure to obtain the compound (123) (71.8 mg, quant).

MS(ESI)m/z:518.40[M+H]⁺

### (19-4-3) Synthesis of pyrene (124)

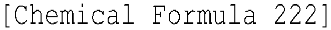

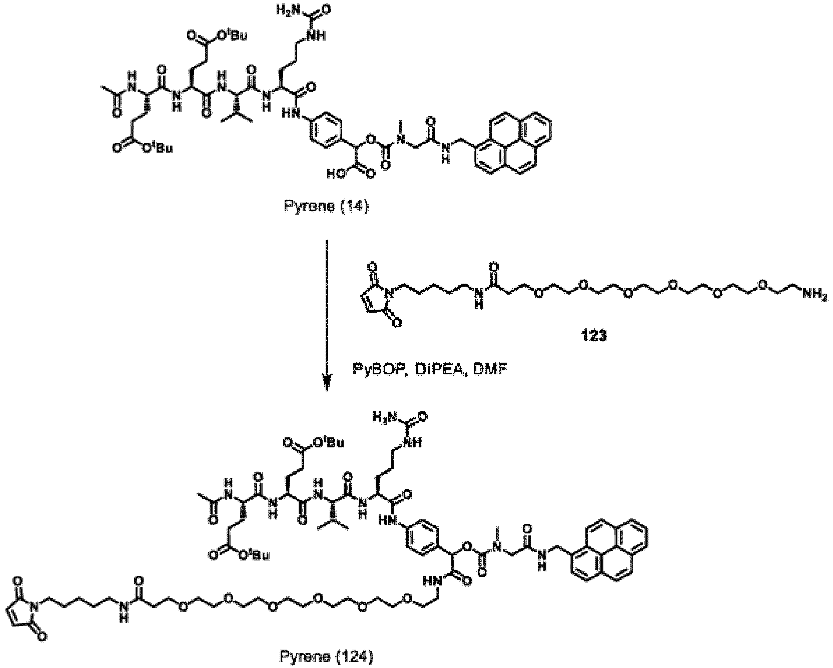

Pyrene (14) (16.0 mg, 14.0 µmol) was dissolved in N,N-dimethylformamide (690 µL). Thereafter, the solution was ice-cooled, and N,N-diisopropylethylamine (9.3 µL, 55.0 µmol) and 1H-benzotriazol-1-yloxy tripyrrolidinophosphonium hexafluorophosphate (11 mg, 21.0 µmol) were added thereto. Next, PEG6 (11.0 mg, 21.0 µmol) was added thereto, the temperature was returned to room temperature, and the mixture was stirred for two hours and a half. After completion of the reaction, purification was performed by reverse phase preparative chromatography. A fraction comprising a product was collected and concentrated under reduced pressure to remove acetonitrile. Thereafter, the resulting residue was freeze-dried to obtain pyrene (124) (11.2 mg, 6.73 µmol).

MS(ESI)m/z:1664.80[M+H]⁺

### (19-4-4) Synthesis of Linker-payload mimic (120)

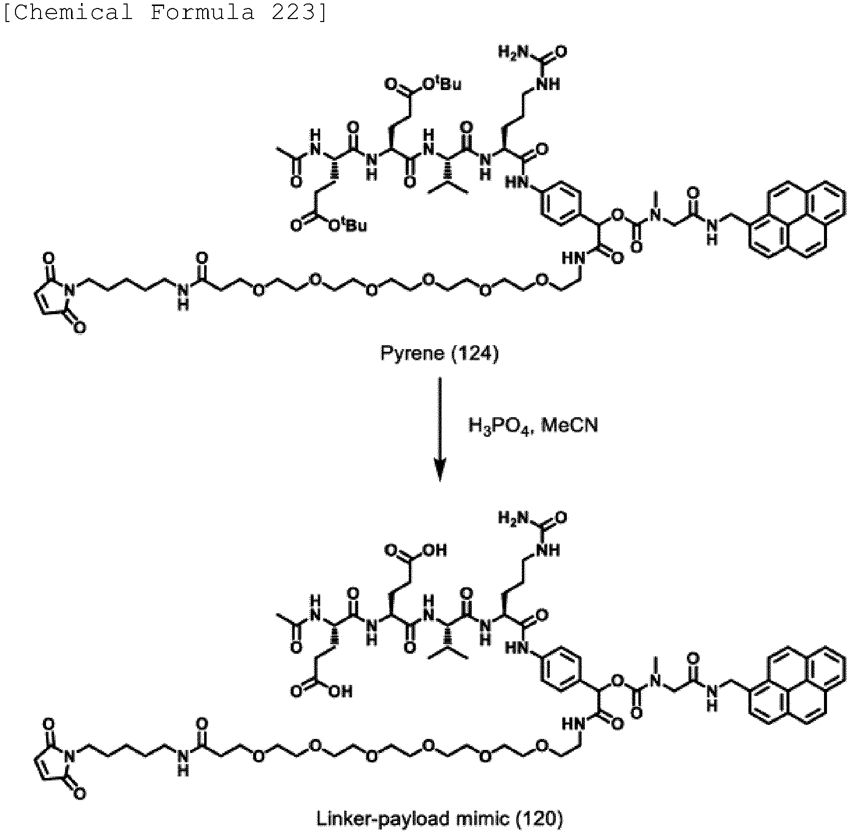

To pyrene (124) (5.0 mg, 3.0 µmol), acetonitrile (200 µL) was added, an 85% phosphoric acid solution (60.0 µL, 880 µmol) was added thereto under ice cooling, and the mixture was stirred at room temperature for 25 hours. After completion of the reaction, purification was performed by reverse phase preparative chromatography. A fraction comprising a product was collected and concentrated under reduced pressure to remove acetonitrile. Thereafter, the residue was freeze-dried to obtain Linker-payload mimic (120) (2.4 mg, 1.5 µmol).

MS(ESI)m/z:1552.65[M+H]⁺

### Example 20: Synthesis of ADC mimic

### (20-1) Synthesis of ADC mimic

In the following Example, ADC mimic was prepared in a similar manner to Example 2.

ADC mimic 22 having the following structure was synthesized from Linker-payload mimic (56) of (19-1) and the thiol-comprising antibody. ESI-TOFMS analysis was performed. For the reaction product, a peak was observed at 150322 indicating a product with two Linker-payload mimics (56) introduced.

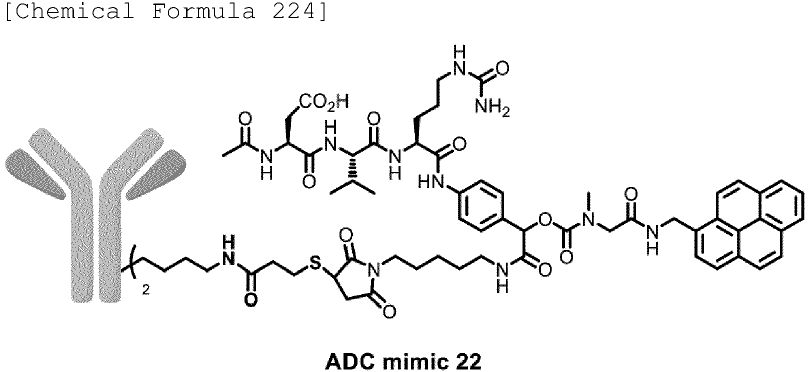

Similarly, ADC mimic 24 having the following structure was synthesized from Linker-payload mimic (66) of (19-2) and the thiol-comprising antibody. ESI-TOFMS analysis was performed. For the reaction product, a peak was observed at 151125 indicating a product with two Linker-payload mimics (66) introduced.

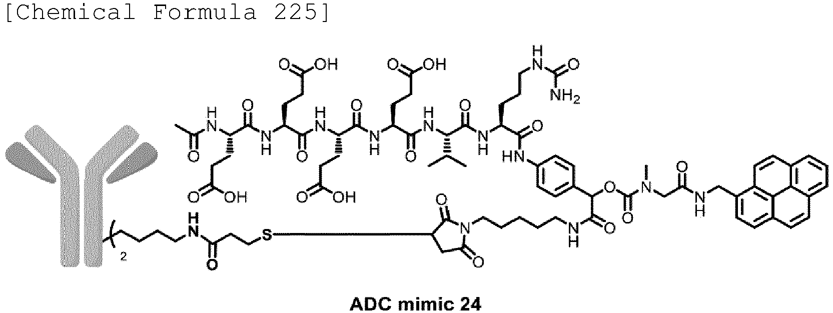

Similarly, ADC mimic 33 having the following 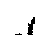 structure was synthesized from Linker-payload mimic (120) of (19-4) and the thiol-comprising antibody. ESI-TOFMS analysis was performed. For the reaction product, a peak was observed at 151279 indicating a product with two Linker-payload mimics (120) introduced.

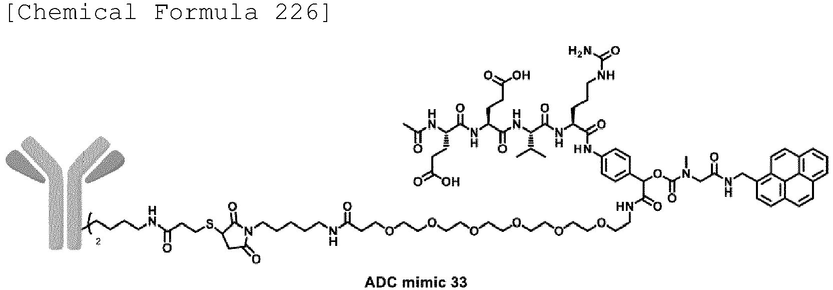

### (20-2) DAR analysis of ADC mimic

ADC mimic synthesized in Example 20-1 was subjected to ESI-TOFMS analysis according to a previous report (WO 2019/240287 A1) and confirmed to have a DAR of 2.

**[Table 19]**

| Table 19. DAR of ADC mimic | | | |
|---|---|---|---|
| | Linker-payload mimic | Example/ Comparative Example | DAR |
| ADC mimic 22 | Linker-payload mimic (56) | Example 19-1 | 2 |
| ADC mimic 24 | Linker-payload mimic (66) | Example 19-2 | 2 |
| ADC mimic 33 | Linker-payload mimic (120) | Example 19-4 | 2 |
| ADC mimic 5 | Linker-payload mimic (26) | Comparative Example 1 | 2 |

### Example 21: Evaluation of degrees of hydrophobicity of ADC and ADC mimic by hydrophobic column chromatography (HIC-HPLC)

According to a previous report (Anal. Chem., 2019, 91, 20, 12724-12732), HIC-HPLC analysis was performed. Measurement was performed using the following conditions. Degrees of hydrophobicity of an ADC can be evaluated by retention time of the ADC in HIC chromatogram.

### Measurement system: Chromaster (registered trademark) (manufactured by Hitachi, Ltd.)

Column: Tosoh Biobuthyl NPR 2.5 µm 4.6 × 35 mm column manufactured by Tosoh Bio-Sciences Inc.
Gradient: linear gradient of eluent A/B
Flow rate: 0.8 mL/min
Eluent A: 1.1 M (NH₄)₂SO₄, 25mM Na₂HPO₄/NaH₂PO₄ (pH 6. 0)
Eluent B: 25 mM Na₂HPO₄/NaH₂PO₄ (pH 6.0, 25 v/v% isopropanol added)
Detector: UV (280 nm)

**[Table 20]**

| Table 20. Evaluation of degrees of hydrophobicity of ADC and ADC mimic using HIC-HPLC | | | |
|---|---|---|---|
| | Linker-payload mimic or Linker-payload | Example/ Comparative Example | Retention time |
| ADC mimic 22 | Linker-payload mimic (56) | Example 19-1 | 10.8 minutes |
| ADC mimic 24 | Linker-payload mimic (66) | Example 19-2 | 9.9 minutes |
| ADC mimic 33 | Linker-payload mimic (120) | Example 19-4 | 9.2 minutes |
| ADC mimic 5 | Linker-payload mimic (26) | Comparative Example 1 | 13.6 minutes |

As a result, it has been confirmed that retention times of the ADC mimics synthesized in Examples 19-1, 19-2, and 19-4 tend to be early, and it is found that the ADC mimics synthesized in Examples 19-1, 19-2, and 19-4 have high degrees of hydrophilicity. Therefore, it has been confirmed that the ADC mimics synthesized in Examples 19-1, 19-2, and 19-4 are preferable ADCs because it is considered that the ADC mimics synthesized in Examples 19-1, 19-2, and 19-4 have a slow plasma clearance and a long time during which the ADC mimics remain in the body.

### Example 22: Evaluation of aggregation ratios of ADC and ADC mimic by size exclusion chromatography (SEC-HPLC)

SEC-HPLC analysis was performed according to a previous report (Chemistry Select, 2020, 5, 8435-8439). Measurement was performed using the following conditions.

### Measurement system: 1260 HPLC system (manufactured by Agilent)

Column: AdvanceBio SEC 300 Å, 2.7 µm, 4.6 mm × 150 mm, manufactured by Agilent Technologies
Flow rate: 0.25 mL/min
Eluent: 100 mM sodium dihydrogen phosphate/sodium hydrogen phosphate, aqueous solution of 250 mM sodium chloride (pH 6.8), 10% v/v isopropanol
Detector: UV (280 nm)

**[Table 21]**

| Table 21. Evaluation of aggregation ratios of ADC and ADC mimic using SEC-HPLC | | | |
|---|---|---|---|
| | Linker-payload mimic or Linker-payload | Example/ Comparative Example | Aggregation ratio |
| ADC mimic 22 | Linker-payload mimic (56) | Example 19-1 | 1.7% |
| ADC mimic 24 | Linker-payload mimic (66) | Example 19-2 | 1.2% |
| ADC mimic 33 | Linker-payload mimic (120) | Example 19-4 | 1.3% |
| ADC mimic 5 | Linker-payload mimic (26) | Comparative Example 1 | 2.7% |

### Example 23: Evaluation of ADC mimic using enzyme cathepsin B

Cleavabilities for various ADC mimics by cathepsin B were evaluated by analyzing the amount of fluorescent molecules dropped from the ADC mimics as described below.

### (23-1) Cathepsin B cleavability test

A test was performed in a similar manner to Example 5.

### (23-2) Analysis of amount of dropped fluorescent molecules using HPLC analysis

Analysis was performed in a similar manner to Example 5.

**[Table 22]**

| Table 22. Evaluation of Cathepsin B cleavability of ADC mimic | | | |
|---|---|---|---|
| | Linker-payload mimic | Example/ Comparative Example | Dropping ratio of fluorescent molecules in six hours |
| ADC mimic 22 | Linker-payload mimic (56) | Example 19-1 | 180% |
| ADC mimic 24 | Linker-payload mimic (66) | Example 19-2 | 163% |
| ADC mimic 33 | Linker-payload mimic (120) | Example 19-4 | 200% |
| ADC mimic 5 | Linker-payload mimic (26) | Comparative Example 1 | 200% |

As presented in Table 22, the synthesized ADC mimic was found to have sufficient cathepsin B cleavage.

### Example 24: Evaluation of ADC mimic using mouse plasma

### (24-1) Test for stability of ADC mimic in plasma

A test was performed in a similar manner to Example 6.

### (24-2) Analysis of amount of dropped fluorescent molecules using HPLC analysis

Analysis was performed in a similar manner to Example 6.

**[Table 23]**

| Table 23. Results of test for stability of ADC mimic in plasma | | | |
|---|---|---|---|
| | Linker-payload mimic | Example/ Comparative Example | Dropping ratio of payload at Day=4 |
| ADC mimic 22 | Linker-payload mimic (56) | Example 19-1 | 3% |
| ADC mimic 24 | Linker-payload mimic (66) | Example 19-2 | 4% |
| ADC mimic 33 | Linker-payload mimic (120) | Example 19-4 | 12% |
| ADC mimic 5 | Linker-payload mimic (26) | Comparative Example 1 | 52% |

As a result, the ADC mimic synthesized in Examples 19-4 exhibited stability of 2 times or more, and the ADC mimics synthesized in Examples 19-1 and 19-2 exhibited stability of 10 times or more, as compared with the ADC mimic synthesized in Comparative Example 1.

### Example 25: Synthesis of Linker-payload

NMR spectrum data of Linker-payload (42) synthesized in Example (12-3-5) is as follows.

¹H NMR (300MHz;DMSO-d₆) δ12.06(brs,2H),10.05-10.07(m,1H),8.47-8.28(m,1H),8.29-8.19(m,1H),8.13-8.04(m,3H),7.91-7.56(m,5H),7.39-7.17(m,7H),6.99(s,2H),5.99(s,1H),5.86-5.67(m,1H),5.43-5.35(m,3H),4.77-4.16(m,6H),4.00-3.98(m,2H),3.80-3.76(m,1H),3.52-3.18(m,12H),3.01-2.73(m,9H),2.26-2.14(m,6H),2.12-2.09(m,2H),1.97-1.90(m,2H),1.84(s,6H),1.76-1.69(m,5H),1.43-1.35(m,10H),1.23-1.14(m,3H),1.01-0.96(m,7H),0.83-0.68(m,24H).

### Example 26: Synthesis of ADC

### (26-1-1) Synthesis of linker intermediate (115)

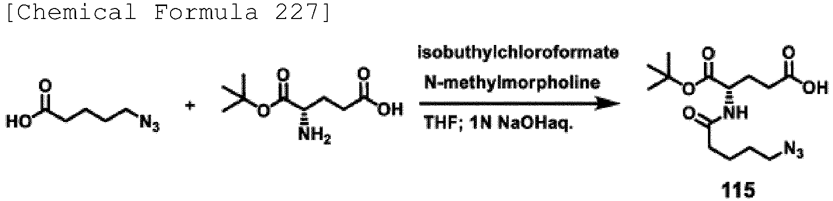

5-Azidopentanoic acid (800 mg, 5.59 mmol) was dissolved in THF (14 mL), isobutyl chloroformate (808 µL, 6.15 mmol) and N-methylmorpholine (873 µL, 8.39 mmol) were added thereto, and the mixture was stirred at 0°C for 30 minutes. Thereafter, hydrazine hydrate (1.36 g, 6.71 mmol) dissolved in a 1 M NaOH aqueous solution (4 mL) was added thereto, and the mixture was stirred at room temperature for three hours. The mixture was concentrated under reduced pressure. Thereafter, a 1 M NaOH aqueous solution was added thereto, the pH in the system was adjusted to pH 10, and the mixture was washed with ethyl acetate. Thereafter, a 1 M HCl aqueous solution was added to the aqueous layer, the pH in the system was adjusted to 3.0, ethyl acetate was added thereto to wash the mixture, and sodium sulfate was added to the obtained ethyl acetate solution. Sodium sulfate was removed by filtration, and the residue was purified by concentrated column chromatography (dichloromethane : methanol = 10 : 1) under reduced pressure. A fraction comprising a product was collected and concentrated under a reduced pressure to obtain linker intermediate (115).

¹H NMR(400MHz,Chloroform-d)δ6.29(d,J=7.7Hz,1H),4.56(td,J=8.0,4.9Hz,1H),3.32(t,J=6.6H z,2H),2.53-2.38(m,3H),2.36-2.16(m,3H),2.12(s,2H),1.96(dq,J=14.7,7.6Hz,1H),1.84-1.59(m,4H),1.50(s,9H).

MS(ESI)m/z:329[M+H]⁺

### (26-1-2) Synthesis of linker intermediate (116)

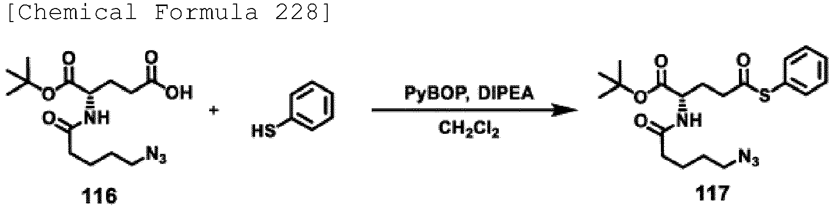

Linker intermediate (116) (2.41 g, 5.59 mmol) was dissolved in dichloromethane (28 mL). Thiophenol (627 µL, 6.15 mmol), benzotriazol-1-yloxy (3.49 g, 6.71 mmol), and DIPEA (1.42 mL, 8.39 mmol) were added thereto, and the mixture was stirred at room temperature for two hours. Thereafter, the mixture was concentrated under reduced pressure, and then purified by column chromatography (hexane : ethyl acetate = 4 : 1). A fraction comprising a product was collected and concentrated under a reduced pressure to obtain linker intermediate (117) (2.20 g, 5.23 mmol).

¹H NMR(400MHz,Chloroform-d)δ7.43(s,5H),6.10(d,J=7.8Hz,1H),4.55(td,J=7.7,4.9Hz,1H),3. 31(t,J=6.7Hz,2H),2.87-2.63(m,2H),2.28(dd,J=8.7,5.9Hz,2H),2.16-1.98(m,1H),1.83-1.58(m,4H),1.50(s,9H),1.37-1.22(m,2H),0.91(t,J=6.7Hz,1H).

MS(ESI)m/z:421[M+H]⁺

### (26-1-3) Synthesis of linker intermediate (118)

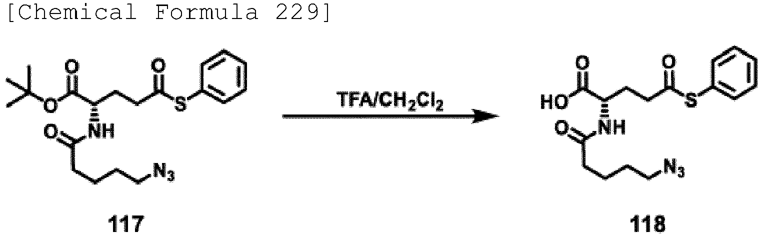

Linker intermediate (117) (2.20 g, 5.23 mmol) was dissolved in dichloromethane (10 mL), and trifluoroacetic acid (10 mL) was added thereto. The mixture was stirred at room temperature for one hour, and then concentrated under reduced pressure to remove dichloromethane. Water was added thereto, and the mixture was freeze-dried to obtain linker intermediate (118) (1.98 g, 5.43 mmo).

¹H NMR(400MHz,Chloroform-d)δ7.44(s,J=6.3,4.6,2.4Hz,5H),6.76(s,1H),4.62(td,J=7.5,4.9H z,1H),3.31(t,J=6.6Hz,2H),2.88(qt,J=16.8,6.8Hz,2H),2.33(dt,J =12.4,6.8Hz,3H),2.18(dq,J=14.4,7.4Hz,1H),1.74(dq,J=11.8,7.5, 6.9Hz,2H),1.63(ddd,J=17.7,10.5,4.8Hz,2H).

MS(ESI)m/z:365[M+H]⁺

### (26-1-4) Synthesis of linker intermediate (119)

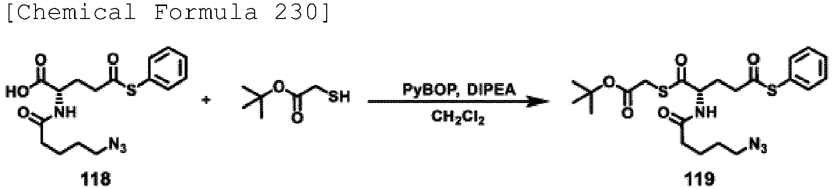

Linker intermediate (118) (100 mg, 0.274mmo) was dissolved in dichloromethane (3 mL), (40.6 µL, 0.280 mmol). Benzotriazol-1-yloxy (150 mg, 0.288 mmol) and DIPEA (70.1 µL, 0.412 mmol) were added thereto, and the mixture was stirred at room temperature for two hours. A 1 M HCl aqueous solution was added thereto to adjust the pH in the system to 3. Dichloromethane was added thereto to dilute the mixture. The diluted mixture was washed with water and saline, and then sodium sulfate was added thereto. Sodium sulfate was removed by filtration. Thereafter, the residue was concentrated under reduced pressure and purified by column chromatography (hexane : ethyl acetate = 4 : 1). A fraction comprising a product was collected and concentrated under a reduced pressure to obtain linker intermediate (119) (84.7 mg, 0.171 mmol).

¹H NMR(400MHz,Chloroform-d)δ7.50-7.38(m,5H),6.33(d,J=8.4Hz,1H),4.78(tdd,J=7.8,4.6,3.0Hz,1H), 3.70-3.54(m,2H),3.32(dt,J=9.1,6.7Hz,2H),2.96-2.67(m,2H),2.30(pd,J=7.1,4.5Hz,2H),1.85-1.60(m,6H),1.49(d,J=2.8Hz,9H).

MS(ESI)m/z:495[M+H]⁺

### (26-1-5) Synthesis of linker intermediate (120)

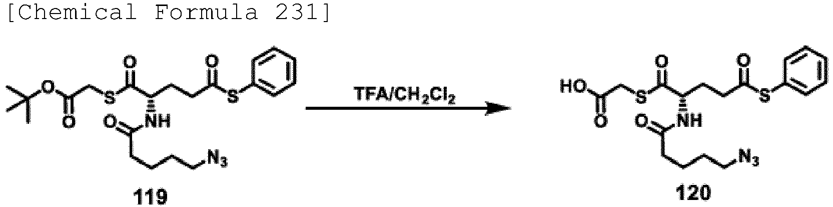

Linker intermediate (119) (84.7 mg, 0.171 mmol) was dissolved in dichloromethane (5 mL), and trifluoroacetic acid (5 mL) was added thereto. The mixture was stirred at room temperature for one hour, and then concentrated under reduced pressure to remove dichloromethane. Water was added thereto, and the mixture was freeze-dried and then purified by column chromatography (dichloromethane : methanol = 10 : 1). A fraction comprising a product was collected and concentrated under a reduced pressure to obtain linker intermediate (120) (46.8 mg, 0.107 mmo).

¹H NMR(400MHz,Methanol-d4)δ7.44(dq,J=2.3,1.5Hz,5H),4.69-4.57(m,1H),3.79-3.67(m,2H),3.40-3.30(m,2H),2.89-2.71(m,2H),2.44-2.23(m,4H),2.08-1.95(m,1H),1.82-1.61(m,4H).

MS(ESI)m/z:439[M+H]⁺

### (26-2) Preparation of affinity reagent (18) having azide group

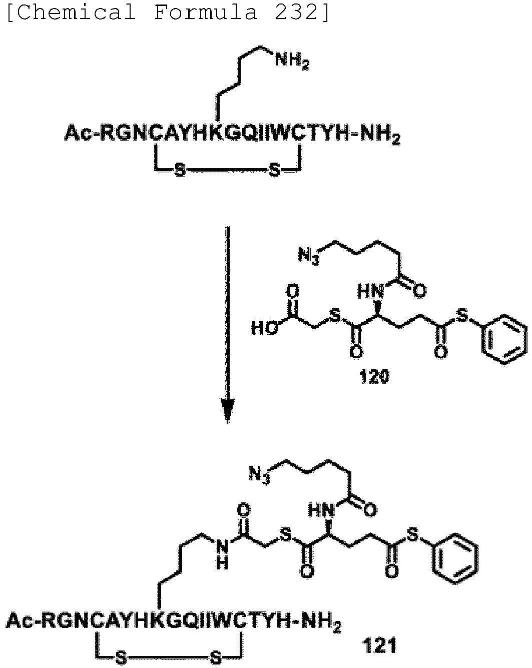

(The above amino acid sequence is an amino acid sequence of SEQ ID NO: 1.)

Ac-RGNCAYHKGQIIWCTYH-NH2 (SEQ ID NO: 1, 30.9 mg, 14.9 µmol, provided that the two cysteines at positions 4 and 14 are each disulfide-bonded in the molecule) described in a previous report (WO 2019/240287 A1) was dissolved in N,N'-dimethylformamide (468 µL). Linker intermediate (120) synthesized in Example 6-1-5 (46.8 mg, 0.107 mmol) and WSC•HCl (29.7 mg, 0.155 mmol) were added thereto. The mixture was stirred at room temperature for five hours, and then eluted by reverse phase preparative chromatography. A fraction comprising a product was collected and concentrated under reduced pressure to remove acetonitrile. Thereafter, the residue was freeze-dried to obtain the modifying reagent (121) (15.1 mg, 6.02 µmol).

### (26-3) Introduction of two-molecule peptide reagent into Trastuzumab

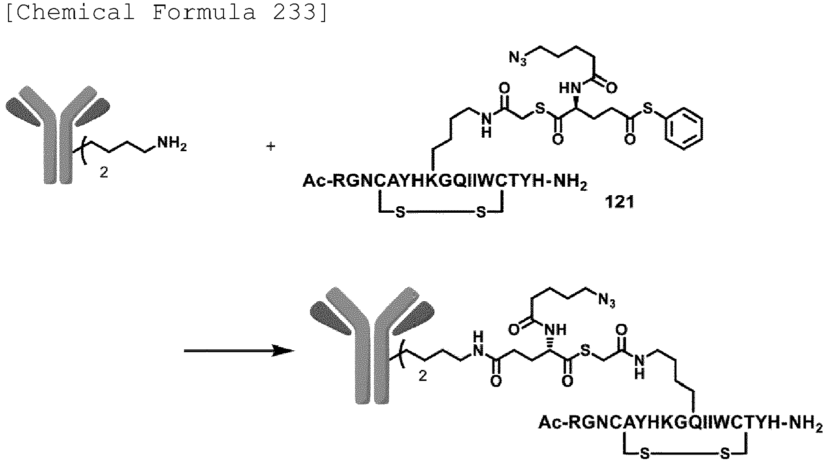

(The above amino acid sequence is an amino acid sequence of SEQ ID NO: 1.)

Subsequently, using the peptide reagent (121) prepared in Example 26-2, conjugation was performed on trastuzumab in accordance with a method of a previous report (WO 2019/240287 A1). As a result, an antibody into which the modifying reagent (121) had been introduced was obtained. The DAR analysis of the antibody into which the peptide reagent (121) had been introduced was performed in accordance with a previous report (Anal. Chem., 2019, 91, 20, 12724-12732), and HIC-HPLC analysis was performed to confirm that two peptide reagents had been introduced.

### (26-4) Synthesis of trastuzumab (T-1) into which azide group has been introduced

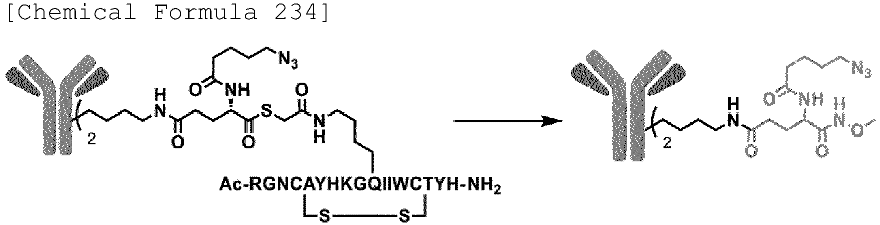

(The above amino acid sequence is an amino acid sequence of SEQ ID NO: 1.)

With reference to a method of a previous report (WO 2019/240287 A1), a methoxyamine solution was added to the antibody into which the modifying reagent (121) obtained in Example 26-3 had been introduced, and the mixture was shaken at room temperature for three hours to cause a cleavage reaction. As a result, an antibody into which an azide group had been introduced was obtained. Analysis was performed in accordance with a previous report (Anal. Chem., 2019, 91, 20, 12724-12732), and HIC-HPLC analysis was performed to confirm that an azide group had been introduced.

### (26-5) Synthesis of ADC7

To the azide-introduced antibody, Linker-payload (40) was added to obtain ADC (7). ESI-TOFMS analysis was performed. For the reaction product, a peak was observed at 151276 indicating a product with two Linker-payloads (40) introduced. In addition, ESI-TOFMS analysis was performed according to a previous report (WO 2019/240287 A1), and DAR was confirmed to be 2.

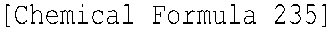

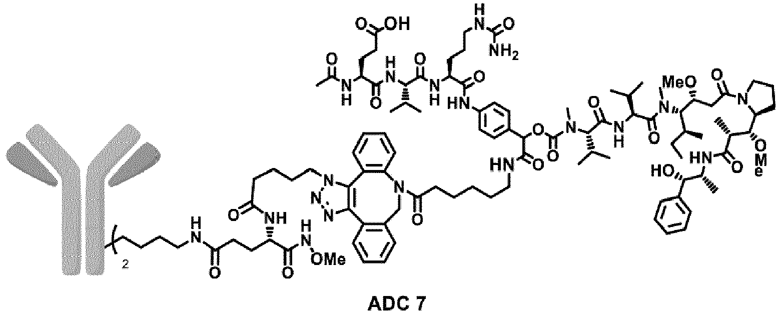

### Example 27: Evaluation of ADC using mouse plasma

### (27-1) Test for stability of ADC in plasma

An ADC mimic was added to 500 µL of mouse plasma (manufactured by Charles River) so as to have a concentration of 0.1 mg/mL, and then the mixture was subjected to sterile filtration. 50 µL of this solution was poured into each of six Eppendorf tubes. Three of the six samples were stored in an incubator set at 37°C for four days. The remaining three samples were stored in a freezer at -80°C for four days similarly. To each of the samples, 100 µL of acetonitrile was added. The mixture was stirred by vortex, and then centrifuged to obtain a precipitate. The resulting supernatant solution was collected and subjected to HPLC analysis.

### (27-2) Analysis of amount of dropped payload using HPLC analysis

The amount of payload dropped from an ADC was measured using liquid chromatography mass spectrometry (comprising tandem mass spectrometry). The samples similarly stored in a freezer at -80°C for four days in Example 27-1 were taken as Day 0 samples, and the three samples incubated at 37°C for four days in Example 27-1 were taken as Day 4 samples. MS intensities of payloads detected from the Day 4 samples and the Day 0 samples were calculated by an extracted ion chromatogram, and a difference therebetween was analyzed.

Separately, a correlation between the area of a TIC by HPLC and a concentration was calculated using MMAE. TIC of a fluorescence intensity of each of the ADCs was converted into a concentration using the calculation formula. When the concentration at Day 0 was set to 100%, a ratio of the above-described difference in ion chromatogram was calculated as a dropping ratio.

**[Table 24]**

| Table 24. Results of test for stability of ADC in plasma | | | |
|---|---|---|---|
| | Linker-payload mimic | Example/ Comparative Example | Dropping ratio of payload at Day=4 |
| ADC 4 | Linker-payload (35) | Example 12-1 | 5% |
| ADC 5 | Linker-payload (42) | Example 12-3 | 0% |
| ADC 7 | Linker-payload (40) | Example 12-2 | 2% |

As a result, it was found that the ADCs synthesized in Examples 12-1, 12-2, and 12-3 had high stability.

Example 28: Evaluation of ADC using mouse plasma

### (28-1) Test for stability of ADC in plasma

An ADC mimic was added to 500 µL of mouse plasma (manufactured by Charles River) so as to have a concentration of 0.1 mg/mL, and then the mixture was subjected to sterile filtration. 50 µL of this solution was poured into each of six Eppendorf tubes. Three of the six samples were stored in an incubator set at 37°C for four days. The remaining three samples were stored in a freezer at -80°C for four days similarly. To each of the samples, 100 µL of acetonitrile was added. The mixture was stirred by vortex, and then centrifuged to obtain a precipitate. The resulting supernatant solution was collected and subjected to HPLC analysis.

### (28-2) Analysis of amount of dropped payload using HPLC analysis

The amount of payload dropped from an ADC was measured using liquid chromatography mass spectrometry (comprising tandem mass spectrometry). The samples similarly stored in a freezer at -80°C for four days in Example 28-1 were taken as Day 0 samples, and the three samples incubated at 37°C for four days in Example 28-1 were taken as Day 4 samples. MS intensities of payloads detected from the Day 4 samples and the Day 0 samples were calculated by an extracted ion chromatogram, and a difference therebetween was analyzed. A dropping ratio of payload was calculated according to Example 27-2.

**[Table 25]**

| Table 25. Results of test for stability of ADC in plasma | | | |
|---|---|---|---|
| | Linker-payload mimic | Example/ Comparative Example | Dropping ratio of payload at Day=4 |
| ADC 2 | Linker-payload (42) | Example 12-3 | 7% |
| ADC 9 | Linker-payload (125) | Example 12-5 | 14% |

As a result, it was found that the ADCs synthesized in Examples 12-3 and 12-5 had high stability.

[Sequence Listing]

## Claims

1. A conjugate of an antibody and a functional substance or functional substances, comprising a structural unit represented by the following Formula (1): 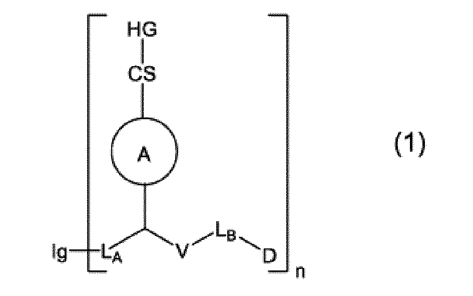 wherein
Ig represents an immunoglobulin unit comprising two heavy chains and two light chains, and is bonded to L_{A} adjacent to Ig via a thiol group in side chains of a plurality of cysteine residues in the two heavy chains and the two light chains,
HG represents a hydrophilic group or a monovalent group comprising a hydrophilic group,
CS represents a divalent group comprising a cleavable site,
ring A represents a divalent aromatic ring group optionally having a substituent wherein the divalent aromatic ring group constitutes a π electron conjugated system with the cleavable site,
V represents an oxygen atom, a sulfur atom, or an amino group (NH),
L_{A} and L_{B} each independently represent a divalent group,
D represents the functional substance, and
an average number n of the bonds per the immunoglobulin unit is 1.5 or more, or
a salt thereof.

2. The conjugate or salt thereof according to claim 1, wherein the immunoglobulin unit is a human immunoglobulin unit.

3. The conjugate or salt thereof according to claim 2, wherein the human immunoglobulin unit is a human IgG antibody.

4. The conjugate or salt thereof according to claim 1, wherein n is 2.0 to 8.0.

5. The conjugate or salt thereof according to claim 1, wherein n is 6.0 to 8.0.

6. The conjugate or salt thereof according to claim 1, wherein n is 7.0 to 8.0.

7. The conjugate or salt thereof according to claim 1, wherein the hydrophilic group is one or more groups selected from the group consisting of a carboxylic acid group, a sulfonate group, a hydroxy group, a polyethylene glycol group, a polysarcosine group, and a sugar portion.

8. The conjugate or salt thereof according to claim 1, wherein the cleavable site is a cleavable site by an enzyme.

9. The conjugate or salt thereof according to claim 8, wherein the enzyme is cathepsin B.

10. The conjugate or salt thereof according to claim 1, wherein
the divalent group comprising a cleavable site satisfies the following conditions (i) to (iii):
(i) the cleavable site is -CO-W- wherein hyphen (-) represents a bond, and W represents an oxygen atom, a sulfur atom, or an amino group (NH), and is bonded to the divalent aromatic ring group;
(ii) a bond between CO and W is a site to be cleaved; and
(iii) the divalent aromatic ring group constitutes a π electron conjugated system with W.

11. The conjugate or salt thereof according to claim 1, wherein ring A is a phenylene group optionally having a substituent.

12. The conjugate or salt thereof according to claim 1, wherein the functional substance is a medicament, a labelling substance, or a stabilizer.

13. The conjugate or salt thereof according to claim 1, wherein the conjugate exhibits an aggregation ratio of 2.6% or less when being analyzed by size exclusion chromatography.

14. The conjugate or salt thereof according to claim 1, wherein
the structural unit represented by Formula (1) comprises a structural unit represented by the following Formula (1a): 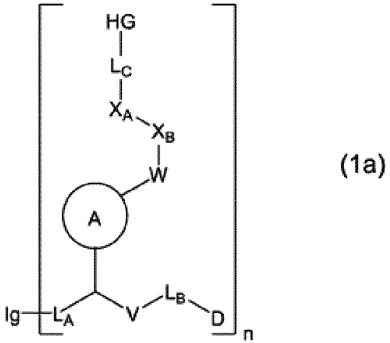 wherein
Ig, HG, ring A, V, L_{A}, L_{B}, D, and n are the same as those represented in Formula (1), respectively,
L_{C} represents a bond or a divalent group,
X_{A} and X_{B} each independently represent an amino acid residue, and
W represents an oxygen atom, a sulfur atom, or an amino group (NH).

15. The conjugate or salt thereof according to claim 14, wherein
X_{A} represents a valine residue, a phenylalanine residue, a threonine residue, a leucine residue, or an alanine residue, and
X_{B} represents a citrulline residue, an alanine residue, a glutamic acid residue, a glutamine residue, a lysine residue, an arginine residue, a threonine residue, or a methionine residue.

16. The conjugate or salt thereof according to claim 14, wherein
the structural unit represented by Formula (1a) comprises a structural unit represented by the following Formula (1b): 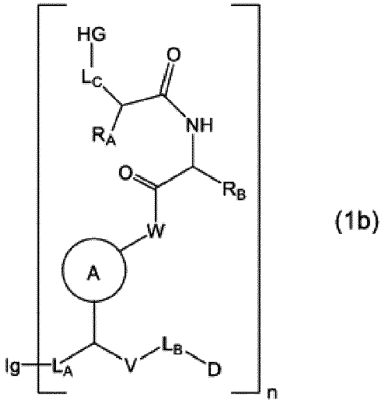 wherein
Ig, HG, ring A, V, L_{A}, L_{B}, D, and n are the same as those represented in Formula (1), respectively,
L_{C} and W are the same as those represented in Formula (1a), respectively,
R_{A} represents a side chain of a valine residue, a phenylalanine residue, a threonine residue, a leucine residue, or an alanine residue, and
R_{B} represents a side chain of a citrulline residue, an alanine residue, a glutamic acid residue, a glutamine residue, a lysine residue, an arginine residue, a threonine residue, or a methionine residue.

17. The conjugate or salt thereof according to claim 16, wherein
the structural unit represented by Formula (1b) comprises a structural unit represented by the following Formula (1c): 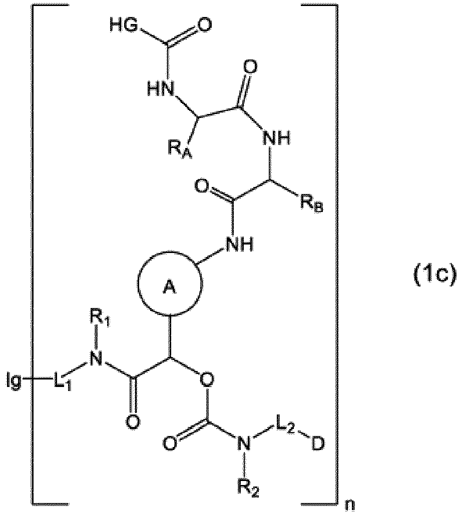 wherein
Ig, HG, ring A, D, and n are the same as those represented in Formula (1), respectively,
R_{A} and R_{B} are the same as those represented in Formula (1b), respectively,
R₁ and R₂ each independently represent a hydrogen atom or a monovalent group, and
L₁ and L₂ each independently represent a divalent group.

18. The conjugate or salt thereof according to claim 17, wherein
the structural unit represented by Formula (1c) comprises a structural unit represented by the following Formula (1d): 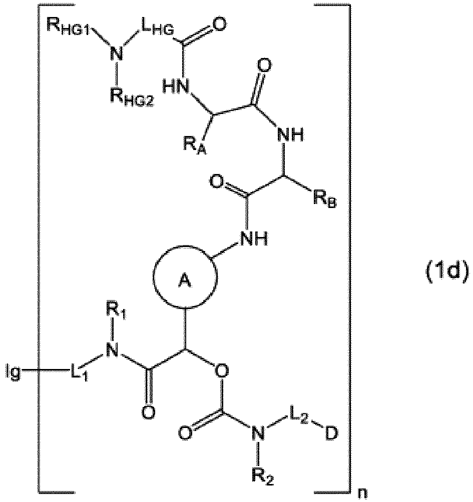 wherein
Ig, ring A, D, and n are the same as those represented in Formula (1), respectively,
R_{A} and R_{B} are the same as those represented in Formula (1b), respectively,
R₁, R₂, L₁, and L₂ are the same as those represented in Formula (1c), respectively,
L_{HG} represents a bond or a divalent group optionally comprising a hydrophilic group,
R_{HG1} and R_{HG2} each independently represent a hydrogen atom, a hydrophilic group, or a monovalent group optionally comprising a hydrophilic group, and
at least one hydrophilic group is comprised in one or more sites selected from the group consisting of L_{HG}, R_{HG1}, and R_{HG2}.

19. The conjugate or salt thereof according to claim 18, wherein
the divalent group (-L_{HG}-) optionally comprising a hydrophilic group is a divalent group represented by the following Formula (a):
-(C(R_{HG})₂)ₙ₁-(C=O)ₙ₂-(NR_{HG})ₙ₃-(C(R_{HG})₂)ₙ₄- (a),
wherein
a plurality of R_{HG} each independently represent a hydrogen atom, a hydrophilic group, or a monovalent group optionally comprising a hydrophilic group,
n1 is an integer of 0 to 3,
n2 is an integer of 0 or 1,
n3 is an integer of 0 or 1, and
n4 is an integer of 0 to 3.

20. The conjugate or salt thereof according to claim 19, wherein
the divalent group represented by Formula (a) is a divalent group represented by the following Formula (a1), (a2), or (a3) :
(a1) -(C(R_{HG})₂)-;
(a2) -(C(R_{HG})₂)-(C=O)-(NR_{HG})-(C(R_{HG})₂)-;
or
(a3) -(C=O)-(C(R_{HG})₂)₂-,
wherein
a plurality of R_{HG} each independently represent a hydrogen atom, a hydrophilic group, or a C₁₋₆ alkyl group comprising a hydrophilic group.

21. The conjugate or salt thereof according to any one of claims 1 to 20, wherein the hydrophilic groups are each independently a carboxylic acid group, a sulfonate group, or a hydroxy group.

22. The conjugate or salt thereof according to claim 21, wherein the hydrophilic group is a carboxylic acid group.

23. An antibody derivative having a bioorthogonal functional group or bioorthogonal functional groups, comprising a structural unit represented by the following Formula (2): 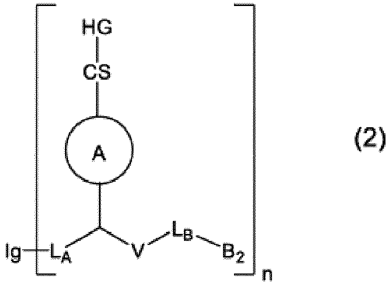 wherein
Ig represents an immunoglobulin unit comprising two heavy chains and two light chains, and is bonded to L_{A} adjacent to Ig via a thiol group in side chains of a plurality of cysteine residues in the two heavy chains and the two light chains,
HG represents a hydrophilic group or a monovalent group comprising a hydrophilic group,
CS represents a divalent group comprising a cleavable site,
ring A represents a divalent aromatic ring group optionally having a substituent wherein the divalent aromatic ring group constitutes a π electron conjugated system with the cleavable site,
V represents an oxygen atom, a sulfur atom, or an amino group (NH),
L_{A} and L_{B} each independently represent a divalent group,
B₂ represents a bioorthogonal functional group, and
an average number n of the bonds per the immunoglobulin unit is 1.5 or more, or
a salt thereof.

24. The antibody derivative or salt thereof according to claim 23, wherein the bioorthogonal functional group is a furan residue, an alkene residue, an alkyne residue, an azide residue, or a tetrazine residue.

25. The antibody derivative or salt thereof according to claim 23, wherein the structural unit represented by Formula (2) comprises a structural unit represented by the following Formula (2a): 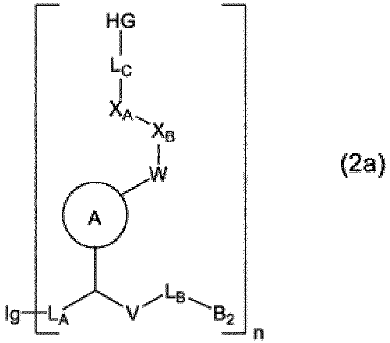 wherein
Ig, HG, ring A, V, L_{A}, L_{B}, B₂, and n are the same as those represented in Formula (2), respectively,
L_{C} represents a bond or a divalent group,
X_{A} and X_{B} each independently represent an amino acid residue, and
W represents an oxygen atom, a sulfur atom, or an amino group (NH).

26. The antibody derivative or salt thereof according to claim 25, wherein
the structural unit represented by Formula (2a) comprises a structural unit represented by the following Formula (2b): 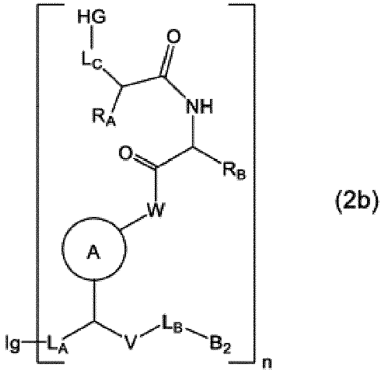 wherein
Ig, HG, ring A, V, L_{A}, L_{B}, B₂, and n are the same as those represented in Formula (2), respectively,
L_{C} and W are the same as those represented in Formula (2a), respectively,
R_{A} represents a side chain of a valine residue, a phenylalanine residue, a threonine residue, a leucine residue, or an alanine residue, and
R_{B} represents a side chain of a citrulline residue, an alanine residue, a glutamic acid residue, a glutamine residue, a lysine residue, an arginine residue, a threonine residue, or a methionine residue.

27. The antibody derivative or salt thereof according to claim 26, wherein
the structural unit represented by Formula (2b) comprises a structural unit represented by the following Formula (2c): 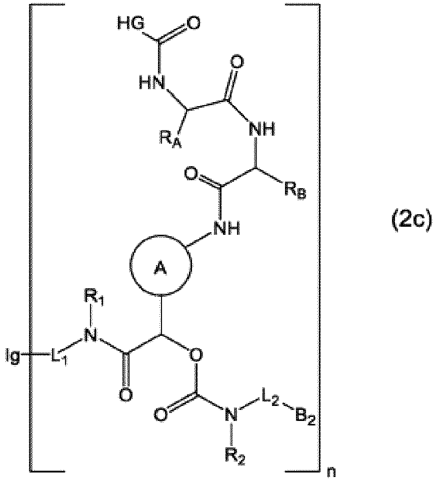 wherein
Ig, HG, ring A, B₂, and n are the same as those represented in Formula (2), respectively,
R_{A} and R_{B} are the same as those represented in Formula (2b), respectively,
R₁ and R₂ each independently represent a hydrogen atom or a monovalent group, and
L₁ and L₂ each independently represent a divalent group.

28. The antibody derivative or salt thereof according to claim 27, wherein
the structural unit represented by Formula (2c) comprises a structural unit represented by the following Formula (2d): 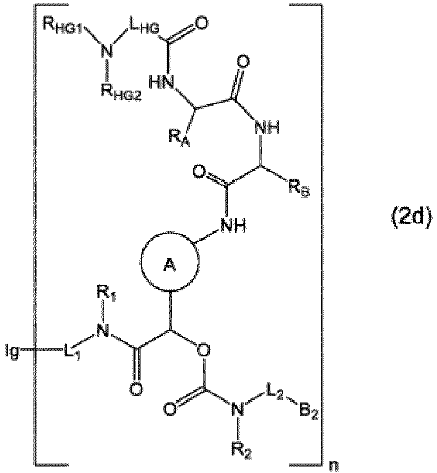 wherein
Ig, ring A, B₂, and n are the same as those represented in Formula (2), respectively,
R_{A} and R_{B} are the same as those represented in Formula (2b), respectively,
R₁, R₂, L₁, and L₂ are the same as those represented in Formula (2c), respectively,
L_{HG} represents a bond or a divalent group optionally comprising a hydrophilic group,
R_{HG1} and R_{HG2} each independently represent a hydrogen atom, a hydrophilic group, or a monovalent group optionally comprising a hydrophilic group, and
at least one hydrophilic group is comprised in one or more sites selected from the group consisting of L_{HG}, R_{HG1}, and R_{HG2}.

29. A compound having a bioorthogonal functional group and a functional substance, represented by the following Formula (3): 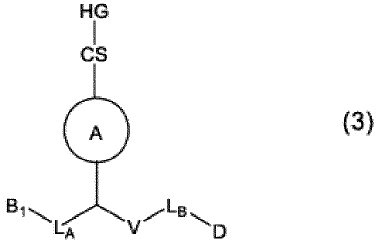 wherein
HG represents a hydrophilic group or a monovalent group comprising a hydrophilic group,
CS represents a divalent group comprising a cleavable site,
ring A represents a divalent aromatic ring group optionally having a substituent wherein the divalent aromatic ring group constitutes a π electron conjugated system with the cleavable site,
V represents an oxygen atom, a sulfur atom, or an amino group (NH),
L_{A} and L_{B} each independently represent a divalent group,
B₁ represents a bioorthogonal functional group, and
D represents a functional substance, or
a salt thereof.

30. The compound or salt thereof according to claim 29, wherein
the compound represented by Formula (3) is represented by the following Formula (3a): 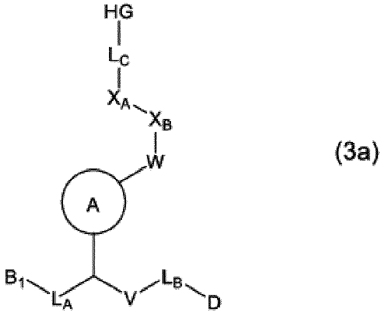 wherein
HG, ring A, V, L_{A}, L_{B}, B₁, and D are the same as those represented in Formula (3), respectively,
L_{C} represents a bond or a divalent group,
X_{A} and X_{B} each independently represent an amino acid residue, and
W represents an oxygen atom, a sulfur atom, or an amino group (NH).

31. The compound or salt thereof according to claim 30, wherein the compound represented by Formula (3a) is represented by the following Formula (3b): 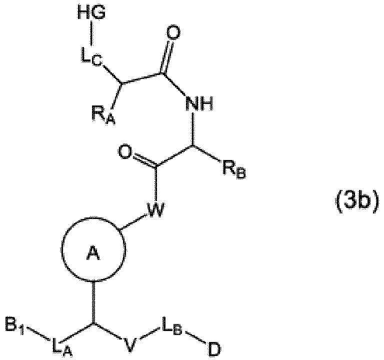 wherein
HG, ring A, V, L_{A}, L_{B}, B₁, and D are the same as those represented in Formula (3), respectively,
L_{C} and W are the same as those represented in Formula (3a), respectively,
R_{A} represents a side chain of a valine residue, a phenylalanine residue, a threonine residue, a leucine residue, or an alanine residue, and
R_{B} represents a side chain of a citrulline residue, an alanine residue, a glutamic acid residue, a glutamine residue, a lysine residue, an arginine residue, a threonine residue, or a methionine residue.

32. The compound or salt thereof according to claim 31, wherein
the compound represented by Formula (3b) is represented by the following Formula (3c): 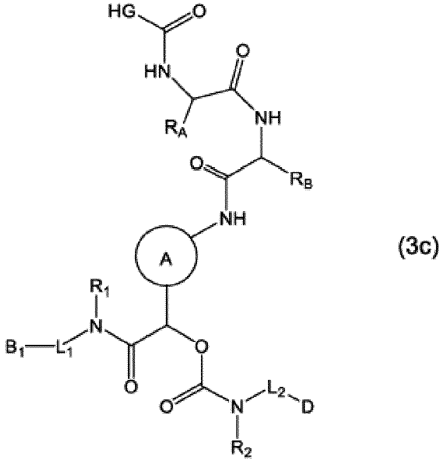 wherein
HG, ring A, B₁, and D are the same as those represented in Formula (3), respectively,
R_{A} and R_{B} are the same as those represented in Formula (3b), respectively,
R₁ and R₂ each independently represent a hydrogen atom or a monovalent group, and
L₁ and L₂ each independently represent a divalent group.

33. The compound or salt thereof according to claim 32, wherein the compound represented by Formula (3c) is represented by the following Formula (3d): 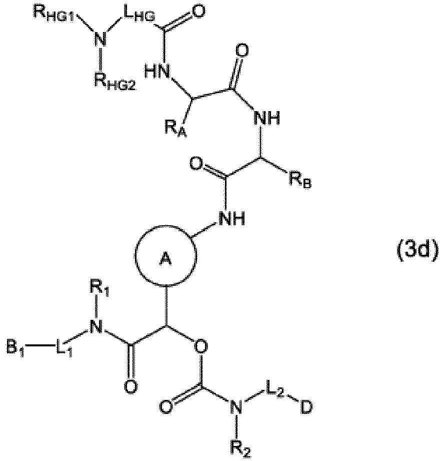 wherein
ring A, B₁, and D are the same as those represented in Formula (3), respectively,
R_{A} and R_{B} are the same as those represented in Formula (3b), respectively,
R₁, R₂, L₁, and L₂ are the same as those represented in Formula (3c), respectively,
L_{HG} represents a bond or a divalent group optionally comprising a hydrophilic group,
R_{HG1} and R_{HG2} each independently represent a hydrogen atom, a hydrophilic group, or a monovalent group optionally comprising a hydrophilic group, and
at least one hydrophilic group is comprised in one or more sites selected from the group consisting of L_{HG}, R_{HG1}, and R_{HG2}.

34. A reagent for derivatizing an antibody, the reagent comprising the compound or salt thereof according to any one of claims 29 to 33.

35. A compound having a first bioorthogonal functional group and a second bioorthogonal functional group, represented by the following Formula (4): 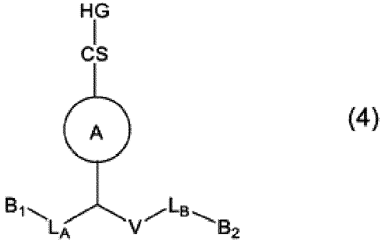 wherein
HG represents a hydrophilic group or a monovalent group comprising a hydrophilic group,
CS represents a divalent group comprising a cleavable site,
ring A represents a divalent aromatic ring group optionally having a substituent wherein the divalent aromatic ring group constitutes a π electron conjugated system with the cleavable site,
V represents an oxygen atom, a sulfur atom, or an amino group (NH),
L_{A} and L_{B} each independently represent a divalent group,
B₁ represents a first bioorthogonal functional group, and
B₂ represents a second bioorthogonal functional group, or
a salt thereof.

36. The compound or salt thereof according to claim 35, wherein
the compound represented by Formula (4) is represented by the following Formula (4a): 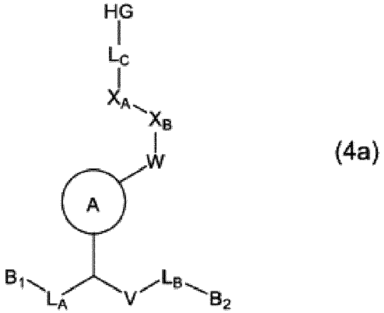 wherein
HG, ring A, V, L_{A}, L_{B}, B₁, and B₂ are the same as those represented in Formula (4), respectively,
L_{C} represents a bond or a divalent group,
X_{A} and X_{B} each independently represent an amino acid residue, and
W represents an oxygen atom, a sulfur atom, or an amino group (NH).

37. The compound or salt thereof according to claim 36, wherein
the compound represented by Formula (4a) is represented by the following Formula (4b): 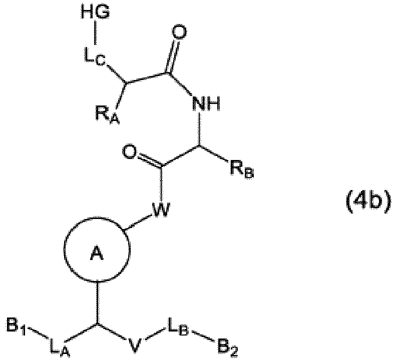 wherein
HG, ring A, V, L_{A}, L_{B}, B₁, and B₂ are the same as those represented in Formula (4), respectively,
L_{C} and W are the same as those represented in Formula (4a), respectively,
R_{A} represents a side chain of a valine residue, a phenylalanine residue, a threonine residue, a leucine residue, or an alanine residue, and
R_{B} represents a side chain of a citrulline residue, an alanine residue, a glutamic acid residue, a glutamine residue, a lysine residue, an arginine residue, a threonine residue, or a methionine residue.

38. The compound or salt thereof according to claim 37, wherein
the compound represented by Formula (4b) is represented by the following Formula (4c): 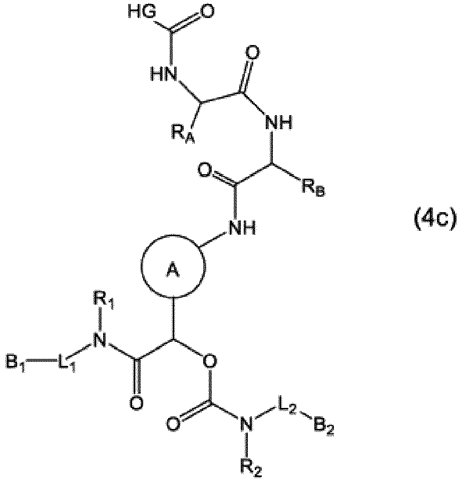 wherein
HG, ring A, B₁, and B₂ are the same as those represented in Formula (4), respectively,
R_{A} and R_{B} are the same as those represented in Formula (4b), respectively,
R₁ and R₂ each independently represent a hydrogen atom or a monovalent group, and
L₁ and L₂ each independently represent a divalent group.

39. The compound or salt thereof according to claim 38, wherein
the compound represented by Formula (4c) is represented by the following Formula (4d): 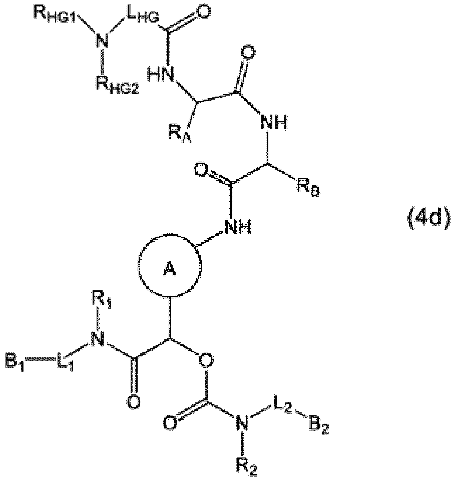 wherein
ring A, B₁, and B₂ are the same as those represented in Formula (4), respectively,
R_{A} and R_{B} are the same as those represented in Formula (4b), respectively,
R₁, R₂, L₁, and L₂ are the same as those represented in Formula (4c), respectively,
L_{HG} represents a bond or a divalent group optionally comprising a hydrophilic group,
R_{HG1} and R_{HG2} each independently represent a hydrogen atom, a hydrophilic group, or a monovalent group optionally comprising a hydrophilic group, and
at least one hydrophilic group is comprised in one or more sites selected from the group consisting of L_{HG}, R_{HG1}, and R_{HG2}.

40. A reagent for derivatizing an antibody or a functional substance, the reagent comprising the compound or salt thereof according to any one of claims 35 to 39.

41. A compound or a salt thereof of the following (A), (B), or (C):
(A) a compound represented by the following Formula (5) : 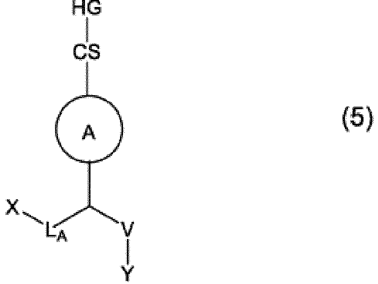 wherein
HG represents a hydrophilic group or a monovalent group comprising a hydrophilic group,
CS represents a divalent group comprising a cleavable site,
ring A represents a divalent aromatic ring group optionally having a substituent wherein the divalent aromatic ring group constitutes a π electron conjugated system with the cleavable site,
V represents an oxygen atom, a sulfur atom, or an amino group (NH),
L_{A} represents a divalent group, and
X and Y each independently represent a monovalent group, or
a salt thereof;
(B) a compound having a bioorthogonal functional group represented by the following Formula (6): 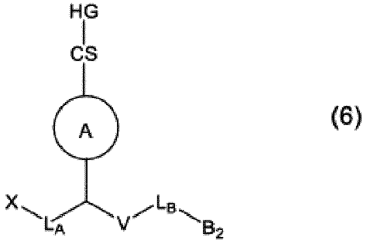 wherein
HG, CS, ring A, V, L_{A}, and X are the same as those represented in Formula (5), respectively,
L_{B} represents a divalent group, and
B₂ represents a bioorthogonal functional group, or
a salt thereof; or
(C) a compound having a bioorthogonal functional group represented by the following Formula (7): 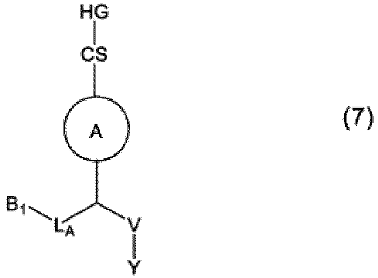 wherein
HG, CS, Ring A, V, L_{A}, and Y are the same as those represented in Formula (5), respectively, and
B₁ represents a bioorthogonal functional group, or
a salt thereof.

42. The compound or salt thereof according to claim 41, wherein
the compound of (A), (B), or (C) is a following compound of (A-1), (B-1), or (C-1), respectively:
(A-1) a compound represented by the following Formula (5a) : 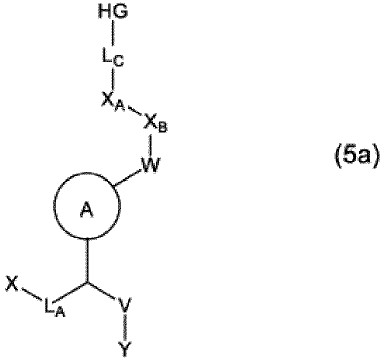 wherein
HG, ring A, V, L_{A}, X, and Y are the same as those represented in Formula (5), respectively,
L_{C} represents a bond or a divalent group,
X_{A} and X_{B} each independently represent an amino acid residue, and
W represents an oxygen atom, a sulfur atom, or an amino group (NH), or
a salt thereof;
(B-1) a compound represented by the following Formula (6a) : 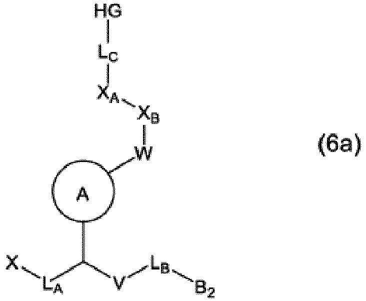 wherein
HG, ring A, V, L_{A}, and X are the same as those represented in Formula (5), respectively,
L_{C}, X_{A}, X_{B}, and W are the same as those represented in Formula (5a), respectively, and
L_{B} and B₂ are the same as those represented in Formula (6), respectively, or
a salt thereof; and
(C-1) a compound represented by the following Formula (7a) : 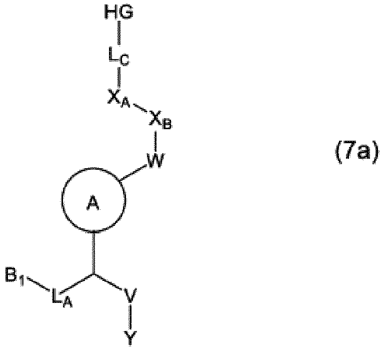 wherein
HG, ring A, V, L_{A}, and Y are the same as those represented in Formula (5), respectively,
L_{C}, X_{A}, X_{B}, and W are the same as those represented in Formula (5a), respectively, and
B₁ is the same as that represented in Formula (7), or
a salt thereof.

43. The compound or salt thereof according to claim 42, wherein
the compound of (A-1), (B-1), or (C-1) is a following compound of (A-2), (B-2), or (C-2), respectively:
(A-2) a compound represented by the following Formula (5b) : 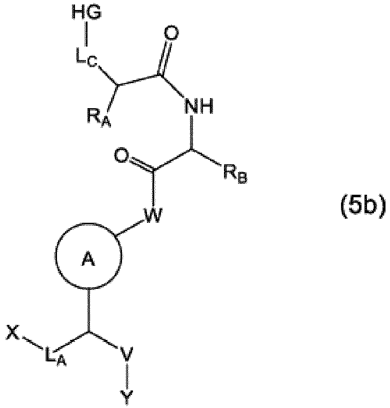 wherein
HG, ring A, V, L_{A}, X, and Y are the same as those represented in Formula (5), respectively,
L_{C} and W are the same as those represented in Formula (5a), respectively,
R_{A} represents a side chain of a valine residue, a phenylalanine residue, a threonine residue, a leucine residue, or an alanine residue, and
R_{B} represents a side chain of a citrulline residue, an alanine residue, a glutamic acid residue, a glutamine residue, a lysine residue, an arginine residue, a threonine residue, or a methionine residue, or
a salt thereof;
(B-2) a compound represented by the following Formula (6b) : 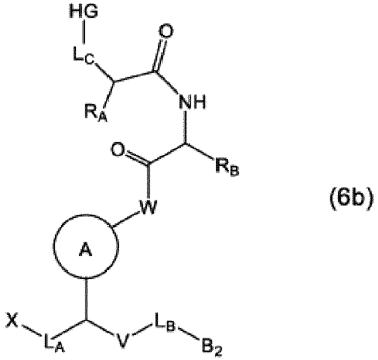 wherein
HG, ring A, V, L_{A}, and X are the same as those represented in Formula (5), respectively,
L_{C} and W are the same as those represented in Formula (5a), respectively,
R_{A} and R_{B} are the same as those represented in Formula (5b), respectively, and
L_{B} and B₂ are the same as those represented in Formula (6), respectively, or
a salt thereof; and
(C-2) a compound represented by the following Formula (7b) : 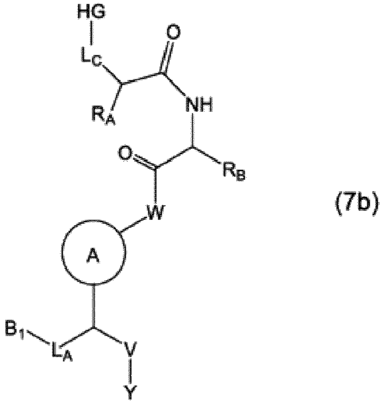 wherein
HG, ring A, V, L_{A}, and Y are the same as those represented in Formula (5), respectively,
L_{C}, X_{A}, X_{B}, and W are the same as those represented in Formula (5a), respectively,
R_{A} and R_{B} are the same as those represented in Formula (5b), respectively, and
B₁ is the same as that represented in Formula (7), or
a salt thereof.

44. The compound or salt thereof according to claim 43, wherein
the compound of (A-2), (B-2), or (C-2) is a following compound of (A-3), (B-3), or (C-3), respectively:
(A-3) a compound represented by the following Formula (5c) : 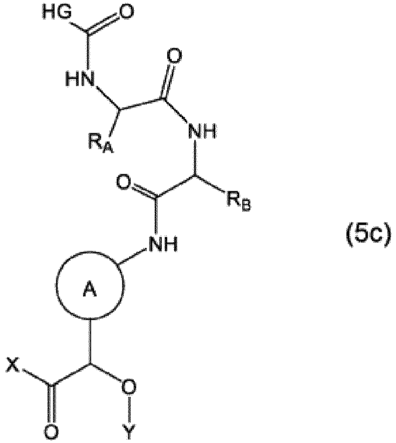 wherein
HG, ring A, X, and Y are the same as those represented in Formula (5), respectively, and
R_{A} and R_{B} are the same as those represented in Formula (5b), respectively, or
a salt thereof;
(B-3) a compound represented by the following Formula (6c) : 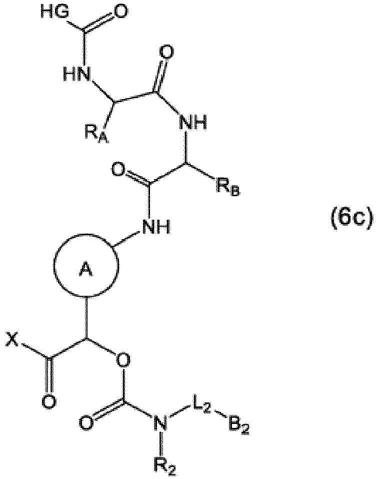 wherein
HG, ring A, and X are the same as those represented in Formula (5), respectively,
R_{A} and R_{B} are the same as those represented in Formula (5b), respectively,
B₂ is the same as that represented in Formula (6),
R₂ represents a hydrogen atom or a monovalent group, and
L₂ represents a divalent group, or
a salt thereof; and
(C-3) a compound represented by the following Formula (7c) : 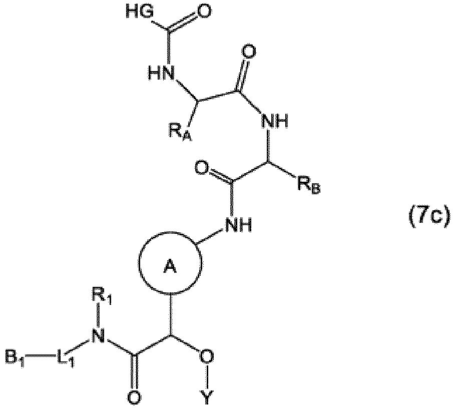 wherein
HG, ring A, and Y are the same as those represented in Formula (5), respectively,
R_{A} and R_{B} are the same as those represented in Formula (5b), respectively,
B₁ is the same as that represented in Formula (7),
R₁ represents a hydrogen atom or a monovalent group, and
L₁ represents a divalent group, or
a salt thereof.

45. The compound or salt thereof according to claim 44, wherein
the compound of (A-3), (B-3), or (C-3) is a following compound of (A-4), (B-4), or (C-4), respectively:
(A-4) a compound represented by the following Formula (5d) : 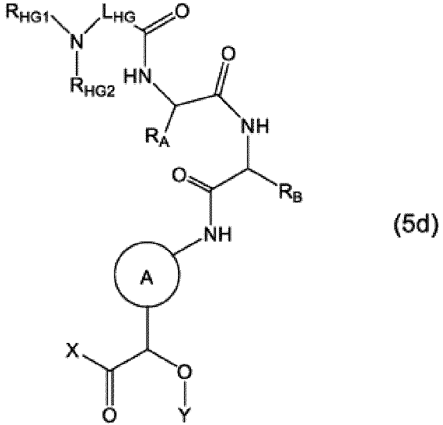 wherein
ring A, X, and Y are the same as those represented in Formula (5), respectively,
R_{A} and R_{B} are the same as those represented in Formula (5b), respectively,
L_{HG} represents a bond or a divalent group optionally comprising a hydrophilic group,
R_{HG1} and R_{HG2} each independently represent a hydrogen atom, a hydrophilic group, or a monovalent group optionally comprising a hydrophilic group, and
at least one hydrophilic group is comprised in one or more sites selected from the group consisting of L_{HG}, R_{HG1}, and R_{HG2}, or
a salt thereof;
(B-4) a compound represented by the following Formula (6d) : 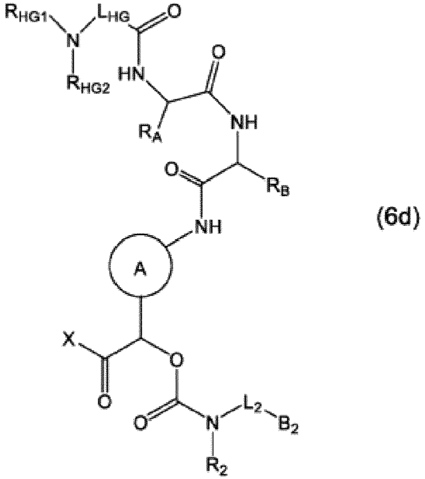 wherein
ring A and X are the same as those represented in Formula (5), respectively,
R_{A} and R_{B} are the same as those represented in Formula (5b), respectively,
L_{HG}, R_{HG1}, and R_{HG2} are the same as those represented in Formula (5d),
B₂ is the same as that represented in Formula (6), and
R₂ and L₂ are the same as those represented in Formula (6c), or
a salt thereof; and
(C-4) a compound represented by the following Formula (7d) : 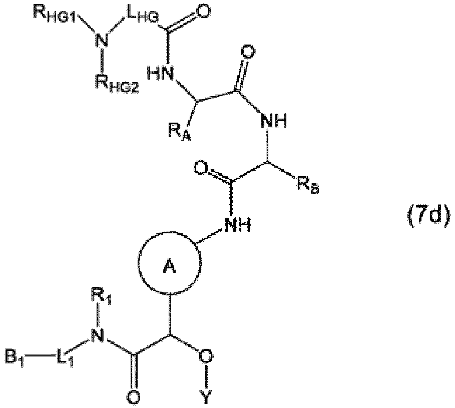 wherein
ring A and Y are the same as those represented in Formula (5), respectively,
R_{A} and R_{B} are the same as those represented in Formula (5b), respectively,
L_{HG}, R_{HG1}, and R_{HG2} are the same as those represented in Formula (5d),
B₁ is the same as that represented in Formula (7), and
R₁ and L₁ are the same as those represented in Formula (7c), or
a salt thereof.
